# EUROPEAN PATENT APPLICATION

(11) **EP 3 279 330 A2**
(43) Date of publication of application: **07.02.2018**
(21) Application number: 16773434.2
(22) Date of filing: 30.03.2016
(51) Int. Cl.: C12Q 1/02, C12Q 1/68

(54) **NOVEL GENE REGULATING VIRULENCE OFCRYPTOCOCCUS NEOFORMANS**

(30) Priority: 30.03.2015 KR 20150044621; 30.03.2015 KR 20150044617
(71) Applicant: Amtixbio Co., Ltd., Seoul 05836 (KR)
(72) Inventor: BAHN, Yong-Sun, Seoul 138-879 (KR); JUNG, Kwang-Woo, Seoul 02609 (KR); YANG, Dong-Hoon, Namyangju-si Gyeonggi-do 12109 (KR)
(74) Representative: Leifert & Steffan
(86) International application number: PCT/KR2016/003255
(87) International publication number: WO 2016/159653

(57) **Abstract**

The present invention relates to a novel gene regulating the virulence of *Cryptococcus neoformans,* and a use thereof. According to the present invention, anti-Cryptococcal or anti-fungal drug candidate materials can be effectively screened for. In addition, the present invention relates to a method for screening for drug candidate materials, which can bring a synergistic effect by being co-administered with a commercially available anti-Cryptococcal drug or anti-fungal drug. Furthermore, provided is a pharmaceutical composition having an anti-Cryptococcal or anti-fungal effect by increasing or decreasing the expression of transcription factors. The present inventors have performed a large-scale virulence test by using insect and animal models so as to identity transcription factors, and have analyzed a complex correlation between the transcription factors and in vivo and in vitro phenotypes of pathogenicity.

### Sequence Listing

<110>    ORIGEN BIOTICS INC.

<120>    Novel genes for regulating the virulence of Cryptococcus
         neoformans and their use

<130>    AMTIX16-0001-WO

<150>    KR 10-2015-0044617
<151>    2015-03-30

<150>    KR 10-2015-0044621
<151>    2015-03-30

<160>    1380

<170>    KopatentIn 2.0

<210>    1
<211>    19
<212>    DNA
<213>    CNAG_03132 5 flanking region primer 1

<400>    1
gcgtggcatt tagagagtg                                                    19


<210>    2
<211>    38
<212>    DNA
<213>    CNAG_03132 5 flanking region primer 2

<400>    2
tcactggccg tcgttttacg agcgtattgg atgaggag                               38


<210>    3
<211>    39
<212>    DNA
<213>    CNAG_03132 3 flanking region primer 1

<400> 3
catggtcata gctgtttcct gaaacctcgc tttgagacg 39

<210> 4
<211> 18
<212> DNA
<213> CNAG_03132 3 flanking region primer 2

<400> 4
tgacgaagcg acagaatc 18

<210> 5
<211> 20
<212> DNA
<213> CNAG_03132 diagnostic screening primer, pairing with B79

<400> 5
cccaaacact attatcgcag 20

<210> 6
<211> 18
<212> DNA
<213> CNAG_03132 Southern blot probe primer

<400> 6
tactcgtatg aatcggcg 18

<210> 7
<211> 20
<212> DNA
<213> NAT#5 STM primer

<400> 7
tgctagaggg cgggagagtt 20

<210> 8
<211> 24
<212> DNA
<213> STM common primer

<400> 8
gcatgccctg cccctaagaa ttcg 24


<210> 9
<211> 20
<212> DNA
<213> CNAG_05380 5 flanking region primer 1

<400> 9
cctactgctt ggcacattac 20


<210> 10
<211> 35
<212> DNA
<213> CNAG_05380 5 flanking region primer 2

<400> 10
ctggccgtcg ttttaccgtc gtttcttttt gggag 35


<210> 11
<211> 37
<212> DNA
<213> CNAG_05380 3 flanking region primer 1

<400> 11
gtcatagctg tttcctgtcc atctccagtc tgtgaac 37


<210> 12
<211> 20
<212> DNA
<213> CNAG_05380 3 flanking region primer 2

<400> 12
cgcttcttag gacctcactc 20

<210> 13
<211> 20
<212> DNA
<213> CNAG_05380 diagnostic screening primer, pairing with B79

<400> 13
tctgaaggcg gaatctggac 20

<210> 14
<211> 24
<212> DNA
<213> CNAG_05380 Southern blot probe primer

<400> 14
ggagcaagga actaaagatg gtag 24

<210> 15
<211> 20
<212> DNA
<213> NAT#5 STM primer

<400> 15
tgctagaggg cgggagagtt 20

<210> 16
<211> 24
<212> DNA
<213> STM common primer

<400> 16
gcatgccctg cccctaagaa ttcg 24

<210> 17
<211> 20
<212> DNA
<213> CNAG_06719 5 flanking region primer 1

<400> 17
tcgccaactg aacaaacccc 20

<210> 18
<211> 36
<212> DNA
<213> CNAG_06719 5 flanking region primer 2

<400> 18
ctggccgtcg ttttactccc gcctaacact ttcgtg 36

<210> 19
<211> 36
<212> DNA
<213> CNAG_06719 3 flanking region primer 1

<400> 19
gtcatagctg tttcctgtgt caagccataa agagcc 36

<210> 20
<211> 20
<212> DNA
<213> CNAG_06719 3 flanking region primer 2

<400> 20
ctgatagttt tgccacttgc 20

<210> 21
<211> 22
<212> DNA

<213>    CNAG_06719 diagnostic screening primer, pairing with B79

<400>    21
aataccctcc ttacttcccc gc                                                22

<210>    22
<211>    18
<212>    DNA
<213>    CNAG_06719 Southern blot probe primer

<400>    22
gtaaaagttt gggcgtgg                                                     18

<210>    23
<211>    20
<212>    DNA
<213>    NAT#5 STM primer

<400>    23
tgctagaggg cgggagagtt                                                   20

<210>    24
<211>    24
<212>    DNA
<213>    STM common primer

<400>    24
gcatgccctg cccctaagaa ttcg                                              24

<210>    25
<211>    20
<212>    DNA
<213>    CNAG_04093 5 flanking region primer 1

<400>    25
tccaggcgta ttcatcccag                                                   20

<210> 26
<211> 38
<212> DNA
<213> CNAG_04093 5 flanking region primer 2

<400> 26
ctggccgtcg ttttacaagg attgtgacct caggctcg 38

<210> 27
<211> 36
<212> DNA
<213> CNAG_04093 3 flanking region primer 1

<400> 27
gtcatagctg tttcctgatg tttctcctcc agagcc 36

<210> 28
<211> 19
<212> DNA
<213> CNAG_04093 3 flanking region primer 2

<400> 28
gcaagaacca aatgacagc 19

<210> 29
<211> 24
<212> DNA
<213> CNAG_04093 diagnostic screening primer, pairing with B79

<400> 29
atcattcctc tgacaagtcg ggcg 24

<210> 30
<211> 19

<212>    DNA
<213>    CNAG_04093 Southern blot probe primer

<400>    30
atgtgtcctg gatggcttg                                                    19


<210>    31
<211>    20
<212>    DNA
<213>    NAT#5 STM primer

<400>    31
tgctagaggg cgggagagtt                                                   20


<210>    32
<211>    24
<212>    DNA
<213>    STM common primer

<400>    32
gcatgccctg cccctaagaa ttcg                                              24


<210>    33
<211>    18
<212>    DNA
<213>    CNAG_04518 5 flanking region primer 1

<400>    33
tgtcctttct tgtgaccg                                                     18


<210>    34
<211>    39
<212>    DNA
<213>    CNAG_04518 5 flanking region primer 2

<400>    34

tcactggccg tcgttttacc agtaacgaag aacgcaatc 39

<210> 35
<211> 40
<212> DNA
<213> CNAG_04518 3 flanking region primer 1

<400> 35
catggtcata gctgtttcct gcacaagcca cgattattgc 40

<210> 36
<211> 20
<212> DNA
<213> CNAG_04518 3 flanking region primer 2

<400> 36
tgtatctgtg tgtggactgc 20

<210> 37
<211> 18
<212> DNA
<213> CNAG_04518 diagnostic screening primer, pairing with B79

<400> 37
acacaaaggg aggaatgc 18

<210> 38
<211> 19
<212> DNA
<213> CNAG_04518 Southern blot probe primer

<400> 38
catacactgt gccacgaac 19

<210> 39

<211>    20
<212>    DNA
<213>    NAT#6 STM primer

<400>    39
atagctacca cacgatagct                                                    20


<210>    40
<211>    24
<212>    DNA
<213>    STM common primer

<400>    40
gcatgccctg cccctaagaa ttcg                                               24


<210>    41
<211>    20
<212>    DNA
<213>    CNAG_01858 5 flanking region primer 1

<400>    41
tcttcaggaa cataggatgg                                                    20


<210>    42
<211>    38
<212>    DNA
<213>    CNAG_01858 5 flanking region primer 2

<400>    42
tcactggccg tcgttttacg agattgttgg gaggattg                                38


<210>    43
<211>    39
<212>    DNA
<213>    CNAG_01858 3 flanking region primer 1

<400> 43
catggtcata gctgtttcct ggggcgaata aggtttctg 39

<210> 44
<211> 18
<212> DNA
<213> CNAG_01858 3 flanking region primer 2

<400> 44
tgttttcgct catccgtc 18

<210> 45
<211> 19
<212> DNA
<213> CNAG_01858 diagnostic screening primer, pairing with B79

<400> 45
aacacagcgg aatgacctc 19

<210> 46
<211> 18
<212> DNA
<213> CNAG_01858 Southern blot probe primer

<400> 46
cacttggttc attgctgg 18

<210> 47
<211> 20
<212> DNA
<213> NAT#43 STM primer

<400> 47
ccagctacca atcacgctac 20

<210>    48
<211>    24
<212>    DNA
<213>    STM common primer

<400>    48
gcatgccctg cccctaagaa ttcg                                              24


<210>    49
<211>    22
<212>    DNA
<213>    CNAG_02305 5 flanking region primer 1

<400>    49
ccagaaggat gtgatactga gg                                                22


<210>    50
<211>    36
<212>    DNA
<213>    CNAG_02305 5 flanking region primer 2

<400>    50
ctggccgtcg ttttacgaaa tcgttctgaa actgcg                                 36


<210>    51
<211>    39
<212>    DNA
<213>    CNAG_02305 3 flanking region primer 1

<400>    51
gtcatagctg tttcctgatc gttcacaggc tcaaggctg                              39


<210>    52
<211>    22
<212>    DNA
<213>    CNAG_02305 3 flanking region primer 2

<400> 52
gaggtcatta gagggtattt gc 22

<210> 53
<211> 19
<212> DNA
<213> CNAG_02305 diagnostic screening primer, pairing with B79

<400> 53
gcgtggcgaa tacatacag 19

<210> 54
<211> 19
<212> DNA
<213> CNAG_02305 Southern blot probe primer

<400> 54
aggtgctaca aagactcgg 19

<210> 55
<211> 24
<212> DNA
<213> NAT#58 STM primer

<400> 55
cgcaaaatca ctagccctat agcg 24

<210> 56
<211> 24
<212> DNA
<213> STM common primer

<400> 56
gcatgccctg cccctaagaa ttcg 24

<210> 57
<211> 19
<212> DNA
<213> CNAG_06156 5 flanking region primer 1

<400> 57
aagatagcac ctccgacag 19

<210> 58
<211> 41
<212> DNA
<213> CNAG_06156 5 flanking region primer 2

<400> 58
gctcactggc cgtcgtttta cgctgttgcg aaagataaag g 41

<210> 59
<211> 41
<212> DNA
<213> CNAG_06156 3 flanking region primer 1

<400> 59
catggtcata gctgtttcct gccaggaatg aactaccaag g 41

<210> 60
<211> 19
<212> DNA
<213> CNAG_06156 3 flanking region primer 2

<400> 60
ttgaagcagg actctccac 19

<210> 61
<211> 19
<212> DNA

<213>    CNAG_06156 diagnostic screening primer, pairing with B79

<400>    61
tgcttgtcgt ttggttagg                                                    19

<210>    62
<211>    19
<212>    DNA
<213>    CNAG_06156 Southern blot probe primer

<400>    62
ggtgaaaatg acggtccag                                                    19

<210>    63
<211>    24
<212>    DNA
<213>    NAT#119 STM primer

<400>    63
ctccccacat aaagagagct aaac                                              24

<210>    64
<211>    24
<212>    DNA
<213>    STM common primer

<400>    64
gcatgccctg cccctaagaa ttcg                                              24

<210>    65
<211>    21
<212>    DNA
<213>    CNAG_06188 5 flanking region primer 1

<400>    65
ctcttgtctt ctgcctattc c                                                 21

<210> 66
<211> 35
<212> DNA
<213> CNAG_06188 5 flanking region primer 2

<400> 66
ctggccgtcg ttttacggac atagtgaaac tgggg 35

<210> 67
<211> 38
<212> DNA
<213> CNAG_06188 3 flanking region primer 1

<400> 67
gtcatagctg tttcctgtca tcctccgact ctaaactg 38

<210> 68
<211> 20
<212> DNA
<213> CNAG_06188 3 flanking region primer 2

<400> 68
gttttcctac ttttgtgggc 20

<210> 69
<211> 20
<212> DNA
<213> CNAG_06188 diagnostic screening primer, pairing with B79

<400> 69
agaagacgac tgtggtaggc 20

<210> 70
<211> 20

<212>    DNA
<213>    CNAG_06188 Southern blot probe primer

<400>    70
gagtcagcag cagaagattc                                                    20

<210>    71
<211>    24
<212>    DNA
<213>    NAT#122 STM primer

<400>    71
acagctccaa acctcgctaa acag                                               24

<210>    72
<211>    24
<212>    DNA
<213>    STM common primer

<400>    72
gcatgccctg cccctaagaa ttcg                                               24

<210>    73
<211>    19
<212>    DNA
<213>    CNAG_05785 5 flanking region primer 1

<400>    73
catttctact tggttgccg                                                     19

<210>    74
<211>    36
<212>    DNA
<213>    CNAG_05785 5 flanking region primer 2

<400>    74

ctggccgtcg ttttacagga gaaagtgatg gtcggc 36

<210> 75
<211> 39
<212> DNA
<213> CNAG_05785 3 flanking region primer 1

<400> 75
gtcatagctg tttcctgtta tcgtgagcct tcaagcccc 39

<210> 76
<211> 21
<212> DNA
<213> CNAG_05785 3 flanking region primer 2

<400> 76
ccattgtaga tgtcggttac g 21

<210> 77
<211> 22
<212> DNA
<213> CNAG_05785 diagnostic screening primer, pairing with B79

<400> 77
ttgcggtctt tgtcccttgt gg 22

<210> 78
<211> 24
<212> DNA
<213> CNAG_05785 Southern blot probe primer

<400> 78
ttctccactt gctcgcctat cctc 24

<210> 79

<211>    24
<212>    DNA
<213>    NAT#125 STM primer

<400>    79
cgctacagcc agcgcgcgca agcg                                              24


<210>    80
<211>    24
<212>    DNA
<213>    STM common primer

<400>    80
gcatgccctg cccctaagaa ttcg                                              24


<210>    81
<211>    20
<212>    DNA
<213>    CNAG_05019 5 flanking region primer 1

<400>    81
aaccaccaca atgtcttcag                                                   20


<210>    82
<211>    40
<212>    DNA
<213>    CNAG_05019 5 flanking region primer 2

<400>    82
gctcactggc cgtcgtttta cagacccttg tttggttctg                             40


<210>    83
<211>    41
<212>    DNA
<213>    CNAG_05019 3 flanking region primer 1

<400> 83
catggtcata gctgtttcct gagtggagag ggagagcaat g 41

<210> 84
<211> 18
<212> DNA
<213> CNAG_05019 3 flanking region primer 2

<400> 84
tttacgaaga gcagcgac 18

<210> 85
<211> 18
<212> DNA
<213> CNAG_05019 diagnostic screening primer, pairing with B79

<400> 85
agatagcaac ggctttgg 18

<210> 86
<211> 20
<212> DNA
<213> CNAG_05019 Southern blot probe primer

<400> 86
cgcaaaaggg aagatggata 20

<210> 87
<211> 20
<212> DNA
<213> NAT#150 STM primer

<400> 87
acatacaccc ccatcccccc 20

<210> 88
<211> 24
<212> DNA
<213> STM common primer

<400> 88
gcatgccctg cccctaagaa ttcg 24


<210> 89
<211> 18
<212> DNA
<213> CNAG_02877 5 flanking region primer 1

<400> 89
gtttaccatt tcaccgcc 18


<210> 90
<211> 39
<212> DNA
<213> CNAG_02877 5 flanking region primer 2

<400> 90
tcactggccg tcgttttact gatgaggata cattcggag 39


<210> 91
<211> 41
<212> DNA
<213> CNAG_02877 3 flanking region primer 1

<400> 91
catggtcata gctgtttcct gggcttcaac aatcaccatt c 41


<210> 92
<211> 18
<212> DNA
<213> CNAG_02877 3 flanking region primer 2

<400> 92
acgaaaagcg gatagcag 18

<210> 93
<211> 20
<212> DNA
<213> CNAG_02877 diagnostic screening primer, pairing with B79

<400> 93
agccttatta tgaccaaccc 20

<210> 94
<211> 20
<212> DNA
<213> CNAG_02877 Southern blot probe primer

<400> 94
caagttcctt tgcctacacc 20

<210> 95
<211> 21
<212> DNA
<213> NAT#159 STM primer

<400> 95
acgcaccaga cacacaacca g 21

<210> 96
<211> 24
<212> DNA
<213> STM common primer

<400> 96
gcatgccctg cccctaagaa ttcg 24

<210> 97
<211> 20
<212> DNA
<213> CNAG_00039 5 flanking region primer 1

<400> 97
tcactctttc ggggcatcag 20

<210> 98
<211> 38
<212> DNA
<213> CNAG_00039 5 flanking region primer 2

<400> 98
ctggccgtcg ttttacctaa ctggggatga aatgtctg 38

<210> 99
<211> 39
<212> DNA
<213> CNAG_00039 3 flanking region primer 1

<400> 99
gtcatagctg tttcctgggg gaagagcaga aagagtaac 39

<210> 100
<211> 22
<212> DNA
<213> CNAG_00039 3 flanking region primer 2

<400> 100
gcaatcgtaa acaacaccct tg 22

<210> 101
<211> 20
<212> DNA

<213>    CNAG_00039 diagnostic screening primer, pairing with B79

<400>    101
cgtctgtcgt ttatcggttg                                                    20

<210>    102
<211>    19
<212>    DNA
<213>    CNAG_00039 Southern blot probe primer

<400>    102
accctgtaac atttgtccg                                                     19

<210>    103
<211>    19
<212>    DNA
<213>    CNAG_00039 Southern blot probe primer

<400>    103
acttaccctt cacgccaag                                                     19

<210>    104
<211>    20
<212>    DNA
<213>    NAT#177 STM primer

<400>    104
caccaactcc ccatctccat                                                    20

<210>    105
<211>    24
<212>    DNA
<213>    STM common primer

<400>    105
gcatgccctg cccctaagaa ttcg                                               24

<210>	106
<211>	20
<212>	DNA
<213>	CNAG_00039 5 flanking region primer 1

<400>	106
agacattggg cttgacggtg	20

<210>	107
<211>	39
<212>	DNA
<213>	CNAG_00039 5 flanking region primer 2

<400>	107
tcactggccg tcgttttaca cgagttggag aggggttac	39

<210>	108
<211>	39
<212>	DNA
<213>	CNAG_00039 3 flanking region primer 1

<400>	108
catggtcata gctgtttcct gtagaaagga atcggggac	39

<210>	109
<211>	20
<212>	DNA
<213>	CNAG_00039 3 flanking region primer 2

<400>	109
ggagaacgaa aacaaagtgc	20

<210>	110
<211>	19

<212>    DNA
<213>    CNAG_00039 diagnostic screening primer, pairing with B79

<400>    110
ggttcttgtt tatgccctg                                                    19


<210>    111
<211>    20
<212>    DNA
<213>    CNAG_00039 Southern blot probe primer

<400>    111
aacgagggta gattgcgtcg                                                   20


<210>    112
<211>    20
<212>    DNA
<213>    NAT#177 STM primer

<400>    112
caccaactcc ccatctccat                                                   20


<210>    113
<211>    24
<212>    DNA
<213>    STM common primer

<400>    113
gcatgccctg cccctaagaa ttcg                                              24


<210>    114
<211>    19
<212>    DNA
<213>    CNAG_01014 5 flanking region primer 1

<400>    114

tatcgccgac atctctcag 19

<210> 115
<211> 38
<212> DNA
<213> CNAG_01014 5 flanking region primer 2

<400> 115
tcactggccg tcgttttact ttaggacgga ggggattg 38

<210> 116
<211> 39
<212> DNA
<213> CNAG_01014 3 flanking region primer 1

<400> 116
catggtcata gctgtttcct ggagccgaat caggaaatc 39

<210> 117
<211> 19
<212> DNA
<213> CNAG_01014 3 flanking region primer 2

<400> 117
cgcaggcttt tctactctc 19

<210> 118
<211> 30
<212> DNA
<213> CNAG_01014 diagnostic screening primer, pairing with B79

<400> 118
cgcgcggccg cgaaaacctc cacagagcag 30

<210> 119

<211>    19
<212>    DNA
<213>    CNAG_01014 Southern blot probe primer

<400>    119
ttgcttgctg tctcgtaac                                                    19


<210>    120
<211>    18
<212>    DNA
<213>    CNAG_01014 Southern blot probe primer

<400>    120
gatttcctga ttcggctc                                                     18


<210>    121
<211>    20
<212>    DNA
<213>    CTAGAGCCCGCCACAACGCT

<400>    121
ctagagcccg ccacaacgct                                                   20


<210>    122
<211>    24
<212>    DNA
<213>    STM common primer

<400>    122
gcatgccctg cccctaagaa ttcg                                              24


<210>    123
<211>    21
<212>    DNA
<213>    CNAG_06762 5 flanking region primer 1

<400>    123
tgggtgagag aaagatgtca g                                                21

<210>    124
<211>    39
<212>    DNA
<213>    CNAG_06762 5 flanking region primer 2

<400>    124
tcactggccg tcgttttact gtgagtggtt gtgctgtgg                             39

<210>    125
<211>    41
<212>    DNA
<213>    CNAG_06762 3 flanking region primer 1

<400>    125
catggtcata gctgtttcct gggctcatag gtgttgtaac g                          41

<210>    126
<211>    20
<212>    DNA
<213>    CNAG_06762 3 flanking region primer 2

<400>    126
agtattcggt ggtgacaaag                                                  20

<210>    127
<211>    21
<212>    DNA
<213>    CNAG_06762 diagnostic screening primer, pairing with B79

<400>    127
tgggtgagag aaagatgtca g                                                21

<210> 128
<211> 19
<212> DNA
<213> CNAG_06762 Southern blot probe primer

<400> 128
tgttccaccc cttgctaac 19

<210> 129
<211> 20
<212> DNA
<213> NAT#218 STM primer

<400> 129
ctccacatcc atcgctccaa 20

<210> 130
<211> 24
<212> DNA
<213> STM common primer

<400> 130
gcatgccctg cccctaagaa ttcg 24

<210> 131
<211> 18
<212> DNA
<213> CNAG_04630 5 flanking region primer 1

<400> 131
atggaaggac tttgacgg 18

<210> 132
<211> 40
<212> DNA
<213> CNAG_04630 5 flanking region primer 2

<400>    132
tcactggccg tcgttttact gactcacact tgattctccc                                40

<210>    133
<211>    40
<212>    DNA
<213>    CNAG_04630 3 flanking region primer 1

<400>    133
gatggtcata gctgtttcct gctcattttc cctttcgtcg                                40

<210>    134
<211>    18
<212>    DNA
<213>    CNAG_04630 3 flanking region primer 2

<400>    134
taaaggaatg cggctgag                                                        18

<210>    135
<211>    19
<212>    DNA
<213>    CNAG_04630 diagnostic screening primer, pairing with B79

<400>    135
ccaacagcaa cttcgtttc                                                       19

<210>    136
<211>    21
<212>    DNA
<213>    CNAG_04630 Southern blot probe primer

<400>    136
tctcttctcc ttggaactga g                                                    21

<210>    137
<211>    20
<212>    DNA
<213>    NAT#218 STM primer

<400>    137
ctccacatcc atcgctccaa                                                    20


<210>    138
<211>    24
<212>    DNA
<213>    STM common primer

<400>    138
gcatgccctg cccctaagaa ttcg                                               24


<210>    139
<211>    19
<212>    DNA
<213>    CNAG_03401 5 flanking region primer 1

<400>    139
ttcctcgtcc aagtaaacc                                                     19


<210>    140
<211>    41
<212>    DNA
<213>    CNAG_03401 5 flanking region primer 2

<400>    140
gctcactggc cgtcgtttta caatctcgca aaccgcacac g                            41


<210>    141
<211>    43
<212>    DNA

<213>    CNAG_03401 3 flanking region primer 1

<400>    141
catggtcata gctgtttcct gagcgcgaac agggaaatac acc                43

<210>    142
<211>    18
<212>    DNA
<213>    CNAG_03401 3 flanking region primer 2

<400>    142
tcgtgtagga atgggtag                                            18

<210>    143
<211>    20
<212>    DNA
<213>    CNAG_03401 diagnostic screening primer, pairing with B79

<400>    143
tggtctcccc gacgatttac                                          20

<210>    144
<211>    19
<212>    DNA
<213>    CNAG_03401 Southern blot probe primer

<400>    144
tatggtcagg aggaagcag                                           19

<210>    145
<211>    20
<212>    DNA
<213>    NAT#220 STM primer

<400>    145
cagatctcga acgataccca                                          20

<210>    146
<211>    24
<212>    DNA
<213>    STM common primer

<400>    146
   gcatgccctg cccctaagaa ttcg                                                24

<210>    147
<211>    22
<212>    DNA
<213>    CNAG_04594 5 flanking region primer 1

<400>    147
tggcatcgtg ctgttacaag tc                                                   22

<210>    148
<211>    45
<212>    DNA
<213>    CNAG_04594 5 flanking region primer 2

<400>    148
gctcactggc cgtcgtttta catttgcgga tggacctgcc ttagg                          45

<210>    149
<211>    41
<212>    DNA
<213>    CNAG_04594 3 flanking region primer 1

<400>    149
catggtcata gctgtttcct gatcaggatt caggacacga c                              41

<210>    150
<211>    18

<212>	DNA
<213>	CNAG_04594 3 flanking region primer 2

<400>	150
tttggttctc tctcccac	18

<210>	151
<211>	19
<212>	DNA
<213>	CNAG_04594 diagnostic screening primer, pairing with B79

<400>	151
attgcttgag accagcgtc	19

<210>	152
<211>	18
<212>	DNA
<213>	CNAG_04594 Southern blot probe primer

<400>	152
acggttgctt tgctatgc	18

<210>	153
<211>	20
<212>	DNA
<213>	NAT#220 STM primer

<400>	153
cagatctcga acgataccca	20

<210>	154
<211>	24
<212>	DNA
<213>	STM common primer

<400>	154

gcatgccctg cccctaagaa ttcg                                          24

<210>    155
<211>    19
<212>    DNA
<213>    CNAG_07901 5 flanking region primer 1

<400>    155
actcaccttc ccgctttag                                                19

<210>    156
<211>    34
<212>    DNA
<213>    CNAG_07901 5 flanking region primer 2

<400>    156
ctggccgtcg ttttacgttt gagttgaggt tgcg                               34

<210>    157
<211>    36
<212>    DNA
<213>    CNAG_07901 3 flanking region primer 1

<400>    157
gtcatagctg tttcctgaga atgttgatgg tagcgg                             36

<210>    158
<211>    19
<212>    DNA
<213>    CNAG_07901 3 flanking region primer 2

<400>    158
tccaccgaag aaactgaag                                                19

<210>    159

<211>    18
<212>    DNA
<213>    CNAG_07901 diagnostic screening primer, pairing with B79

<400>    159
gggatttgga tacgcttg                                                    18

<210>    160
<211>    18
<212>    DNA
<213>    CNAG_07901 Southern blot probe primer

<400>    160
ttgcggtggg aaagtatc                                                    18

<210>    161
<211>    20
<212>    DNA
<213>    NAT#225 STM primer

<400>    161
ccatagaact agctaaagca                                                  20

<210>    162
<211>    24
<212>    DNA
<213>    STM common primer

<400>    162
gcatgccctg cccctaagaa ttcg                                             24

<210>    163
<211>    19
<212>    DNA
<213>    CNAG_04588 5 flanking region primer 1

<400> 163
aaaagaggtc cgatgagtg 19

<210> 164
<211> 40
<212> DNA
<213> CNAG_04588 5 flanking region primer 2

<400> 164
gctcactggc cgtcgtttta cgtgaatgat aaaggcgacc 40

<210> 165
<211> 39
<212> DNA
<213> CNAG_04588 3 flanking region primer 1

<400> 165
catggtcata gctgtttcct gtgctgtttc actatccgc 39

<210> 166
<211> 19
<212> DNA
<213> CNAG_04588 3 flanking region primer 2

<400> 166
cgaaagcgac tggagtaac 19

<210> 167
<211> 20
<212> DNA
<213> CNAG_04588 diagnostic screening primer, pairing with B79

<400> 167
gaagatgctg aagcgtattc 20

<210> 168
<211> 18
<212> DNA
<213> CNAG_04588 Southern blot probe primer

<400> 168
acacttatcc gccattcc 18

<210> 169
<211> 20
<212> DNA
<213> NAT#225 STM primer

<400> 169
ccatagaact agctaaagca 20

<210> 170
<211> 24
<212> DNA
<213> STM common primer

<400> 170
gcatgccctg cccctaagaa ttcg 24

<210> 171
<211> 18
<212> DNA
<213> CNAG_03998 5 flanking region primer 1

<400> 171
gccattgtga aagcatag 18

<210> 172
<211> 43
<212> DNA
<213> CNAG_03998 5 flanking region primer 2

<400> 172
gctcactggc cgtcgtttta cacctgtcta tctcactgtc cag 43

<210> 173
<211> 39
<212> DNA
<213> CNAG_03998 3 flanking region primer 1

<400> 173
catggtcata gctgtttcct gtgttcagca gcaaaaggc 39

<210> 174
<211> 18
<212> DNA
<213> CNAG_03998 3 flanking region primer 2

<400> 174
gcggagagaa ggattatc 18

<210> 175
<211> 20
<212> DNA
<213> CNAG_03998 diagnostic screening primer, pairing with B79

<400> 175
gctgacaact tggaatcatc 20

<210> 176
<211> 20
<212> DNA
<213> CNAG_03998 Southern blot probe primer

<400> 176
gggcttctct tcttcttcac 20

<210> 177
<211> 20
<212> DNA
<213> NAT#234 STM primer

<400> 177
ccagccagca ctccgatata 20

<210> 178
<211> 24
<212> DNA
<213> STM common primer

<400> 178
gcatgccctg cccctaagaa ttcg 24

<210> 179
<211> 20
<212> DNA
<213> CNAG_04804 5 flanking region primer 1

<400> 179
taggtagttc ctgctcgtgg 20

<210> 180
<211> 40
<212> DNA
<213> CNAG_04804 5 flanking region primer 2

<400> 180
tcactggccg tcgttttacc atcttgtcct gtaatgaggc 40

<210> 181
<211> 39
<212> DNA

<213>    CNAG_04804 3 flanking region primer 1

<400>    181
catggtcata gctgtttcct ggattggagg gcgatttag                              39


<210>    182
<211>    20
<212>    DNA
<213>    CNAG_04804 3 flanking region primer 2

<400>    182
caacaaggtc caagaaactg                                                   20


<210>    183
<211>    21
<212>    DNA
<213>    CNAG_04804 diagnostic screening primer, pairing with B79

<400>    183
agaggactga gacttttgtc g                                                 21


<210>    184
<211>    19
<212>    DNA
<213>    CNAG_04804 Southern blot probe primer

<400>    184
cgaaggggaa ctggttatc                                                    19


<210>    185
<211>    20
<212>    DNA
<213>    NAT#240 STM primer

<400>    185
ggtgttggat cggggtggat                                                   20

<210> 186
<211> 24
<212> DNA
<213> STM common primer

<400> 186
gcatgccctg cccctaagaa ttcg 24

<210> 187
<211> 20
<212> DNA
<213> CNAG_06134 5 flanking region primer 1

<400> 187
gtttgaggct ggtaaaaagg 20

<210> 188
<211> 39
<212> DNA
<213> CNAG_06134 5 flanking region primer 2

<400> 188
gctcactggc cgtcgtttta catggggaat gaaagcgtg 39

<210> 189
<211> 41
<212> DNA
<213> CNAG_06134 3 flanking region primer 1

<400> 189
catggtcata gctgtttcct gaaggggcga gagtagttca g 41

<210> 190
<211> 21

<212>    DNA
<213>    CNAG_06134 3 flanking region primer 2

<400>    190
gactgtaaag gagggcataa g                                                21

<210>    191
<211>    19
<212>    DNA
<213>    CNAG_06134 diagnostic screening primer, pairing with B79

<400>    191
aactcttctc agccttcgg                                                   19

<210>    192
<211>    19
<212>    DNA
<213>    CNAG_06134 Southern blot probe primer

<400>    192
cgttctccgt cttgatagc                                                   19

<210>    193
<211>    21
<212>    DNA
<213>    NAT#295 STM primer

<400>    193
acacctacat caaaccctcc c                                                21

<210>    194
<211>    24
<212>    DNA
<213>    STM common primer

<400>    194

gcatgccctg cccctaagaa ttcg                                                24

<210>   195
<211>   19
<212>   DNA
<213>   CNAG_04798 5 flanking region primer 1

<400>   195
caatgattag gcttgcgtc                                                      19

<210>   196
<211>   38
<212>   DNA
<213>   CNAG_04798 5 flanking region primer 2

<400>   196
tcactggccg tcgttttact gttgttgggc agtggtag                                 38

<210>   197
<211>   39
<212>   DNA
<213>   CNAG_04798 3 flanking region primer 1

<400>   197
catggtcata gctgtttcct gtttagggaa gaggaagcg                                39

<210>   198
<211>   18
<212>   DNA
<213>   CNAG_04798 3 flanking region primer 2

<400>   198
tgtcatccag cagattgg                                                       18

<210>   199

<211>    19
<212>    DNA
<213>    CNAG_04798 diagnostic screening primer, pairing with B79

<400>    199
tgaatgactg atggacccc                                                    19

<210>    200
<211>    18
<212>    DNA
<213>    CNAG_04798 Southern blot probe primer

<400>    200
tcggttctcg ttttggag                                                     18

<210>    201
<211>    20
<212>    DNA
<213>    CNAG_04864 5 flanking region primer 1

<400>    201
tcatttcttc gtcctctgag                                                   20

<210>    202
<211>    36
<212>    DNA
<213>    CNAG_04864 5 flanking region primer 2

<400>    202
ctggccgtcg ttttacgctc cccataatca gactcc                                 36

<210>    203
<211>    36
<212>    DNA
<213>    CNAG_04864 3 flanking region primer 1

<400>    203
gtcatagctg tttcctgacg aatggagttt ctggag                                36

<210>    204
<211>    20
<212>    DNA
<213>    CNAG_04864 3 flanking region primer 2

<400>    204
gcgttctcca tagttgtagc                                                  20

<210>    205
<211>    20
<212>    DNA
<213>    CNAG_04864 diagnostic screening primer, pairing with B79

<400>    205
atgtagataa agcacgcacc                                                  20

<210>    206
<211>    20
<212>    DNA
<213>    CNAG_04864 Southern blot probe primer

<400>    206
cgagttattg taggttgggc                                                  20

<210>    207
<211>    20
<212>    DNA
<213>    CNAG_01973 5 flanking region primer 1

<400>    207
gcttgaggtt gaaacaggtc                                                  20

<210> 208
<211> 41
<212> DNA
<213> CNAG_01973 5 flanking region primer 2

<400> 208
tcactggccg tcgttttacc agatgtgtgt aagagggtca g 41

<210> 209
<211> 39
<212> DNA
<213> CNAG_01973 3 flanking region primer 1

<400> 209
catggtcata gctgtttcct gtaccgagaa cgatttggc 39

<210> 210
<211> 20
<212> DNA
<213> CNAG_01973 3 flanking region primer 2

<400> 210
tcccataaga ccacacactg 20

<210> 211
<211> 19
<212> DNA
<213> CNAG_01973 diagnostic screening primer, pairing with B79

<400> 211
tgatgctgaa tagacgggg 19

<210> 212
<211> 19
<212> DNA
<213> CNAG_01973 Southern blot probe primer

<400> 212
agccttgacg acatacctg 19

<210> 213
<211> 20
<212> DNA
<213> NAT#6 STM primer

<400> 213
atagctacca cacgatagct 20

<210> 214
<211> 24
<212> DNA
<213> STM common primer

<400> 214
gcatgccctg cccctaagaa ttcg 24

<210> 215
<211> 19
<212> DNA
<213> CNAG_03914 5 flanking region primer 1

<400> 215
gatgtcgttg ttccagagc 19

<210> 216
<211> 35
<212> DNA
<213> CNAG_03914 5 flanking region primer 2

<400> 216
ctggccgtcg ttttactttg gatgtaagag gtcgg 35

<210>    217
<211>    37
<212>    DNA
<213>    CNAG_03914 3 flanking region primer 1

<400>    217
gtcatagctg tttcctgcga gtatccttac cgtgtcg                                37

<210>    218
<211>    19
<212>    DNA
<213>    CNAG_03914 3 flanking region primer 2

<400>    218
acactcctgc ccaagaaac                                                    19

<210>    219
<211>    19
<212>    DNA
<213>    CNAG_03914 diagnostic screening primer, pairing with B79

<400>    219
actccactcc atccaaagc                                                    19

<210>    220
<211>    20
<212>    DNA
<213>    CNAG_03914 Southern blot probe primer

<400>    220
ataagaggtc cttttgctcc                                                   20

<210>    221
<211>    20
<212>    DNA

<213>    NAT#43 STM primer

<400>    221
ccagctacca atcacgctac                                                    20

<210>    222
<211>    24
<212>    DNA
<213>    STM common primer

<400>    222
gcatgccctg cccctaagaa ttcg                                               24

<210>    223
<211>    19
<212>    DNA
<213>    CNAG_00828 5 flanking region primer 1

<400>    223
accagtggca aggttagag                                                     19

<210>    224
<211>    39
<212>    DNA
<213>    CNAG_00828 5 flanking region primer 2

<400>    224
tcactggccg tcgttttact ggcagaagca caagattag                               39

<210>    225
<211>    40
<212>    DNA
<213>    CNAG_00828 3 flanking region primer 1

<400>    225
catggtcata gctgtttcct ggccatttct cgtagcgaac                              40

<210> 226
<211> 19
<212> DNA
<213> CNAG_00828 3 flanking region primer 2

<400> 226
actgcttatc cccacatcg 19

<210> 227
<211> 20
<212> DNA
<213> CNAG_00828 diagnostic screening primer, pairing with B79

<400> 227
tgtgaagtgt gatgatgtcc 20

<210> 228
<211> 19
<212> DNA
<213> CNAG_00828 Southern blot probe primer

<400> 228
ggttgccact gataaaagc 19

<210> 229
<211> 24
<212> DNA
<213> NAT#58 STM primer

<400> 229
cgcaaaatca ctagccctat agcg 24

<210> 230
<211> 24

<212>    DNA
<213>    STM common primer

<400>    230
   gcatgccctg cccctaagaa ttcg                                          24

<210>    231
<211>    19
<212>    DNA
<213>    CNAG_00871 5 flanking region primer 1

<400>    231
atagcgggca agtgtctac                                                 19

<210>    232
<211>    38
<212>    DNA
<213>    CNAG_00871 5 flanking region primer 2

<400>    232
tcactggccg tcgttttacc caacctttca ttccagtc                            38

<210>    233
<211>    42
<212>    DNA
<213>    CNAG_00871 3 flanking region primer 1

<400>    233
catggtcata gctgtttcct gggatacaaa tctcaacccc tg                       42

<210>    234
<211>    19
<212>    DNA
<213>    CNAG_00871 3 flanking region primer 2

<400>    234

ggttttcact tcaagagcg 19

<210> 235
<211> 18
<212> DNA
<213> CNAG_00871 diagnostic screening primer, pairing with B79

<400> 235
tccaatagca ccacaacc 18

<210> 236
<211> 19
<212> DNA
<213> CNAG_00871 Southern blot probe primer

<400> 236
aacatccctt tactcggcg 19

<210> 237
<211> 24
<212> DNA
<213> NAT#102 STM primer

<400> 237
ccatagcgat atctacccca atct 24

<210> 238
<211> 24
<212> DNA
<213> STM common primer

<400> 238
gcatgccctg cccctaagaa ttcg 24

<210> 239

<211>    19
<212>    DNA
<213>    CNAG_07464 5 flanking region primer 1

<400>    239
ggcatcagga tagaaacgc                                                    19

<210>    240
<211>    41
<212>    DNA
<213>    CNAG_07464 5 flanking region primer 2

<400>    240
gctcactggc cgtcgtttta cggggtgaga tttgaaggta g                           41

<210>    241
<211>    40
<212>    DNA
<213>    CNAG_07464 3 flanking region primer 1

<400>    241
catggtcata gctgtttcct ggatgtgact tggtcttggg                             40

<210>    242
<211>    20
<212>    DNA
<213>    CNAG_07464 3 flanking region primer 2

<400>    242
tggtccagtc tcctcattac                                                   20

<210>    243
<211>    18
<212>    DNA
<213>    CNAG_07464 diagnostic screening primer, pairing with B79

<400>    243
tcttccatct cagcatcg                                                    18

<210>    244
<211>    24
<212>    DNA
<213>    CNAG_07464 Southern blot probe primer

<400>    244
ccatagcgat atctacccca atct                                             24

<210>    245
<211>    20
<212>    DNA
<213>    NAT#150 STM primer

<400>    245
acatacaccc ccatcccccc                                                  20

<210>    246
<211>    24
<212>    DNA
<213>    STM common primer

<400>    246
gcatgccctg cccctaagaa ttcg                                             24

<210>    247
<211>    23
<212>    DNA
<213>    CNAG_00460 5 flanking region primer 1

<400>    247
cgtgggagag tgagtaaagt atg                                              23

<210> 248
<211> 42
<212> DNA
<213> CNAG_00460 5 flanking region primer 2

<400> 248
gctcactggc cgtcgtttta ccgggtaaga gtggatgtat gg 42

<210> 249
<211> 39
<212> DNA
<213> CNAG_00460 3 flanking region primer 1

<400> 249
catggtcata gctgtttcct gaagggcacc gttcattag 39

<210> 250
<211> 19
<212> DNA
<213> CNAG_00460 3 flanking region primer 2

<400> 250
ttcttaggat gcgggactc 19

<210> 251
<211> 18
<212> DNA
<213> CNAG_00460 diagnostic screening primer, pairing with B79

<400> 251
tgcgttgaca aagtcgtg 18

<210> 252
<211> 22
<212> DNA
<213> CNAG_00460 Southern blot probe primer

<400>    252
tagactccag taaggtccaa tc                                                22

<210>    253
<211>    24
<212>    DNA
<213>    NAT#213 STM primer

<400>    253
ctggggattt tgatgtgtct atgt                                              24

<210>    254
<211>    24
<212>    DNA
<213>    STM common primer

<400>    254
gcatgccctg cccctaagaa ttcg                                              24

<210>    255
<211>    18
<212>    DNA
<213>    CNAG_01431 5 flanking region primer 1

<400>    255
tccatttggg ggtttcag                                                     18

<210>    256
<211>    39
<212>    DNA
<213>    CNAG_01431 5 flanking region primer 2

<400>    256
tcactggccg tcgttttact gtcttcgttc ttgccctcc                              39

<210> 257
<211> 42
<212> DNA
<213> CNAG_01431 3 flanking region primer 1

<400> 257
catggtcata gctgtttcct gcgttgacag aagaggacaa gg 42

<210> 258
<211> 20
<212> DNA
<213> CNAG_01431 3 flanking region primer 2

<400> 258
tgacagatga tgagcagagg 20

<210> 259
<211> 19
<212> DNA
<213> CNAG_01431 diagnostic screening primer, pairing with B79

<400> 259
attccagagc ctcacttgc 19

<210> 260
<211> 21
<212> DNA
<213> CNAG_01431 Southern blot probe primer

<400> 260
cctgcttgtc actacactga g 21

<210> 261
<211> 24
<212> DNA

<213>    NAT#213 STM primer

<400>    261
ctggggattt tgatgtgtct atgt                                              24


<210>    262
<211>    24
<212>    DNA
<213>    STM common primer

<400>    262
gcatgccctg cccctaagaa ttcg                                              24


<210>    263
<211>    21
<212>    DNA
<213>    CNAG_03894 5 flanking region primer 1

<400>    263
catctatctc aacggggtat c                                                 21


<210>    264
<211>    39
<212>    DNA
<213>    CNAG_03894 5 flanking region primer 2

<400>    264
gctcactggc cgtcgtttta ctttttcggg agaacgcag                              39


<210>    265
<211>    40
<212>    DNA
<213>    CNAG_03894 3 flanking region primer 1

<400>    265
catggtcata gctgtttcct gaggaccaag aatgctgagg                             40

<210> 266
<211> 22
<212> DNA
<213> CNAG_03894 3 flanking region primer 2

<400> 266
ttctcccttg tcgccgataa gc 22

<210> 267
<211> 20
<212> DNA
<213> CNAG_03894 diagnostic screening primer, pairing with B79

<400> 267
tcttatcgct caccacatcc 20

<210> 268
<211> 19
<212> DNA
<213> CNAG_03894 Southern blot probe primer

<400> 268
ggaacattgg gaaaaggtg 19

<210> 269
<211> 20
<212> DNA
<213> NAT#220 STM primer

<400> 269
cagatctcga acgataccca 20

<210> 270
<211> 24

<212>    DNA
<213>    STM common primer

<400>    270
gcatgccctg cccctaagaa ttcg                                              24


<210>    271
<211>    18
<212>    DNA
<213>    CNAG_06483 5 flanking region primer 1

<400>    271
tggagcagat ggagtttg                                                     18


<210>    272
<211>    35
<212>    DNA
<213>    CNAG_06483 5 flanking region primer 2

<400>    272
ctggccgtcg ttttacgctt ctccctattt ccagc                                  35


<210>    273
<211>    36
<212>    DNA
<213>    CNAG_06483 3 flanking region primer 1

<400>    273
gtcatagctg tttcctgaat ggagcccaga gaacag                                 36


<210>    274
<211>    19
<212>    DNA
<213>    CNAG_06483 3 flanking region primer 2

<400>    274

tctcgctgag ggaaaactc 19

<210> 275
<211> 18
<212> DNA
<213> CNAG_06483 diagnostic screening primer, pairing with B79

<400> 275
ccctttctct gcgttttg 18

<210> 276
<211> 18
<212> DNA
<213> CNAG_06483 Southern blot probe primer

<400> 276
caatgaagaa ctggtgcg 18

<210> 277
<211> 20
<212> DNA
<213> NAT#227 STM primer

<400> 277
tcgtggttta gagggagcgc 20

<210> 278
<211> 24
<212> DNA
<213> STM common primer

<400> 278
gcatgccctg cccctaagaa ttcg 24

<210> 279

<211>    18
<212>    DNA
<213>    CNAG_00896 5 flanking region primer 1

<400>    279
aaagggtcac gatggaac                                                    18

<210>    280
<211>    35
<212>    DNA
<213>    CNAG_00896 5 flanking region primer 2

<400>    280
ctggccgtcg ttttacgcga ttctttactc ctggc                                 35

<210>    281
<211>    36
<212>    DNA
<213>    CNAG_00896 3 flanking region primer 1

<400>    281
gtcatagctg tttcctgttc tgagacctcc gtaacc                                36

<210>    282
<211>    19
<212>    DNA
<213>    CNAG_00896 3 flanking region primer 2

<400>    282
ctgtatcgct ttagccacc                                                   19

<210>    283
<211>    19
<212>    DNA
<213>    CNAG_00896 diagnostic screening primer, pairing with B79

<400> 283
tacaaggacc tcacgaacg 19

<210> 284
<211> 20
<212> DNA
<213> CNAG_00896 Southern blot probe primer

<400> 284
acgagcagag ggacagatag 20

<210> 285
<211> 20
<212> DNA
<213> NAT#231 STM primer

<400> 285
gagagatccc aacatcacgc 20

<210> 286
<211> 24
<212> DNA
<213> STM common primer

<400> 286
gcatgccctg cccctaagaa ttcg 24

<210> 287
<211> 18
<212> DNA
<213> CNAG_01977 5 flanking region primer 1

<400> 287
tgggaaaacg aagcactc 18

<210> 288
<211> 35
<212> DNA
<213> CNAG_01977 5 flanking region primer 2

<400> 288
ctggccgtcg ttttactagg aggtcacttt cgctc 35

<210> 289
<211> 36
<212> DNA
<213> CNAG_01977 3 flanking region primer 1

<400> 289
gtcatagctg tttcctgcgg caaagaacaa ggtatg 36

<210> 290
<211> 20
<212> DNA
<213> CNAG_01977 3 flanking region primer 2

<400> 290
tgatgggctt gaacgaacgc 20

<210> 291
<211> 19
<212> DNA
<213> CNAG_01977 diagnostic screening primer, pairing with B79

<400> 291
tgtcggcaca catctactg 19

<210> 292
<211> 22
<212> DNA
<213> CNAG_01977 Southern blot probe primer

<400>    292
gtctcagcct cttgttacag at                                              22

<210>    293
<211>    20
<212>    DNA
<213>    NAT#231 STM primer

<400>    293
gagagatccc aacatcacgc                                                 20

<210>    294
<211>    24
<212>    DNA
<213>    STM common primer

<400>    294
gcatgccctg cccctaagaa ttcg                                            24

<210>    295
<211>    19
<212>    DNA
<213>    CNAG_01069 5 flanking region primer 1

<400>    295
cttttatcta tggcgagcg                                                  19

<210>    296
<211>    39
<212>    DNA
<213>    CNAG_01069 5 flanking region primer 2

<400>    296
gctcactggc cgtcgtttta ctatcgcact gctggaatc                            39

<210>    297
<211>    40
<212>    DNA
<213>    CNAG_01069 3 flanking region primer 1

<400>    297
catggtcata gctgtttcct gctactttcc ctcacatccg                                40


<210>    298
<211>    19
<212>    DNA
<213>    CNAG_01069 3 flanking region primer 2

<400>    298
tggagggtag atgcgttac                                                       19


<210>    299
<211>    20
<212>    DNA
<213>    CNAG_01069 diagnostic screening primer, pairing with B79

<400>    299
agagaagcgg aagagaaaac                                                      20


<210>    300
<211>    20
<212>    DNA
<213>    CNAG_01069 Southern blot probe primer

<400>    300
ccttgcgaaa atgtcttctc                                                      20


<210>    301
<211>    21
<212>    DNA

<213>    NAT#292 STM primer

<400>    301
atttcgatgt atatatgtag c                                                21

<210>    302
<211>    24
<212>    DNA
<213>    STM common primer

<400>    302
gcatgccctg cccctaagaa ttcg                                             24

<210>    303
<211>    19
<212>    DNA
<213>    CNAG_06276 5 flanking region primer 1

<400>    303
tcaagtctac gcagtcgtg                                                   19

<210>    304
<211>    40
<212>    DNA
<213>    CNAG_06276 5 flanking region primer 2

<400>    304
gctcactggc cgtcgtttta cgcagcaatc aactcactcg                            40

<210>    305
<211>    41
<212>    DNA
<213>    CNAG_06276 3 flanking region primer 1

<400>    305
catggtcata gctgtttcct gaggagagtg ggagtatgga g                          41

<210> 306
<211> 18
<212> DNA
<213> CNAG_06276 3 flanking region primer 2

<400> 306
acccatcaaa aggcgtag 18

<210> 307
<211> 20
<212> DNA
<213> CNAG_06276 diagnostic screening primer, pairing with B79

<400> 307
accatttgtc aactgtcagg 20

<210> 308
<211> 20
<212> DNA
<213> CNAG_06276 Southern blot probe primer

<400> 308
tcgctcactg acaagaagac 20

<210> 309
<211> 21
<212> DNA
<213> NAT#292 STM primer

<400> 309
atttcgatgt atatatgtag c 21

<210> 310
<211> 24

<212>    DNA
<213>    STM common primer

<400>    310
gcatgccctg cccctaagaa ttcg                                              24


<210>    311
<211>    19
<212>    DNA
<213>    CNAG_02476 5 flanking region primer 1

<400>    311
tctgctcaat gaaacgggc                                                    19


<210>    312
<211>    45
<212>    DNA
<213>    CNAG_02476 5 flanking region primer 2

<400>    312
gctcactggc cgtcgtttta cgcctttgct tcctttgctt cttac                       45


<210>    313
<211>    40
<212>    DNA
<213>    CNAG_02476 3 flanking region primer 1

<400>    313
catggtcata gctgtttcct ggtgtcttga atccaatgcc                             40


<210>    314
<211>    20
<212>    DNA
<213>    CNAG_02476 3 flanking region primer 2

<400>    314

ctgctccata caaggcttag                                                    20

<210>    315
<211>    20
<212>    DNA
<213>    CNAG_02476 diagnostic screening primer, pairing with B79

<400>    315
tcgtcagcaa ttgaaaacct                                                    20

<210>    316
<211>    19
<212>    DNA
<213>    CNAG_02476 Southern blot probe primer

<400>    316
atcaagtcag accacgagg                                                     19

<210>    317
<211>    20
<212>    DNA
<213>    NAT#219 STM primer

<400>    317
ccctaaaacc ctacagcaat                                                    20

<210>    318
<211>    24
<212>    DNA
<213>    STM common primer

<400>    318
gcatgccctg cccctaagaa ttcg                                               24

<210>    319

<211>    20
<212>    DNA
<213>    CNAG_01841 5 flanking region primer 1

<400>    319
ccattatcat ccgtcaccac                                                    20


<210>    320
<211>    37
<212>    DNA
<213>    CNAG_01841 5 flanking region primer 2

<400>    320
ctggccgtcg ttttacgaag agggaagaga gggtaac                                 37


<210>    321
<211>    36
<212>    DNA
<213>    CNAG_01841 3 flanking region primer 1

<400>    321
gtcatagctg tttcctgtga ggctgaggaa acagag                                  36


<210>    322
<211>    18
<212>    DNA
<213>    CNAG_01841 3 flanking region primer 2

<400>    322
tccttttccc aacctgtc                                                      18


<210>    323
<211>    21
<212>    DNA
<213>    CNAG_01841 diagnostic screening primer, pairing with B79

<400>    323
ttcagtggtt ggtctctaca g                                                21

<210>    324
<211>    21
<212>    DNA
<213>    CNAG_01841 Southern blot probe primer

<400>    324
tggtaaaggt ctatcagtgc c                                                21

<210>    325
<211>    20
<212>    DNA
<213>    NAT#230 STM primer

<400>    325
atgtaggtag ggtgataggt                                                  20

<210>    326
<211>    24
<212>    DNA
<213>    STM common primer

<400>    326
gcatgccctg cccctaagaa ttcg                                             24

<210>    327
<211>    19
<212>    DNA
<213>    CNAG_04457 5 flanking region primer 1

<400>    327
cttcaaaatc cagagcagg                                                   19

<210>    328
<211>    38
<212>    DNA
<213>    CNAG_04457 5 flanking region primer 2

<400>    328
ctggccgtcg ttttacctgg tctgatagtt cgtattcg                                38

<210>    329
<211>    39
<212>    DNA
<213>    CNAG_04457 3 flanking region primer 1

<400>    329
gtcatagctg tttcctggga cttgaacaat ggtatggac                               39

<210>    330
<211>    19
<212>    DNA
<213>    CNAG_04457 3 flanking region primer 2

<400>    330
tttagagcct acggtggtc                                                     19

<210>    331
<211>    22
<212>    DNA
<213>    CNAG_04457 diagnostic screening primer, pairing with B79

<400>    331
ttgcccgacg agatgctatg ac                                                 22

<210>    332
<211>    20
<212>    DNA
<213>    CNAG_04457 Southern blot probe primer

<400> 332
tccgtctccg atgatttgcg 20

<210> 333
<211> 20
<212> DNA
<213> NAT#230 STM primer

<400> 333
atgtaggtag ggtgataggt 20

<210> 334
<211> 24
<212> DNA
<213> STM common primer

<400> 334
gcatgccctg cccctaagaa ttcg 24

<210> 335
<211> 19
<212> DNA
<213> CNAG_06252 5 flanking region primer 1

<400> 335
tctgacaggt tcaatcgcc 19

<210> 336
<211> 41
<212> DNA
<213> CNAG_06252 5 flanking region primer 2

<400> 336
tcactggccg tcgttttacg catttctaca ccgtgattag c 41

<210> 337
<211> 40
<212> DNA
<213> CNAG_06252 3 flanking region primer 1

<400> 337
catggtcata gctgtttcct gggatgtgcg gttttgttag 40

<210> 338
<211> 19
<212> DNA
<213> CNAG_06252 3 flanking region primer 2

<400> 338
taacgaagtt ctccccgtg 19

<210> 339
<211> 20
<212> DNA
<213> CNAG_06252 diagnostic screening primer, pairing with B79

<400> 339
tgagagggaa cgaaggatac 20

<210> 340
<211> 20
<212> DNA
<213> CNAG_06252 Southern blot probe primer

<400> 340
cttcgcctgt tggaaataag 20

<210> 341
<211> 20
<212> DNA

<213>    CNAG_06327 5 flanking region primer 1

<400>    341
ggagaaggga aagatgggtg                                                    20


<210>    342
<211>    41
<212>    DNA
<213>    CNAG_06327 5 flanking region primer 2

<400>    342
gctcactggc cgtcgtttta ctgtttccac tattgggaga g                            41


<210>    343
<211>    40
<212>    DNA
<213>    CNAG_06327 3 flanking region primer 1

<400>    343
catggtcata gctgtttcct gaggtgaagg tgtgagattg                             40


<210>    344
<211>    18
<212>    DNA
<213>    CNAG_06327 3 flanking region primer 2

<400>    344
caataatgtc ctcgccag                                                      18


<210>    345
<211>    19
<212>    DNA
<213>    CNAG_06327 diagnostic screening primer, pairing with B79

<400>    345
gtatgaaggg aaggtgctg                                                     19

<210>    346
<211>    18
<212>    DNA
<213>    CNAG_06327 Southern blot probe primer

<400>    346
acgggttacg agaaatgc                                                    18


<210>    347
<211>    19
<212>    DNA
<213>    CNAG_03790 5 flanking region primer 1

<400>    347
tgagggttgt tgagggaag                                                   19


<210>    348
<211>    40
<212>    DNA
<213>    CNAG_03790 5 flanking region primer 2

<400>    348
gctcactggc cgtcgtttta cagcactgta tcgccgaaac                            40


<210>    349
<211>    42
<212>    DNA
<213>    CNAG_03790 3 flanking region primer 1

<400>    349
catggtcata gctgtttcct gctcgtagca atgtagatgt gc                         42


<210>    350
<211>    21

<212>    DNA
<213>    CNAG_03790 3 flanking region primer 2

<400>    350
tcagatgaag acgactacga c                                                21


<210>    351
<211>    19
<212>    DNA
<213>    CNAG_03790 diagnostic screening primer, pairing with B79

<400>    351
cttccacaaa gccagattc                                                   19


<210>    352
<211>    18
<212>    DNA
<213>    CNAG_03790 Southern blot probe primer

<400>    352
agattgcgtg aaacccac                                                    18


<210>    353
<211>    19
<212>    DNA
<213>    CNAG_05112 5 flanking region primer 1

<400>    353
atgtgcgaag tccaagagc                                                   19


<210>    354
<211>    40
<212>    DNA
<213>    CNAG_05112 5 flanking region primer 2

<400>    354

gctcactggc cgtcgtttta cgggtgaaga caacagatgg 40

<210> 355
<211> 41
<212> DNA
<213> CNAG_05112 3 flanking region primer 1

<400> 355
catggtcata gctgtttcct gcagaccaac tggcgtaata g 41

<210> 356
<211> 19
<212> DNA
<213> CNAG_05112 3 flanking region primer 2

<400> 356
acgaccaaat gctcttcgc 19

<210> 357
<211> 19
<212> DNA
<213> CNAG_05112 diagnostic screening primer, pairing with B79

<400> 357
tcggatacag gcgatagag 19

<210> 358
<211> 19
<212> DNA
<213> CNAG_05112 Southern blot probe primer

<400> 358
tcaacagcag tttcaggtg 19

<210> 359

<211>    24
<212>    DNA
<213>    NAT#122 STM primer

<400>    359
acagctccaa acctcgctaa acag                                              24

<210>    360
<211>    24
<212>    DNA
<213>    STM common primer

<400>    360
gcatgccctg cccctaagaa ttcg                                              24

<210>    361
<211>    19
<212>    DNA
<213>    CNAG_00068 5 flanking region primer 1

<400>    361
tctatcacct ctgcgttgc                                                    19

<210>    362
<211>    39
<212>    DNA
<213>    CNAG_00068 5 flanking region primer 2

<400>    362
tcactggccg tcgttttaca ttcctaccac tatcccctg                              39

<210>    363
<211>    40
<212>    DNA
<213>    CNAG_00068 3 flanking region primer 1

<400> 363
catggtcata gctgtttcct gcggataaga tgcctgagac 40

<210> 364
<211> 22
<212> DNA
<213> CNAG_00068 3 flanking region primer 2

<400> 364
ggtatgtttc tcggatgtaa gg 22

<210> 365
<211> 20
<212> DNA
<213> CNAG_00068 diagnostic screening primer, pairing with B79

<400> 365
catcatcagg tatcagcagc 20

<210> 366
<211> 20
<212> DNA
<213> CNAG_00068 Southern blot probe primer

<400> 366
cttccaactt caatgttccc 20

<210> 367
<211> 24
<212> DNA
<213> NAT#58 STM primer

<400> 367
cgcaaaatca ctagccctat agcg 24

<210>    368
<211>    24
<212>    DNA
<213>    STM common primer

<400>    368
gcatgccctg cccctaagaa ttcg                                                24

<210>    369
<211>    19
<212>    DNA
<213>    CNAG_04184 5 flanking region primer 1

<400>    369
tcgttcacat aactgccac                                                      19

<210>    370
<211>    38
<212>    DNA
<213>    CNAG_04184 5 flanking region primer 2

<400>    370
tcactggccg tcgttttact cattgtgagc ctcgtatg                                 38

<210>    371
<211>    39
<212>    DNA
<213>    CNAG_04184 3 flanking region primer 1

<400>    371
catggtcata gctgtttcct gaatggtgac ggttcaagg                                39

<210>    372
<211>    18
<212>    DNA
<213>    CNAG_04184 3 flanking region primer 2

<400> 372
   cttttccact gaatccgc 18

<210> 373
<211> 19
<212> DNA
<213> CNAG_04184 diagnostic screening primer, pairing with B79

<400> 373
tggtccgatg ccgtaaatc 19

<210> 374
<211> 19
<212> DNA
<213> CNAG_04184 Southern blot probe primer

<400> 374
caagggaatg actcactcc 19

<210> 375
<211> 24
<212> DNA
<213> NAT#102 STM primer

<400> 375
ccatagcgat atctacccca atct 24

<210> 376
<211> 24
<212> DNA
<213> STM common primer

<400> 376
gcatgccctg cccctaagaa ttcg 24

<210> 377
<211> 18
<212> DNA
<213> CNAG_03018 5 flanking region primer 1

<400> 377
agccgtttgt cattctgc 18

<210> 378
<211> 37
<212> DNA
<213> CNAG_03018 5 flanking region primer 2

<400> 378
tcactggccg tcgttttacg tcgttgctca ttttgcg 37

<210> 379
<211> 39
<212> DNA
<213> CNAG_03018 3 flanking region primer 1

<400> 379
catggtcata gctgtttcct ggcgaacctc caataaagc 39

<210> 380
<211> 18
<212> DNA
<213> CNAG_03018 3 flanking region primer 2

<400> 380
cccttttttc cctgcttc 18

<210> 381
<211> 18
<212> DNA

<213>    CNAG_03018 diagnostic screening primer, pairing with B79

<400>    381
aatagggatg ccaaccag                                                    18

<210>    382
<211>    18
<212>    DNA
<213>    CNAG_03018 Southern blot probe primer

<400>    382
caaaaagtgt cagcacgc                                                    18

<210>    383
<211>    20
<212>    DNA
<213>    NAT#150 STM primer

<400>    383
acatacaccc ccatcccccc                                                  20

<210>    384
<211>    24
<212>    DNA
<213>    STM common primer

<400>    384
gcatgccctg cccctaagaa ttcg                                             24

<210>    385
<211>    20
<212>    DNA
<213>    CNAG_01708 5 flanking region primer 1

<400>    385
ccgactcgtt cgtctatctg                                                  20

<210> 386
<211> 41
<212> DNA
<213> CNAG_01708 5 flanking region primer 2

<400> 386
tcactggccg tcgttttact ggagaggata gatgtgatga c 41

<210> 387
<211> 41
<212> DNA
<213> CNAG_01708 3 flanking region primer 1

<400> 387
catggtcata gctgtttcct gaacttctac ccaggtcgtg c 41

<210> 388
<211> 18
<212> DNA
<213> CNAG_01708 3 flanking region primer 2

<400> 388
aactgccctt cttcaagc 18

<210> 389
<211> 20
<212> DNA
<213> CNAG_01708 diagnostic screening primer, pairing with B79

<400> 389
tagcctctcc caatgtccac 20

<210> 390
<211> 20

<212>    DNA
<213>    CNAG_01708 Southern blot probe primer

<400>    390
cgattctcct ttgctgagtc                                                    20


<210>    391
<211>    21
<212>    DNA
<213>    NAT#159 STM primer

<400>    391
acgcaccaga cacacaacca g                                                  21


<210>    392
<211>    24
<212>    DNA
<213>    STM common primer

<400>    392
gcatgccctg cccctaagaa ttcg                                               24


<210>    393
<211>    19
<212>    DNA
<213>    CNAG_05861 5 flanking region primer 1

<400>    393
ggttttggtc acttcaccg                                                     19


<210>    394
<211>    38
<212>    DNA
<213>    CNAG_05861 5 flanking region primer 2

<400>    394

tcactggccg tcgttttaca gcagaaagga gatgatgc 38

<210> 395
<211> 40
<212> DNA
<213> CNAG_05861 3 flanking region primer 1

<400> 395
catggtcata gctgtttcct ggacgacttt tacgaccagg 40

<210> 396
<211> 18
<212> DNA
<213> CNAG_05861 3 flanking region primer 2

<400> 396
aaatggaaca agcccgtc 18

<210> 397
<211> 18
<212> DNA
<213> CNAG_05861 diagnostic screening primer, pairing with B79

<400> 397
tctgaagggc gtaaatgc 18

<210> 398
<211> 18
<212> DNA
<213> CNAG_05861 Southern blot probe primer

<400> 398
aaggaatggg ttgttggg 18

<210> 399

<211>    24
<212>    DNA
<213>    NAT#184 STM primer

<400>    399
atatatggct cgagctagat agag                                              24


<210>    400
<211>    24
<212>    DNA
<213>    STM common primer

<400>    400
gcatgccctg cccctaagaa ttcg                                              24


<210>    401
<211>    20
<212>    DNA
<213>    CNAG_02066 5 flanking region primer 1

<400>    401
ccccattgta aacattggtg                                                   20


<210>    402
<211>    36
<212>    DNA
<213>    CNAG_02066 5 flanking region primer 2

<400>    402
ctggccgtcg ttttaccgtg atgtggagtt gattcg                                 36


<210>    403
<211>    36
<212>    DNA
<213>    CNAG_02066 3 flanking region primer 1

<400> 403
gtcatagctg tttcctgtgc tgccaatgga aggttc 36

<210> 404
<211> 20
<212> DNA
<213> CNAG_02066 3 flanking region primer 2

<400> 404
atagtctcgc tccttggtgc 20

<210> 405
<211> 19
<212> DNA
<213> CNAG_02066 diagnostic screening primer, pairing with B79

<400> 405
gttcgttatc cgctctttg 19

<210> 406
<211> 19
<212> DNA
<213> CNAG_02066 Southern blot probe primer

<400> 406
acataggcga gacagttgc 19

<210> 407
<211> 20
<212> DNA
<213> NAT#191 STM primer

<400> 407
atatggatgt ttttagcgag 20

<210> 408
<211> 24
<212> DNA
<213> STM common primer

<400> 408
gcatgccctg cccctaagaa ttcg 24

<210> 409
<211> 19
<212> DNA
<213> CNAG_03527 5 flanking region primer 1

<400> 409
ccatctcacg gttgatacg 19

<210> 410
<211> 35
<212> DNA
<213> CNAG_03527 5 flanking region primer 2

<400> 410
ctggccgtcg ttttactcaa acccttatcc caccg 35

<210> 411
<211> 37
<212> DNA
<213> CNAG_03527 3 flanking region primer 1

<400> 411
gtcatagctg tttcctgtct ttccctgctt tgcctgc 37

<210> 412
<211> 20
<212> DNA
<213> CNAG_03527 3 flanking region primer 2

<400>    412
cgtcatccgt cgtgtaactg                                                    20

<210>    413
<211>    19
<212>    DNA
<213>    CNAG_03527 diagnostic screening primer, pairing with B79

<400>    413
tctcgcaagg tcagagttc                                                     19

<210>    414
<211>    21
<212>    DNA
<213>    CNAG_03527 Southern blot probe primer

<400>    414
ctatgagcga cattcacaga c                                                  21

<210>    415
<211>    20
<212>    DNA
<213>    NAT#204 STM primer

<400>    415
gatctctcgc gcttggggga                                                    20

<210>    416
<211>    24
<212>    DNA
<213>    STM common primer

<400>    416
gcatgccctg cccctaagaa ttcg                                               24

<210> 417
<211> 18
<212> DNA
<213> CNAG_04263 5 flanking region primer 1

<400> 417
tgtttccaca caccgatg 18

<210> 418
<211> 36
<212> DNA
<213> CNAG_04263 5 flanking region primer 2

<400> 418
ctggccgtcg ttttacggtg gtggtcatct gtatgc 36

<210> 419
<211> 37
<212> DNA
<213> CNAG_04263 3 flanking region primer 1

<400> 419
gtcatagctg tttcctgaga agatggagac gcacacg 37

<210> 420
<211> 19
<212> DNA
<213> CNAG_04263 3 flanking region primer 2

<400> 420
gctattgcgt gaagactcc 19

<210> 421
<211> 20
<212> DNA

<213>    CNAG_04263 diagnostic screening primer, pairing with B79

<400>    421
cgtgattgag agtagcaacc                                                    20

<210>    422
<211>    19
<212>    DNA
<213>    CNAG_04263 Southern blot probe primer

<400>    422
gtaatgtcag tgagtgcgg                                                     19

<210>    423
<211>    24
<212>    DNA
<213>    NAT#205 STM primer

<400>    423
tatccccctc tccgctctct agca                                               24

<210>    424
<211>    24
<212>    DNA
<213>    STM common primer

<400>    424
gcatgccctg cccctaagaa ttcg                                               24

<210>    425
<211>    20
<212>    DNA
<213>    CNAG_00018 5 flanking region primer 1

<400>    425
tttgcctcaa tctctctcag                                                    20

<210>    426
<211>    41
<212>    DNA
<213>    CNAG_00018 5 flanking region primer 2

<400>    426
gctcactggc cgtcgtttta cggaatacaa catccacctc c                41

<210>    427
<211>    42
<212>    DNA
<213>    CNAG_00018 3 flanking region primer 1

<400>    427
catggtcata gctgtttcct ggcacgggtt acattctaca tc               42

<210>    428
<211>    20
<212>    DNA
<213>    CNAG_00018 3 flanking region primer 2

<400>    428
ggggaggact tgaacttgag                                        20

<210>    429
<211>    18
<212>    DNA
<213>    CNAG_00018 diagnostic screening primer, pairing with B79

<400>    429
aacaaactgc cttcctcc                                          18

<210>    430
<211>    19

<212>    DNA
<213>    CNAG_00018 Southern blot probe primer

<400>    430
gcttggtcgg ataaagatg                                                    19


<210>    431
<211>    20
<212>    DNA
<213>    NAT#211 STM primer

<400>    431
gcggtcgctt tatagcgatt                                                   20


<210>    432
<211>    24
<212>    DNA
<213>    STM common primer

<400>    432
gcatgccctg cccctaagaa ttcg                                              24


<210>    433
<211>    19
<212>    DNA
<213>    CNAG_05176 5 flanking region primer 1

<400>    433
tgagaaggag gatggtcac                                                    19


<210>    434
<211>    37
<212>    DNA
<213>    CNAG_05176 5 flanking region primer 2

<400>    434

tcactggccg tcgttttaca tgtggatagt gttgcgg 37

<210> 435
<211> 40
<212> DNA
<213> CNAG_05176 3 flanking region primer 1

<400> 435
catggtcata gctgtttcct ggcaaaaagg gagaatgtcg 40

<210> 436
<211> 19
<212> DNA
<213> CNAG_05176 3 flanking region primer 2

<400> 436
tcatacagcc gagttgacc 19

<210> 437
<211> 19
<212> DNA
<213> CNAG_05176 diagnostic screening primer, pairing with B79

<400> 437
tgtgagtatg cctatcccg 19

<210> 438
<211> 19
<212> DNA
<213> CNAG_05176 Southern blot probe primer

<400> 438
tcaccgagac taatggcac 19

<210> 439

<211>	20
<212>	DNA
<213>	NAT#211 STM primer

<400>	439
gcggtcgctt tatagcgatt	20

<210>	440
<211>	24
<212>	DNA
<213>	STM common primer

<400>	440
gcatgccctg cccctaagaa ttcg	24

<210>	441
<211>	19
<212>	DNA
<213>	CNAG_06339 5 flanking region primer 1

<400>	441
tagtgaaaga ctcggcacc	19

<210>	442
<211>	37
<212>	DNA
<213>	CNAG_06339 5 flanking region primer 2

<400>	442
tcactggccg tcgttttacc agtcaccgca aagattc	37

<210>	443
<211>	41
<212>	DNA
<213>	CNAG_06339 3 flanking region primer 1

<400>    443
catggtcata gctgtttcct gcgcttcgtt attacctgtt c                41

<210>    444
<211>    22
<212>    DNA
<213>    CNAG_06339 3 flanking region primer 2

<400>    444
ctccaggata agtagtgtga gg                                     22

<210>    445
<211>    20
<212>    DNA
<213>    CNAG_06339 diagnostic screening primer, pairing with B79

<400>    445
gggaaagtag ggtaaccacc                                        20

<210>    446
<211>    20
<212>    DNA
<213>    CNAG_06339 Southern blot probe primer

<400>    446
gaggagaggt gaataatgcc                                        20

<210>    447
<211>    24
<212>    DNA
<213>    NAT#213 STM primer

<400>    447
ctggggattt tgatgtgtct atgt                                   24

<210> 448
<211> 24
<212> DNA
<213> STM common primer

<400> 448
gcatgccctg cccctaagaa ttcg 24

<210> 449
<211> 19
<212> DNA
<213> CNAG_07593 5 flanking region primer 1

<400> 449
gattgcttgg atggacttc 19

<210> 450
<211> 39
<212> DNA
<213> CNAG_07593 5 flanking region primer 2

<400> 450
tcactggccg tcgttttact gcctttcttg ccgaactcg 39

<210> 451
<211> 40
<212> DNA
<213> CNAG_07593 3 flanking region primer 1

<400> 451
catggtcata gctgtttcct gtttgaagga ccaggagcag 40

<210> 452
<211> 19
<212> DNA
<213> CNAG_07593 3 flanking region primer 2

<400> 452
tcaagtgaca cagtgccac 19

<210> 453
<211> 19
<212> DNA
<213> CNAG_07593 diagnostic screening primer, pairing with B79

<400> 453
ccataatcag acagcgttg 19

<210> 454
<211> 19
<212> DNA
<213> CNAG_07593 Southern blot probe primer

<400> 454
ggtttggggg aatagtgag 19

<210> 455
<211> 20
<212> DNA
<213> NAT#218 STM primer

<400> 455
ctccacatcc atcgctccaa 20

<210> 456
<211> 24
<212> DNA
<213> STM common primer

<400> 456
gcatgccctg cccctaagaa ttcg 24

<210> 457
<211> 20
<212> DNA
<213> CNAG_00670 5 flanking region primer 1

<400> 457
tgatgaagat gctgatgagg 20

<210> 458
<211> 41
<212> DNA
<213> CNAG_00670 5 flanking region primer 2

<400> 458
gctcactggc cgtcgtttta ctcatttggc gtctcactta c 41

<210> 459
<211> 39
<212> DNA
<213> CNAG_00670 3 flanking region primer 1

<400> 459
catggtcata gctgtttcct gtgtgaaagg ggagtgatg 39

<210> 460
<211> 19
<212> DNA
<213> CNAG_00670 3 flanking region primer 2

<400> 460
agaatacatc gtggttggc 19

<210> 461
<211> 22
<212> DNA

<213>    CNAG_00670 diagnostic screening primer, pairing with B79

<400>    461
ggaagtgtag agtcaggaga tg                                                22

<210>    462
<211>    21
<212>    DNA
<213>    CNAG_00670 Southern blot probe primer

<400>    462
ggatgaaagg tgaaaggaca c                                                 21

<210>    463
<211>    19
<212>    DNA
<213>    NAT#224 STM primer

<400>    463
aacctttaaa tgggtagag                                                    19

<210>    464
<211>    24
<212>    DNA
<213>    STM common primer

<400>    464
gcatgccctg cccctaagaa ttcg                                              24

<210>    465
<211>    18
<212>    DNA
<213>    CNAG_05010 5 flanking region primer 1

<400>    465
tccttccttt ctaagccg                                                     18

<210> 466
<211> 41
<212> DNA
<213> CNAG_05010 5 flanking region primer 2

<400> 466
gctcactggc cgtcgtttta ctgtccccta cttttactcc g 41

<210> 467
<211> 41
<212> DNA
<213> CNAG_05010 3 flanking region primer 1

<400> 467
catggtcata gctgtttcct gtgagggttt gataccttgt g 41

<210> 468
<211> 18
<212> DNA
<213> CNAG_05010 3 flanking region primer 2

<400> 468
aagagcaaca tccgcaag 18

<210> 469
<211> 19
<212> DNA
<213> CNAG_05010 diagnostic screening primer, pairing with B79

<400> 469
aacagccttg ccttacagc 19

<210> 470
<211> 19

<212>    DNA
<213>    CNAG_05010 Southern blot probe primer

<400>    470
agtggtgaag ggctaaatg                                                    19


<210>    471
<211>    19
<212>    DNA
<213>    NAT#224 STM primer

<400>    471
aacctttaaa tgggtagag                                                    19


<210>    472
<211>    24
<212>    DNA
<213>    STM common primer

<400>    472
gcatgccctg cccctaagaa ttcg                                              24


<210>    473
<211>    18
<212>    DNA
<213>    CNAG_04345 5 flanking region primer 1

<400>    473
aaaaggcagt cgtcttcg                                                     18


<210>    474
<211>    40
<212>    DNA
<213>    CNAG_04345 5 flanking region primer 2

<400>    474

ctggccgtcg ttttaccctt ttagatactc acacaagccc 40

<210> 475
<211> 36
<212> DNA
<213> CNAG_04345 3 flanking region primer 1

<400> 475
gtcatagctg tttcctgttc tgggatgact tcacgc 36

<210> 476
<211> 19
<212> DNA
<213> CNAG_04345 3 flanking region primer 2

<400> 476
ttactgctcc tggtttggg 19

<210> 477
<211> 18
<212> DNA
<213> CNAG_04345 diagnostic screening primer, pairing with B79

<400> 477
cctgcttcac aaaatggc 18

<210> 478
<211> 22
<212> DNA
<213> CNAG_04345 Southern blot probe primer

<400> 478
gagtagtggt gtcagtgtga tg 22

<210> 479

<211> 20
<212> DNA
<213> NAT#225 STM primer

<400> 479
ccatagaact agctaaagca 20

<210> 480
<211> 24
<212> DNA
<213> STM common primer

<400> 480
gcatgccctg cccctaagaa ttcg 24

<210> 481
<211> 19
<212> DNA
<213> CNAG_02774 5 flanking region primer 1

<400> 481
ttgagccagc ctaagcaag 19

<210> 482
<211> 35
<212> DNA
<213> CNAG_02774 5 flanking region primer 2

<400> 482
ctggccgtcg ttttactctt cttttctgcg gtcac 35

<210> 483
<211> 36
<212> DNA
<213> CNAG_02774 3 flanking region primer 1

<400> 483
gtcatagctg tttcctggct tgatttcttg gaccac 36

<210> 484
<211> 19
<212> DNA
<213> CNAG_02774 3 flanking region primer 2

<400> 484
cgagataaga agcaacgcc 19

<210> 485
<211> 19
<212> DNA
<213> CNAG_02774 diagnostic screening primer, pairing with B79

<400> 485
tgccagccaa cctaatctg 19

<210> 486
<211> 20
<212> DNA
<213> CNAG_02774 Southern blot probe primer

<400> 486
gagaggcagg gagataactc 20

<210> 487
<211> 20
<212> DNA
<213> NAT#230 STM primer

<400> 487
atgtaggtag ggtgataggt 20

<210> 488
<211> 24
<212> DNA
<213> STM common primer

<400> 488
gcatgccctg cccctaagaa ttcg 24

<210> 489
<211> 20
<212> DNA
<213> CNAG_06425 5 flanking region primer 1

<400> 489
ctttcacgaa cgcttgattc 20

<210> 490
<211> 35
<212> DNA
<213> CNAG_06425 5 flanking region primer 2

<400> 490
ctggccgtcg ttttacttgg acagtttttc ggcgg 35

<210> 491
<211> 36
<212> DNA
<213> CNAG_06425 3 flanking region primer 1

<400> 491
gtcatagctg tttcctgatg tgcctgcgga tttctc 36

<210> 492
<211> 19
<212> DNA
<213> CNAG_06425 3 flanking region primer 2

<400> 492
tgagaccgct gaaagttcc 19

<210> 493
<211> 19
<212> DNA
<213> CNAG_06425 diagnostic screening primer, pairing with B79

<400> 493
taatcgccga caaagggtg 19

<210> 494
<211> 18
<212> DNA
<213> CNAG_06425 Southern blot probe primer

<400> 494
cccttttttt gtgcgacc 18

<210> 495
<211> 24
<212> DNA
<213> NAT#288 STM primer

<400> 495
ctatccaact agacctctag ctac 24

<210> 496
<211> 24
<212> DNA
<213> STM common primer

<400> 496
gcatgccctg cccctaagaa ttcg 24

<210> 497
<211> 22
<212> DNA
<213> CNAG_05255 5 flanking region primer 1

<400> 497
atggtcaccg tcacaaggaa gc 22

<210> 498
<211> 40
<212> DNA
<213> CNAG_05255 5 flanking region primer 2

<400> 498
ctggccgtcg ttttactaca cattcggctt ggttgcggac 40

<210> 499
<211> 38
<212> DNA
<213> CNAG_05255 3 flanking region primer 1

<400> 499
gtcatagctg tttcctgcga ggtttgtatc ccagtcag 38

<210> 500
<211> 20
<212> DNA
<213> CNAG_05255 3 flanking region primer 2

<400> 500
aggggctatc tctgcttcac 20

<210> 501
<211> 24
<212> DNA

<213>    CNAG_05255 diagnostic screening primer, pairing with B79

<400>    501
ttctgtgccc acctctactc tcag                                              24

<210>    502
<211>    20
<212>    DNA
<213>    CNAG_05255 Southern blot probe primer

<400>    502
accaaaactc gtcgtaggcg                                                   20

<210>    503
<211>    24
<212>    DNA
<213>    NAT#288 STM primer

<400>    503
ctatccaact agacctctag ctac                                              24

<210>    504
<211>    24
<212>    DNA
<213>    STM common primer

<400>    504
gcatgccctg cccctaagaa ttcg                                              24

<210>    505
<211>    19
<212>    DNA
<213>    CNAG_05049 5 flanking region primer 1

<400>    505
atcaggacga gtggcattg                                                    19

<210> 506
<211> 39
<212> DNA
<213> CNAG_05049 5 flanking region primer 2

<400> 506
tcactggccg tcgttttacg agtcttcttt gggcatagc 39

<210> 507
<211> 40
<212> DNA
<213> CNAG_05049 3 flanking region primer 1

<400> 507
catggtcata gctgtttcct gagtggatga tgaggactcg 40

<210> 508
<211> 21
<212> DNA
<213> CNAG_05049 3 flanking region primer 2

<400> 508
gtttcctatc ttcagtcgga g 21

<210> 509
<211> 20
<212> DNA
<213> CNAG_05049 diagnostic screening primer, pairing with B79

<400> 509
gatagcagag gagaaaaggc 20

<210> 510
<211> 18

<212>	DNA
<213>	CNAG_05049 Southern blot probe primer

<400>	510
tatttggggt tgaggctg	18

<210>	511
<211>	20
<212>	DNA
<213>	NAT#123 STM primer

<400>	511
ctatcgacca accaacacag	20

<210>	512
<211>	24
<212>	DNA
<213>	STM common primer

<400>	512
gcatgccctg cccctaagaa ttcg	24

<210>	513
<211>	18
<212>	DNA
<213>	CNAG_05222 5 flanking region primer 1

<400>	513
gagggtcctt tggttttg	18

<210>	514
<211>	37
<212>	DNA
<213>	CNAG_05222 5 flanking region primer 2

<400>	514

tcactggccg tcgttttact ttcacactca cggttgg 37

<210> 515
<211> 39
<212> DNA
<213> CNAG_05222 3 flanking region primer 1

<400> 515
catggtcata gctgtttcct ggttgcccct tttgacaag 39

<210> 516
<211> 19
<212> DNA
<213> CNAG_05222 3 flanking region primer 2

<400> 516
aacttacggc gacaacaac 19

<210> 517
<211> 18
<212> DNA
<213> CNAG_05222 diagnostic screening primer, pairing with B79

<400> 517
tccacataat agccaccg 18

<210> 518
<211> 20
<212> DNA
<213> CNAG_05222 Southern blot probe primer

<400> 518
gctatgttca ggagcgatac 20

<210> 519

<211>    20
<212>    DNA
<213>    NAT#123 STM primer

<400>    519
ctatcgacca accaacacag                                                    20


<210>    520
<211>    24
<212>    DNA
<213>    STM common primer

<400>    520
gcatgccctg cccctaagaa ttcg                                               24


<210>    521
<211>    18
<212>    DNA
<213>    CNAG_04130 5 flanking region primer 1

<400>    521
cattgacgcc atagcaag                                                      18


<210>    522
<211>    38
<212>    DNA
<213>    CNAG_04130 5 flanking region primer 2

<400>    522
tcactggccg tcgttttacg tgtttggaat gcggtttc                                38


<210>    523
<211>    41
<212>    DNA
<213>    CNAG_04130 3 flanking region primer 1

<400> 523
catggtcata gctgtttcct gtggatgagg ttgaggggaa c 41

<210> 524
<211> 21
<212> DNA
<213> CNAG_04130 3 flanking region primer 2

<400> 524
gtagtgtaag ggcaggaaga g 21

<210> 525
<211> 21
<212> DNA
<213> CNAG_04130 diagnostic screening primer, pairing with B79

<400> 525
ttctactcat aagggctacg c 21

<210> 526
<211> 21
<212> DNA
<213> CNAG_04130 Southern blot probe primer

<400> 526
ttctactcat aagggctacg c 21

<210> 527
<211> 19
<212> DNA
<213> CNAG_00193 5 flanking region primer 1

<400> 527
gggcagaaat agttggcag 19

<210> 528
<211> 34
<212> DNA
<213> CNAG_00193 5 flanking region primer 2

<400> 528
ctggccgtcg ttttactttt tccccccgat gtag 34


<210> 529
<211> 36
<212> DNA
<213> CNAG_00193 3 flanking region primer 1

<400> 529
gtcatagctg tttcctgttc ccgcaagaac agtgtc 36


<210> 530
<211> 20
<212> DNA
<213> CNAG_00193 3 flanking region primer 2

<400> 530
ttcaggatgt ccaaactcag 20


<210> 531
<211> 19
<212> DNA
<213> CNAG_00193 diagnostic screening primer, pairing with B79

<400> 531
aaggggtaag aaatcgggc 19


<210> 532
<211> 19
<212> DNA
<213> CNAG_00193 Southern blot probe primer

<400>    532
tgctgagaga cagggaatc                                                    19

<210>    533
<211>    20
<212>    DNA
<213>    NAT#227 STM primer

<400>    533
tcgtggttta gagggagcgc                                                   20

<210>    534
<211>    24
<212>    DNA
<213>    STM common primer

<400>    534
gcatgccctg cccctaagaa ttcg                                              24

<210>    535
<211>    20
<212>    DNA
<213>    CNAG_02700 5 flanking region primer 1

<400>    535
tgaaatcaga ctccagcctc                                                   20

<210>    536
<211>    38
<212>    DNA
<213>    CNAG_02700 5 flanking region primer 2

<400>    536
tcactggccg tcgttttacg cgaccacatt tacactcg                               38

<210>    537
<211>    41
<212>    DNA
<213>    CNAG_02700 3 flanking region primer 1

<400>    537
catggtcata gctgtttcct ggccacagaa caataactgg g                41

<210>    538
<211>    19
<212>    DNA
<213>    CNAG_02700 3 flanking region primer 2

<400>    538
gcgtatgaat aagcgttgg                19

<210>    539
<211>    18
<212>    DNA
<213>    CNAG_02700 diagnostic screening primer, pairing with B79

<400>    539
agcaaaacag aagcagcg                18

<210>    540
<211>    18
<212>    DNA
<213>    CNAG_02700 Southern blot probe primer

<400>    540
agaaagcagc atttgggg                18

<210>    541
<211>    20
<212>    DNA

<213>    NAT#230 STM primer

<400>    541
atgtaggtag ggtgataggt                                                    20


<210>    542
<211>    24
<212>    DNA
<213>    STM common primer

<400>    542
gcatgccctg cccctaagaa ttcg                                               24


<210>    543
<211>    19
<212>    DNA
<213>    CNAG_04586 5 flanking region primer 1

<400>    543
tccatctccc aatccacag                                                     19


<210>    544
<211>    38
<212>    DNA
<213>    CNAG_04586 5 flanking region primer 2

<400>    544
tcactggccg tcgttttacg cttgattagg cggataag                               38


<210>    545
<211>    41
<212>    DNA
<213>    CNAG_04586 3 flanking region primer 1

<400>    545
catggtcata gctgtttcct gtggacgaaa agacccctac c                            41

<210> 546
<211> 18
<212> DNA
<213> CNAG_04586 3 flanking region primer 2

<400> 546
aacactggaa tgtgcgtg 18

<210> 547
<211> 20
<212> DNA
<213> CNAG_04586 diagnostic screening primer, pairing with B79

<400> 547
ttggtggtgc ggttttgtgc 20

<210> 548
<211> 18
<212> DNA
<213> CNAG_04586 Southern blot probe primer

<400> 548
tgatgttggt tcgtgacc 18

<210> 549
<211> 21
<212> DNA
<213> NAT#159 STM primer

<400> 549
acgcaccaga cacacaacca g 21

<210> 550
<211> 24

<212>    DNA
<213>    STM common primer

<400>    550
gcatgccctg cccctaagaa ttcg                                              24


<210>    551
<211>    18
<212>    DNA
<213>    CNAG_05153 5 flanking region primer 1

<400>    551
aaaaccaagt ggaacccg                                                     18


<210>    552
<211>    35
<212>    DNA
<213>    CNAG_05153 5 flanking region primer 2

<400>    552
ctggccgtcg ttttactggt tgacgctgtt agcac                                  35


<210>    553
<211>    37
<212>    DNA
<213>    CNAG_05153 3 flanking region primer 1

<400>    553
gtcatagctg tttcctgata ggcaggctgg tggaaac                                37


<210>    554
<211>    19
<212>    DNA
<213>    CNAG_05153 3 flanking region primer 2

<400>    554

accacatttg cttccgagc 19

<210> 555
<211> 21
<212> DNA
<213> CNAG_05153 diagnostic screening primer, pairing with JOHE12579

<400> 555
tggctgaact gtaggttagt g 21

<210> 556
<211> 18
<212> DNA
<213> CNAG_05153 Southern blot probe primer

<400> 556
cggcgataag gtttttgc 18

<210> 557
<211> 24
<212> DNA
<213> NAT#290 STM primer

<400> 557
accgacagct cgaacaagca agag 24

<210> 558
<211> 24
<212> DNA
<213> STM common primer

<400> 558
gcatgccctg cccctaagaa ttcg 24

<210> 559

<211>    20
<212>    DNA
<213>    CNAG_02723 5 flanking region primer 1

<400>    559
gcttccttat cggtagggac                                                    20


<210>    560
<211>    39
<212>    DNA
<213>    CNAG_02723 5 flanking region primer 2

<400>    560
tcactggccg tcgttttacg actggacatt gacgaagac                               39


<210>    561
<211>    40
<212>    DNA
<213>    CNAG_02723 3 flanking region primer 1

<400>    561
catggtcata gctgtttcct gtggtgacag tgacgcttac                              40


<210>    562
<211>    19
<212>    DNA
<213>    CNAG_02723 3 flanking region primer 2

<400>    562
attgtcagga tgtgggttc                                                     19


<210>    563
<211>    21
<212>    DNA
<213>    CNAG_02723 diagnostic screening primer, pairing with B79

<400>    563
gcttactccc aaactcacac c                                                21

<210>    564
<211>    18
<212>    DNA
<213>    CNAG_02723 Southern blot probe primer

<400>    564
atggatttgg cagaacgg                                                    18

<210>    565
<211>    24
<212>    DNA
<213>    NAT#201 STM primer

<400>    565
caccctctat ctcgagaaag ctcc                                             24

<210>    566
<211>    24
<212>    DNA
<213>    STM common primer

<400>    566
gcatgccctg cccctaagaa ttcg                                             24

<210>    567
<211>    19
<212>    DNA
<213>    CNAG_03741 5 flanking region primer 1

<400>    567
gccgtctcct gatgtaatc                                                   19

<210> 568
<211> 36
<212> DNA
<213> CNAG_03741 5 flanking region primer 2

<400> 568
ctggccgtcg ttttaccgac tgtcatcaac gactgg 36

<210> 569
<211> 36
<212> DNA
<213> CNAG_03741 3 flanking region primer 1

<400> 569
ctggccgtcg ttttacgcgt caacaggaat aaaacc 36

<210> 570
<211> 22
<212> DNA
<213> CNAG_03741 3 flanking region primer 2

<400> 570
ctgtgccttt ctccaagata ag 22

<210> 571
<211> 22
<212> DNA
<213> CNAG_03741 diagnostic screening primer, pairing with B79

<400> 571
ggataagcag aacatagttg cg 22

<210> 572
<211> 21
<212> DNA
<213> CNAG_03741 Southern blot probe primer

<400>    572
tctcttcttc ttctgacctg c                                                21

<210>    573
<211>    24
<212>    DNA
<213>    NAT#273 STM primer

<400>    573
gagatctttc gggaggtctg gatt                                             24

<210>    574
<211>    24
<212>    DNA
<213>    STM common primer

<400>    574
gcatgccctg cccctaagaa ttcg                                             24

<210>    575
<211>    18
<212>    DNA
<213>    CNAG_07411 5 flanking region primer 1

<400>    575
agacccgcca aaacaatc                                                    18

<210>    576
<211>    35
<212>    DNA
<213>    CNAG_07411 5 flanking region primer 2

<400>    576
ctggccgtcg ttttacttgg acctacgctg acttc                                 35

<210> 577
<211> 37
<212> DNA
<213> CNAG_07411 3 flanking region primer 1

<400> 577
gtcatagctg tttcctggac ttgcgttctg attttgg 37

<210> 578
<211> 20
<212> DNA
<213> CNAG_07411 3 flanking region primer 2

<400> 578
gctgttatca agagaatgcc 20

<210> 579
<211> 19
<212> DNA
<213> CNAG_07411 diagnostic screening primer, pairing with B79

<400> 579
acgactggct atcaagcag 19

<210> 580
<211> 19
<212> DNA
<213> CNAG_07411 Southern blot probe primer

<400> 580
tacgaggatt gagtctggc 19

<210> 581
<211> 24
<212> DNA

<213>    NAT#288 STM primer

<400>    581
ctatccaact agacctctag ctac                                              24


<210>    582
<211>    24
<212>    DNA
<213>    STM common primer

<400>    582
gcatgccctg cccctaagaa ttcg                                              24


<210>    583
<211>    20
<212>    DNA
<213>    CNAG_03849 5 flanking region primer 1

<400>    583
tagtggttcc cgtttgtgcg                                                   20


<210>    584
<211>    40
<212>    DNA
<213>    CNAG_03849 5 flanking region primer 2

<400>    584
gctcactggc cgtcgtttta ccttacgcct ttttccatcg                             40


<210>    585
<211>    41
<212>    DNA
<213>    CNAG_03849 3 flanking region primer 1

<400>    585
catggtcata gctgtttcct gtcaaacggc gattggtggt c                           41

<210> 586
<211> 20
<212> DNA
<213> CNAG_03849 3 flanking region primer 2

<400> 586
tggtgaaggg gaagattggg 20

<210> 587
<211> 19
<212> DNA
<213> CNAG_03849 diagnostic screening primer, pairing with B79

<400> 587
aacaagccga ggaatgagg 19

<210> 588
<211> 19
<212> DNA
<213> CNAG_03849 Southern blot probe primer

<400> 588
tgagtgaacg aagagtgcc 19

<210> 589
<211> 20
<212> DNA
<213> NAT#6 STM primer

<400> 589
atagctacca cacgatagct 20

<210> 590
<211> 24

<212>    DNA
<213>    STM common primer

<400>    590
   gcatgccctg cccctaagaa ttcg                                                24

<210>    591
<211>    19
<212>    DNA
<213>    CNAG_04916 5 flanking region primer 1

<400>    591
ttgcgttgag acatcactg                                                       19

<210>    592
<211>    43
<212>    DNA
<213>    CNAG_04916 5 flanking region primer 2

<400>    592
gctcactggc cgtcgtttta cgctcctatg ttaccaatgt tgc                            43

<210>    593
<211>    40
<212>    DNA
<213>    CNAG_04916 3 flanking region primer 1

<400>    593
catggtcata gctgtttcct gacccatttg ctttcctggc                                40

<210>    594
<211>    20
<212>    DNA
<213>    CNAG_04916 3 flanking region primer 2

<400>    594

tctcaacgag tcgctttcgc                                                    20

<210>  595
<211>  20
<212>  DNA
<213>  CNAG_04916 diagnostic screening primer, pairing with B79

<400>  595
aaaagcatcc tgcccagcac                                                    20

<210>  596
<211>  20
<212>  DNA
<213>  CNAG_04916 Southern blot probe primer

<400>  596
gttgaaagta gttgcgttgg                                                    20

<210>  597
<211>  20
<212>  DNA
<213>  NAT#212 STM primer

<400>  597
agagcgatcg cgttatagat                                                    20

<210>  598
<211>  24
<212>  DNA
<213>  STM common primer

<400>  598
gcatgccctg cccctaagaa ttcg                                               24

<210>  599

<211> 19
<212> DNA
<213> CNAG_04012 5 flanking region primer 1

<400> 599
ttggtggagt gattgaccc 19

<210> 600
<211> 36
<212> DNA
<213> CNAG_04012 5 flanking region primer 2

<400> 600
ctggccgtcg ttttacaatc aaaccagaag gagagc 36

<210> 601
<211> 39
<212> DNA
<213> CNAG_04012 3 flanking region primer 1

<400> 601
gtcatagctg tttcctgagg aggataaagg aagagagtg 39

<210> 602
<211> 19
<212> DNA
<213> CNAG_04012 3 flanking region primer 2

<400> 602
ttatgctggt ggtgcctac 19

<210> 603
<211> 19
<212> DNA
<213> CNAG_04012 diagnostic screening primer, pairing with B79

<400> 603
accgaagagg atgaggttg 19

<210> 604
<211> 20
<212> DNA
<213> CNAG_04012 Southern blot probe primer

<400> 604
cagtggtggt tgagattgac 20

<210> 605
<211> 20
<212> DNA
<213> NAT#212 STM primer

<400> 605
agagcgatcg cgttatagat 20

<210> 606
<211> 24
<212> DNA
<213> STM common primer

<400> 606
gcatgccctg cccctaagaa ttcg 24

<210> 607
<211> 20
<212> DNA
<213> CNAG_04090 5 flanking region primer 1

<400> 607
gagtagagga gtggattggg 20

<210> 608
<211> 39
<212> DNA
<213> CNAG_04090 5 flanking region primer 2

<400> 608
gctcactggc cgtcgtttta cccattgttc aagcagagc 39

<210> 609
<211> 42
<212> DNA
<213> CNAG_04090 3 flanking region primer 1

<400> 609
catggtcata gctgtttcct gggcttactg ctatcttgat gc 42

<210> 610
<211> 24
<212> DNA
<213> CNAG_04090 3 flanking region primer 2

<400> 610
gaaagaggaa ctacatactg ggtc 24

<210> 611
<211> 20
<212> DNA
<213> CNAG_04090 diagnostic screening primer, pairing with B79

<400> 611
gtcttttgct ccttgaaacc 20

<210> 612
<211> 20
<212> DNA
<213> CNAG_04090 Southern blot probe primer

<400>    612
accaggtgta aacccagtcc                                                    20

<210>    613
<211>    20
<212>    DNA
<213>    NAT#220 STM primer

<400>    613
cagatctcga acgataccca                                                    20

<210>    614
<211>    24
<212>    DNA
<213>    STM common primer

<400>    614
gcatgccctg cccctaagaa ttcg                                               24

<210>    615
<211>    21
<212>    DNA
<213>    CNAG_05375 5 flanking region primer 1

<400>    615
ggaagcatta ctgtcccata g                                                  21

<210>    616
<211>    43
<212>    DNA
<213>    CNAG_05375 5 flanking region primer 2

<400>    616
gctcactggc cgtcgtttta cagaaacagg agagagagta gcc                          43

<210> 617
<211> 39
<212> DNA
<213> CNAG_05375 3 flanking region primer 1

<400> 617
catggtcata gctgtttcct gtcttggatt tcaacgggg 39

<210> 618
<211> 20
<212> DNA
<213> CNAG_05375 3 flanking region primer 2

<400> 618
gtagttccgt tatttgctgg 20

<210> 619
<211> 22
<212> DNA
<213> CNAG_05375 diagnostic screening primer, pairing with B79

<400> 619
ggttgaagat ggacgagtag ac 22

<210> 620
<211> 20
<212> DNA
<213> CNAG_05375 Southern blot probe primer

<400> 620
cattcacctc ccttagaagc 20

<210> 621
<211> 19
<212> DNA

<213>    NAT#224 STM primer

<400>    621
aacctttaaa tgggtagag                                                    19


<210>    622
<211>    24
<212>    DNA
<213>    STM common primer

<400>    622
gcatgccctg cccctaagaa ttcg                                              24


<210>    623
<211>    20
<212>    DNA
<213>    CNAG_07797 5 flanking region primer 1

<400>    623
agggagaaca ccacaggatg                                                   20


<210>    624
<211>    38
<212>    DNA
<213>    CNAG_07797 5 flanking region primer 2

<400>    624
tcactggccg tcgttttaca ggaatggagg tggtgaac                               38


<210>    625
<211>    40
<212>    DNA
<213>    CNAG_07797 3 flanking region primer 1

<400>    625
catggtcata gctgtttcct gggaaagtca gtgatgctcc                             40

<210> 626
<211> 18
<212> DNA
<213> CNAG_07797 3 flanking region primer 2

<400> 626
gctttcttcc ttttcccg 18

<210> 627
<211> 18
<212> DNA
<213> CNAG_07797 diagnostic screening primer, pairing with B79

<400> 627
atcaatggtt tcgtcggc 18

<210> 628
<211> 18
<212> DNA
<213> CNAG_07797 Southern blot probe primer

<400> 628
ggggcgttga aaatgatg 18

<210> 629
<211> 20
<212> DNA
<213> NAT#231 STM primer

<400> 629
gagagatccc aacatcacgc 20

<210> 630
<211> 24

<212>    DNA
<213>    STM common primer

<400>    630
gcatgccctg cccctaagaa ttcg                                              24


<210>    631
<211>    22
<212>    DNA
<213>    CNAG_05538 5 flanking region primer 1

<400>    631
ggtctcaaca aagcacttac tg                                                22


<210>    632
<211>    40
<212>    DNA
<213>    CNAG_05538 5 flanking region primer 2

<400>    632
tcactggccg tcgttttacg taatagtcag gcggtttctc                             40


<210>    633
<211>    41
<212>    DNA
<213>    CNAG_05538 3 flanking region primer 1

<400>    633
catggtcata gctgtttcct gagttaccag aggaggagtg g                           41


<210>    634
<211>    20
<212>    DNA
<213>    CNAG_05538 3 flanking region primer 2

<400>    634

gattctcttt accctcagcc                                                    20

<210>    635
<211>    20
<212>    DNA
<213>    CNAG_05538 diagnostic screening primer, pairing with B79

<400>    635
tccgattcta ttgactctgc                                                    20

<210>    636
<211>    20
<212>    DNA
<213>    CNAG_05538 Southern blot probe primer

<400>    636
gtatcatcca ttttcctggc                                                    20

<210>    637
<211>    20
<212>    DNA
<213>    NAT#240 STM primer

<400>    637
ggtgttggat cggggtggat                                                    20

<210>    638
<211>    24
<212>    DNA
<213>    STM common primer

<400>    638
gcatgccctg cccctaagaa ttcg                                               24

<210>    639

<211>    18
<212>    DNA
<213>    CNAG_00332 5 flanking region primer 1

<400>    639
tgattgtcgt ccttgtgg                                                    18


<210>    640
<211>    39
<212>    DNA
<213>    CNAG_00332 5 flanking region primer 2

<400>    640
gctcactggc cgtcgtttta ctttgcgtga ctgttctgc                             39


<210>    641
<211>    42
<212>    DNA
<213>    CNAG_00332 3 flanking region primer 1

<400>    641
catggtcata gctgtttcct gcggtttgcg aagatacaga ac                         42


<210>    642
<211>    18
<212>    DNA
<213>    CNAG_00332 3 flanking region primer 2

<400>    642
tatggatgcg tgggaatg                                                    18


<210>    643
<211>    18
<212>    DNA
<213>    CNAG_00332 diagnostic screening primer, pairing with B79

<400> 643
gccagaccaa tcaaatgc 18

<210> 644
<211> 19
<212> DNA
<213> CNAG_00332 Southern blot probe primer

<400> 644
gaagaagggt tgaatgtcg 19

<210> 645
<211> 24
<212> DNA
<213> NAT#290 STM primer

<400> 645
accgacagct cgaacaagca agag 24

<210> 646
<211> 24
<212> DNA
<213> STM common primer

<400> 646
gcatgccctg cccctaagaa ttcg 24

<210> 647
<211> 19
<212> DNA
<213> CNAG_04023 5 flanking region primer 1

<400> 647
agcaggatag acagcgaag 19

<210> 648
<211> 35
<212> DNA
<213> CNAG_04023 5 flanking region primer 2

<400> 648
ctggccgtcg ttttactgtc cttgataggt cggag 35

<210> 649
<211> 35
<212> DNA
<213> CNAG_04023 3 flanking region primer 1

<400> 649
gtcatagctg tttcctgtgg aacacattgg aggag 35

<210> 650
<211> 18
<212> DNA
<213> CNAG_04023 3 flanking region primer 2

<400> 650
aatgggtgta tgatggcg 18

<210> 651
<211> 19
<212> DNA
<213> CNAG_04023 diagnostic screening primer, pairing with B79

<400> 651
cagaacgcat tgtttgtgc 19

<210> 652
<211> 19
<212> DNA
<213> CNAG_04023 Southern blot probe primer

<400>    652
gtagtaaacg ccttgagcc                                                    19

<210>    653
<211>    20
<212>    DNA
<213>    CNAG_05311 5 flanking region primer 1

<400>    653
caacaagttc catcacatcg                                                   20

<210>    654
<211>    40
<212>    DNA
<213>    CNAG_05311 5 flanking region primer 2

<400>    654
gctcactggc cgtcgtttta cgggtaaaac ctggcttagg                             40

<210>    655
<211>    39
<212>    DNA
<213>    CNAG_05311 3 flanking region primer 1

<400>    655
catggtcata gctgtttcct gggatttgga tgaacctgg                              39

<210>    656
<211>    21
<212>    DNA
<213>    CNAG_05311 3 flanking region primer 2

<400>    656
gagaaagagg tttgtcgtga g                                                 21

<210> 657
<211> 18
<212> DNA
<213> CNAG_05311 diagnostic screening primer, pairing with B79

<400> 657
ttctactttc cgccaagg 18

<210> 658
<211> 19
<212> DNA
<213> CNAG_05311 Southern blot probe primer

<400> 658
gagatgggtc actaatcgc 19

<210> 659
<211> 19
<212> DNA
<213> CNAG_03086 5 flanking region primer 1

<400> 659
aatctccttt gcccgactc 19

<210> 660
<211> 38
<212> DNA
<213> CNAG_03086 5 flanking region primer 2

<400> 660
tcactggccg tcgttttaca taccttgtct ttgcgggc 38

<210> 661
<211> 41
<212> DNA

<213>    CNAG_03086 3 flanking region primer 1

<400>    661
catggtcata gctgtttcct gattgaaggt ggcagtagat g                41

<210>    662
<211>    18
<212>    DNA
<213>    CNAG_03086 3 flanking region primer 2

<400>    662
aaaatgtggg cggatgag                                          18

<210>    663
<211>    19
<212>    DNA
<213>    CNAG_03086 diagnostic screening primer, pairing with B79

<400>    663
tcagtcggga agagtttgc                                         19

<210>    664
<211>    19
<212>    DNA
<213>    CNAG_03086 Southern blot probe primer

<400>    664
aggcgagagt aagaatgcg                                         19

<210>    665
<211>    20
<212>    DNA
<213>    NAT#191 STM primer

<400>    665
atatggatgt ttttagcgag                                        20

<210> 666
<211> 24
<212> DNA
<213> STM common primer

<400> 666
gcatgccctg cccctaagaa ttcg 24

<210> 667
<211> 18
<212> DNA
<213> CNAG_03336 5 flanking region primer 1

<400> 667
gtgccttatt ccaatggg 18

<210> 668
<211> 34
<212> DNA
<213> CNAG_03336 5 flanking region primer 2

<400> 668
ctggccgtcg ttttacttct tgctgcctgt ttgg 34

<210> 669
<211> 35
<212> DNA
<213> CNAG_03336 3 flanking region primer 1

<400> 669
ctggccgtcg ttttacccaa caataatcac gcagg 35

<210> 670
<211> 20

<212>    DNA
<213>    CNAG_03336 3 flanking region primer 2

<400>    670
ccatcctttt tttagcggtc                                                    20


<210>    671
<211>    19
<212>    DNA
<213>    CNAG_03336 diagnostic screening primer, pairing with B79

<400>    671
cgaggtgatt gatgtgagc                                                     19


<210>    672
<211>    20
<212>    DNA
<213>    CNAG_03336 Southern blot probe primer

<400>    672
ttcaagagcg gactggacag                                                    20


<210>    673
<211>    20
<212>    DNA
<213>    NAT#204 STM primer

<400>    673
gatctctcgc gcttggggga                                                    20


<210>    674
<211>    24
<212>    DNA
<213>    STM common primer

<400>    674

gcatgccctg cccctaagaa ttcg 24

<210> 675
<211> 19
<212> DNA
<213> CNAG_03229 5 flanking region primer 1

<400> 675
gtgggttact ttgggtgag 19

<210> 676
<211> 37
<212> DNA
<213> CNAG_03229 5 flanking region primer 2

<400> 676
tcactggccg tcgttttaca aaagggactt gctgtcg 37

<210> 677
<211> 40
<212> DNA
<213> CNAG_03229 3 flanking region primer 1

<400> 677
catggtcata gctgtttcct ggaaagaaag gaaatcgccg 40

<210> 678
<211> 18
<212> DNA
<213> CNAG_03229 3 flanking region primer 2

<400> 678
tggaagatgg tgacgaag 18

<210> 679

<211>    18
<212>    DNA
<213>    CNAG_03229 diagnostic screening primer, pairing with B79

<400>    679
ttgcgatttc cgactacc                                                    18


<210>    680
<211>    18
<212>    DNA
<213>    CNAG_03229 Southern blot probe primer

<400>    680
tggaggcgaa taatggtc                                                    18


<210>    681
<211>    20
<212>    DNA
<213>    NAT#227 STM primer

<400>    681
tcgtggttta gagggagcgc                                                  20


<210>    682
<211>    24
<212>    DNA
<213>    STM common primer

<400>    682
gcatgccctg cccctaagaa ttcg                                             24


<210>    683
<211>    19
<212>    DNA
<213>    CNAG_01883 5 flanking region primer 1

<400> 683
tactcgctgc ttagggttc 19

<210> 684
<211> 35
<212> DNA
<213> CNAG_01883 5 flanking region primer 2

<400> 684
ctggccgtcg ttttacatgg catactccag gtctg 35

<210> 685
<211> 36
<212> DNA
<213> CNAG_01883 3 flanking region primer 1

<400> 685
gtcatagctg tttcctgccc tccatttact tctccg 36

<210> 686
<211> 22
<212> DNA
<213> CNAG_01883 3 flanking region primer 2

<400> 686
tagcagtagg gctcctgatt cg 22

<210> 687
<211> 19
<212> DNA
<213> CNAG_01883 diagnostic screening primer, pairing with B79

<400> 687
ttggaggtct ttatcagcg 19

<210> 688
<211> 19
<212> DNA
<213> CNAG_01883 Southern blot probe primer

<400> 688
ggagaagtaa atggagggg 19


<210> 689
<211> 24
<212> DNA
<213> NAT#125 STM primer

<400> 689
cgctacagcc agcgcgcgca agcg 24


<210> 690
<211> 24
<212> DNA
<213> STM common primer

<400> 690
gcatgccctg cccctaagaa ttcg 24


<210> 691
<211> 20
<212> DNA
<213> CNAG_04837 5 flanking region primer 1

<400> 691
tgaaacagtg aatcaggcac 20


<210> 692
<211> 40
<212> DNA
<213> CNAG_04837 5 flanking region primer 2

<400> 692
gctcactggc cgtcgtttta ctcagtgtca acggtcattg 40

<210> 693
<211> 42
<212> DNA
<213> CNAG_04837 3 flanking region primer 1

<400> 693
catggtcata gctgtttcct ggaaaggggt agggataagt tg 42

<210> 694
<211> 20
<212> DNA
<213> CNAG_04837 3 flanking region primer 2

<400> 694
actgaacgac aagagacgac 20

<210> 695
<211> 18
<212> DNA
<213> CNAG_04837 diagnostic screening primer, pairing with B79

<400> 695
taagagaaaa ggggctgc 18

<210> 696
<211> 21
<212> DNA
<213> CNAG_04837 Southern blot probe primer 1

<400> 696
tgtccccatc aactctacaa c 21

<210> 697
<211> 20
<212> DNA
<213> CNAG_04837 Southern blot probe primer 2

<400> 697
cttctgcttt caccattagc 20


<210> 698
<211> 21
<212> DNA
<213> NAT#146 STM primer

<400> 698
actagccccc cctcaccacc t 21


<210> 699
<211> 24
<212> DNA
<213> STM common primer

<400> 699
gcatgccctg cccctaagaa ttcg 24


<210> 700
<211> 19
<212> DNA
<213> CNAG_00514 5 flanking region primer 1

<400> 700
ggtgtttcca tacgaaccg 19


<210> 701
<211> 38
<212> DNA

<213>    CNAG_00514 5 flanking region primer 2

<400>    701
ctggccgtcg ttttacggag ccatttctta cttatgcg                                38

<210>    702
<211>    35
<212>    DNA
<213>    CNAG_00514 3 flanking region primer 1

<400>    702
gtcatagctg tttcctgaat cccaaggacc atctg                                   35

<210>    703
<211>    20
<212>    DNA
<213>    CNAG_00514 3 flanking region primer 2

<400>    703
gctgagaagg agaggatagc                                                    20

<210>    704
<211>    20
<212>    DNA
<213>    CNAG_00514 diagnostic screening primer, pairing with B79

<400>    704
gtggttgaaa gtattaggcg                                                    20

<210>    705
<211>    19
<212>    DNA
<213>    CNAG_00514 Southern blot probe primer

<400>    705
gaggattggt gatggactg                                                     19

<210>    706
<211>    24
<212>    DNA
<213>    NAT#201 STM primer

<400>    706
caccctctat ctcgagaaag ctcc                                                24

<210>    707
<211>    24
<212>    DNA
<213>    STM common primer

<400>    707
gcatgccctg cccctaagaa ttcg                                                24

<210>    708
<211>    20
<212>    DNA
<213>    CNAG_03409 5 flanking region primer 1

<400>    708
cctcaccgat tcattcactg                                                     20

<210>    709
<211>    38
<212>    DNA
<213>    CNAG_03409 5 flanking region primer 2

<400>    709
ctggccgtcg ttttactttt ctcccttctt cgcccgac                                 38

<210>    710
<211>    37

<212>    DNA
<213>    CNAG_03409 3 flanking region primer 1

<400>    710
gtcatagctg tttcctgagg ttcaagccca gactcag                                37


<210>    711
<211>    20
<212>    DNA
<213>    CNAG_03409 3 flanking region primer 2

<400>    711
agtatctacg gtgctggtgc                                                   20


<210>    712
<211>    20
<212>    DNA
<213>    CNAG_03409 diagnostic screening primer, pairing with B79

<400>    712
tgaggatgat gactggggac                                                   20


<210>    713
<211>    20
<212>    DNA
<213>    CNAG_03409 Southern blot probe primer 1

<400>    713
acttccaatg ctcaaccccg                                                   20


<210>    714
<211>    20
<212>    DNA
<213>    CNAG_03409 Southern blot probe primer 2

<400>    714

gcaggacaag aaggagagtg                                                    20

<210>  715
<211>  24
<212>  DNA
<213>  NAT#201 STM primer

<400>  715
caccctctat ctcgagaaag ctcc                                              24

<210>  716
<211>  24
<212>  DNA
<213>  STM common primer

<400>  716
gcatgccctg cccctaagaa ttcg                                              24

<210>  717
<211>  19
<212>  DNA
<213>  CNAG_05392 5 flanking region primer 1

<400>  717
aagttgtgaa accaccagg                                                    19

<210>  718
<211>  38
<212>  DNA
<213>  CNAG_05392 5 flanking region primer 2

<400>  718
tcactggccg tcgttttact caccctgtag atgtgtgg                               38

<210>  719

<211>    42
<212>    DNA
<213>    CNAG_05392 3 flanking region primer 1

<400>    719
catggtcata gctgtttcct gggagtccct gtgaagtaaa ag                    42


<210>    720
<211>    20
<212>    DNA
<213>    CNAG_05392 3 flanking region primer 2

<400>    720
tgagccaaag tagcagtcag                                              20


<210>    721
<211>    19
<212>    DNA
<213>    CNAG_05392 diagnostic screening primer, pairing with B79

<400>    721
gaatggacct tggaaaacg                                               19


<210>    722
<211>    20
<212>    DNA
<213>    CNAG_05392 Southern blot probe primer

<400>    722
cggtctgtgt gcttcttcac                                              20


<210>    723
<211>    20
<212>    DNA
<213>    NAT#204 STM primer

<400> 723
   gatctctcgc gcttggggga 20

<210> 724
<211> 24
<212> DNA
<213> STM common primer

<400> 724
gcatgccctg cccctaagaa ttcg 24

<210> 725
<211> 20
<212> DNA
<213> CNAG_04895 5 flanking region primer 1

<400> 725
ttcgtagtta gccagcagag 20

<210> 726
<211> 38
<212> DNA
<213> CNAG_04895 5 flanking region primer 2

<400> 726
tcactggccg tcgttttacg ttcttgatgt ggaggagc 38

<210> 727
<211> 40
<212> DNA
<213> CNAG_04895 3 flanking region primer 1

<400> 727
catggtcata gctgtttcct gcccaaccct tatgcgtatg 40

<210> 728
<211> 18
<212> DNA
<213> CNAG_04895 3 flanking region primer 2

<400> 728
agcgtctccc tcaaaaac 18

<210> 729
<211> 18
<212> DNA
<213> CNAG_04895 diagnostic screening primer, pairing with B79

<400> 729
ttagcgaccg aaaaaggg 18

<210> 730
<211> 19
<212> DNA
<213> CNAG_04895 Southern blot probe primer

<400> 730
aagatgacgc agaactggg 19

<210> 731
<211> 20
<212> DNA
<213> NAT#204 STM primer

<400> 731
gatctctcgc gcttggggga 20

<210> 732
<211> 24
<212> DNA
<213> STM common primer

<400>    732
gcatgccctg cccctaagaa ttcg                                              24

<210>    733
<211>    19
<212>    DNA
<213>    CNAG_04176 5 flanking region primer 1

<400>    733
ttgacacggg gactaatcg                                                    19

<210>    734
<211>    40
<212>    DNA
<213>    CNAG_04176 5 flanking region primer 2

<400>    734
gctcactggc cgtcgtttta cgattagccc agtcttagcg                            40

<210>    735
<211>    41
<212>    DNA
<213>    CNAG_04176 3 flanking region primer 1

<400>    735
catggtcata gctgtttcct gagaagcagg gagcaaaacg g                          41

<210>    736
<211>    19
<212>    DNA
<213>    CNAG_04176 3 flanking region primer 2

<400>    736
ccgaaaagtt gagcaagac                                                    19

<210> 737
<211> 23
<212> DNA
<213> CNAG_04176 diagnostic screening primer, pairing with B79

<400> 737
cgatagcctt tgtagtaggt ctc 23

<210> 738
<211> 20
<212> DNA
<213> CNAG_04176 Southern blot probe primer

<400> 738
gggatttgat tatctggagg 20

<210> 739
<211> 24
<212> DNA
<213> NAT#205 STM primer

<400> 739
tatccccctc tccgctctct agca 24

<210> 740
<211> 24
<212> DNA
<213> STM common primer

<400> 740
gcatgccctg cccctaagaa ttcg 24

<210> 741
<211> 19
<212> DNA

<213>    CNAG_07724 5 flanking region primer 1

<400>    741
caaagaaaac gctcactcc                                                    19


<210>    742
<211>    40
<212>    DNA
<213>    CNAG_07724 5 flanking region primer 2

<400>    742
tcactggccg tcgttttacg gatagaacaa gacaagagcg                             40


<210>    743
<211>    41
<212>    DNA
<213>    CNAG_07724 3 flanking region primer 1

<400>    743
catggtcata gctgtttcct gacttcagtc gttaccagca c                           41


<210>    744
<211>    19
<212>    DNA
<213>    CNAG_07724 3 flanking region primer 2

<400>    744
tctttgagca catcaggtg                                                    19


<210>    745
<211>    19
<212>    DNA
<213>    CNAG_07724 diagnostic screening primer, pairing with B79

<400>    745
ccgtcattat cgtgtctcc                                                    19

<210> 746
<211> 19
<212> DNA
<213> CNAG_07724 Southern blot probe primer

<400> 746
gcttcggagg agttgatag 19

<210> 747
<211> 24
<212> DNA
<213> NAT#205 STM primer

<400> 747
tatccccctc tccgctctct agca 24

<210> 748
<211> 24
<212> DNA
<213> STM common primer

<400> 748
gcatgccctg cccctaagaa ttcg 24

<210> 749
<211> 19
<212> DNA
<213> CNAG_06751 5 flanking region primer 1

<400> 749
aaaaacggct gaaggctcg 19

<210> 750
<211> 40

<212>    DNA
<213>    CNAG_06751 5 flanking region primer 2

<400>    750
gctcactggc cgtcgtttta ctggcgagtt ggtatctgag                    40

<210>    751
<211>    40
<212>    DNA
<213>    CNAG_06751 3 flanking region primer 1

<400>    751
catggtcata gctgtttcct gaagatgaga gagaaggggg                    40

<210>    752
<211>    20
<212>    DNA
<213>    CNAG_06751 3 flanking region primer 2

<400>    752
agaagtggta tgaccgtttg                                          20

<210>    753
<211>    19
<212>    DNA
<213>    CNAG_06751 diagnostic screening primer, pairing with B79

<400>    753
tccatacatt gttgagggc                                           19

<210>    754
<211>    18
<212>    DNA
<213>    CNAG_06751 Southern blot probe primer

<400>    754

attgcccaag cggataag 18

<210> 755
<211> 22
<212> DNA
<213> NAT#208 STM primer

<400> 755
tggtcgcggg agatcgtggt tt 22

<210> 756
<211> 24
<212> DNA
<213> STM common primer

<400> 756
gcatgccctg cccctaagaa ttcg 24

<210> 757
<211> 18
<212> DNA
<213> CNAG_01438 5 flanking region primer 1

<400> 757
atcacacgca atcccatc 18

<210> 758
<211> 39
<212> DNA
<213> CNAG_01438 5 flanking region primer 2

<400> 758
gctcactggc cgtcgtttta cagcaagatg gaaggaagc 39

<210> 759

<211>    39
<212>    DNA
<213>    CNAG_01438 3 flanking region primer 1

<400>    759
catggtcata gctgtttcct ggcgacgaaa tcataaggc                                39

<210>    760
<211>    19
<212>    DNA
<213>    CNAG_01438 3 flanking region primer 2

<400>    760
tgagaactgg acgaactgc                                                      19

<210>    761
<211>    18
<212>    DNA
<213>    CNAG_01438 diagnostic screening primer, pairing with B79

<400>    761
aatgatgcgt cggacttc                                                       18

<210>    762
<211>    20
<212>    DNA
<213>    CNAG_01438 Southern blot probe primer

<400>    762
ggaagagaaa gacttgcgag                                                     20

<210>    763
<211>    24
<212>    DNA
<213>    NAT#122 STM primer

<400>    763
acagctccaa acctcgctaa acag                                              24

<210>    764
<211>    24
<212>    DNA
<213>    STM common primer

<400>    764
gcatgccctg cccctaagaa ttcg                                              24

<210>    765
<211>    20
<212>    DNA
<213>    CNAG_00791 5 flanking region primer 1

<400>    765
gcatccattt tatcggttcg                                                   20

<210>    766
<211>    41
<212>    DNA
<213>    CNAG_00791 5 flanking region primer 2

<400>    766
gctcactggc cgtcgtttta cttctgcttg aaggagtcgg g                           41

<210>    767
<211>    40
<212>    DNA
<213>    CNAG_00791 3 flanking region primer 1

<400>    767
catggtcata gctgtttcct gcggttatct tgcgtaaagc                             40

<210> 768
<211> 19
<212> DNA
<213> CNAG_00791 3 flanking region primer 2

<400> 768
tgtctcaccc atcacgaac 19

<210> 769
<211> 18
<212> DNA
<213> CNAG_00791 diagnostic screening primer, pairing with B79

<400> 769
attcgctcag gcagtttc 18

<210> 770
<211> 19
<212> DNA
<213> CNAG_00791 Southern blot probe primer

<400> 770
gcgatgatga ggactcttg 19

<210> 771
<211> 21
<212> DNA
<213> NAT#146 STM primer

<400> 771
actagccccc cctcaccacc t 21

<210> 772
<211> 24
<212> DNA
<213> STM common primer

<400>    772
   gcatgccctg cccctaagaa ttcg                                                24

<210>    773
<211>    19
<212>    DNA
<213>    CNAG_02241 5 flanking region primer 1

<400>    773
atagcaaacc catacctcg                                                       19

<210>    774
<211>    37
<212>    DNA
<213>    CNAG_02241 5 flanking region primer 2

<400>    774
tcactggccg tcgttttact ttgtttggta gtggggg                                   37

<210>    775
<211>    40
<212>    DNA
<213>    CNAG_02241 3 flanking region primer 1

<400>    775
catggtcata gctgtttcct gcgagaactc tggagcattc                                40

<210>    776
<211>    21
<212>    DNA
<213>    CNAG_02241 3 flanking region primer 2

<400>    776
gatgtgttgt ggactgtatg c                                                    21

<210> 777
<211> 20
<212> DNA
<213> CNAG_02241 diagnostic screening primer, pairing with B79

<400> 777
tcccatcata ggagtggacg 20

<210> 778
<211> 20
<212> DNA
<213> CNAG_02241 Southern blot probe primer

<400> 778
cttgggagca aataagtgtg 20

<210> 779
<211> 20
<212> DNA
<213> NAT#219 STM primer

<400> 779
ccctaaaacc ctacagcaat 20

<210> 780
<211> 24
<212> DNA
<213> STM common primer

<400> 780
gcatgccctg cccctaagaa ttcg 24

<210> 781
<211> 19
<212> DNA

<213>    CNAG_02566 5 flanking region primer 1

<400>    781
tcgcacggca taggattag                                                    19

<210>    782
<211>    38
<212>    DNA
<213>    CNAG_02566 5 flanking region primer 2

<400>    782
tcactggccg tcgttttaca tgccgccaga atgttgag                                38

<210>    783
<211>    40
<212>    DNA
<213>    CNAG_02566 3 flanking region primer 1

<400>    783
catggtcata gctgtttcct gtgatgttga tggagactgg                              40

<210>    784
<211>    20
<212>    DNA
<213>    CNAG_02566 3 flanking region primer 2

<400>    784
actgcttctt caccctccac                                                    20

<210>    785
<211>    18
<212>    DNA
<213>    CNAG_02566 diagnostic screening primer, pairing with B79

<400>    785
atctgccgca acattcac                                                      18

<210> 786
<211> 21
<212> DNA
<213> CNAG_02566 Southern blot probe primer

<400> 786
gttggtgctt catagtgact g 21

<210> 787
<211> 20
<212> DNA
<213> NAT#219 STM primer

<400> 787
ccctaaaacc ctacagcaat 20

<210> 788
<211> 24
<212> DNA
<213> STM common primer

<400> 788
gcatgccctg cccctaagaa ttcg 24

<210> 789
<211> 19
<212> DNA
<213> CNAG_01626 5 flanking region primer 1

<400> 789
ggatgatgga atcgtatgc 19

<210> 790
<211> 38

<212>    DNA
<213>    CNAG_01626 5 flanking region primer 2

<400>    790
tcactggccg tcgttttaca ttatccaccc tcggcttc                                38

<210>    791
<211>    42
<212>    DNA
<213>    CNAG_01626 3 flanking region primer 1

<400>    791
catggtcata gctgtttcct ggcgaacggt atgacaacta tg                           42

<210>    792
<211>    22
<212>    DNA
<213>    CNAG_01626 3 flanking region primer 2

<400>    792
ggacgaaaac attgctctca ac                                                 22

<210>    793
<211>    19
<212>    DNA
<213>    CNAG_01626 diagnostic screening primer, pairing with B79

<400>    793
tcagcagcag caggtaaac                                                     19

<210>    794
<211>    18
<212>    DNA
<213>    CNAG_01626 Southern blot probe primer

<400>    794

cccagaccgt tttgaatg 18

<210> 795
<211> 18
<212> DNA
<213> NAT#232 STM primer

<400> 795
ctttaaaggt ggtttgtg 18

<210> 796
<211> 24
<212> DNA
<213> STM common primer

<400> 796
gcatgccctg cccctaagaa ttcg 24

<210> 797
<211> 20
<212> DNA
<213> CNAG_05940 5 flanking region primer 1

<400> 797
tcttgaccgt cgccagttac 20

<210> 798
<211> 39
<212> DNA
<213> CNAG_05940 5 flanking region primer 2

<400> 798
tcactggccg tcgttttact ggctcaaagg caaagtcgg 39

<210> 799

<211>    40
<212>    DNA
<213>    CNAG_05940 3 flanking region primer 1

<400>    799
catggtcata gctgtttcct gaggtgattt tgggagtcag                                40

<210>    800
<211>    19
<212>    DNA
<213>    CNAG_05940 3 flanking region primer 2

<400>    800
ggctgagaat gatgtgacg                                                       19

<210>    801
<211>    19
<212>    DNA
<213>    CNAG_05940 diagnostic screening primer, pairing with B79

<400>    801
ggaaagaaga cggatagcg                                                       19

<210>    802
<211>    19
<212>    DNA
<213>    CNAG_05940 Southern blot probe primer

<400>    802
gtctccagga aagttcgtg                                                       19

<210>    803
<211>    18
<212>    DNA
<213>    NAT#232 STM primer

<400> 803
ctttaaaggt ggtttgtg 18

<210> 804
<211> 24
<212> DNA
<213> STM common primer

<400> 804
gcatgccctg cccctaagaa ttcg 24

<210> 805
<211> 22
<212> DNA
<213> CNAG_04352 5 flanking region primer 1

<400> 805
gtgattatgt gaacccgtta cc 22

<210> 806
<211> 39
<212> DNA
<213> CNAG_04352 5 flanking region primer 2

<400> 806
tcactggccg tcgttttact tttagaggtg atgactgcg 39

<210> 807
<211> 41
<212> DNA
<213> CNAG_04352 3 flanking region primer 1

<400> 807
catggtcata gctgtttcct gtgctgctgg aggaagagaa c 41

<210>    808
<211>    19
<212>    DNA
<213>    CNAG_04352 3 flanking region primer 2

<400>    808
agtgagtcgt tgatgtggc                                                    19


<210>    809
<211>    19
<212>    DNA
<213>    CNAG_04352 diagnostic screening primer, pairing with B79

<400>    809
atagtgaata cgggtcggg                                                    19


<210>    810
<211>    19
<212>    DNA
<213>    CNAG_04352 Southern blot probe primer

<400>    810
gctgaaacag gaggcatag                                                    19


<210>    811
<211>    20
<212>    DNA
<213>    NAT#234 STM primer

<400>    811
ccagccagca ctccgatata                                                   20


<210>    812
<211>    24
<212>    DNA
<213>    STM common primer

<400>    812
gcatgccctg cccctaagaa ttcg                                              24

<210>    813
<211>    18
<212>    DNA
<213>    CNAG_03768 5 flanking region primer 1

<400>    813
aatgtccttg cggttgac                                                     18

<210>    814
<211>    40
<212>    DNA
<213>    CNAG_03768 5 flanking region primer 2

<400>    814
gctcactggc cgtcgtttta caatagtccc cgtcgttgtc                             40

<210>    815
<211>    41
<212>    DNA
<213>    CNAG_03768 3 flanking region primer 1

<400>    815
catggtcata gctgtttcct gcgatggatg tttggtggta g                           41

<210>    816
<211>    19
<212>    DNA
<213>    CNAG_03768 3 flanking region primer 2

<400>    816
aaaggggttt tggtggctc                                                    19

<210> 817
<211> 20
<212> DNA
<213> CNAG_03768 diagnostic screening primer, pairing with B79

<400> 817
tggatttggc agagaagaag 20

<210> 818
<211> 19
<212> DNA
<213> CNAG_03768 Southern blot probe primer

<400> 818
aagacacttg gctctccag 19

<210> 819
<211> 20
<212> DNA
<213> NAT#234 STM primer

<400> 819
ccagccagca ctccgatata 20

<210> 820
<211> 24
<212> DNA
<213> STM common primer

<400> 820
gcatgccctg cccctaagaa ttcg 24

<210> 821
<211> 20
<212> DNA

<213>    CNAG_02322 5 flanking region primer 1

<400>    821
cgctcactca cctttcaaac                                                    20

<210>    822
<211>    35
<212>    DNA
<213>    CNAG_02322 5 flanking region primer 2

<400>    822
ctggccgtcg ttttacacat cggcttttac ggagc                                   35

<210>    823
<211>    37
<212>    DNA
<213>    CNAG_02322 3 flanking region primer 1

<400>    823
gtcatagctg tttcctgctc ggttaccagt cactacg                                 37

<210>    824
<211>    20
<212>    DNA
<213>    CNAG_02322 3 flanking region primer 2

<400>    824
ccaccaagat tcagagttcg                                                    20

<210>    825
<211>    19
<212>    DNA
<213>    CNAG_02322 diagnostic screening primer, pairing with B79

<400>    825
gagatttcgg tggatttcc                                                     19

<210> 826
<211> 18
<212> DNA
<213> CNAG_02322 Southern blot probe primer

<400> 826
aagcaaccct cagcattg 18

<210> 827
<211> 20
<212> DNA
<213> NAT#240 STM primer

<400> 827
ggtgttggat cggggtggat 20

<210> 828
<211> 24
<212> DNA
<213> STM common primer

<400> 828
gcatgccctg cccctaagaa ttcg 24

<210> 829
<211> 20
<212> DNA
<213> CNAG_06818 5 flanking region primer 1

<400> 829
tgtattctgc ccacctccac 20

<210> 830
<211> 39

<212>    DNA
<213>    CNAG_06818 5 flanking region primer 2

<400>    830
tcactggccg tcgttttacc ctctttgctg gtctctgag                                39

<210>    831
<211>    41
<212>    DNA
<213>    CNAG_06818 3 flanking region primer 1

<400>    831
catggtcata gctgtttcct gtctgctgct acatcaggtt c                             41

<210>    832
<211>    19
<212>    DNA
<213>    CNAG_06818 3 flanking region primer 2

<400>    832
aacctctgta tcgctgacg                                                      19

<210>    833
<211>    22
<212>    DNA
<213>    CNAG_06818 diagnostic screening primer, pairing with B79

<400>    833
gggtgaaaga caataggtaa gg                                                  22

<210>    834
<211>    21
<212>    DNA
<213>    CNAG_06818 Southern blot probe primer

<400>    834

aaagtgaaga gaggaggtca g                                                21

<210>    835
<211>    20
<212>    DNA
<213>    NAT#240 STM primer

<400>    835
ggtgttggat cggggtggat                                                  20

<210>    836
<211>    24
<212>    DNA
<213>    STM common primer

<400>    836
gcatgccctg cccctaagaa ttcg                                             24

<210>    837
<211>    19
<212>    DNA
<213>    CNAG_07011 5 flanking region primer 1

<400>    837
ccaacaaata gcaaagccg                                                   19

<210>    838
<211>    38
<212>    DNA
<213>    CNAG_07011 5 flanking region primer 2

<400>    838
tcactggccg tcgttttaca caccaaagcc aagaacac                              38

<210>    839

<211>    41
<212>    DNA
<213>    CNAG_07011 3 flanking region primer 1

<400>    839
catggtcata gctgtttcct ggctaatgcc tgaaacagac c                41

<210>    840
<211>    19
<212>    DNA
<213>    CNAG_07011 3 flanking region primer 2

<400>    840
tcggacaccc aatcaatac                19

<210>    841
<211>    18
<212>    DNA
<213>    CNAG_07011 diagnostic screening primer, pairing with B79

<400>    841
ttgtgttgtt cggtgagg                18

<210>    842
<211>    20
<212>    DNA
<213>    CNAG_07011 Southern blot probe primer

<400>    842
tgggtgatac agaggaaaac                20

<210>    843
<211>    24
<212>    DNA
<213>    NAT#273 STM primer

<400>    843
gagatctttc gggaggtctg gatt                                              24

<210>    844
<211>    24
<212>    DNA
<213>    STM common primer

<400>    844
gcatgccctg cccctaagaa ttcg                                              24

<210>    845
<211>    20
<212>    DNA
<213>    CNAG_04036 5 flanking region primer 1

<400>    845
ccagttgaac gcttctaaac                                                   20

<210>    846
<211>    39
<212>    DNA
<213>    CNAG_04036 5 flanking region primer 2

<400>    846
tcactggccg tcgttttact gatggtgtgg gaacaatac                              39

<210>    847
<211>    40
<212>    DNA
<213>    CNAG_04036 3 flanking region primer 1

<400>    847
catggtcata gctgtttcct gttctccttg cctatgtgcc                             40

<210>    848
<211>    20
<212>    DNA
<213>    CNAG_04036 3 flanking region primer 2

<400>    848
cagcaactta ctttggaggc                                                    20

<210>    849
<211>    19
<212>    DNA
<213>    CNAG_04036 diagnostic screening primer, pairing with B79

<400>    849
atagacagca gcgggatac                                                     19

<210>    850
<211>    18
<212>    DNA
<213>    CNAG_04036 Southern blot probe primer

<400>    850
tcacagatgc ctttctcg                                                      18

<210>    851
<211>    24
<212>    DNA
<213>    NAT#273 STM primer

<400>    851
gagatctttc gggaggtctg gatt                                               24

<210>    852
<211>    24
<212>    DNA
<213>    STM common primer

<400>    852
gcatgccctg cccctaagaa ttcg                                              24

<210>    853
<211>    19
<212>    DNA
<213>    CNAG_07506 5 flanking region primer 1

<400>    853
cgtaatgaca ctggagagc                                                    19

<210>    854
<211>    42
<212>    DNA
<213>    CNAG_07506 5 flanking region primer 2

<400>    854
gctcactggc cgtcgtttta ctgttgccgt aggaaccgct tc                          42

<210>    855
<211>    40
<212>    DNA
<213>    CNAG_07506 3 flanking region primer 1

<400>    855
catggtcata gctgtttcct gtcccaaagc ctcttctcag                            40

<210>    856
<211>    18
<212>    DNA
<213>    CNAG_07506 3 flanking region primer 2

<400>    856
gtgagaccct gatagacc                                                     18

<210> 857
<211> 19
<212> DNA
<213> CNAG_07506 diagnostic screening primer, pairing with B79

<400> 857
gactcaaagg gagatgacg 19

<210> 858
<211> 19
<212> DNA
<213> CNAG_07506 Southern blot probe primer

<400> 858
gcttttgcct gctattgac 19

<210> 859
<211> 20
<212> DNA
<213> NAT#296 STM primer

<400> 859
cgcccgccct cactatccac 20

<210> 860
<211> 24
<212> DNA
<213> STM common primer

<400> 860
gcatgccctg cccctaagaa ttcg 24

<210> 861
<211> 19
<212> DNA

<213>    CNAG_06223 5 flanking region primer 1

<400>    861
ggatgaaggg gaagacttg                                                    19

<210>    862
<211>    38
<212>    DNA
<213>    CNAG_06223 5 flanking region primer 2

<400>    862
tcactggccg tcgttttacc ttccgttcaa aatcgtcg                               38

<210>    863
<211>    41
<212>    DNA
<213>    CNAG_06223 3 flanking region primer 1

<400>    863
catggtcata gctgtttcct ggtctaccac caccatcaca c                           41

<210>    864
<211>    20
<212>    DNA
<213>    CNAG_06223 3 flanking region primer 2

<400>    864
gccgtccatt catacatctg                                                   20

<210>    865
<211>    19
<212>    DNA
<213>    CNAG_06223 diagnostic screening primer, pairing with B79

<400>    865
ggtgtcaata gttgggtgg                                                    19

<210> 866
<211> 19
<212> DNA
<213> CNAG_06223 Southern blot probe primer

<400> 866
agagcagaag caaatgacc 19

<210> 867
<211> 20
<212> DNA
<213> NAT#210 STM primer

<400> 867
ctagagcccg ccacaacgct 20

<210> 868
<211> 24
<212> DNA
<213> STM common primer

<400> 868
gcatgccctg cccctaagaa ttcg 24

<210> 869
<211> 24
<212> DNA
<213> CNAG_06283 5 flanking region primer 1

<400> 869
ccctttgtcc ttacccacat tttc 24

<210> 870
<211> 39

<212>	DNA
<213>	CNAG_06283 5 flanking region primer 2

<400>	870
tcactggccg tcgttttact cgttcgggca cttcttgac	39

<210>	871
<211>	41
<212>	DNA
<213>	CNAG_06283 3 flanking region primer 1

<400>	871
catggtcata gctgtttcct gcgagtaagg agacggttga g	41

<210>	872
<211>	22
<212>	DNA
<213>	CNAG_06283 3 flanking region primer 2

<400>	872
ggttaggggg ataaaatagg ag	22

<210>	873
<211>	21
<212>	DNA
<213>	CNAG_06283 diagnostic screening primer, pairing with B79

<400>	873
gagacattct ttctgacgag c	21

<210>	874
<211>	20
<212>	DNA
<213>	CNAG_06283 Southern blot probe primer

<400>	874

tggcttgaag ggtcttggtc                                                    20

<210>    875
<211>    20
<212>    DNA
<213>    NAT#234 STM primer

<400>    875
ccagccagca ctccgatata                                                    20

<210>    876
<211>    24
<212>    DNA
<213>    STM common primer

<400>    876
gcatgccctg cccctaagaa ttcg                                               24

<210>    877
<211>    22
<212>    DNA
<213>    CNAG_04583 5 flanking region primer 1

<400>    877
gctcgctaca ttttcaggta tc                                                 22

<210>    878
<211>    39
<212>    DNA
<213>    CNAG_04583 5 flanking region primer 2

<400>    878
tcactggccg tcgttttacg agagggtcgg aaatagaag                              39

<210>    879

<211>    41
<212>    DNA
<213>    CNAG_04583 3 flanking region primer 1

<400>    879
catggtcata gctgtttcct ggggaaggag taatgcctga c                41

<210>    880
<211>    18
<212>    DNA
<213>    CNAG_04583 3 flanking region primer 2

<400>    880
aaccgtgatg gagattgg                18

<210>    881
<211>    20
<212>    DNA
<213>    CNAG_04583 diagnostic screening primer, pairing with B79

<400>    881
cggtgtgcga ttctaacttc                20

<210>    882
<211>    19
<212>    DNA
<213>    CNAG_04583 Southern blot probe primer

<400>    882
ggggactgtt ttgatgacc                19

<210>    883
<211>    24
<212>    DNA
<213>    NAT#102 STM primer

<400>    883
ccatagcgat atctacccca atct                                              24

<210>    884
<211>    24
<212>    DNA
<213>    STM common primer

<400>    884
gcatgccctg cccctaagaa ttcg                                              24

<210>    885
<211>    19
<212>    DNA
<213>    CNAG_03261 5 flanking region primer 1

<400>    885
ggcgataaag acgacgaac                                                    19

<210>    886
<211>    35
<212>    DNA
<213>    CNAG_03261 5 flanking region primer 2

<400>    886
ctggccgtcg ttttacggac tattcgtggc ttagc                                  35

<210>    887
<211>    35
<212>    DNA
<213>    CNAG_03261 3 flanking region primer 1

<400>    887
gtcatagctg tttcctgtga gaccaatgtg ttgcc                                  35

<210> 888
<211> 24
<212> DNA
<213> CNAG_03261 3 flanking region primer 2

<400> 888
cctcaacccc tttctgattt atcc 24

<210> 889
<211> 22
<212> DNA
<213> CNAG_03261 diagnostic screening primer, pairing with B79

<400> 889
tagtcggtga tgagagttgc gg 22

<210> 890
<211> 20
<212> DNA
<213> CNAG_03261 Southern blot probe primer

<400> 890
gcaaatctgc ttctgggatg 20

<210> 891
<211> 19
<212> DNA
<213> CNAG_04908 5 flanking region primer 1

<400> 891
gatgacgaat agcacggtc 19

<210> 892
<211> 37
<212> DNA
<213> CNAG_04908 5 flanking region primer 2

<400> 892
tcactggccg tcgttttacg gatgggaaga ttgttgg 37

<210> 893
<211> 39
<212> DNA
<213> CNAG_04908 3 flanking region primer 1

<400> 893
gatggtcata gctgtttcct gttgcttacg cctttgtcc 39

<210> 894
<211> 18
<212> DNA
<213> CNAG_04908 3 flanking region primer 2

<400> 894
atggttgttc acgcacag 18

<210> 895
<211> 18
<212> DNA
<213> CNAG_04908 diagnostic screening primer, pairing with B79

<400> 895
aaacaaaagc cgagggag 18

<210> 896
<211> 19
<212> DNA
<213> CNAG_04908 Southern blot probe primer

<400> 896
aacttcccac cccttcatc 19

<210> 897
<211> 24
<212> DNA
<213> NAT#242 STM primer

<400> 897
gtagcgatag gggtgtcgct ttag 24

<210> 898
<211> 24
<212> DNA
<213> STM common primer

<400> 898
gcatgccctg cccctaagaa ttcg 24

<210> 899
<211> 19
<212> DNA
<213> CNAG_01317 5 flanking region primer 1

<400> 899
aagcaccaag aagcaagtg 19

<210> 900
<211> 37
<212> DNA
<213> CNAG_01317 5 flanking region primer 2

<400> 900
tcactggccg tcgttttaca ttgacacctg ccgattc 37

<210> 901
<211> 40
<212> DNA

<213>    CNAG_01317 3 flanking region primer 1

<400>    901
catggtcata gctgtttcct gtactatcag cagaagcggc                                40

<210>    902
<211>    20
<212>    DNA
<213>    CNAG_01317 3 flanking region primer 2

<400>    902
aatgataggc ggaagtaagg                                                      20

<210>    903
<211>    19
<212>    DNA
<213>    CNAG_01317 diagnostic screening primer, pairing with B79

<400>    903
ccacctcgca gtatcaaac                                                       19

<210>    904
<211>    20
<212>    DNA
<213>    CNAG_01317 Southern blot probe primer

<400>    904
agggtcaggg agatgatgtg                                                      20

<210>    905
<211>    19
<212>    DNA
<213>    CNAG_00559 5 flanking region primer 1

<400>    905
ggtgtttcca tacgaaccg                                                       19

<210>    906
<211>    38
<212>    DNA
<213>    CNAG_00559 5 flanking region primer 2

<400>    906
ctggccgtcg ttttacggag ccatttctta cttatgcg                                38

<210>    907
<211>    35
<212>    DNA
<213>    CNAG_00559 3 flanking region primer 1

<400>    907
gtcatagctg tttcctgaat cccaaggacc atctg                                   35

<210>    908
<211>    20
<212>    DNA
<213>    CNAG_00559 3 flanking region primer 2

<400>    908
gctgagaagg agaggatagc                                                    20

<210>    909
<211>    20
<212>    DNA
<213>    CNAG_00559 diagnostic screening primer, pairing with B79

<400>    909
gtggttgaaa gtattaggcg                                                    20

<210>    910
<211>    19

<212>    DNA
<213>    CNAG_00559 Southern blot probe primer

<400>    910
tctcttcgtt tgctacacg                                                    19


<210>    911
<211>    24
<212>    DNA
<213>    NAT#242 STM primer

<400>    911
   gtagcgatag gggtgtcgct ttag                                               24


<210>    912
<211>    24
<212>    DNA
<213>    STM common primer

<400>    912
gcatgccctg cccctaagaa ttcg                                              24


<210>    913
<211>    18
<212>    DNA
<213>    CNAG_07940 5 flanking region primer 1

<400>    913
caaggtaatg attggggc                                                     18


<210>    914
<211>    38
<212>    DNA
<213>    CNAG_07940 5 flanking region primer 2

<400>    914

tcactggccg tcgttttacg caataatgga aggtctcg 38

<210> 915
<211> 39
<212> DNA
<213> CNAG_07940 3 flanking region primer 1

<400> 915
catggtcata gctgtttcct gatcaagaat gacacgccg 39

<210> 916
<211> 19
<212> DNA
<213> CNAG_07940 3 flanking region primer 2

<400> 916
aacttgtatg gtggtgccg 19

<210> 917
<211> 19
<212> DNA
<213> CNAG_07940 diagnostic screening primer, pairing with B79

<400> 917
tcacttgctc ttctccctg 19

<210> 918
<211> 18
<212> DNA
<213> CNAG_07940 Southern blot probe primer

<400> 918
gtgacgaatg cgaaactc 18

<210> 919

<211>    20
<212>    DNA
<213>    NAT#191 STM primer

<400>    919
atatggatgt ttttagcgag                                                    20


<210>    920
<211>    24
<212>    DNA
<213>    STM common primer

<400>    920
gcatgccctg cccctaagaa ttcg                                               24


<210>    921
<211>    18
<212>    DNA
<213>    CNAG_05535 5 flanking region primer 1

<400>    921
agcaacaacg gcatagtg                                                      18


<210>    922
<211>    39
<212>    DNA
<213>    CNAG_05535 5 flanking region primer 2

<400>    922
tcactggccg tcgttttaca cgacagtgag agtcctgac                               39


<210>    923
<211>    43
<212>    DNA
<213>    CNAG_05535 3 flanking region primer 1

<400>    923
catggtcata gctgtttcct gctggagcga ggaaatagta atg    43

<210>    924
<211>    18
<212>    DNA
<213>    CNAG_05535 3 flanking region primer 2

<400>    924
acacaaagcg agtggttg    18

<210>    925
<211>    19
<212>    DNA
<213>    CNAG_05535 diagnostic screening primer, pairing with B79

<400>    925
ggcagtgtca gatgaaagc    19

<210>    926
<211>    19
<212>    DNA
<213>    CNAG_05535 Southern blot probe primer

<400>    926
catcggctac atcttcctc    19

<210>    927
<211>    21
<212>    DNA
<213>    CNAG_02516 5 flanking region primer 1

<400>    927
gcatcacacg aacgattcta c    21

<210>    928
<211>    38
<212>    DNA
<213>    CNAG_02516 5 flanking region primer 2

<400>    928
tcactggccg tcgttttaca ttgttggcgg tggaaacg                                38

<210>    929
<211>    41
<212>    DNA
<213>    CNAG_02516 3 flanking region primer 1

<400>    929
catggtcata gctgtttcct gaggtttggg agcagtaagg c                            41

<210>    930
<211>    19
<212>    DNA
<213>    CNAG_02516 3 flanking region primer 2

<400>    930
gatgtttgtg accgattcg                                                     19

<210>    931
<211>    19
<212>    DNA
<213>    CNAG_02516 diagnostic screening primer, pairing with B79

<400>    931
gcgaaggttt accgataag                                                     19

<210>    932
<211>    20
<212>    DNA
<213>    CNAG_02516 Southern blot probe primer

<400>    932
tatggggtta ggagcgttgg                                                    20

<210>    933
<211>    20
<212>    DNA
<213>    NAT#210 STM primer

<400>    933
ctagagcccg ccacaacgct                                                    20

<210>    934
<211>    24
<212>    DNA
<213>    STM common primer

<400>    934
gcatgccctg cccctaagaa ttcg                                               24

<210>    935
<211>    19
<212>    DNA
<213>    CNAG_04226 5 flanking region primer 1

<400>    935
   gccctttttg gtatgacag                                                      19

<210>    936
<211>    38
<212>    DNA
<213>    CNAG_04226 5 flanking region primer 2

<400>    936
tcactggccg tcgttttact gtgagggatt ccttgttc                                38

<210> 937
<211> 39
<212> DNA
<213> CNAG_04226 3 flanking region primer 1

<400> 937
catggtcata gctgtttcct ggttggaaac aagcagtcg 39

<210> 938
<211> 20
<212> DNA
<213> CNAG_04226 3 flanking region primer 2

<400> 938
tcagaagaat caagaggtcg 20

<210> 939
<211> 19
<212> DNA
<213> CNAG_04226 diagnostic screening primer, pairing with B79

<400> 939
acttacattg ccttcctcg 19

<210> 940
<211> 19
<212> DNA
<213> CNAG_04226 Southern blot probe primer

<400> 940
atacatcagc ctgtcctgc 19

<210> 941
<211> 19
<212> DNA

<213>    CNAG_05170 5 flanking region primer 1

<400>    941
tgccttcgct ctgtaactc                                                    19

<210>    942
<211>    34
<212>    DNA
<213>    CNAG_05170 5 flanking region primer 2

<400>    942
ctggccgtcg ttttaccctt tttgaggctc gctg                                   34

<210>    943
<211>    36
<212>    DNA
<213>    CNAG_05170 3 flanking region primer 1

<400>    943
gtcatagctg tttcctgaaa tgaaggagtg tgcggg                                 36

<210>    944
<211>    20
<212>    DNA
<213>    CNAG_05170 3 flanking region primer 2

<400>    944
aataggacct caacaccagg                                                   20

<210>    945
<211>    18
<212>    DNA
<213>    CNAG_05170 diagnostic screening primer, pairing with B79

<400>    945
aacaagaagc ccctttcg                                                     18

<210> 946
<211> 18
<212> DNA
<213> CNAG_05170 Southern blot probe primer

<400> 946
agtttttgga cccgagtg 18

<210> 947
<211> 18
<212> DNA
<213> NAT#232 STM primer

<400> 947
ctttaaaggt ggtttgtg 18

<210> 948
<211> 24
<212> DNA
<213> STM common primer

<400> 948
gcatgccctg cccctaagaa ttcg 24

<210> 949
<211> 20
<212> DNA
<213> CNAG_03902 5 flanking region primer 1

<400> 949
gcaaagtcaa gaaggtcagg 20

<210> 950
<211> 35

<212>    DNA
<213>    CNAG_03902 5 flanking region primer 2

<400>    950
ctggccgtcg ttttacttct tctttggctc gtccg                                35

<210>    951
<211>    39
<212>    DNA
<213>    CNAG_03902 3 flanking region primer 1

<400>    951
gtcatagctg tttcctgggg agatgaaatg tgcgtttag                            39

<210>    952
<211>    22
<212>    DNA
<213>    CNAG_03902 3 flanking region primer 2

<400>    952
atgctttcgc cgtcatctcg tc                                              22

<210>    953
<211>    20
<212>    DNA
<213>    CNAG_03902 diagnostic screening primer, pairing with B79

<400>    953
tgattacccc tgtcaaactc                                                 20

<210>    954
<211>    19
<212>    DNA
<213>    CNAG_03902 Southern blot probe primer

<400>    954

cagaatcggg aagtaatgg                                                    19

<210>    955
<211>    18
<212>    DNA
<213>    NAT#232 STM primer

<400>    955
ctttaaaggt ggtttgtg                                                     18

<210>    956
<211>    24
<212>    DNA
<213>    STM common primer

<400>    956
gcatgccctg cccctaagaa ttcg                                              24

<210>    957
<211>    20
<212>    DNA
<213>    CNAG_06097 5 flanking region primer 1

<400>    957
cagtccgaag tttacaatcg                                                   20

<210>    958
<211>    36
<212>    DNA
<213>    CNAG_06097 5 flanking region primer 2

<400>    958
ctggccgtcg ttttactttc gtcgtgtgga gttggc                                 36

<210>    959

<211>    37
<212>    DNA
<213>    CNAG_06097 3 flanking region primer 1

<400>    959
gtcatagctg tttcctgaga ttggagggtt tgagggc                                37

<210>    960
<211>    19
<212>    DNA
<213>    CNAG_06097 3 flanking region primer 2

<400>    960
tccaccgaag aaactgaag                                                    19

<210>    961
<211>    20
<212>    DNA
<213>    CNAG_06097 diagnostic screening primer, pairing with B79

<400>    961
aggtttgaga ggtttggaag                                                   20

<210>    962
<211>    18
<212>    DNA
<213>    CNAG_06097 Southern blot probe primer

<400>    962
attatcggag cgttgtgg                                                     18

<210>    963
<211>    20
<212>    DNA
<213>    CNAG_03431 5 flanking region primer 1

<400>    963
aagcactctg tagcctgttg                                                    20

<210>    964
<211>    39
<212>    DNA
<213>    CNAG_03431 5 flanking region primer 2

<400>    964
gctcactggc cgtcgtttta ccttgaagat gatgcgtgc                               39

<210>    965
<211>    40
<212>    DNA
<213>    CNAG_03431 3 flanking region primer 1

<400>    965
catggtcata gctgtttcct gcgctcatag agaatgctgg                              40

<210>    966
<211>    19
<212>    DNA
<213>    CNAG_03431 3 flanking region primer 2

<400>    966
taggagtgtc tgccaaagc                                                     19

<210>    967
<211>    19
<212>    DNA
<213>    CNAG_03431 diagnostic screening primer, pairing with B79

<400>    967
tcatcctcat cccgaatac                                                     19

<210>    968
<211>    19
<212>    DNA
<213>    CNAG_03431 Southern blot probe primer

<400>    968
actcttcgtg agacgcatc                                                    19

<210>    969
<211>    24
<212>    DNA
<213>    NAT#290 STM primer

<400>    969
accgacagct cgaacaagca agag                                              24

<210>    970
<211>    24
<212>    DNA
<213>    STM common primer

<400>    970
gcatgccctg cccctaagaa ttcg                                              24

<210>    971
<211>    21
<212>    DNA
<213>    CNAG_00830 5 flanking region primer 1

<400>    971
ccgactaata ggtgtgctgt c                                                 21

<210>    972
<211>    41
<212>    DNA
<213>    CNAG_00830 5 flanking region primer 2

<400>    972
gctcactggc cgtcgtttta cgagatgaga tgagttgcgt g                41

<210>    973
<211>    41
<212>    DNA
<213>    CNAG_00830 3 flanking region primer 1

<400>    973
catggtcata gctgtttcct gttgtgtccc catcactacc g                41

<210>    974
<211>    19
<212>    DNA
<213>    CNAG_00830 3 flanking region primer 2

<400>    974
catccaaaga agcgaaacg                                         19

<210>    975
<211>    24
<212>    DNA
<213>    CNAG_00830 diagnostic screening primer, pairing with B79

<400>    975
gaagagaagt agtggataga ctcg                                   24

<210>    976
<211>    19
<212>    DNA
<213>    CNAG_00830 Southern blot probe primer

<400>    976
tgatgccgta aatcgtagg                                         19

<210>    977
<211>    21
<212>    DNA
<213>    NAT#292 STM primer

<400>    977
atttcgatgt atatatgtag c                                                21

<210>    978
<211>    24
<212>    DNA
<213>    STM common primer

<400>    978
gcatgccctg cccctaagaa ttcg                                             24

<210>    979
<211>    20
<212>    DNA
<213>    CNAG_07443 5 flanking region primer 1

<400>    979
atctcttctc tcggcgtgtc                                                  20

<210>    980
<211>    40
<212>    DNA
<213>    CNAG_07443 5 flanking region primer 2

<400>    980
gctcactggc cgtcgtttta cacaggaaaa ggaggagtcg                            40

<210>    981
<211>    40
<212>    DNA

<213>    CNAG_07443 3 flanking region primer 1

<400>    981
catggtcata gctgtttcct gaagtctttc cgtctgaccc                                40

<210>    982
<211>    18
<212>    DNA
<213>    CNAG_07443 3 flanking region primer 2

<400>    982
tcgcatccct tgtattcg                                                        18

<210>    983
<211>    19
<212>    DNA
<213>    CNAG_07443 diagnostic screening primer, pairing with B79

<400>    983
tcaataatca gccgcctac                                                       19

<210>    984
<211>    19
<212>    DNA
<213>    CNAG_07443 Southern blot probe primer

<400>    984
ttagattccg cataggtcc                                                       19

<210>    985
<211>    21
<212>    DNA
<213>    NAT#295 STM primer

<400>    985
acacctacat caaaccctcc c                                                    21

<210>    986
<211>    24
<212>    DNA
<213>    STM common primer

<400>    986
gcatgccctg cccctaagaa ttcg                                                24

<210>    987
<211>    19
<212>    DNA
<213>    CNAG_05186 5 flanking region primer 1

<400>    987
acaacggtca agaagggtc                                                      19

<210>    988
<211>    39
<212>    DNA
<213>    CNAG_05186 5 flanking region primer 2

<400>    988
gctcactggc cgtcgtttta caagtaaagg aacgcacgg                                39

<210>    989
<211>    41
<212>    DNA
<213>    CNAG_05186 3 flanking region primer 1

<400>    989
catggtcata gctgtttcct gcgcttgaga cgggagtaat c                             41

<210>    990
<211>    20

<212>    DNA
<213>    CNAG_05186 3 flanking region primer 2

<400>    990
gaggtaagga tgattggacc                                                    20


<210>    991
<211>    23
<212>    DNA
<213>    CNAG_05186 diagnostic screening primer, pairing with B79

<400>    991
gtgtgaagtg agtagtaacg agc                                                23


<210>    992
<211>    22
<212>    DNA
<213>    CNAG_05186 Southern blot probe primer

<400>    992
ttccctatct ctcttcatac cc                                                 22


<210>    993
<211>    20
<212>    DNA
<213>    NAT#296 STM primer

<400>    993
cgcccgccct cactatccac                                                    20


<210>    994
<211>    24
<212>    DNA
<213>    STM common primer

<400>    994

gcatgccctg cccctaagaa ttcg                                                24

<210>    995
<211>    20
<212>    DNA
<213>    CNAG_03561 5 flanking region primer 1

<400>    995
tgggcgagac gagatagaac                                                      20

<210>    996
<211>    36
<212>    DNA
<213>    CNAG_03561 5 flanking region primer 2

<400>    996
ctggccgtcg ttttacggat aaagatgatt cgggtc                                    36

<210>    997
<211>    36
<212>    DNA
<213>    CNAG_03561 3 flanking region primer 1

<400>    997
gtcatagctg tttcctgtga tggctgattt cccgtg                                    36

<210>    998
<211>    36
<212>    DNA
<213>    CNAG_03561 3 flanking region primer 2

<400>    998
gtcatagctg tttcctgtga tggctgattt cccgtg                                    36

<210>    999

<211>    20
<212>    DNA
<213>    CNAG_03561 diagnostic screening primer, pairing with B79

<400>    999
aagaaagacg gtagcggtgg                                                    20

<210>    1000
<211>    20
<212>    DNA
<213>    CNAG_03561 Southern blot probe primer

<400>    1000
ccacctcttc atccaatagc                                                    20

<210>    1001
<211>    20
<212>    DNA
<213>    NAT#43 STM primer

<400>    1001
ccagctacca atcacgctac                                                    20

<210>    1002
<211>    24
<212>    DNA
<213>    STM common primer

<400>    1002
gcatgccctg cccctaagaa ttcg                                               24

<210>    1003
<211>    19
<212>    DNA
<213>    CNAG_04878 5 flanking region primer 1

<400>    1003
aagtccgtgc tggctaatc                                                    19

<210>    1004
<211>    35
<212>    DNA
<213>    CNAG_04878 5 flanking region primer 2

<400>    1004
ctggccgtcg ttttaccttt tcgctgcctt attcg                                  35

<210>    1005
<211>    41
<212>    DNA
<213>    CNAG_04878 3 flanking region primer 1

<400>    1005
gtcatagctg tttcctgaaa acagggcata cggtgtcgtg g                           41

<210>    1006
<211>    20
<212>    DNA
<213>    CNAG_04878 3 flanking region primer 2

<400>    1006
gctgaccatc tcttccctac                                                   20

<210>    1007
<211>    22
<212>    DNA
<213>    CNAG_04878 diagnostic screening primer, pairing with B79

<400>    1007
ccttgtttgt gtctaccgta tc                                                22

<210>    1008
<211>    19
<212>    DNA
<213>    CNAG_04878 Southern blot probe primer

<400>    1008
agtgggaatc gtcgttgac                                                    19

<210>    1009
<211>    20
<212>    DNA
<213>    NAT#116 STM primer

<400>    1009
gcacccaaga gctccatctc                                                   20

<210>    1010
<211>    24
<212>    DNA
<213>    STM common primer

<400>    1010
gcatgccctg cccctaagaa ttcg                                              24

<210>    1011
<211>    19
<212>    DNA
<213>    CNAG_03279 5 flanking region primer 1

<400>    1011
cgtttccagt tatcgcttc                                                    19

<210>    1012
<211>    41
<212>    DNA
<213>    CNAG_03279 5 flanking region primer 2

<400> 1012
gctcactggc cgtcgtttta cgcaatgacc cactttttga c 41

<210> 1013
<211> 41
<212> DNA
<213> CNAG_03279 3 flanking region primer 1

<400> 1013
catggtcata gctgtttcct ggctttgcct gatgagtttt g 41

<210> 1014
<211> 19
<212> DNA
<213> CNAG_03279 3 flanking region primer 2

<400> 1014
gtaacccact cggaagttg 19

<210> 1015
<211> 19
<212> DNA
<213> CNAG_03279 diagnostic screening primer, pairing with B79

<400> 1015
ttgtctgtga ggtggaagg 19

<210> 1016
<211> 20
<212> DNA
<213> CNAG_03279 Southern blot probe primer

<400> 1016
caggctcgtt tgtagccttc 20

<210> 1017
<211> 24
<212> DNA
<213> NAT#122 STM primer

<400> 1017
acagctccaa acctcgctaa acag 24

<210> 1018
<211> 24
<212> DNA
<213> STM common primer

<400> 1018
gcatgccctg cccctaagaa ttcg 24

<210> 1019
<211> 18
<212> DNA
<213> CNAG_06921 5 flanking region primer 1

<400> 1019
gacaaagcaa caacagcg 18

<210> 1020
<211> 37
<212> DNA
<213> CNAG_06921 5 flanking region primer 2

<400> 1020
tcactggccg tcgttttact cggatgacag atggttg 37

<210> 1021
<211> 39
<212> DNA

<213>    CNAG_06921 3 flanking region primer 1

<400>    1021
catggtcata gctgtttcct gaaagattgg gcgtctctg                                39

<210>    1022
<211>    18
<212>    DNA
<213>    CNAG_06921 3 flanking region primer 2

<400>    1022
atcatcagcg acatccag                                                       18

<210>    1023
<211>    18
<212>    DNA
<213>    CNAG_06921 diagnostic screening primer, pairing with B79

<400>    1023
aaagaacata cacgccgc                                                       18

<210>    1024
<211>    18
<212>    DNA
<213>    CNAG_06921 Southern blot probe primer

<400>    1024
accatctgga tttctgcc                                                       18

<210>    1025
<211>    21
<212>    DNA
<213>    NAT#159 STM primer

<400>    1025
acgcaccaga cacacaacca g                                                   21

<210>    1026
<211>    24
<212>    DNA
<213>    STM common primer

<400>    1026
gcatgccctg cccctaagaa ttcg                                                24

<210>    1027
<211>    20
<212>    DNA
<213>    CNAG_02555 5 flanking region primer 1

<400>    1027
agaggaacac ggtatggtcg                                                     20

<210>    1028
<211>    40
<212>    DNA
<213>    CNAG_02555 5 flanking region primer 2

<400>    1028
gctcactggc cgtcgtttta ccaacaacgg tgaaccaaag                              40

<210>    1029
<211>    41
<212>    DNA
<213>    CNAG_02555 3 flanking region primer 1

<400>    1029
catggtcata gctgtttcct gcagagtgga ggtagaaacg g                             41

<210>    1030
<211>    18

<212>    DNA
<213>    CNAG_02555 3 flanking region primer 2

<400>    1030
tctgaaatgc tggctgtc                                                    18


<210>    1031
<211>    20
<212>    DNA
<213>    CNAG_02555 diagnostic screening primer, pairing with B79

<400>    1031
cccctcacaa cttgctaatg                                                  20


<210>    1032
<211>    20
<212>    DNA
<213>    CNAG_02555 Southern blot probe primer

<400>    1032
ccgatgtcga cgtatgattg                                                  20


<210>    1033
<211>    18
<212>    DNA
<213>    NAT#270 STM primer

<400>    1033
ggtggtttgt gtgggtag                                                    18


<210>    1034
<211>    24
<212>    DNA
<213>    STM common primer

<400>    1034

gcatgccctg cccctaagaa ttcg                                                24

<210>    1035
<211>    19
<212>    DNA
<213>    CNAG_03710 5 flanking region primer 1

<400>    1035
aagacaagaa gcagcagcg                                                       19

<210>    1036
<211>    40
<212>    DNA
<213>    CNAG_03710 5 flanking region primer 2

<400>    1036
gctcactggc cgtcgtttta ctatcattcc catccgtcgc                                40

<210>    1037
<211>    42
<212>    DNA
<213>    CNAG_03710 3 flanking region primer 1

<400>    1037
catggtcata gctgtttcct gcgctcaata acatcgtcag ac                             42

<210>    1038
<211>    20
<212>    DNA
<213>    CNAG_03710 3 flanking region primer 2

<400>    1038
ccagccttca tctctcttcc                                                      20

<210>    1039

<211>    20
<212>    DNA
<213>    CNAG_03710 diagnostic screening primer, pairing with B79

<400>    1039
cagaactgat aactggctgc                                                    20

<210>    1040
<211>    20
<212>    DNA
<213>    CNAG_03710 Southern blot probe primer

<400>    1040
tcctctgcac caagactgtg                                                    20

<210>    1041
<211>    24
<212>    DNA
<213>    NAT#290 STM primer

<400>    1041
accgacagct cgaacaagca agag                                               24

<210>    1042
<211>    24
<212>    DNA
<213>    STM common primer

<400>    1042
gcatgccctg cccctaagaa ttcg                                               24

<210>    1043
<211>    20
<212>    DNA
<213>    CNAG_03183 5 flanking region primer 1

<400>    1043
ctctatgagg gcaaagtcag                                                    20

<210>    1044
<211>    40
<212>    DNA
<213>    CNAG_03183 5 flanking region primer 2

<400>    1044
gctcactggc cgtcgtttta ctgttgttgc tgttggctcg                              40

<210>    1045
<211>    40
<212>    DNA
<213>    CNAG_03183 3 flanking region primer 1

<400>    1045
catggtcata gctgtttcct gttcttccaa tcccagccac                              40

<210>    1046
<211>    19
<212>    DNA
<213>    CNAG_03183 3 flanking region primer 2

<400>    1046
tacgcttcac agattacgc                                                     19

<210>    1047
<211>    18
<212>    DNA
<213>    CNAG_03183 diagnostic screening primer, pairing with B79

<400>    1047
tatacgtcca agacatcc                                                      18

<210>    1048
<211>    20
<212>    DNA
<213>    CNAG_03183 Southern blot probe primer

<400>    1048
ctgattccga ggcttactcg                                                    20


<210>    1049
<211>    21
<212>    DNA
<213>    NAT#292 STM primer

<400>    1049
atttcgatgt atatatgtag c                                                  21


<210>    1050
<211>    24
<212>    DNA
<213>    STM common primer

<400>    1050
gcatgccctg cccctaagaa ttcg                                               24


<210>    1051
<211>    20
<212>    DNA
<213>    CNAG_04637 5 flanking region primer 1

<400>    1051
ccttcaagcc aaaatgagac                                                    20


<210>    1052
<211>    40
<212>    DNA
<213>    CNAG_04637 5 flanking region primer 2

<400> 1052
gctcactggc cgtcgtttta cgagatggag agaagcaacg 40

<210> 1053
<211> 43
<212> DNA
<213> CNAG_04637 3 flanking region primer 1

<400> 1053
catggtcata gctgtttcct gggttgacgg atttactgaa gtc 43

<210> 1054
<211> 20
<212> DNA
<213> CNAG_04637 3 flanking region primer 2

<400> 1054
tggaagatga ctcactcggg 20

<210> 1055
<211> 19
<212> DNA
<213> CNAG_04637 diagnostic screening primer, pairing with B79

<400> 1055
tccatcaaag atacacccg 19

<210> 1056
<211> 20
<212> DNA
<213> CNAG_04637 Southern blot probe primer

<400> 1056
ttacatcttg aggcttagcg 20

<210> 1057
<211> 20
<212> DNA
<213> NAT#296 STM primer

<400> 1057
cgcccgccct cactatccac 20

<210> 1058
<211> 24
<212> DNA
<213> STM common primer

<400> 1058
gcatgccctg cccctaagaa ttcg 24

<210> 1059
<211> 19
<212> DNA
<213> CNAG_04398 5 flanking region primer 1

<400> 1059
agtctggaga aaaggggtc 19

<210> 1060
<211> 34
<212> DNA
<213> CNAG_04398 5 flanking region primer 2

<400> 1060
ctggccgtcg ttttacgcaa acacacattc tcgg 34

<210> 1061
<211> 36
<212> DNA

<213>    CNAG_04398 3 flanking region primer 1

<400>    1061
gtcatagctg tttcctgttg gctaatgtag ggttgg                                36

<210>    1062
<211>    18
<212>    DNA
<213>    CNAG_04398 3 flanking region primer 2

<400>    1062
ccccttccca tttttcac                                                    18

<210>    1063
<211>    22
<212>    DNA
<213>    CNAG_04398 diagnostic screening primer, pairing with B79

<400>    1063
gccactttct gatttccgta tc                                               22

<210>    1064
<211>    18
<212>    DNA
<213>    CNAG_04398 Southern blot probe primer

<400>    1064
tatttgctgc ctgaccac                                                    18

<210>    1065
<211>    20
<212>    DNA
<213>    NAT#225 STM primer

<400>    1065
ccatagaact agctaaagca                                                  20

<210> 1066
<211> 24
<212> DNA
<213> STM common primer

<400> 1066
gcatgccctg cccctaagaa ttcg 24

<210> 1067
<211> 20
<212> DNA
<213> CNAG_01454 5 flanking region primer 1

<400> 1067
ggaagtttgg aggaggatag 20

<210> 1068
<211> 37
<212> DNA
<213> CNAG_01454 5 flanking region primer 2

<400> 1068
tcactggccg tcgttttacc agattcgtgg tcaaagc 37

<210> 1069
<211> 40
<212> DNA
<213> CNAG_01454 3 flanking region primer 1

<400> 1069
catggtcata gctgtttcct ggccttgaat gatgaccagc 40

<210> 1070
<211> 18

<212>    DNA
<213>    CNAG_01454 3 flanking region primer 2

<400>    1070
cggatttgag atgatgcc                                                    18

<210>    1071
<211>    22
<212>    DNA
<213>    CNAG_01454 diagnostic screening primer, pairing with B79

<400>    1071
gttagaggag tcattctttg gc                                               22

<210>    1072
<211>    20
<212>    DNA
<213>    CNAG_01454 Southern blot probe primer

<400>    1072
caaaaggtca aggaaaggtg                                                  20

<210>    1073
<211>    24
<212>    DNA
<213>    NAT#58 STM primer

<400>    1073
cgcaaaatca ctagccctat agcg                                             24

<210>    1074
<211>    24
<212>    DNA
<213>    STM common primer

<400>    1074

gcatgccctg cccctaagaa ttcg 24

<210> 1075
<211> 18
<212> DNA
<213> CNAG_04268 5 flanking region primer 1

<400> 1075
tcgtctttgt gtggttgc 18

<210> 1076
<211> 38
<212> DNA
<213> CNAG_04268 5 flanking region primer 2

<400> 1076
tcactggccg tcgttttaca cttatggcga cttacggg 38

<210> 1077
<211> 40
<212> DNA
<213> CNAG_04268 3 flanking region primer 1

<400> 1077
catggtcata gctgtttcct ggcaatgtct gtatcaccgc 40

<210> 1078
<211> 19
<212> DNA
<213> CNAG_04268 3 flanking region primer 2

<400> 1078
cgtccgcctg tattttatc 19

<210> 1079

<211>    18
<212>    DNA
<213>    CNAG_04268 diagnostic screening primer, pairing with B79

<400>    1079
atgatgctgt tttgcccc                                                    18

<210>    1080
<211>    18
<212>    DNA
<213>    CNAG_04268 Southern blot probe primer

<400>    1080
ttctttccac atcggtcc                                                    18

<210>    1081
<211>    24
<212>    DNA
<213>    NAT#102 STM primer

<400>    1081
ccatagcgat atctacccca atct                                             24

<210>    1082
<211>    24
<212>    DNA
<213>    STM common primer

<400>    1082
gcatgccctg cccctaagaa ttcg                                             24

<210>    1083
<211>    22
<212>    DNA
<213>    CNAG_00031 5 flanking region primer 1

<400>    1083
atccaatcat cggcttccca gc                                                22

<210>    1084
<211>    35
<212>    DNA
<213>    CNAG_00031 5 flanking region primer 2

<400>    1084
ctggccgtcg ttttacactt gaactcccat ctgcg                                  35

<210>    1085
<211>    37
<212>    DNA
<213>    CNAG_00031 3 flanking region primer 1

<400>    1085
gtcatagctg tttcctggaa atgcgatgaa tgggttg                                37

<210>    1086
<211>    21
<212>    DNA
<213>    CNAG_00031 3 flanking region primer 2

<400>    1086
cctcttgccc taatgagata g                                                 21

<210>    1087
<211>    18
<212>    DNA
<213>    CNAG_00031 diagnostic screening primer, pairing with B79

<400>    1087
ccgtgttcgc aatctatg                                                     18

<210>    1088
<211>    19
<212>    DNA
<213>    CNAG_00031 Southern blot probe primer

<400>    1088
gaatcagcat ttgggttcg                                                    19

<210>    1089
<211>    20
<212>    DNA
<213>    NAT#116 STM primer

<400>    1089
gcacccaaga gctccatctc                                                   20

<210>    1090
<211>    24
<212>    DNA
<213>    STM common primer

<400>    1090
gcatgccctg cccctaagaa ttcg                                              24

<210>    1091
<211>    19
<212>    DNA
<213>    CNAG_07922 5 flanking region primer 1

<400>    1091
cctcttccgc tattttgtg                                                    19

<210>    1092
<211>    39
<212>    DNA
<213>    CNAG_07922 5 flanking region primer 2

<400>    1092
gctcactggc cgtcgtttta ccattgctgt tgtgggaac                                39

<210>    1093
<211>    41
<212>    DNA
<213>    CNAG_07922 3 flanking region primer 1

<400>    1093
catggtcata gctgtttcct gaagtggagt tggaagaggc g                             41

<210>    1094
<211>    22
<212>    DNA
<213>    CNAG_07922 3 flanking region primer 2

<400>    1094
caactatcca atacctgttc cg                                                  22

<210>    1095
<211>    19
<212>    DNA
<213>    CNAG_07922 diagnostic screening primer, pairing with B79

<400>    1095
cacggcttgt cttgaagtc                                                      19

<210>    1096
<211>    18
<212>    DNA
<213>    CNAG_07922 Southern blot probe primer

<400>    1096
ttgacggttt tggcacag                                                       18

<210> 1097
<211> 20
<212> DNA
<213> NAT#116 STM primer

<400> 1097
gcacccaaga gctccatctc 20

<210> 1098
<211> 24
<212> DNA
<213> STM common primer

<400> 1098
gcatgccctg cccctaagaa ttcg 24

<210> 1099
<211> 19
<212> DNA
<213> CNAG_04841 5 flanking region primer 1

<400> 1099
tgaggtgttc cgtgtattg 19

<210> 1100
<211> 35
<212> DNA
<213> CNAG_04841 5 flanking region primer 2

<400> 1100
ctggccgtcg ttttacctct tgactggtaa tgggg 35

<210> 1101
<211> 35
<212> DNA

<213>    CNAG_04841 3 flanking region primer 1

<400>    1101
gtcatagctg tttcctgatt cccaaggtga cgaag                                35

<210>    1102
<211>    20
<212>    DNA
<213>    CNAG_04841 3 flanking region primer 2

<400>    1102
tctccaataa gttcaggcac                                                 20

<210>    1103
<211>    22
<212>    DNA
<213>    CNAG_04841 diagnostic screening primer, pairing with B79

<400>    1103
taaagacgga ggtgggagta gg                                              22

<210>    1104
<211>    18
<212>    DNA
<213>    CNAG_04841 Southern blot probe primer

<400>    1104
tgagattggg aaagagcg                                                   18

<210>    1105
<211>    24
<212>    DNA
<213>    NAT#119 STM primer

<400>    1105
ctccccacat aaagagagct aaac                                            24

<210>    1106
<211>    24
<212>    DNA
<213>    STM common primer

<400>    1106
gcatgccctg cccctaagaa ttcg                                                24

<210>    1107
<211>    20
<212>    DNA
<213>    CNAG_01948 5 flanking region primer 1

<400>    1107
cttggtaaat ccgtattccg                                                     20

<210>    1108
<211>    35
<212>    DNA
<213>    CNAG_01948 5 flanking region primer 2

<400>    1108
ctggccgtcg ttttaccctt tcgcttcaac ctacg                                    35

<210>    1109
<211>    35
<212>    DNA
<213>    CNAG_01948 3 flanking region primer 1

<400>    1109
gtcatagctg tttcctgtga tttggcgact ctgag                                    35

<210>    1110
<211>    20

<212>    DNA
<213>    CNAG_01948 3 flanking region primer 2

<400>    1110
tgtgtttctc atcgtaagcg                                                    20

<210>    1111
<211>    22
<212>    DNA
<213>    CNAG_01948 diagnostic screening primer, pairing with B79

<400>    1111
ttgacttccc aggttacact ac                                                 22

<210>    1112
<211>    19
<212>    DNA
<213>    CNAG_01948 Southern blot probe primer

<400>    1112
cattgggact cttgtaggg                                                     19

<210>    1113
<211>    24
<212>    DNA
<213>    NAT#119 STM primer

<400>    1113
ctccccacat aaagagagct aaac                                               24

<210>    1114
<211>    24
<212>    DNA
<213>    STM common primer

<400>    1114

gcatgccctg cccctaagaa ttcg 24

<210> 1115
<211> 19
<212> DNA
<213> CNAG_07435 5 flanking region primer 1

<400> 1115
caaccttcaa agtcttgcc 19

<210> 1116
<211> 39
<212> DNA
<213> CNAG_07435 5 flanking region primer 2

<400> 1116
tcactggccg tcgttttaca acgaggagtc atactgtgc 39

<210> 1117
<211> 41
<212> DNA
<213> CNAG_07435 3 flanking region primer 1

<400> 1117
catggtcata gctgtttcct gaaaggcgtc aaagggcgaa g 41

<210> 1118
<211> 20
<212> DNA
<213> CNAG_07435 3 flanking region primer 2

<400> 1118
ggtttggagc gtattgattg 20

<210> 1119

<211>    19
<212>    DNA
<213>    CNAG_07435 diagnostic screening primer, pairing with B79

<400>    1119
aagagcggca ttgaatcgg                                                    19

<210>    1120
<211>    20
<212>    DNA
<213>    CNAG_07435 Southern blot probe primer

<400>    1120
gcgtcacttt ggacatactc                                                   20

<210>    1121
<211>    20
<212>    DNA
<213>    NAT#123 STM primer

<400>    1121
ctatcgacca accaacacag                                                   20

<210>    1122
<211>    24
<212>    DNA
<213>    STM common primer

<400>    1122
gcatgccctg cccctaagaa ttcg                                              24

<210>    1123
<211>    22
<212>    DNA
<213>    CNAG_00505 5 flanking region primer 1

<400> 1123
tttcaggcac caacaggcac ag 22

<210> 1124
<211> 38
<212> DNA
<213> CNAG_00505 5 flanking region primer 2

<400> 1124
ctggccgtcg ttttacttcc gatggtgccc ttgagtag 38

<210> 1125
<211> 38
<212> DNA
<213> CNAG_00505 3 flanking region primer 1

<400> 1125
gtcatagctg tttcctgggt cttccaaatg tcacagtc 38

<210> 1126
<211> 19
<212> DNA
<213> CNAG_00505 3 flanking region primer 2

<400> 1126
aaagaagaga cgcctcacg 19

<210> 1127
<211> 20
<212> DNA
<213> CNAG_00505 diagnostic screening primer, pairing with B79

<400> 1127
tgatagacac cacggcagtc 20

<210>	1128
<211>	20
<212>	DNA
<213>	CNAG_00505 Southern blot probe primer

<400>	1128
cgaggattgt caaatgaagg	20

<210>	1129
<211>	24
<212>	DNA
<213>	NAT#125 STM primer

<400>	1129
cgctacagcc agcgcgcgca agcg	24

<210>	1130
<211>	24
<212>	DNA
<213>	STM common primer

<400>	1130
gcatgccctg cccctaagaa ttcg	24

<210>	1131
<211>	18
<212>	DNA
<213>	CNAG_03115 5 flanking region primer 1

<400>	1131
cgaaggaagt aaaacggg	18

<210>	1132
<211>	37
<212>	DNA
<213>	CNAG_03115 5 flanking region primer 2

<400>    1132
ctggccgtcg ttttacaagc actcaccact ctcctac                                37

<210>    1133
<211>    36
<212>    DNA
<213>    CNAG_03115 3 flanking region primer 1

<400>    1133
gtcatagctg tttcctgtgc ttggatagac gacgag                                 36

<210>    1134
<211>    20
<212>    DNA
<213>    CNAG_03115 3 flanking region primer 2

<400>    1134
tcaatcaaag cgttcggggg                                                   20

<210>    1135
<211>    20
<212>    DNA
<213>    CNAG_03115 diagnostic screening primer, pairing with B79

<400>    1135
aatccgactt ctcagccctg                                                   20

<210>    1136
<211>    20
<212>    DNA
<213>    CNAG_03115 Southern blot probe primer

<400>    1136
atctgaggga agggctacag                                                   20

<210>    1137
<211>    20
<212>    DNA
<213>    NAT#177 STM primer

<400>    1137
caccaactcc ccatctccat                                                    20

<210>    1138
<211>    24
<212>    DNA
<213>    STM common primer

<400>    1138
gcatgccctg cccctaagaa ttcg                                               24

<210>    1139
<211>    19
<212>    DNA
<213>    CNAG_04807 5 flanking region primer 1

<400>    1139
gcgtttcctc gtgaacttc                                                     19

<210>    1140
<211>    37
<212>    DNA
<213>    CNAG_04807 5 flanking region primer 2

<400>    1140
ctggccgtcg ttttacggaa gaagggttga gacactc                                 37

<210>    1141
<211>    36
<212>    DNA

<213>    CNAG_04807 3 flanking region primer 1

<400>    1141
gtcatagctg tttcctgggc aagcataatg gcaaag                                36.

<210>    1142
<211>    18
<212>    DNA
<213>    CNAG_04807 3 flanking region primer 2

<400>    1142
ggatttccga agccaaac                                                    18

<210>    1143
<211>    19
<212>    DNA
<213>    CNAG_04807 diagnostic screening primer, pairing with B79

<400>    1143
tggcacagtt caatgttcc                                                   19

<210>    1144
<211>    18
<212>    DNA
<213>    CNAG_04807 Southern blot probe primer

<400>    1144
cgacaataca aacacgcc                                                    18

<210>    1145
<211>    20
<212>    DNA
<213>    NAT#177 STM primer

<400>    1145
caccaactcc ccatctccat                                                  20

<210>	1146
<211>	24
<212>	DNA
<213>	STM common primer

<400>	1146
gcatgccctg cccctaagaa ttcg	24

<210>	1147
<211>	19
<212>	DNA
<213>	CNAG_02435 5 flanking region primer 1

<400>	1147
atcgttagca gggtgagtc	19

<210>	1148
<211>	42
<212>	DNA
<213>	CNAG_02435 5 flanking region primer 2

<400>	1148
gctcactggc cgtcgtttta ctcctattca gggactatca cc	42

<210>	1149
<211>	39
<212>	DNA
<213>	CNAG_02435 3 flanking region primer 1

<400>	1149
catggtcata gctgtttcct gacctatttt ccacgcagc	39

<210>	1150
<211>	18

<212>    DNA
<213>    CNAG_02435 3 flanking region primer 2

<400>    1150
tcagcctcgc acataatc                                                    18


<210>    1151
<211>    18
<212>    DNA
<213>    CNAG_02435 diagnostic screening primer, pairing with B79

<400>    1151
cgagccgaaa agaacaag                                                    18


<210>    1152
<211>    20
<212>    DNA
<213>    CNAG_02435 Southern blot probe primer

<400>    1152
gattatctgt ttggttcggc                                                  20


<210>    1153
<211>    24
<212>    DNA
<213>    NAT#184 STM primer

<400>    1153
atatatggct cgagctagat agag                                             24


<210>    1154
<211>    24
<212>    DNA
<213>    STM common primer

<400>    1154

gcatgccctg cccctaagaa ttcg 24

<210> 1155
<211> 20
<212> DNA
<213> CNAG_02364 5 flanking region primer 1

<400> 1155
gaagtcaaca ggaagaagcc 20

<210> 1156
<211> 34
<212> DNA
<213> CNAG_02364 5 flanking region primer 2

<400> 1156
ctggccgtcg ttttactttg cgtgaaggtg tagg 34

<210> 1157
<211> 36
<212> DNA
<213> CNAG_02364 3 flanking region primer 1

<400> 1157
gtcatagctg tttcctgtgc tcatcttcac ggctac 36

<210> 1158
<211> 19
<212> DNA
<213> CNAG_02364 3 flanking region primer 2

<400> 1158
cattatccgc ctgatgttg 19

<210> 1159

<211>    19
<212>    DNA
<213>    CNAG_02364 diagnostic screening primer, pairing with B79

<400>    1159
gcacttccaa caaagccac                                                    19

<210>    1160
<211>    19
<212>    DNA
<213>    CNAG_02364 Southern blot probe primer

<400>    1160
gactttttgc gtgaaggtg                                                    19

<210>    1161
<211>    24
<212>    DNA
<213>    NAT#184 STM primer

<400>    1161
atatatggct cgagctagat agag                                              24

<210>    1162
<211>    24
<212>    DNA
<213>    STM common primer

<400>    1162
gcatgccctg cccctaagaa ttcg                                              24

<210>    1163
<211>    20
<212>    DNA
<213>    CNAG_05420 5 flanking region primer 1

<400>    1163
cgcctttgag gtattcactc                                                    20

<210>    1164
<211>    39
<212>    DNA
<213>    CNAG_05420 5 flanking region primer 2

<400>    1164
tcactggccg tcgttttacg gtgttggtca cgaatctac                               39

<210>    1165
<211>    40
<212>    DNA
<213>    CNAG_05420 3 flanking region primer 1

<400>    1165
catggtcata gctgtttcct gcggctttac gaaatgagtg                              40

<210>    1166
<211>    21
<212>    DNA
<213>    CNAG_05420 3 flanking region primer 2

<400>    1166
cctgaaagac atagcgacaa g                                                  21

<210>    1167
<211>    19
<212>    DNA
<213>    CNAG_05420 diagnostic screening primer, pairing with B79

<400>    1167
atcctgtctt gctgtggtc                                                     19

<210> 1168
<211> 18
<212> DNA
<213> CNAG_05420 Southern blot probe primer

<400> 1168
tccagcatcg tcattgag 18

<210> 1169
<211> 20
<212> DNA
<213> NAT#191 STM primer

<400> 1169
atatggatgt ttttagcgag 20

<210> 1170
<211> 24
<212> DNA
<213> STM common primer

<400> 1170
gcatgccctg cccctaagaa ttcg 24

<210> 1171
<211> 19
<212> DNA
<213> CNAG_05093 5 flanking region primer 1

<400> 1171
aatgtattgc caccgtgcc 19

<210> 1172
<211> 41
<212> DNA
<213> CNAG_05093 5 flanking region primer 2

<400>    1172
tcactggccg tcgttttact tggggtgatg tgttctcgtc c                41

<210>    1173
<211>    41
<212>    DNA
<213>    CNAG_05093 3 flanking region primer 1

<400>    1173
catggtcata gctgtttcct gacaggcaga ttgggaagtg c                41

<210>    1174
<211>    19
<212>    DNA
<213>    CNAG_05093 3 flanking region primer 2

<400>    1174
tgtatgccgt gggaacaag                                         19

<210>    1175
<211>    24
<212>    DNA
<213>    CNAG_05093 diagnostic screening primer, pairing with B79

<400>    1175
cgaagtctca aacctatcta ctcg                                   24

<210>    1176
<211>    22
<212>    DNA
<213>    CNAG_05093 Southern blot probe primer

<400>    1176
ttgctgaggc gaacttgttg gc                                     22

<210> 1177
<211> 24
<212> DNA
<213> NAT#201 STM primer

<400> 1177
caccctctat ctcgagaaag ctcc 24

<210> 1178
<211> 24
<212> DNA
<213> STM common primer

<400> 1178
gcatgccctg cccctaagaa ttcg 24

<210> 1179
<211> 19
<212> DNA
<213> CNAG_06814 5 flanking region primer 1

<400> 1179
tgggaatggc aaaaggcac 19

<210> 1180
<211> 35
<212> DNA
<213> CNAG_06814 5 flanking region primer 2

<400> 1180
ctggccgtcg ttttacggta ttttcgtcca gtaaa 35

<210> 1181
<211> 35
<212> DNA

<213>    CNAG_06814 3 flanking region primer 1

<400>    1181
gtcatagctg tttcctgtgc agcctatgcc tcctt                                35

<210>    1182
<211>    19
<212>    DNA
<213>    CNAG_06814 3 flanking region primer 2

<400>    1182
taagtgtgtt cacggtgcg                                                  19

<210>    1183
<211>    19
<212>    DNA
<213>    CNAG_06814 diagnostic screening primer, pairing with B79

<400>    1183
tggttaccga agtagggag                                                  19

<210>    1184
<211>    19
<212>    DNA
<213>    CNAG_06814 Southern blot probe primer

<400>    1184
atttggaggt ggagttctg                                                  19

<210>    1185
<211>    22
<212>    DNA
<213>    NAT#208 STM primer

<400>    1185
tggtcgcggg agatcgtggt tt                                              22

<210> 1186
<211> 24
<212> DNA
<213> STM common primer

<400> 1186
gcatgccctg cccctaagaa ttcg 24

<210> 1187
<211> 21
<212> DNA
<213> CNAG_05431 5 flanking region primer 1

<400> 1187
catcagtctt gcttcttctg c 21

<210> 1188
<211> 40
<212> DNA
<213> CNAG_05431 5 flanking region primer 2

<400> 1188
tcactggccg tcgttttaca gagtcagaat aggtgttggg 40

<210> 1189
<211> 40
<212> DNA
<213> CNAG_05431 3 flanking region primer 1

<400> 1189
catggtcata gctgtttcct gagggagagg attgaagtgg 40

<210> 1190
<211> 20

<212>	DNA
<213>	CNAG_05431 3 flanking region primer 2

<400>	1190
cattactttg actgggaggg	20

<210>	1191
<211>	19
<212>	DNA
<213>	CNAG_05431 diagnostic screening primer, pairing with B79

<400>	1191
cacggcaact tatgctctc	19

<210>	1192
<211>	20
<212>	DNA
<213>	CNAG_05431 Southern blot probe primer

<400>	1192
actcggtgtt ggtgaaacgg	20

<210>	1193
<211>	22
<212>	DNA
<213>	NAT#208 STM primer

<400>	1193
tggtcgcggg agatcgtggt tt	22

<210>	1194
<211>	24
<212>	DNA
<213>	STM common primer

<400>	1194

gcatgccctg cccctaagaa ttcg                                                24

<210>    1195
<211>    20
<212>    DNA
<213>    CNAG_05642 5 flanking region primer 1

<400>    1195
agagggataa gagcctgacg                                                      20

<210>    1196
<211>    38
<212>    DNA
<213>    CNAG_05642 5 flanking region primer 2

<400>    1196
tcactggccg tcgttttaca gaaggaagtt gggaagcg                                  38

<210>    1197
<211>    41
<212>    DNA
<213>    CNAG_05642 3 flanking region primer 1

<400>    1197
catggtcata gctgtttcct gatgattaca gaggggagga c                              41

<210>    1198
<211>    19
<212>    DNA
<213>    CNAG_05642 3 flanking region primer 2

<400>    1198
tgtatcccaa gcagcaaag                                                       19

<210>    1199

<211>	20
<212>	DNA
<213>	CNAG_05642 diagnostic screening primer, pairing with B79

<400>	1199
ctcggaaata aatgtggagc	20

<210>	1200
<211>	20
<212>	DNA
<213>	CNAG_05642 Southern blot probe primer

<400>	1200
tacctgccag tgcttcaacc	20

<210>	1201
<211>	20
<212>	DNA
<213>	NAT#210 STM primer

<400>	1201
ctagagcccg ccacaacgct	20

<210>	1202
<211>	24
<212>	DNA
<213>	STM common primer

<400>	1202
gcatgccctg cccctaagaa ttcg	24

<210>	1203
<211>	19
<212>	DNA
<213>	CNAG_03212 5 flanking region primer 1

<400> 1203
gacacccaaa caagcaaac 19

<210> 1204
<211> 39
<212> DNA
<213> CNAG_03212 5 flanking region primer 2

<400> 1204
gctcactggc cgtcgtttta cctggggtgt ttgttttcg 39

<210> 1205
<211> 41
<212> DNA
<213> CNAG_03212 3 flanking region primer 1

<400> 1205
catggtcata gctgtttcct ggggttcaga ttttggatac c 41

<210> 1206
<211> 19
<212> DNA
<213> CNAG_03212 3 flanking region primer 2

<400> 1206
ggcgaggtgt tgtaaatcc 19

<210> 1207
<211> 22
<212> DNA
<213> CNAG_03212 diagnostic screening primer, pairing with B79

<400> 1207
gcgtatctgg catcttgtta tc 22

<210> 1208
<211> 18
<212> DNA
<213> CNAG_03212 Southern blot probe primer

<400> 1208
tgaggaatgc caatggtg 18

<210> 1209
<211> 20
<212> DNA
<213> NAT#211 STM primer

<400> 1209
gcggtcgctt tatagcgatt 20

<210> 1210
<211> 24
<212> DNA
<213> STM common primer

<400> 1210
gcatgccctg cccctaagaa ttcg 24

<210> 1211
<211> 18
<212> DNA
<213> CNAG_03212 5 flanking region primer 1

<400> 1211
gcgaaacttt gtgagtgc 18

<210> 1212
<211> 40
<212> DNA
<213> CNAG_03212 5 flanking region primer 2

<400>    1212
gctcactggc cgtcgtttta ccaagcagca agatggagtc                40

<210>    1213
<211>    40
<212>    DNA
<213>    CNAG_03212 3 flanking region primer 1

<400>    1213
catggtcata gctgtttcct gtcgtaagtt cggtgtcagc                40

<210>    1214
<211>    20
<212>    DNA
<213>    CNAG_03212 3 flanking region primer 2

<400>    1214
aagtcttatt atgggcgagg                                      20

<210>    1215
<211>    19
<212>    DNA
<213>    CNAG_03212 diagnostic screening primer, pairing with B79

<400>    1215
tgtcccgttg ataggtgtc                                       19

<210>    1216
<211>    20
<212>    DNA
<213>    CNAG_03212 Southern blot probe primer

<400>    1216
tcctctccct cctattcagg                                      20

<210> 1217
<211> 20
<212> DNA
<213> NAT#211 STM primer

<400> 1217
gcggtcgctt tatagcgatt 20

<210> 1218
<211> 24
<212> DNA
<213> STM common primer

<400> 1218
gcatgccctg cccctaagaa ttcg 24

<210> 1219
<211> 19
<212> DNA
<213> CNAG_07924 5 flanking region primer 1

<400> 1219
acctaatcag gaaagcccc 19

<210> 1220
<211> 39
<212> DNA
<213> CNAG_07924 5 flanking region primer 2

<400> 1220
tcactggccg tcgttttacc attccttctc cgaaatctc 39

<210> 1221
<211> 40
<212> DNA

<213>    CNAG_07924 3 flanking region primer 1

<400>    1221
catggtcata gctgtttcct gttatcctcc cgaataccgc                                40

<210>    1222
<211>    18
<212>    DNA
<213>    CNAG_07924 3 flanking region primer 2

<400>    1222
ccgcaccaaa ctatttcg                                                        18

<210>    1223
<211>    18
<212>    DNA
<213>    CNAG_07924 diagnostic screening primer, pairing with B79

<400>    1223
atcgcacgaa aggcaaag                                                        18

<210>    1224
<211>    20
<212>    DNA
<213>    CNAG_07924 Southern blot probe primer

<400>    1224
tctaaacccg tctctgactg                                                      20

<210>    1225
<211>    20
<212>    DNA
<213>    NAT#218 STM primer

<400>    1225
ctccacatcc atcgctccaa                                                      20

<210> 1226
<211> 24
<212> DNA
<213> STM common primer

<400> 1226
gcatgccctg cccctaagaa ttcg 24

<210> 1227
<211> 22
<212> DNA
<213> CNAG_00156 5 flanking region primer 1

<400> 1227
cccgattttg ctttttagtg cg 22

<210> 1228
<211> 36
<212> DNA
<213> CNAG_00156 5 flanking region primer 2

<400> 1228
ctggccgtcg ttttacttgc cgtctttgct tgggtc 36

<210> 1229
<211> 36
<212> DNA
<213> CNAG_00156 3 flanking region primer 1

<400> 1229
gtcatagctg tttcctgatc aagtccactc ccgaag 36

<210> 1230
<211> 19

<212>    DNA
<213>    CNAG_00156 3 flanking region primer

<400>    1230
tagatgatga gggcgaacg                                                    19

<210>    1231
<211>    20
<212>    DNA
<213>    CNAG_00156 diagnostic screening primer, pairing with B79

<400>    1231
gcttcgttgg ttcgttagtc                                                   20

<210>    1232
<211>    20
<212>    DNA
<213>    CNAG_00156 Southern blot probe primer

<400>    1232
caatccgttt ctctcacctc                                                   20

<210>    1233
<211>    20
<212>    DNA
<213>    NAT#231 STM primer

<400>    1233
gagagatccc aacatcacgc                                                   20

<210>    1234
<211>    24
<212>    DNA
<213>    STM common primer

<400>    1234

gcatgccctg cccctaagaa ttcg                                                24

<210>    1235
<211>    19
<212>    DNA
<213>    CNAG_01173 5 flanking region primer 1

<400>    1235
agtatcccaa gcatccctc                                                      19

<210>    1236
<211>    39
<212>    DNA
<213>    CNAG_01173 5 flanking region primer 2

<400>    1236
tcactggccg tcgttttacg gcaggagcaa agtgtattc                                39

<210>    1237
<211>    39
<212>    DNA
<213>    CNAG_01173 3 flanking region primer 1

<400>    1237
catggtcata gctgtttcct gtatggtggc atctgaacc                                39

<210>    1238
<211>    20
<212>    DNA
<213>    CNAG_01173 3 flanking region primer 2

<400>    1238
ctgacaagtt taccaggacg                                                     20

<210>    1239

<211>    20
<212>    DNA
<213>    CNAG_01173 diagnostic screening primer, pairing with B79

<400>    1239
gcttctgtgt gtctcctctg                                                    20

<210>    1240
<211>    20
<212>    DNA
<213>    CNAG_01173 Southern blot probe primer

<400>    1240
gagaaagaag gattccgaag                                                    20

<210>    1241
<211>    24
<212>    DNA
<213>    NAT#242 STM primer

<400>    1241
gtagcgatag gggtgtcgct ttag                                               24

<210>    1242
<211>    24
<212>    DNA
<213>    STM common primer

<400>    1242
gcatgccctg cccctaagaa ttcg                                               24

<210>    1243
<211>    19
<212>    DNA
<213>    CNAG_06871 5 flanking region primer 1

<400>    1243
tcaggatggg agtgtttgc                                                    19

<210>    1244
<211>    37
<212>    DNA
<213>    CNAG_06871 5 flanking region primer 2

<400>    1244
tcactggccg tcgttttacc cttctccttt tgcttgg                                37

<210>    1245
<211>    41
<212>    DNA
<213>    CNAG_06871 3 flanking region primer 1

<400>    1245
catggtcata gctgtttcct gcgctcttat taccgcttca g                           41

<210>    1246
<211>    20
<212>    DNA
<213>    CNAG_06871 3 flanking region primer 2

<400>    1246
tcggtatttc agtagggatg                                                   20

<210>    1247
<211>    19
<212>    DNA
<213>    CNAG_06871 diagnostic screening primer, pairing with B79

<400>    1247
cgactgctgg atgtatgag                                                    19

<210>    1248
<211>    18
<212>    DNA
<213>    CNAG_06871 Southern blot probe primer

<400>    1248
cttgactgcc tcatctgc                                                    18

<210>    1249
<211>    21
<212>    DNA
<213>    NAT#295 STM primer

<400>    1249
acacctacat caaaccctcc c                                                21

<210>    1250
<211>    24
<212>    DNA
<213>    STM common primer

<400>    1250
gcatgccctg cccctaagaa ttcg                                             24

<210>    1251
<211>    18
<212>    DNA
<213>    CNAG_04774 5 flanking region primer 1

<400>    1251
atttgaatag ccgcaccc                                                    18

<210>    1252
<211>    35
<212>    DNA
<213>    CNAG_04774 5 flanking region primer 2

<400>    1252
ctggccgtcg ttttacattg ggggatgctg gaaag                                35

<210>    1253
<211>    36
<212>    DNA
<213>    CNAG_04774 3 flanking region primer 1

<400>    1253
gtcatagctg tttcctgggt ggactgccaa actatg                               36

<210>    1254
<211>    19
<212>    DNA
<213>    CNAG_04774 3 flanking region primer 2

<400>    1254
aggcatttct gttcctccg                                                  19

<210>    1255
<211>    19
<212>    DNA
<213>    CNAG_04774 diagnostic screening primer, pairing with B79

<400>    1255
cgatagacgg aggatgaag                                                  19

<210>    1256
<211>    18
<212>    DNA
<213>    CNAG_04774 Southern blot probe primer

<400>    1256
atcgttgctt ccgcttag                                                   18

<210>    1257
<211>    21
<212>    DNA
<213>    NAT#146 STM primer

<400>    1257
actagccccc cctcaccacc t                                                21

<210>    1258
<211>    24
<212>    DNA
<213>    STM common primer

<400>    1258
gcatgccctg cccctaagaa ttcg                                             24

<210>    1259
<211>    18
<212>    DNA
<213>    CNAG_00841 5 flanking region primer 1

<400>    1259
tcggcaagtt tagcaagc                                                    18

<210>    1260
<211>    39
<212>    DNA
<213>    CNAG_00841 5 flanking region primer 2

<400>    1260
gctcactggc cgtcgtttta cttatttctg ggctgtcgc                             39

<210>    1261
<211>    40
<212>    DNA

<213>    CNAG_00841 3 flanking region primer 1

<400>    1261
catggtcata gctgtttcct gtctgtggag tcagccaaac                                40

<210>    1262
<211>    23
<212>    DNA
<213>    CNAG_00841 3 flanking region primer 2

<400>    1262
cggtatcctc gtgagtctat ttc                                                  23

<210>    1263
<211>    21
<212>    DNA
<213>    CNAG_00841 diagnostic screening primer, pairing with B79

<400>    1263
gcagttgctg aagtgaaaga c                                                    21

<210>    1264
<211>    20
<212>    DNA
<213>    CNAG_00841 Southern blot probe primer

<400>    1264
tgggatagaa gaaggctgtg                                                      20

<210>    1265
<211>    24
<212>    DNA
<213>    NAT#205 STM primer

<400>    1265
tatccccctc tccgctctct agca                                                 24

<210> 1266
<211> 24
<212> DNA
<213> STM common primer

<400> 1266
gcatgccctg cccctaagaa ttcg 24

<210> 1267
<211> 18
<212> DNA
<213> CNAG_04836 5 flanking region primer 1

<400> 1267
tagaaaagcc acaacccc 18

<210> 1268
<211> 37
<212> DNA
<213> CNAG_04836 5 flanking region primer 2

<400> 1268
tcactggccg tcgttttacg agtttctttt ggggtgg 37

<210> 1269
<211> 40
<212> DNA
<213> CNAG_04836 3 flanking region primer 1

<400> 1269
catggtcata gctgtttcct gacgagaagg ggaatgatgg 40

<210> 1270
<211> 19

<212>	DNA
<213>	CNAG_04836 3 flanking region primer 2

<400>	1270
tgggcatctt gctcattac	19

<210>	1271
<211>	19
<212>	DNA
<213>	CNAG_04836 diagnostic screening primer, pairing with B79

<400>	1271
cggagagcaa atagcacag	19

<210>	1272
<211>	20
<212>	DNA
<213>	CNAG_04836 Southern blot probe primer

<400>	1272
aggtgtcgta tcgtatttgc	20

<210>	1273
<211>	20
<212>	DNA
<213>	NAT#123 STM primer

<400>	1273
ctatcgacca accaacacag	20

<210>	1274
<211>	24
<212>	DNA
<213>	STM common primer

<400>	1274

gcatgccctg cccctaagaa ttcg                                                24

<210>    1275
<211>    19
<212>    DNA
<213>    CNAG_02603 5 flanking region primer 1

<400>    1275
tcaaaacgcc tttcggaac                                                       19

<210>    1276
<211>    40
<212>    DNA
<213>    CNAG_02603 5 flanking region primer 2

<400>    1276
gctcactggc cgtcgtttta cacaaatggc agttccctcg                                40

<210>    1277
<211>    43
<212>    DNA
<213>    CNAG_02603 3 flanking region primer 1

<400>    1277
catggtcata gctgtttcct gatctctccc tcacttcagt cgg                           43

<210>    1278
<211>    19
<212>    DNA
<213>    CNAG_02603 3 flanking region primer 2

<400>    1278
taggaccaac ttcagaccg                                                       19

<210>    1279

<211>    20
<212>    DNA
<213>    CNAG_02603 diagnostic screening primer, pairing with B79

<400>    1279
gcctatggaa gaagacaacg                                                    20

<210>    1280
<211>    20
<212>    DNA
<213>    CNAG_02603 Southern blot probe primer

<400>    1280
gtcgtgtagt tttgaatggc                                                    20

<210>    1281
<211>    19
<212>    DNA
<213>    NAT#224 STM primer

<400>    1281
aacctttaaa tgggtagag                                                     19

<210>    1282
<211>    24
<212>    DNA
<213>    STM common primer

<400>    1282
gcatgccctg cccctaagaa ttcg                                               24

<210>    1283
<211>    20
<212>    DNA
<213>    CNAG_03059 5 flanking region primer 1

<400>    1283
aatggcgtta ccgacgagag                                                    20

<210>    1284
<211>    36
<212>    DNA
<213>    CNAG_03059 5 flanking region primer 2

<400>    1284
ctggccgtcg ttttacggta ttgagagtgc cgagtg                                  36

<210>    1285
<211>    38
<212>    DNA
<213>    CNAG_03059 3 flanking region primer 1

<400>    1285
gtcatagctg tttcctgcag actccctctt cttacctg                                38

<210>    1286
<211>    24
<212>    DNA
<213>    CNAG_03059 3 flanking region primer 2

<400>    1286
cgttactgcg aggttattac tgtg                                               24

<210>    1287
<211>    19
<212>    DNA
<213>    CNAG_03059 diagnostic screening primer, pairing with B79

<400>    1287
aaagactcac ccttcaacg                                                     19

<210> 1288
<211> 20
<212> DNA
<213> CNAG_03059 Southern blot probe primer

<400> 1288
   cttttttccg ctggtttctc 20

<210> 1289
<211> 18
<212> DNA
<213> NAT#232 STM primer

<400> 1289
ctttaaaggt ggtttgtg 18

<210> 1290
<211> 24
<212> DNA
<213> STM common primer

<400> 1290
gcatgccctg cccctaagaa ttcg 24

<210> 1291
<211> 21
<212> DNA
<213> CNAG_00883 5 flanking region primer 1

<400> 1291
cgacagaaac aagtccaagt g 21

<210> 1292
<211> 36
<212> DNA
<213> CNAG_00883 5 flanking region primer 2

<400>    1292
ctggccgtcg ttttacgtag tgaaatgagg attgcg                                36

<210>    1293
<211>    35
<212>    DNA
<213>    CNAG_00883 3 flanking region primer 1

<400>    1293
gtcatagctg tttcctgtac ctgcttttct tgcgg                                 35

<210>    1294
<211>    18
<212>    DNA
<213>    CNAG_00883 3 flanking region primer 2

<400>    1294
acaagcccga atctcaag                                                    18

<210>    1295
<211>    19
<212>    DNA
<213>    CNAG_00883 diagnostic screening primer, pairing with B79

<400>    1295
cttttcactc aatgtcccg                                                   19

<210>    1296
<211>    19
<212>    DNA
<213>    CNAG_00883 Southern blot probe primer

<400>    1296
taagaactgt tgccctgcc                                                   19

<210>    1297
<211>    18
<212>    DNA
<213>    CNAG_01551 5 flanking region primer 1

<400>    1297
tccgttgaga tagcgttg                                                    18

<210>    1298
<211>    39
<212>    DNA
<213>    CNAG_01551 5 flanking region primer 2

<400>    1298
tcactggccg tcgttttaca tggtggaggt gtaggactg                             39

<210>    1299
<211>    39
<212>    DNA
<213>    CNAG_01551 3 flanking region primer 1

<400>    1299
catggtcata gctgtttcct gttattcccc ctcaagagc                             39

<210>    1300
<211>    20
<212>    DNA
<213>    CNAG_01551 3 flanking region primer 2

<400>    1300
gcggcccatt cttcttgtcg                                                  20

<210>    1301
<211>    18
<212>    DNA

<213>    CNAG_01551 diagnostic screening primer, pairing with B79

<400>    1301
aaccttgcct aacaaccc                                                    18

<210>    1302
<211>    20
<212>    DNA
<213>    CNAG_01551 Southern blot probe primer

<400>    1302
ggagtatggc tgaaatctgg                                                  20

<210>    1303
<211>    24
<212>    DNA
<213>    NAT#273 STM primer

<400>    1303
gagatctttc gggaggtctg gatt                                             24

<210>    1304
<211>    24
<212>    DNA
<213>    STM common primer

<400>    1304
gcatgccctg cccctaagaa ttcg                                             24

<210>    1305
<211>    20
<212>    DNA
<213>    CNAG_04353 5 flanking region primer 1

<400>    1305
tgcgagagag aatgggagac                                                  20

<210> 1306
<211> 38
<212> DNA
<213> CNAG_04353 5 flanking region primer 2

<400> 1306
tcactggccg tcgttttacg gttccatagc gacatcag 38

<210> 1307
<211> 41
<212> DNA
<213> CNAG_04353 3 flanking region primer 1

<400> 1307
catggtcata gctgtttcct gcgaaggttg tttgtgatac g 41

<210> 1308
<211> 20
<212> DNA
<213> CNAG_04353 3 flanking region primer 2

<400> 1308
cccaacaact catcaatctc 20

<210> 1309
<211> 18
<212> DNA
<213> CNAG_04353 diagnostic screening primer, pairing with B79

<400> 1309
gcattatcgt ttcccgac 18

<210> 1310
<211> 22

<212>    DNA
<213>    CNAG_04353 Southern blot probe primer

<400>    1310
ttgaggtagg tggagtgttg cc                                                22

<210>    1311
<211>    20
<212>    DNA
<213>    CNAG_03346 5 flanking region primer 1

<400>    1311
cgccctttct attgttacac                                                   20

<210>    1312
<211>    39
<212>    DNA
<213>    CNAG_03346 5 flanking region primer 2

<400>    1312
tcactggccg tcgttttacg atgacaggag ggatgaatc                              39

<210>    1313
<211>    43
<212>    DNA
<213>    CNAG_03346 3 flanking region primer 1

<400>    1313
catggtcata gctgtttcct ggggagaata acgactcaat gtc                         43

<210>    1314
<211>    18
<212>    DNA
<213>    CNAG_03346 3 flanking region primer 2

<400>    1314

tcattgctga ctgggaag 18

<210> 1315
<211> 18
<212> DNA
<213> CNAG_03346 diagnostic screening primer, pairing with B79

<400> 1315
aaagaggcgg tgttgaag 18

<210> 1316
<211> 19
<212> DNA
<213> CNAG_03346 Southern blot probe primer

<400> 1316
agccaggtaa tcttggagg 19

<210> 1317
<211> 21
<212> DNA
<213> NAT#295 STM primer

<400> 1317
acacctacat caaaccctcc c 21

<210> 1318
<211> 24
<212> DNA
<213> STM common primer

<400> 1318
gcatgccctg cccctaagaa ttcg 24

<210> 1319

<211>    18
<212>    DNA
<213>    CNAG_00239 5 flanking region primer 1

<400>    1319
aggattatcg cctgaacc                                                    18


<210>    1320
<211>    37
<212>    DNA
<213>    CNAG_00239 5 flanking region primer 2

<400>    1320
tcactggccg tcgttttaca tgttgttgaa gagggcg                               37


<210>    1321
<211>    41
<212>    DNA
<213>    CNAG_00239 3 flanking region primer 1

<400>    1321
catggtcata gctgtttcct gaacgacgac catcgcagta g                          41


<210>    1322
<211>    18
<212>    DNA
<213>    CNAG_00239 3 flanking region primer 2

<400>    1322
catcccgagt tggaaaac                                                    18


<210>    1323
<211>    20
<212>    DNA
<213>    CNAG_00239 diagnostic screening primer, pairing with B79

<400>    1323
cactggcgaa gtcaagtatg                                                    20

<210>    1324
<211>    20
<212>    DNA
<213>    CNAG_00239 Southern blot probe primer

<400>    1324
ctactctgtg gtggcgtaag                                                    20

<210>    1325
<211>    20
<212>    DNA
<213>    NAT#296 STM primer

<400>    1325
cgcccgccct cactatccac                                                    20

<210>    1326
<211>    24
<212>    DNA
<213>    STM common primer

<400>    1326
gcatgccctg cccctaagaa ttcg                                               24

<210>    1327
<211>    19
<212>    DNA
<213>    CNAG_03129 5 flanking region primer 1

<400>    1327
gcaggcgaaa actattagg                                                     19

<210> 1328
<211> 41
<212> DNA
<213> CNAG_03129 5 flanking region primer 2

<400> 1328
gctcactggc cgtcgtttta cgtggggaag tcctcaaata c 41

<210> 1329
<211> 42
<212> DNA
<213> CNAG_03129 3 flanking region primer 1

<400> 1329
catggtcata gctgtttcct gcgacgacat ctcaagtcag ag 42

<210> 1330
<211> 20
<212> DNA
<213> CNAG_03129 3 flanking region primer 2

<400> 1330
tgatgggacg ataaaggttc 20

<210> 1331
<211> 19
<212> DNA
<213> CNAG_03129 diagnostic screening primer, pairing with B79

<400> 1331
tgctttctct aatagcggc 19

<210> 1332
<211> 19
<212> DNA
<213> CNAG_03129 Southern blot probe primer

<400>    1332
gtgcttttga agttccgtc                                                    19

<210>    1333
<211>    18
<212>    DNA
<213>    CNAG_03826 5 flanking region primer 1

<400>    1333
aatgctgatt tggcaggg                                                     18

<210>    1334
<211>    39
<212>    DNA
<213>    CNAG_03826 5 flanking region primer 2

<400>    1334
tcactggccg tcgttttacc gatggaagat gttgtcagc                              39

<210>    1335
<211>    39
<212>    DNA
<213>    CNAG_03826 3 flanking region primer 1

<400>    1335
catggtcata gctgtttcct gacaaagcga aaggcagac                              39

<210>    1336
<211>    18
<212>    DNA
<213>    CNAG_03826 3 flanking region primer 2

<400>    1336
cgaaagtgat gcttcagc                                                     18

<210>    1337
<211>    20
<212>    DNA
<213>    CNAG_03826 diagnostic screening primer, pairing with B79

<400>    1337
ggaaggtcag gtcctatttg                                                    20

<210>    1338
<211>    20
<212>    DNA
<213>    CNAG_02671 5 flanking region primer 1

<400>    1338
cgccgagtta tctgacaaag                                                    20

<210>    1339
<211>    41
<212>    DNA
<213>    CNAG_02671 5 flanking region primer 2

<400>    1339
tcactggccg tcgttttact tctccaaaca ccgcccttga c                            41

<210>    1340
<211>    40
<212>    DNA
<213>    CNAG_02671 3 flanking region primer 1

<400>    1340
catggtcata gctgtttcct gtaacggggc ttagaagtgc                              40

<210>    1341
<211>    18
<212>    DNA

<213>    CNAG_02671 3 flanking region primer 2

<400>    1341
ttcatcaaag gtctggcg                                                    18

<210>    1342
<211>    20
<212>    DNA
<213>    CNAG_02671 diagnostic screening primer, pairing with B79

<400>    1342
cctgactcat cggcttactc                                                  20

<210>    1343
<211>    18
<212>    DNA
<213>    CNAG_02671 Southern blot probe primer

<400>    1343
actcctgctt gcgttttc                                                    18

<210>    1344
<211>    20
<212>    DNA
<213>    CNAG_02134 5 flanking region primer 1

<400>    1344
ggggtaacta tcatcatccg                                                  20

<210>    1345
<211>    38
<212>    DNA
<213>    CNAG_02134 5 flanking region primer 2

<400>    1345
tcactggccg tcgttttact ctcgctcctt ttccttcc                              38

<210> 1346
<211> 42
<212> DNA
<213> CNAG_02134 3 flanking region primer 1

<400> 1346
catggtcata gctgtttcct ggcagtagaa ggtgatacaa cg 42

<210> 1347
<211> 19
<212> DNA
<213> CNAG_02134 3 flanking region primer 2

<400> 1347
cgttacggtt ggacaatgg 19

<210> 1348
<211> 19
<212> DNA
<213> CNAG_02134 diagnostic screening primer, pairing with B79

<400> 1348
cgtagaaaca acgcacgag 19

<210> 1349
<211> 21
<212> DNA
<213> CNAG_02134 Southern blot probe primer

<400> 1349
caagtattct ccagggttca g 21

<210> 1350
<211> 20

<212>    DNA
<213>    CNAG_02936 5 flanking region primer 1

<400>    1350
gcgagtgatg acgagagttc                                                    20

<210>    1351
<211>    39
<212>    DNA
<213>    CNAG_02936 5 flanking region primer 2

<400>    1351
tcactggccg tcgttttacg ccttgaccgt ctcttctac                               39

<210>    1352
<211>    42
<212>    DNA
<213>    CNAG_02936 3 flanking region primer 1

<400>    1352
catggtcata gctgtttcct ggtctactcg tcactttcat cg                           42

<210>    1353
<211>    21
<212>    DNA
<213>    CNAG_02936 3 flanking region primer 2

<400>    1353
cgtcacaaca agcactctca c                                                  21

<210>    1354
<211>    19
<212>    DNA
<213>    CNAG_02936 diagnostic screening primer, pairing with B79

<400>    1354

ggatgttcag gtgccaatc 19

<210> 1355
<211> 18
<212> DNA
<213> CNAG_07460 5 flanking region primer 1

<400> 1355
tcggcattgg catacttg 18

<210> 1356
<211> 39
<212> DNA
<213> CNAG_07460 5 flanking region primer 2

<400> 1356
gctcactggc cgtcgtttta cgtgatttga gcggtgaag 39

<210> 1357
<211> 42
<212> DNA
<213> CNAG_07460 3 flanking region primer 1

<400> 1357
catggtcata gctgtttcct gccaaagtgg actcaaatag gg 42

<210> 1358
<211> 22
<212> DNA
<213> CNAG_07460 3 flanking region primer 2

<400> 1358
ccgttacttt ccaaatcagg ag 22

<210> 1359

<211>    20
<212>    DNA
<213>    CNAG_07460 diagnostic screening primer, pairing with B79

<400>    1359
cgtctgaacc tgattctgag                                                    20


<210>    1360
<211>    19
<212>    DNA
<213>    CNAG_07460 Southern blot probe primer

<400>    1360
ccgatgacca aaagtaggc                                                     19


<210>    1361
<211>    19
<212>    DNA
<213>    CNAG_07460 Southern blot probe primer

<400>    1361
cagagttgat tgccgaaag                                                     19


<210>    1362
<211>    19
<212>    DNA
<213>    CNAG_05436 5 flanking region primer 1

<400>    1362
gcccttttgc tttaccttc                                                     19


<210>    1363
<211>    41
<212>    DNA
<213>    CNAG_05436 5 flanking region primer 2

<400>    1363
tcactggccg tcgttttacc gtagagggat gattttgaga c                41

<210>    1364
<211>    40
<212>    DNA
<213>    CNAG_05436 3 flanking region primer 1

<400>    1364
catggtcata gctgtttcct ggcatccttc gtgctaacag                40

<210>    1365
<211>    18
<212>    DNA
<213>    CNAG_05436 3 flanking region primer 2

<400>    1365
gccttgaaga acaaacgg                18

<210>    1366
<211>    19
<212>    DNA
<213>    CNAG_05436 diagnostic screening primer, pairing with B79

<400>    1366
gggataggag gcattgatg                19

<210>    1367
<211>    20
<212>    DNA
<213>    CNAG_05436 Southern blot probe primer

<400>    1367
caccaaagtg gatttccttg                20

<210>	1368
<211>	21
<212>	DNA
<213>	CNAG_05436 Southern blot probe primer

<400>	1368
ctcggacaaa tcactctact g	21

<210>	1369
<211>	21
<212>	DNA
<213>	CNAG_02698 5 flanking region primer 1

<400>	1369
ctacagataa tgtgctcagc c	21

<210>	1370
<211>	37
<212>	DNA
<213>	CNAG_02698 5 flanking region primer 2

<400>	1370
tcactggccg tcgttttact ttgttccata gccagcg	37

<210>	1371
<211>	40
<212>	DNA
<213>	CNAG_02698 3 flanking region primer 1

<400>	1371
catggtcata gctgtttcct ggttctgtct tgaagggtgc	40

<210>	1372
<211>	20
<212>	DNA
<213>	CNAG_02698 3 flanking region primer 2

<400>    1372
cggtgaagtt ggctaagaac                                                    20

<210>    1373
<211>    19
<212>    DNA
<213>    CNAG_02698 diagnostic screening primer, pairing with B79

<400>    1373
gaaagggcta agaagcgac                                                     19

<210>    1374
<211>    20
<212>    DNA
<213>    CNAG_02698 Southern blot probe primer

<400>    1374
aaactctcca tctgtgtccc                                                    20

<210>    1375
<211>    20
<212>    DNA
<213>    CNAG_04600 5 flanking region primer 1

<400>    1375
gctgttggct gattgtttac                                                    20

<210>    1376
<211>    39
<212>    DNA
<213>    CNAG_04600 5 flanking region primer 2

<400>    1376
tcactggccg tcgttttact tacagtatcc caccgtccc                               39

<210> 1377
<211> 40
<212> DNA
<213> CNAG_04600 3 flanking region primer 1

<400> 1377
catggtcata gctgtttcct gtgggagtga tgtctttggc 40

<210> 1378
<211> 18
<212> DNA
<213> CNAG_04600 3 flanking region primer 2

<400> 1378
ccctcaaaag ttgcgaag 18

<210> 1379
<211> 18
<212> DNA
<213> CNAG_04600 diagnostic screening primer, pairing with B79

<400> 1379
ctggattttg gaatgggc 18

<210> 1380
<211> 18
<212> DNA
<213> CNAG_04600 Southern blot probe primer

<400> 1380
ttacatttgc cagcggac 18

## Description

### [Technical Field]

The present invention relates to novel genes that regulate the virulence of a *Cryptococcus neoformans* strain, and to the use thereof. Moreover, the present invention relates to a method for screening an antifungal agent and a method for screening an agent for treating meningitis, in which the methods comprise measuring the expression of genes that are involved in regulation of the virulence of a *Cryptococcus neoformans* strain.

### [Background Art]

In the past, fungal infections were mainly topical infections such as athlete's foot, jock itch, or oral thrush, and rarely systemic infections. However, recently, systemic infections have frequently occurred such that they accounted for a high frequency of total infections in hospitals.

Antifungal agents developed so far can be largely classified according to chemical structure into two groups: those having an azole structure, and those having no azole structure. The azole-based antifungal agents include ketoconazole, fluconazole, itraconazole, voriconazole and the like, and the non-azole-based antifungal agents include terbinafine, flucytosine, amphotericin B, caspofungin and the like.

Ketoconazole, fluconazole, itraconazole and voriconazole, which have an azole structure, and allylamine-based antifungal agents, such as naftifine and terbinafine, have similar mechanisms of action. These two classes of antifungal agents act to inhibit enzymes required in the process in which lanosterol is converted into ergosterol that is the main component of the fungal cell membrane. The azole-based antifungal agents inhibit microsomal enzymes, and the allylamine-based antifungal agents inhibit squalene epoxidase, thereby exhibiting the above-described effect. Flucytocin (5-FC) is a metabolic antagonist that inhibits nucleic acid synthesis, exhibits antifungal activity by non-competitively antagonizing the overlapping coding of fungal RNA and DNA synthesis. Amphotericin B having a polyene structure exhibits antifungal activity by binding to ergosterol in the fungal cell membrane to induce depolarization of the cell membrane and forming a hole to induce loss of intracellular inclusions. Caspofungin, an echinocandin-based antifungal agent, has an activity of reversibly inhibiting fungal cell wall formation, and differs from the above-mentioned antifungal agents, which act on the cell membrane, in that it acts on the cell wall. The azole-based drug, when administered to patients with reduced liver function, may cause death by hepatitis, and for this reason, a liver function test should precede administration of the azole-based drug. It was reported that flutocytosin has dose-dependent bone marrow suppression and liver toxicity, and can cause enterocolitis. Such side effects further increase when renal function is reduced, and for this reason, monitoring of renal function in patients is very important. In addition, flutocytosin is contraindicated for pregnant women. The major toxicity of amphotericin B is glomerular nephrotoxicity resulting from renal artery vasoconstriction, which is dose-dependent. Thus, when the total cumulative dose of amphotericin B is 4 to 5 g, the possibility of permanent renal function impairment will increase. Furthermore, nephrotoxicity, including the excessive loss of potassium, magnesium and bicarbonate caused by renal tubular toxicity, and a decrease in erythropoietin production, may occur. In addition, as acute responses, symptoms, including thrombophlebitis, rigor, tremor and hyperventilation, may appear.

Meanwhile, *Cryptococcus neoformans* is a basidiomycete fungal pathogen that causes meningoencephalitis in immunocompromised populations, and is responsible for more than 600,000 deaths annually worldwide (Non-Patent Document 1). However, limited therapeutic options are available for treating cryptococcosis (Non-Patent Document 2). Thus, a complete understanding of diverse biological features of Cryptococcus is urgently required for developing novel therapeutic targets and methods. To this end, the signaling cascades governing the general biological features and pathogenicity of *Cryptococcus neoformans* have been extensively studied over the past decades. This study has made it possible to understand several key metabolic and signaling pathways in *Cryptococcus neoformans,* including those involving Ras, cAMP/protein kinase A, Rim101, calmodulin/calcineurin, three MAPKs (Cpk1, Mpk1 and Hog1), the unfolded protein response (UPR), and iron/copper uptake (Non-Patent Document 3, Non-Patent Document 4, and Non-Patent Document 5). The present inventors have found that impairment of function of Irel and Hxl1 (HAC1 and XBP1-like gene) proteins, newly identified in Cryptococcus neoformans, and genes encoding the proteins, shows an antifungal effect or a meningitis-treating effect (Patent Document 1 and Patent Document 2). Most of known signaling cascades are composed of sensor/receptor-like proteins and kinases/phosphatases, and are often equipped with unique adaptor or scaffolding proteins to enhance the specificity of each signaling pathway to prevent aberrant crosstalk between the signaling pathways. Nevertheless, each signaling cascade ultimately activates or represses a single transcription factor (TF) or multiple transcription factors, thereby up-regulating or down-regulating effector proteins of transcription factor through transcription factor binding to a specific region of promoter in a target gene that regulates the transcription level of transcription factor. Thus, transcription factor is regarded as a major regulator of gene expression in a given signaling pathway. Particularly, repertoires of transcription factors are often more divergent among species than are those of other signaling components. This appears particularly true in the case of *C. neoformans,* as described in the results of recent genome analyses (Non-Patent Document 6). For example, the UPR signaling pathway which is important in endoplasmic reticulum (ER) stress responses and *Cryptococcus neoformans* virulence is composed of evolutionarily highly conserved Ire1 kinase and Hxl1 transcription factor downstream of the Ire1 kinase. Therefore, *C*. *neoformans* appears to possess numerous evolutionarily conserved signaling cascades featuring divergent sets of TFs, which might govern the characteristics of *C. neoformans* that are unique compared with those of other fungi.

To understand *C*. *neoformans* transcription factor networks on a global scale, the present inventors constructed a high-quality gene-deletion mutant through homologous recombination methods for 155 putative *C*. *neoformans* transcription factors previously predicted, using a DNA-binding domain (DBD) transcription factor database to identify sequence-specific DNA-binding transcription factors in organisms whose genome sequences were analyzed (Non-Patent Document 7 and Non-Patent Document 8). The constructed transcription factor knockout (TFKO) strains were analyzed for 30 distinct *in vitro* phenotypic traits, which cover growth, differentiation, stress responses, antifungal resistance and virulence-factor production. Moreover, the present inventors performed a large-scale virulence test using an insect host model and signature-tagged mutagenesis (STM) scoring in a murine host model, and thus analyzed phenotypes resulting from deletion of various transcription factors in *Cryptococcus neoformans* strains, thereby constructing a comprehensive phenotypic data set (phenome) of the transcription factors, thereby completing the present invention.

Furthermore, the phenome of *Cryptococcus neoformans* transcription factors according to the present invention can be easily accessed online (http://tf.cryptococcus.org), and provides a unique opportunity to understand general biological features of *C*. *neoformans,* and also provides novel targets required for the treatment of cryptococcosis.

### Prior Art Documents

### Patent Documents

(Patent Document 1) Korean Patent No. 10-1311196.
(Patent Document 2) Korean Patent No. 10-1403862.

### Non-Patent Documents

(Non-Patent Document 1) Park, B.J., and et al., 2009, AIDS, 23: 525-530.
(Non-Patent Document 2) Perfect, J.R., et al., 2010, Clinical infectious diseases, 2010 update by the infectious diseases society of America, 50: 291-322.
(Non-Patent Document 3) Bahn, Y.S., and Jung, K.W., 2013, Eukaryot. Cell. 12: 1564-1577.
(Non-Patent Document 4) Jung. W.H. and Kronstad. J.W., 2008, Cell. Microbiol., 10: 277-284.
(Non-Patent Document 5) Ding, C. et al., 2013, Cell Host Microbe, 13: 265-276.
(Non-Patent Document 6) Janbon, G., and et al., 2014, PLoS Genet. 10: el004261.
(Non-Patent Document 7) Kummerfeld, S.K., and Teichmann, S. A., 2006, Nucleic Acids Res., 34: D74-81.
(Non-Patent Document 8) Wilson, D., and et al., 2008, Nucleic Acids Res. 36: D88-D92.
(Non-Patent Document 9) Cheon. S.A., and et al., 2011, PLoS Pathog. 7: el002177.
(Non-Patent Document 10) Kim. M.S., and et al., 2010, Genetics.185: 1207-1219.
(Non-Patent Document 11) Song, M.H., and et al., 2012, Eukaryot. Cell. 11: 53-67.
(Non-Patent Document 12) Liu. O.W. and et al., 2008, Cell. 135: 174-188.
(Non-Patent Document 13) Homann, O.R., and et al., 2009, PLoS Genet. 5: e 1000783.
(Non-Patent Document 14) Inglis. D.O., and et al., 2014, Eukaryot. Cell. 13: 878-883.
(Non-Patent Document 15) Davidson, R.C., and et al., 2002, Microbiology, 148: 2607-2615.
(Non-Patent Document 16) Kim. M.S., and et al., 2009, Biochem. Biophy. Res. Commun. 390: 983-988.
(Non-Patent Document 17) Lin, X., and Heitman, J., 2006, Annu. Rev. Microbiol. 60: 69-105.
(Non-Patent Document 18) Garcia-Rodas, R., and Zaragoza. O., 2012, Med Microbiol. 64: 147-161.
(Non-Patent Document 19) McFadden, D. C., and Casadevall, A. 2001, Med. Mycol. 39 Suppl 1:19-30.
(Non-Patent Document 20) Bahn, Y.S. and et al., 2004, Eukaryotic. Cell. 3: 1476-1491.
(Non-Patent Document 21) Mylonakis, E. et. al., 2005, Infect. Imraun. 73: 3842-3850.
(Non-Patent Document 22) Haynes, B.C., and et al., 2011, PLoS Pathog. 7: el002411.
(Non-Patent Document 23) Wickes, B.L., and et al., 1997, Mol. Microbiol. 26: 951-960.
(Non-Patent Document 24) Bahn, Y.S., and et al., 2006, Mol. Biol. Cell, 17: 3122-3135.
(Non-Patent Document 25) Jiang, N., and et al., 2009, FEMS Microbiol. Lett. 296: 84-90.
(Non-Patent Document 26) Pukkila-Worley, R., and et al., 2005, Eukaryot. Cell 4: 190-201.
(Non-Patent Document 27) Chang, Y.C., and et al., 2007, Mol. Microbiol., 64: 614-629.
(Non-Patent Document 28) Chun, C.D., and et al., 2007, PLoS Pathog., 3: e22.
(Non-Patent Document 29) Cramer, K.L., and et al., 2006, Eukaryot. Cell, 5: 1147-1156.
(Non-Patent Document 30) Idnurm, A., and Heitman, J.2005, PLoS Biol. 3: e95.
(Non-Patent Document 31) Alder, A., and et al., 2011, J. Biol. Chem., 286: 20977-20990.
(Non-Patent Document 32) Chun. C. D., and et al., 2011, Cell host & microbe, 9: 243-251.
(Non-Patent Document 33) Jung, W., and et al., 2011, Fungal Genet. Biol. 48: 154-165.
(Non-Patent Document 34) Livak. K. J. and Schmittgen, T.D., 2001, Methods. 25: 402-408.
(Non-Patent Document 35) Cowen, L.E., 2008, Nat. Rev. Microbiol. 6: 187-198.
(Non-Patent Document 36) Vermes, A., and et al., 2000, J Antimicrob. Chemother. 46: 171-179.
(Non-Patent Document 37) Choi, J. and et al., 2013, Nucleic Acids Res. 41: D714-719.
(Non-Patent Document 38) Hunter, S. et al., 2012, Nucleic Acids Res. 40: D306-312.
(Non-Patent Document 39) Bendtsen, J.D. and et al., 2004, J. Mol. Biol. 340: 783-795.
(Non-Patent Document 40) Nakai, K. and Horton, P., 1999, Trends Biochem. Sci. 24: 34-36.
(Non-Patent Document 41) Emanuelsson, O., and et al., 2000, J. Mol. Biol., 300: 1005-1016.
(Non-Patent Document 42) Emanuelsson, O., and et al., 1999, Protein Sci., 8: 978-984.
(Non-Patent Document 43) Bendtsen, J.D., and et al., 2004, Protein Eng. Des. Sel. 17: 349-356.
(Non-Patent Document 44) Cokol, M., and et al., 2000, EMBO reports 1: 411-415.
(Non-Patent Document 45) Sonnhammer, E.L., and et al., 1998, Proc. Int. Conf. Intelli. Syst. Mol. Biol 6: 175-812.
(Non-Patent Document 46) Jung, K. et al., 2008, BMC Genomics, 9: 586.

### [Disclosure]

### [Technical Problem]

Therefore, it is an object of the present invention to construct transcription factor networks in a *Cryptococcus neoformans* strain and to provide various uses of the transcription factors.

It is an object of the present invention to provide a method for screening an agent for preventing or treating fungal infection caused by a *Cryptococcus neoformans* strain, or a method for screening an agent for treating meningitis.

Another object of the present invention is to provide a method for screening a candidate that may have a synergistic effect when administered in combination with a conventional antifungal agent or meningitis-treating agent.

Still another object of the present invention is to provide a pharmaceutical composition that exhibits an antifungal effect or a meningitis-treating effect by increasing or inhibiting the expression of a transcription factor that regulates the virulence of a *Cryptococcus neoformans* strain and a gene that encodes the transcription factor.

Yet another object of the present invention is to provide a pharmaceutical composition that exhibits an antifungal effect or a meningitis-treating effect by increasing or inhibiting the expression of a transcription factor that regulates the antifungal agent susceptibility of a *Cryptococcus neoformans* strain, a transcription factor that regulates the growth of the strain, a transcription factor that regulates the mating of the strain, a transcription factor that regulates the responses to various external stresses, and genes that encode the transcription factors.

### [Technical Solution]

To achieve the above objects, the present invention provides a *Cryptococcus neoformans* strain deposited under accession number KCCM51291.

The present invention also provides a method for screening an antifungal agent, an antifungal agent for co-administration, or a meningitis-treating agent, the method comprising measuring an increase or decrease in the expression of a virulence regulatory gene in *Cryptococcus neoformans.*

In one embodiment, the present invention provides a method for screening an antifungal agent, comprising the steps of: (a) bringing a sample to be analyzed into contact with a cell line (deposited under accession number KCCM51291) containing an antifungal agent-targeting gene; (b) measuring the expression of the antifungal agent-targeting gene in the cell line; and (c) determining that the sample is the antifungal agent, when the expression of the antifungal agent-targeting gene is measured to be down-regulated or up-regulated, wherein the antifungal agent-targeting gene is any one gene selected from the group consisting of an antifungal agent resistance regulatory gene, a growth regulatory gene, a mating regulatory gene, and a gene that regulates responses to external stress.

In the embodiment of the method for screening an antifungal agent, the antifungal agent resistance regulatory gene is a gene that regulates resistance to any one antifungal agent selected from the group consisting of azole-, polyene-, 5-flucytocin- and phenylpyrazole-based antifungal agents.

In the embodiment of the method for screening an antifungal agent, as the antifungal agent-targeting gene, a gene, which increases sensitivity to the azole-based antifungal agent when its expression is down-regulated, is any one gene selected from the group consisting of *bzp3, hlh3, bzp*/*hxl1, sre1, riw101, bap2, hlh1, yap4, pip2, miz1, mln1, hob6, mbf1, met32, fzc46, bap1, fzc14, fzc2, liv4, hsf2, zfc6, fzc45, fzc30, asg1, stel2, liv1, fzc22, fzc31, pan1, bzp2, sp1*/*crz1, bzp5, hlh2, sxi1 alpha, fzc34, fzc40, fzc38* and *fzcl7;* a gene that increases sensitivity to the polyene-based antifungal agent is any one selected from the group consisting of *hob1, mbs1, jjj1, ert1, ecm22, gat201, zap104, sp1*/*crz1, fzc6, bzp5, hlh1, pip2, hcm1, bzp2, usv101, hob4, ste12, hob5, grf1, hel2, fzc45, asg1, fzc22, hob6, pan1, liv4, cuf1, fzc49, fzc1, bwc2, fap1, fzc44, fzc8, fzc23, gat204, nrg1, pip201, hlh2, rim101, fzc38, hlh3, bzp3, mln1, met32, zfc2, fzc40, fzc31* and *rum1;* a gene that increases sensitivity to the 5-flucytocin-based antifungal agent is any one gene selected from the group consisting of *nrg1, zfc2, bap1, mbs1, fzc6, bap2, bzp3, jjj1, hlh1, pip2, apn2, fzc46, hap2, fzc51, bzp5, hcm1* and *fzc19;* and a gene that increases sensitivity to the phenylpyrazole-based antifungal agent is any one gene selected from the group consisting of *usv101, ada2, bap1, fzc6, hlh1, pip2, fzc46, hap2, bzp1*/*hxl1, fkh2, liv1, bap2, bzp2, fzc21, hlh3, yrm101, bzp5, gln3, zfc8, ddt1, fzc22, hob6, rlm1, mln1, liv4, pan1, fzc35, yrm103, fzc3, asg1, fzc41, fzc43, fzc51, hap1, fzc38, met32* and *fzc32.*

In the embodiment of method for screening an antifungal agent, as the antifungal agent-targeting gene, a gene, which increases sensitivity to the azole-based antifungal agent when its expression is up-regulated, is any one gene selected from the group consisting of *hob1, hap2, skn7, nrg1, mbs1, ppr1, jjj1, hcm1, ada2, fzc9, gat7, ert1, fkh2, ecm22, ddt1, gat1, yrm103, cuf1* and *fzc51*; a gene that increases sensitivity to the polyene-based antifungal agent is any one selected from the group consisting of *sre1, bap1, fzc51, skn7, clr1, bzp5, atf1* and *fzc4;* a gene that increases sensitivity to the 5-flucytocin-based antifungal agent is any one gene selected from the group consisting of *hlh3, rim101, gat204, hob3, fzc50, znf2* and *rds2;* and a gene that increases sensitivity to the phenylpyrazole-based antifungal agent is any one gene selected from the group consisting of *nrg1, jjj1, sp1*/*crz1, skn7, gat7, fap1, zfc2, gat204, znf2, hel2, fzc50* and *sre1.*

In the embodiment of the method for screening an antifungal agent, as the antifungal agent-targeting gene whose expression is down-regulated, a growth regulatory gene is temperature-independent or temperature-dependent. The temperature-independent growth regulatory gene is any one gene selected from the group consisting of *bzp2, cuf1, hob1, gat5, fzc6* and *nrg1;* and the temperature-dependent growth regulatory gene that regulates growth at a temperature of 37°C to 39°C is any one gene selected from the group consisting of *hxl1, crz1, alf1, ada2, liv4, aro80, usv101, fzc31, fzc30, mln1, fzc30, fzc1, miz1, apn2, gat6, mbs2, sre1* and *ert1.* Furthermore, as the antifungal agent-targeting gene whose expression is up-regulated, an antifungal agent-targeting gene that regulates growth at 39°C is any one gene of *mln1* and *fzc46.*

In the embodiment of the method for screening an antifungal agent, as the antifungal agent-targeting gene whose expression is down-regulated, a mating regulatory gene is any one gene selected from the group consisting of *bzp2, usv101, fzc1, zap104* and *skn7;* and as the antifungal agent-targeting gene whose expression is up-regulated, a mating regulatory gene is any one gene selected from the group consisting of *hlh1, hap2, skn7* and *gat1.*

In the embodiment of the method for screening an antifungal agent, the antifungal agent-targeting gene, which regulates responses to external stress when its expression is down-regulated, may be an antifungal agent-targeting gene that regulates responses to an osmotic stress induced by any one selected from the group consisting of 1M to 1.5M sodium chloride (NaCl), 1M to 1.5M potassium chloride (KCl) and 2M sorbitol. Specifically, the antifungal agent-targeting gene that regulates responses to the osmotic stress induced by the sodium chloride is any one gene selected from the group consisting of *rim101, skn7, ada2, fzc42, hcm1, gat7, bzp2, hob1, hap2, hob6, aro8001, pan1, fzc34, bap1, fzcl9, fzc51, fzc43, fzcl3, gat5* and *met32;* the antifungal agent-targeting gene that regulates responses to the osmotic stress induced by the potassium chloride is any one gene selected from the group consisting of *bzp2, hob2, nrg1, hap2, ada2, fzc6, yrm103, fzc44, fzc32, hob1, rim101, bzp4* and *fzc35;* and the antifungal agent-targeting gene that regulates responses to the osmotic stress induced by the sorbitol is any one gene selected from the group consisting of *bzp2, hob1* and *fzc6.* Moreover, among antifungal agent-targeting genes that regulates responses to an oxidative stress induced by any one selected from the group consisting of 2.5 mM to 3.5 mM hydrogen peroxide (H₂O₂), 0.7 mM to 0.8 mM tert-butyl hydroperoxide (TH), 0.02 mM to 0.03 mM menadione, and 2 mM to 3 mM diamide (DA), the antifungal agent-targeting gene that regulates responses to the oxidative stress induced by the hydrogen peroxide is any one gene selected from the group consisting of *bap1, sre1, usv101, fzc50, fzc31, hob1, ada2, cuf1, gat204, ste12, fzc9, gat1, fzc21, nrg1, bzp2, gat5, pan1, met32, fzc4, rim101, hob6, fzcl3, hlh1, fzc46, sip402, fzc27, hob5, hob4, sp1(crz1), fzc22, bzp3, liv1, miz1* and *gat201;* the antifungal agent-targeting gene that regulates responses to the oxidative stress induced by the tert-butyl hydroperoxide is any one gene selected from the group consisting of *sre1, ada2, rim101, bap2, bap1, usv101, fzc31, hob1, fzc34, ecm22, fzcl5, fzc44, zfc4, fzc49, yrm103, fzc21, zfc2, gat5, pan1, met32, hob4, liv1, mwc2, skn7, hcm1, fzc51, fzc1, ppr1, atf1, grf1, bzp5, gat8, clr1, hlh2, rlm1, fzc6, asg1, hob2,* and *zap103;* the antifungal agent-targeting gene that regulates responses to the oxidative stress induced by the menadione is any one gene selected from the group consisting of *bap1, fzc37, usv101, nrg1, bzp2, fzc4, fzc34, fzc35, hel2, ecm22, fzc6, gat6, jjj1, fzc44, fzc3* and *fzc26;* and the antifungal agent-targeting gene that regulates responses to the oxidative stress induced by the diamide is any one gene selected from the group consisting of *bap1, hob1, bap2, hap2, pip2, bzp5, hsf2, sre1, fzc21, zfc2, fzc31, bzp2, gat5, pan1, met32, fzc4, fzc34, hob6, hlh1, fzc46, sip402, fzc27, miz1, fzcl9, hlh3, fkh2, mln1, gat6, fap1, fzc8, fzc49, fzc3, fzc30, rum1* and *fzc38.* In addition, among antifungal agent-targeting genes that regulates responses to endoplasmic reticulum (ER) stress induced by 0.3 µg/ml tunicamycin (TM) or 20 mM dithiothreitol (DTT), the antifungal agent-targeting gene that regulates responses to the endoplasmic reticulum stress induced by the tunicamycin is any one gene selected from the group consisting of *bzp1 (hxl1), sre1, hlh1, bzp3, pip2, rlm1, met32, ste12, rim101, sp1 (crz1), fzc21, gat7, mln1, fzc2, fzc44, liv4, fzc40* and *fzc38;* and the antifungal agent-targeting gene that regulates responses to the endoplasmic reticulum stress induced by the dithiothreitol is any one gene selected from the group consisting of *bzp1 (hxl1), sre1, bzp2, cuf1, hob1 clr1, ada2, rlm1, gat5, hap2, nrg1, usv101, fzc31, gat201, hlh2, apn2, fzc25* and *ddt1.* In addition, among antifungal agent-targeting genes that regulates responses to a genotoxic stress induced by 0.03% to 0.06% methyl methanesulfonate (MM) or 50 mM to 100 mM hydroxyurea (HU), the antifungal agent-targeting gene that regulates responses to the genotoxic stress induced by the methyl methanesulfonate is any one gene selected from the group consisting of *bzp1 (hxl1), fzc6, hob1, sre1, gat5, gat6, miz1, bzp2, jjj1, fzc40, fzc38, fzc4, hcm101, fzc1* and *apn2;* and the antifungal agent-targeting gene that regulates responses to the genotoxic stress induced by the hydroxyurea is any one gene selected from the group consisting of *hob1, sre1, gat5, gat6, mbs1, skn7, ada2, bzp1 (hxl1), fzc6, bzp2, jjj1, hcm1, nrg1* and *hlh2.* In addition, among antifungal agent-targeting genes that regulates responses to a cell wall or cell membrane stress induced by any one selected from the group consisting of 3 mg/ml to 5 mg/ml calcofluor white (CFW), 0.8% to 1% Congo red (CR), and 0.03% sodium dodecyl sulfate (SDS), the antifungal agent-targeting gene that regulates responses to the cell wall or cell membrane stress induced by the CFW is any one gene selected from the group consisting of *bzp1 (hxl1), sp1 (crz1), hob1, hap2, bzp2, nrg1, bap2, rim101* and *pip2;* the antifungal agent-targeting gene that regulates responses to the cell wall or cell membrane stress induced by the CR is any one gene selected from the group consisting of *sp1 (crz1), hob1, bzp1 (hxl1), cuf1, hlh3, hap2, bzp2, nrg1, bap2* and *rim101;* and the antifungal agent-targeting gene that regulates responses to the cell wall or cell membrane stress induced by the SDS is any one gene selected from the group consisting of *sp1 (crz1), hob1, sre1, pip2, fzc21, gat7, hob3, usv101, gat201, fzc7, asg1, rum1, hap2, bzp2, nrg1, cuf1, gat5, gat6, jjj1, pan1, bzp3, rlm1, bap1, clr1, zfc4, clr4, gat1, fzc31, hob5, asg101, ert1, ecm22, zfc6, bzp5, sxi1 alpha, fap1, sip4, rds2, fzc26* and *fzc30.* In addition, an antifungal agent-targeting gene that regulates responses to a heavy-metal stress induced by 20 M to 30 M cadmium sulfate (CdSO₄) is any one gene selected from the group consisting of *cuf1, hap2, fzc6, skn7, fzc37, bzp2, gat5, yox101, mln1, pip2, hcm1, hob6, fzc46, hob5, mbs2, fzc35, aro8001, fzcl9, fzc51, aro80, ccd4, fzc47, bzp4, fap1, fzc8, pip201, gln3, yrm101, zfc8, hob7, rum1* and *fzc10.*

In the embodiment of the method for screening an antifungal agent, the antifungal agent-targeting gene, which regulates responses to external stress when their expression is up-regulated, may be an antifungal agent-targeting genes that regulates an osmotic stress induced by any one selected from the group consisting of 1 M to 1.5 M sodium chloride (NaCl), 1 M to 1.5 M potassium chloride (KCl) and 2 M sorbitol. Specifically, the antifungal agent-targeting gene that regulates responses to the osmotic stress induced by the sodium chloride is any one gene selected from the group consisting of *hlh3, hel2* and *cuf1;* the antifungal agent-targeting gene that regulates responses to the osmotic stress induced by the potassium chloride is any one gene of *fzc36* and *yrm103;* and the antifungal agent-targeting gene that regulates responses to the osmotic stress induced by the sorbitol is *fzc36.* In addition, among antifungal agent-targeting genes that regulate responses to an oxidative stress induced by any one selected from the group consisting of 2.5 mM to 3.5 mM hydrogen peroxide (H₂O₂), 0.7 mM to 0.8 mM tert-butyl hydroperoxide (TH), 0.02 mM to 0.03 Mm menadione, and 2 mM to 3 mM diamide (DA), the antifungal agent-targeting gene that regulates responses to the oxidative stress induced by the hydrogen peroxide is any one gene selected from the group consisting of *fzc45, asg101, mbs2, fzc35, bwc2, fzc7* and *znf2;* the antifungal agent-targeting gene that regulates responses to the oxidative stress induced by the tert-butyl hydroperoxide is any one gene selected from the group consisting of *fzc33, fap1, clr3* and *ddt1;* the antifungal agent-targeting gene that regulates responses to the oxidative stress induced by the menadione is any one gene selected from the group consisting of *zfc2, fzc50, cuf1, hap2* and *sip4;* and the antifungal agent-targeting gene that regulates responses to the oxidative stress induced by the diamide is any one gene selected from the group consisting of *fzc50, sip4, pip201, nrg1, gat1, znf2, asg101, skn7, gat7, jjj1, hlh5, fzc26* and *fzc20.* In addition, among antifungal agent-targeting genes that regulate responses to an endoplasmic reticulum stress induced by 0.3 µg/ml tunicamycin (TM) or 20 mM dithiothreitol (DTT), the antifungal agent-targeting gene that regulates responses to the endoplasmic reticulum stress induced by the tunicamycin is any one gene selected from the group consisting of *bzp2, nrg1, hap2, cuf1, mbs1, ppr1, fzc6, skn7, zfc2, hob1, gat5, clr1, bap1, bwc2, hcm1, hel2, gat6, jjj1, hob3, zfc4, zfc3* and *clr4;* and the antifungal agent-targeting gene that regulates responses to the endoplasmic reticulum stress induced by the dithiothreitol is any one gene selected from the group consisting of *yap4, hlh1, bzp3, pip2, pan1, mbs1, met32, gat1, fkh2, fzcl1, gat203, sip401, stb4* and *fzc20.* In addition, among antifungal agent-targeting genes that regulate responses to a genotoxic stress induced by 0.03% to 0.06% methyl methanesulfonate (MM) or 50 mM to 100 mM hydroxyurea (HU), the antifungal agent-targeting gene that regulates responses to the genotoxic stress induced by the methyl methanesulfonate is *yox1;* and the antifungal agent-targeting gene that regulates responses to the genotoxic stress induced by the hydroxyurea is *fzc20.* In addition, among antifungal agent-targeting genes that regulate responses to a cell wall or cell membrane stress induced by any one selected from the group consisting of 3 mg/ml to 5 mg/ml calcofluor white (CFW), 0.8 % to 1% Congo red (CR), and 0.03% sodium dodecyl sulfate (SDS), the antifungal agent-targeting gene that regulates responses to the cell wall or cell membrane stress induced by the CFW is any one gene of *fzc9* and *grf1;* and the antifungal agent-targeting gene that regulates responses to the cell wall or cell membrane stress induced by the SDS is any one gene selected from the group consisting of *fzc6, fzc1, zfc1, hsf3, bwc2, skn7, fzc50, fzc22, fzc51* and *fzc8.* In addition, an antifungal agent-targeting gene that regulates responses to a heavy-metal stress induced by 20 µM to 30 µM cadmium sulfate (CdSO₄) is any one gene selected from the group consisting of *bzp1 (hxl1), gat201, znf2, sip4, rds2, sre1, gat7, rlm1, clr1, zfc3, ada2, gat204, fzc7, asg101, atf1, hlh2, fzc39* and *hsf3.*

In another embodiment, the present invention provides a method for screening an antifungal agent, an antifungal agent for co-administration or an agent for treating meningitis, the method comprising the steps of: (a) bringing a sample to be analyzed into contact with a cell comprising a virulence regulatory gene; (b) measuring the expression of the virulence regulatory gene in the cell; and (c) determining that the sample is an antifungal agent, when the expression of the virulence regulatory gene is measured to be down-regulated or up-regulated.

In the embodiment of the method for screening an antifungal agent, an antifungal agent for co-administration or an agent for treating meningitis, the virulence regulatory gene may be a gene that regulates *Cryptococcus neoformans* pathogenicity. Specifically, the gene whose expression is down-regulated is any one gene selected from the group consisting of *usv101, fzc1, bap1, hob1, zfc2, fzc50, fzc31, bzp2, fzc9, ddt1, ma113, fzc2, fzc43, fzc22, hih1, mbs2, rum1, fzc5, aro80, clr1, pip2, fzc37, gat5, fzc49, cef3, fzc33, fzcl2* and *zfc5;* and the gene whose expression is up-regulated is any one selected from the group consisting of *fzc17, fzc40, aro8001, fzc38, fzc24* and *ert1.*

In the embodiment of the method for screening an antifungal agent, an antifungal agent for co-administration or an agent for treating meningitis, the virulence regulatory gene regulates the production of any one selected from the group consisting of capsule, melanin and urease.

In the embodiment of the method for screening an antifungal agent, an antifungal agent for co-administration or an agent for treating meningitis, the gene that reduces capsule production is any one gene selected from the group consisting of *bap1, rds2, zap104, fzc47, gat204, fzc33, fzc45, hsf2, bzp4, hob5, fzcl6, hob3, zfc4, mcm1, liv4, hob4* and *liv1;* and the gene that increases capsule production is any one gene selected from the group consisting of *hob7, clr3, fzc51, fzc1, fkh2, nrg1, usv101, fzc29, bzp3, zfc3, fzc14, sre1, fzc30, hlh4, fzc36, crl6, mln1, fzc46, clr1, fzcl7, jjj1, fzc49, fzcl8, hcm1, fzc24, hlh3* and *hpa1.*

In the embodiment of the method for screening an antifungal agent, an antifungal agent for co-administration or an agent for treating meningitis, the gene that reduces melanin production is any one gene selected from the group consisting of *bzp4, fzc8, hob1, usv101, liv1, mbs2, fzc5, fzc25* and *ert1;* and the gene that increases melanin production is any one gene selected from the group consisting of *bzp2, fkh2, bap1, bzp3, hlh1, sip4, rds2, sip401, fzc1, gat1, ada2, nrg1, fzc31* and *hlh2.*

In the embodiment of the method for screening an antifungal agent, an antifungal agent for co-administration or an agent for treating meningitis, the gene that reduces urease production is any one gene selected from the group consisting of *zap104, sre1, gat201, fzc46, hlh1* and *fzc21;* and the gene that increases urease production is any one gene selected from the group consisting of *rim1, atf1, fkh2, fzc1, usv101, bap1, sxi1 alpha, mln1, fzc26, skn7, zfc7, hob7, fzc14* and *hob4.*

In the embodiment of the method for screening an antifungal agent, an antifungal agent for co-administration or an agent for treating meningitis, the cell is *Cryptococcus neoformans.*

The term "sample" as used herein with reference to the screening method means an unknown candidate that is used in screening to examine whether it influences the expression of a gene or the amount or activity of a protein. Examples of the sample include, but are not limited to, chemical substances, nucleotides, antisense-RNA, siRNA (small interference RNA) and natural extracts.

The term "antifungal agent" as used herein is meant to include inorganic antifungal agents, organic natural extract-based antifungal agents, organic aliphatic compound-based antifungal agents, and organic aromatic compound-based antifungal agents, which serve to inhibit the propagation of bacteria and/or fungi. Examples of the inorganic antifungal agents include, but are not limited to, chlorine compounds (especially sodium hypochlorite), peroxides (especially hydrogen peroxide), boric acid compounds (especially boric acid and sodium borate), copper compounds (especially copper sulfate), zinc compounds (especially zinc sulfate and zinc chloride), sulfur-based compounds (especially sulfur, calcium sulfate, and hydrated sulfur), calcium compounds (especially calcium oxide), silver compounds (especially thiosulfite silver complexes, and silver nitrate), iodine, sodium silicon fluoride, and the like. Examples of the organic natural extract-based antifungal agents include hinokithiol, *Phyllostachys pubescens* extracts, creosote oil, and the like.

The term "meningitis" as used herein is meant to include various inflammatory diseases occurring in the subarachnoid space between the arachnoid and the pia mater, for example, those caused by invasion of viruses or bacteria into the subarachnoid space, inflammation caused by a certain chemical substance, and those caused by the spread of cancer cells into the cerebrospinal fluid space.

### [Advantageous Effects]

The present invention may effectively screen a composition having an antifungal effect or a meningitis-treating effect by measuring the expression level of a transcription factor that regulates virulence in a *Cryptococcus neoformans* strain. In addition, the present invention may provide a pharmaceutical composition, which exhibits an antifungal effect or a meningitis-treating effect, by up-regulating or down-regulating the expression of a transcription factor that regulates *Cryptococcus neoformans* virulence.

### [Description of Drawings]

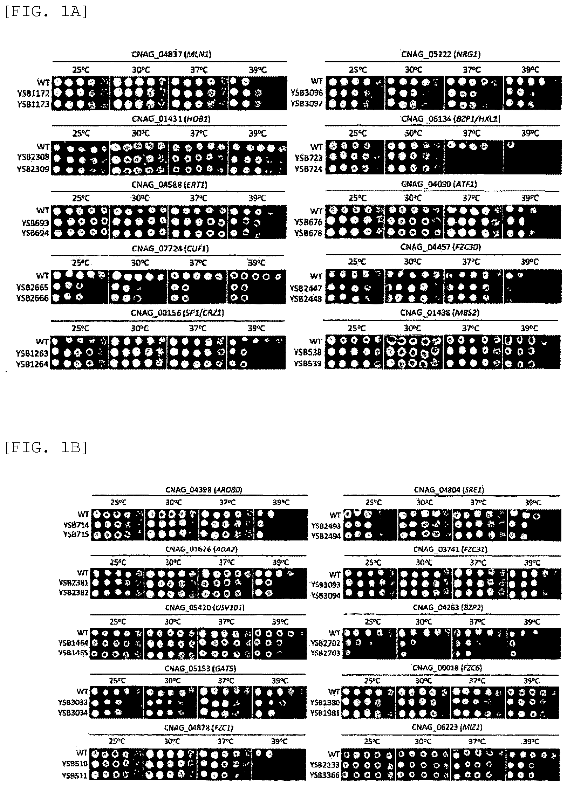

FIGS. 1a and 1b show transcription factors required for the temperature-dependent growth of *Cryptococcus neoformans.*

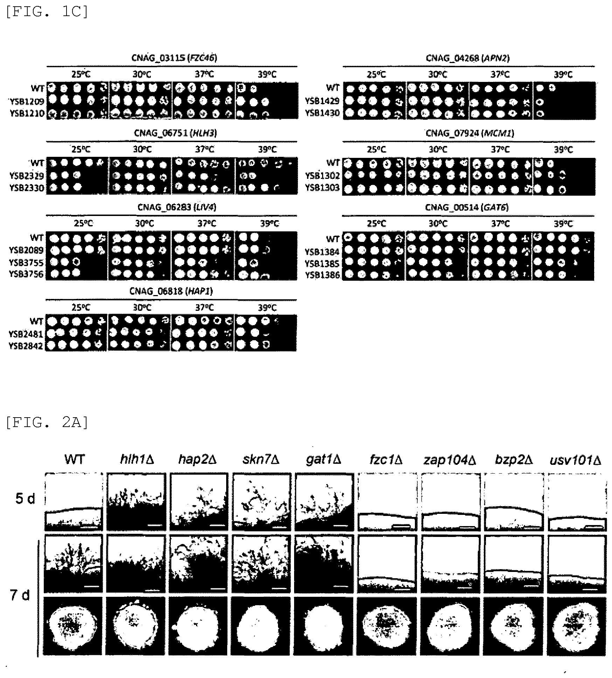

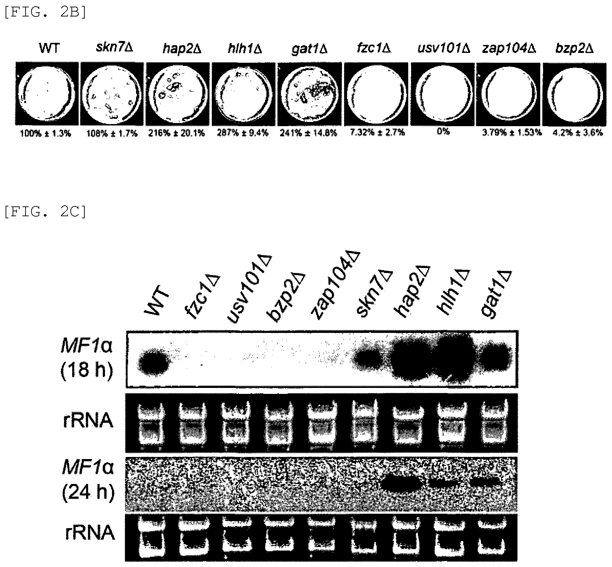

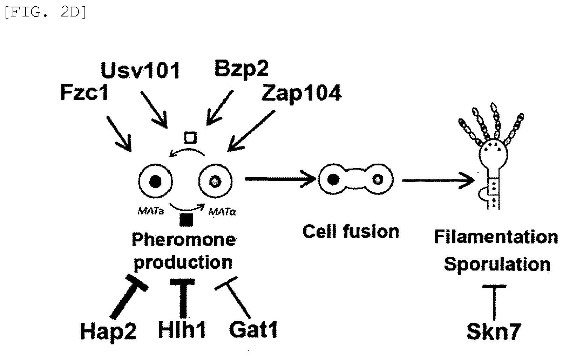

FIGS. 2a to 2d show transcription factors involved in sexual differentiation of *Cryptococcus neoformans.* Specifically, FIG. 2a shows the results of a mating assay in which the WT strain H99 and each TF mutant were cocultured with the opposite mating type KN99a strain on V8 media and incubated at room temperature in the dark for 7 days; FIG. 2b shows the cell-fusion efficiency of each TF mutant calculated relative to that of control strains (NAT-marked wild-type strain (YSB119) × NEO-marked wild-type a strain (YSB121)); FIG. 2c shows transcription factors involved in pheromone gene expression. In FIG. 2c, indicated transcription factor mutants were cocultured with the KN99a strain on V8 medium at room temperature for 18 to 24hours, and then RNA expression was analyzed. FIG. 2d is a schematic diagram showing the role of transcription factors in various mating stages of *Cryptococcus neoformans.*

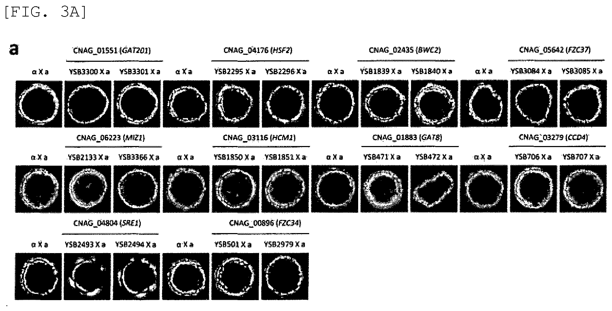

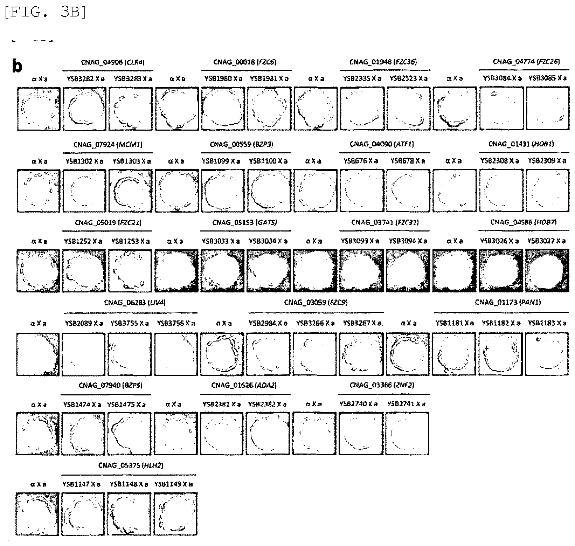

FIGS. 3a and 3b show transcription factors involved in mating efficiency (sexual differentiation) in *Cryptococcus neoformans* (3a-positive regulators; 3b-negative regulators).

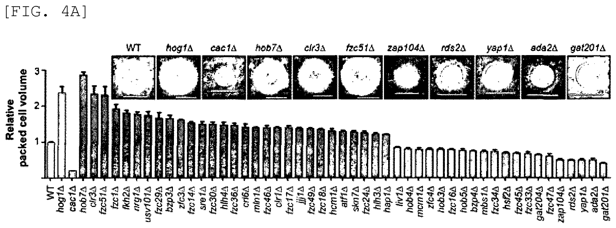

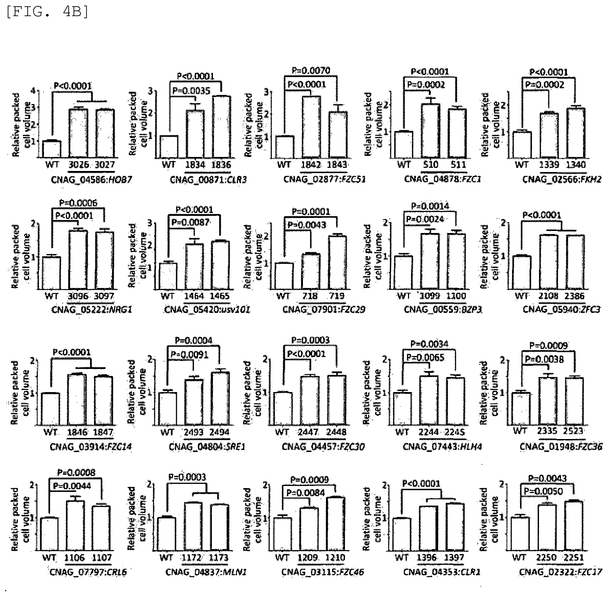

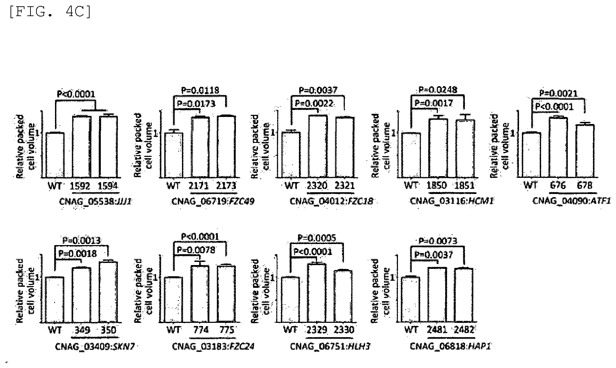

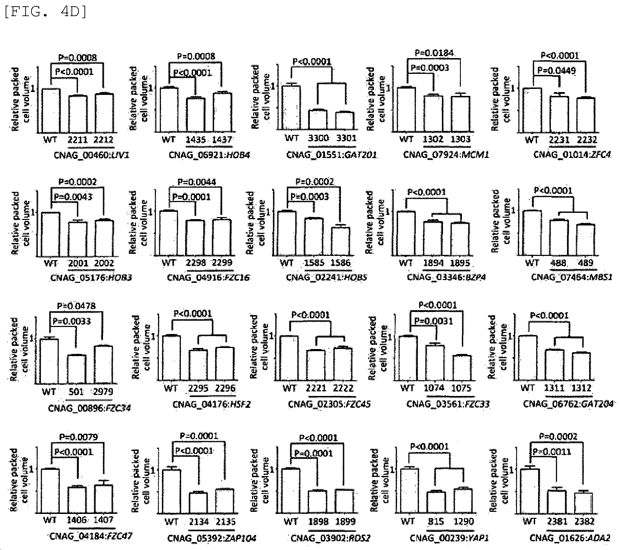

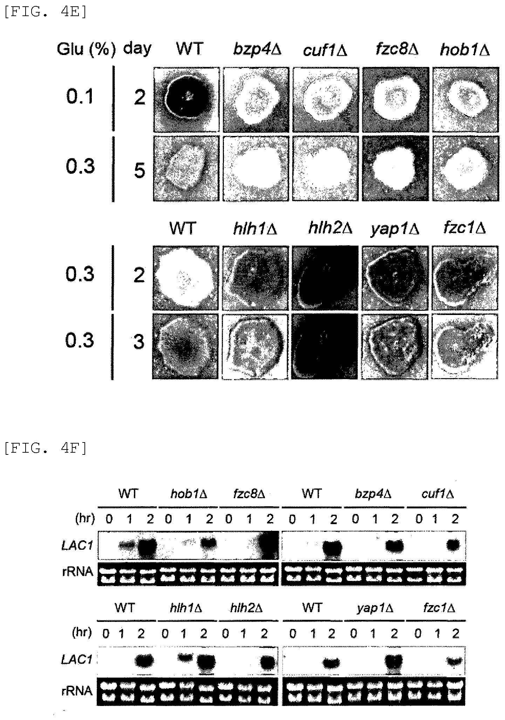

FIGS. 4a to 4f show transcription factors involved in virulence-factor production in *Cryptococcus neoformans.* Specifically, FIG. 4a shows the sizes of capsules produced in the wild-type strain H99 and transcription factor mutants; FIGS. 4b and 4c show transcription factors involved in capsule production in *Cryptococcus neoformans* (4b-negative regulators; 4c-positive regulators); and FIGS. 4d and 4e show the correlation between the expression of LAC1, which is the major laccase involved in melanin synthesis, and transcription factors. Specifically, FIG. 4d shows the results obtained by spotting transcription factor mutants on Niger seed agar medium (containing 0.1 and 0.3% glucose) and photographing the plates while culturing the mutants at 37°C; and FIG. 4e shows the results of Northern blot analyses performed using a LAC1-specific probe for total RNA isolated from cells under glucose-rich (0hr) and glucose-depleted conditions (1 and 2hr).

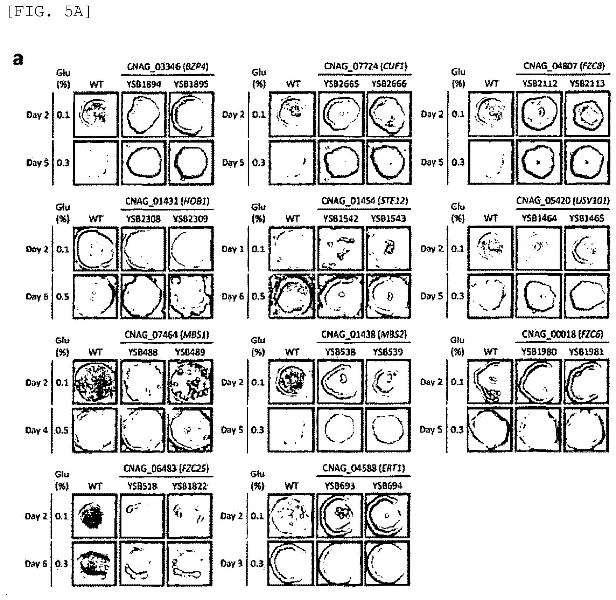

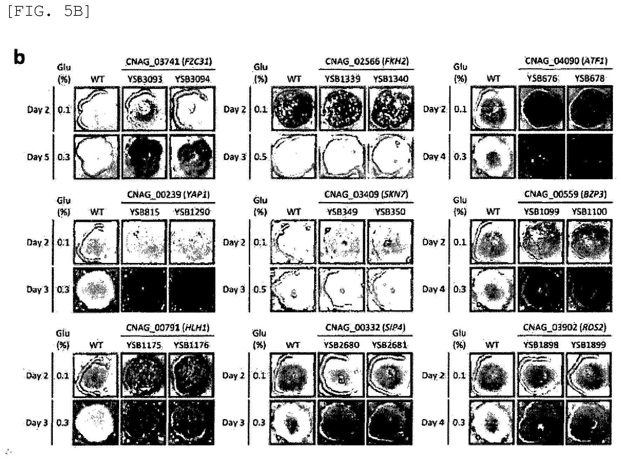

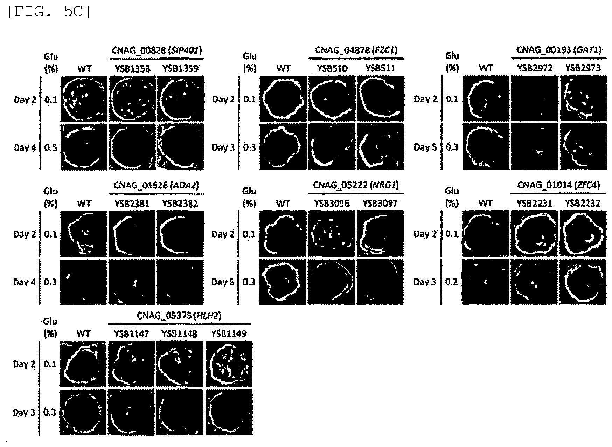

FIGS. 5a to 5c show transcription factors involved in melanin production in *Cryptococcus neoformans* (5a-positive regulators; 5b and 5c-negative regulators).

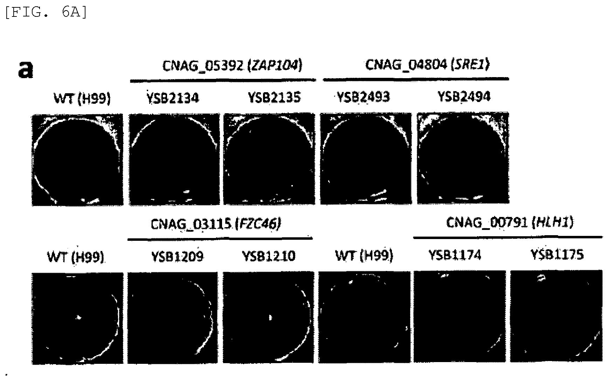

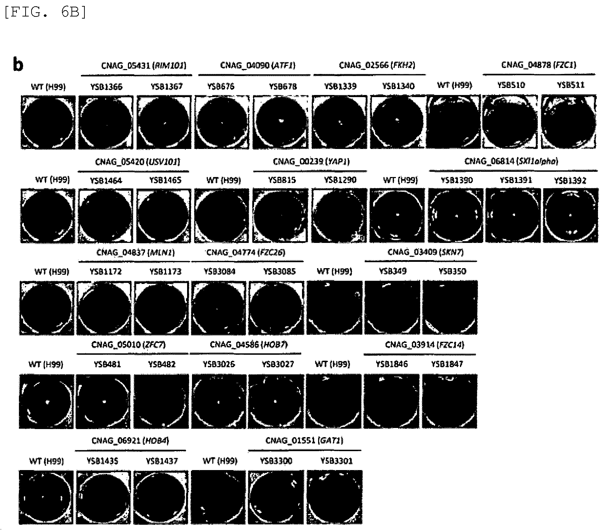

FIGS. 6a and 6b shows transcription factors required for urease production in *Cryptococcus neoformans* (6a-negative regulators; 6b-positive regulators).

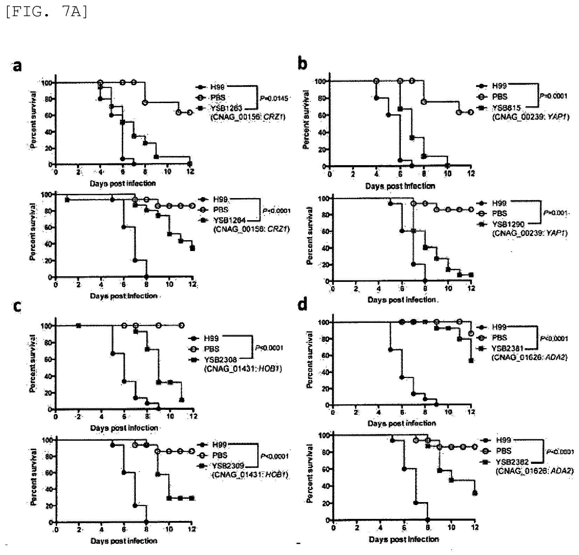

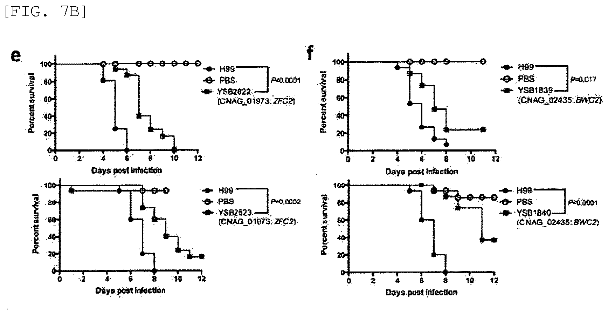

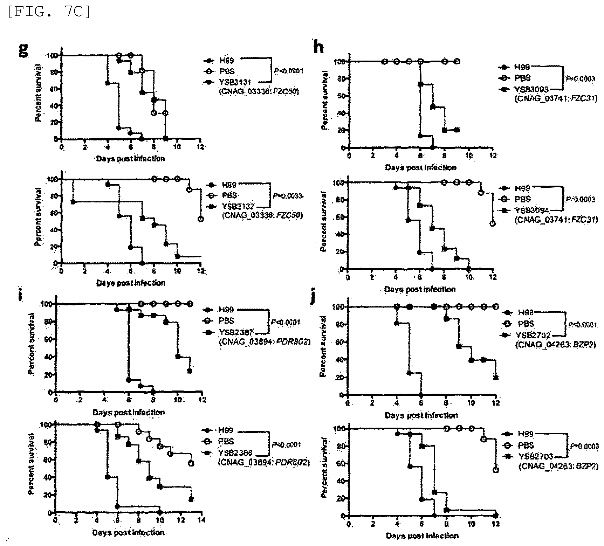

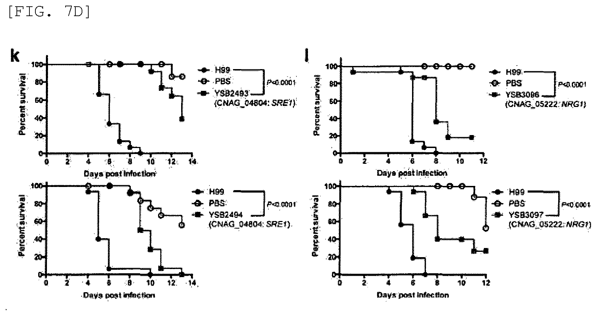

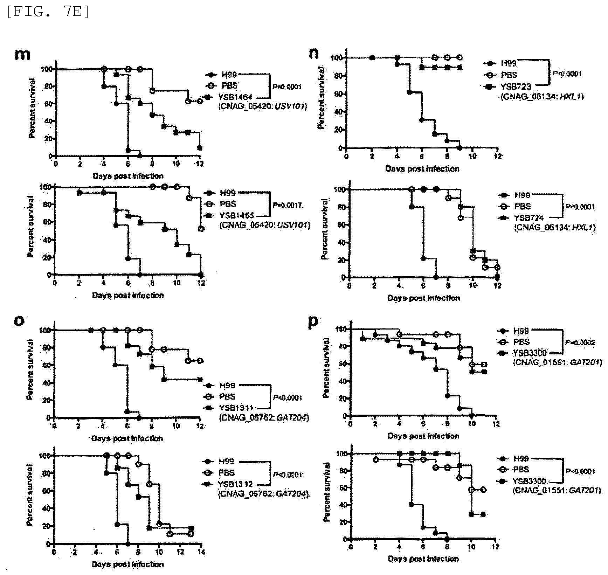

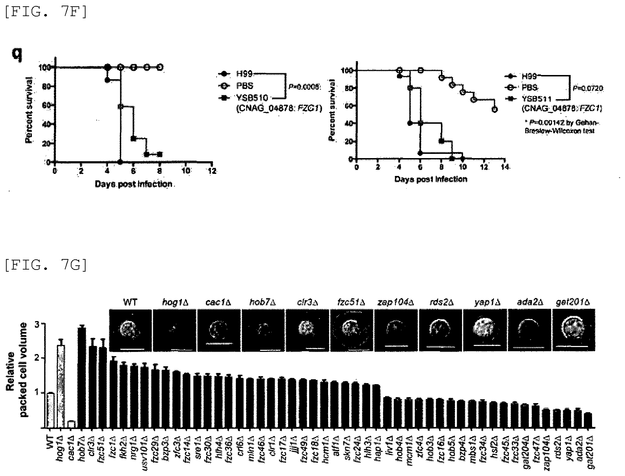

FIGS. 7a to 7g show transcription factors involved in *Cryptococcus neoformans* virulence in *Galleria mellonella* killing assay. Specifically, FIGS. 7a to 7f show the results obtained for various transcription factor deletions, and FIG. 7g shows the results of identifying virulence-related transcription factors in *Cryptococcus neoformans* by signature-tagged mutagenesis (STM)-based murine infectivity assay.

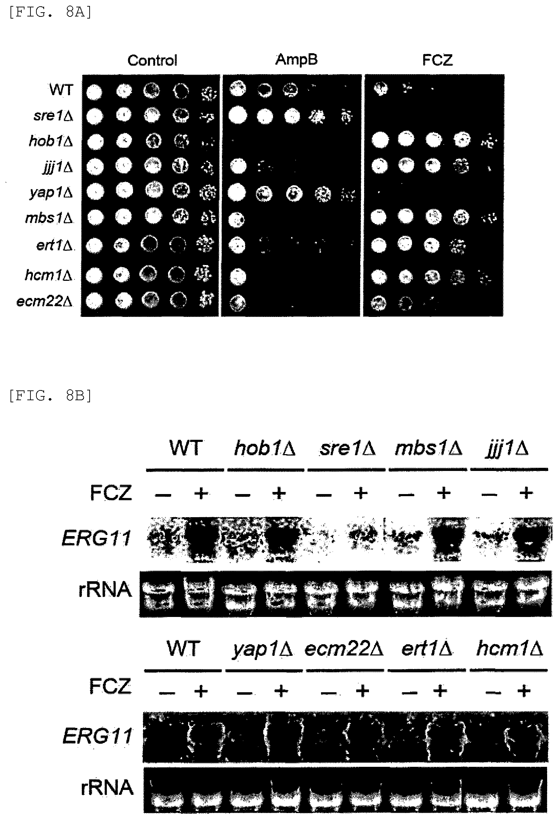

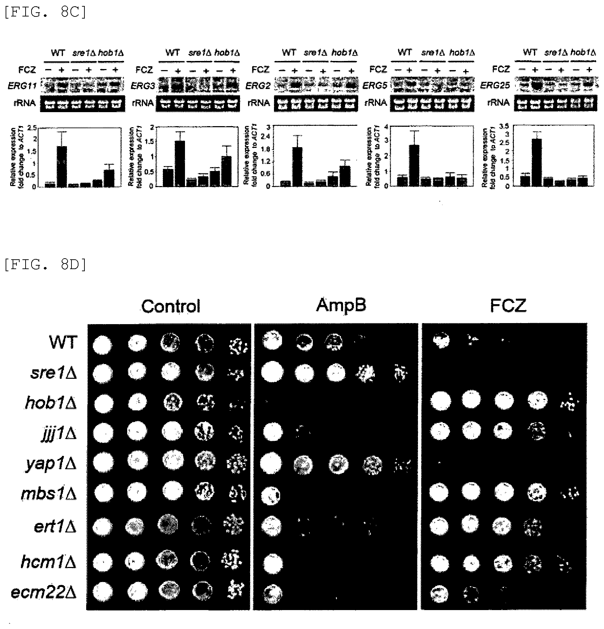

FIGS. 8a to 8d show that transcription factors regulating sterol biosynthesis genes govern general environmental stress responses and adaptation in *Cryptococcus neoformans.* FIG. 8a shows the results of observing the susceptibility of eight transcription factor mutants to antifungal drugs; FIG. 8b shows the results of Northern blot analysis performed using an ERG11-specific probe for the susceptibility of various transcription factor mutants to fluconazole (FCZ); FIG. 8c shows the results of Northern blot analysis performed using an ERG gene-specific probe for the susceptibility of a *sre1* mutant and *hob1* mutant to fluconazole (FCZ); FIG. 8d shows the results of observing the responses of a *sre1* mutant and *hob1* mutant to stress-inducing agents; and FIG. 8e shows a proposed model for the role of Sre1 and Hob1 in the sterol homeostasis and general stress responses of *Cryptococcus neoformans.*
FIGS. 9a and 9b show transcription factors involved in the virulence of *Cryptococcus neoformans.*

### [Mode for Invention]

Hereinafter, the present invention will be described in further detail with reference to examples. It will be obvious to those skilled in the art that these examples are for illustrative purposes and are not intended to limit the scope of the present invention as defined in the appended claims.

### Example 1: Construction of Transcription Factor Mutants

### 1.1: Selection of Transcription Factors and Transcription Factor Mutants

Putative transcription factors were screened using the published DBD transcription factor (TF) prediction database (http://www.transcriptionfactor.org/) (Non-Patent Document 8). The *Cryptococcus neoformans* H99 strain (a serotype A genome-sequence platform strain) contains 188 transcription factors (148 predicted from Pfam and 96 from SUPERFAMILY). Because these transcription factors were predicted based on the first version of the annotated H99 genome database, the present inventors updated this database with reference to the most recent version (version 7) of the annotated H99 genome database (Non-Patent Document 6), which resulted in a final prediction of 155 transcription factors (Table 1 below). The result of Orthologue mapping based on the BLAST e-value matrix demonstrated that *Cryptococcus neoformans* contains several evolutionarily distinct groups of transcription factors. The Cryptococcus DNA binding domain (DBD) transcription factors were classified based on their DNA binding domains (DBDs). 44% of these transcription factors (78) contain a fungal Zn2-Cys6 DBD, and among these, 40 also harbor a fungal-specific transcription factor domain. Several transcription factors contain more than two transcription factor domains (Table 1).

**[Table 1]**

| List of *Cryptococcus neoformans* transcription factors predicted based on DNA-binding domain database | | | |
|---|---|---|---|
| No. | H 9 9 ID | TF domains | Gene Name |
| 1 | 02566 | "Winged helix" DNA-binding domain / Fork head domain | *FKH2* |
| 2 | 00791 | Helix-loop-helix DNA-binding domain | *HLH1* |
| 3 | 01069 | Fungal Zn(2)-Cys(6) binuclear cluster domain / Fungal specific transcription factor domain | *FZC11* |
| 4 | 07464 | APSES domain / Ankyrin repeat#2 | *MBS1* |
| 5 | 03401 | Glucocorticoid receptor-like (DNA-binding domain) / GATA zinc finger | *GAT203* |
| 6 | 04588 | Fungal Zn(2)-Cys(6) binuclear cluster domain | *ERT1* |
| 7 | 00828 | Fungal Zn(2)-Cys(6) binuclear cluster domain / Fungal specific transcription factor domain | *SIP401* |
| 8 | 03561 | Fungal Zn(2)-Cys(6) binuclear cluster domain / Fungal specific transcription factor domain | *FZC33* |
| 9 | 06762 | Glucocorticoid receptor-like (DNA-binding domain) / GATA zinc finger | *GAT204* |
| 10 | 06276 | Fungal Zn(2)-Cys(6) binuclear cluster domain / Fungal specific transcription factor domain | *CEP3* |
| 11 | 05785 | Fungal Zn(2)-Cys(6) binuclear cluster domain | *STB4* |
| 12 | 03132 | Fungal Zn(2)-Cys(6) binuclear cluster domain | *FZC5* |
| 13 | 01438 | KilA-N domain / Ankyrin repeats (many copies) | *MBS2* |
| 14 | 07593 | bZIP transcription factor / Domain of unknown function (DUF3425) | *Y4P4* |
| 15 | 04837 | Helix-loop-helix DNA-binding domain | *MLN1* |
| 16 | 05093 | Homeobox domain | *HOB6* |
| 17 | 05642 | Fungal Zn(2)-Cys(6) binuclear cluster domain / Fungal specific transcription factor domain | *FZC37* |
| 18 | 05431 | Zinc-finger double domain / C2H2-type zinc finger | *RIM101* |
| 19 | 04398 | specific Fungal specific transcription factor domain / Fungal Zn(2)-Cys(6) binuclear cluster domain | *ARO80* |
| 20 | 04878 | Fungal Zn(2)-Cys(6) binuclear cluster domain / Fungal specific transcription factor domain | *FZC1* |
| 21 | 03183 | Fungal Zn(2)-Cys(6) binuclear cluster domain / Fungal specific transcription factor domain | *FZC24* |
| 22 | 03710 | Fungal Zn(2)-Cys(6) binuclear cluster domain / Fungal specific transcription factor domain | *ECM22* |
| 23 | 03279 | Fungal Zn(2)-Cys(6) binuclear cluster domain / Fungal specific transcription factor domain | *CCD4* |
| 24 | 04637 | Helix-turn-helix/lambda repressor-like DNA-binding domains | *MBF1* |
| 25 | 06425 | Fungal Zn(2)-Cys(6) binuclear cluster domain / Fungal specific transcription factor domain | *PPR1* |
| 26 | 04345 | Fungal Zn(2)-Cys(6) binuclear cluster domain | *ARO8001* |
| 27 | 04184 | Fungal Zn(2)-Cys(6) binuclear cluster domain / Fungal specific transcription factor domain | *FZC47* |
| 28 | 02774 | Fungal Zn(2)-Cys(6) binuclear cluster domain / Fungal specific transcription factor domain | *MAL13* |
| 29 | 00670 | Fungal Zn(2)-Cys(6) binuclear cluster domain | *FZC12* |
| 30 | 00068 | Zinc-finger double domain / C2H2-type zinc finger | *MET32* |
| 31 | 05010 | C2H2-type zinc finger | *ZFC7* |
| 32 | 04090 | bZIP transcription factor / Basic region leucine zipper | *ATF1* |
| 33 | 06134 | bZIP transcription factor / Basic region leucine zipper | *BZP1(HXL1)* |
| 34 | 04630 | bZIP transcription factor / Basic region leucine zipper | *BAP2* |
| 35 | 07901 | Fungal Zn(2)-Cys(6) binuclear cluster domain / Fungal specific transcription factor domain | *FZC29* |
| 36 | 01173 | Beta-trefoil DNA-binding domain / p53-like transcription factors / DNA-binding protein LAG-1 (CSL) | *PAN1* |
| 37 | 03115 | Fungal Zn(2)-Cys(6) binuclear cluster domain / Fungal specific transcription factor domain | *FZC46* |
| 38 | 07924 | SRF-type transcription factor (DNA-binding and dimerisation | *MCM1* |
| 39 | 07435 | CCAAT-binding transcription factor (CBF-B/NF-YA) subunit B | *HAP2* |
| 40 | 02555 | Fungal Zn(2)-Cys(6) binuclear cluster domain / Fungal specific transcription factor domain | *SIP402* |
| 41 | 04594 | Fungal Zn(2)-Cys(6) binuclear cluster domain / Fungal specific transcription factor domain | *FZC27* |
| 42 | 06188 | Fungal Zn(2)-Cys(6) binuclear cluster domain | *FZC15* |
| 43 | 05170 | Fungal Zn(2)-Cys(6) binuclear cluster domain | *PIP2* |
| 44 | 02241 | Homeodomain-like / Helix-turn-helix domain | *HOB5* |
| 45 | 06921 | Homeobox domain | *HOB4* |
| 46 | 05186 | GRF zinc finger | *GRF1* |
| 47 | 00896 | Fungal Zn(2)-Cys(6) binuclear cluster domain / Fungal specific transcription factor domain | *FZC34* |
| 48 | 00039 | C2H2-type zinc finger | *ZFC6* |
| 49 | 07940 | Basic region leucine zipper / bZIP transcription factor | *BZP5* |
| 50 | 06814 | Homeobox KN domain | *SXI1alpha* |
| 51 | 01454 | STE like transcription factor / Zinc-finger double domain / C2H2-type zinc finger | *STE12* |
| 52 | 03527 | C2H2-type zinc finger / C3HC4-type zinc finger | *HEL2* |
| 53 | 05255 | Fungal Zn(2)-Cys(6) binuclear cluster domain / Fungal specific transcription factor domain | *FZC2* |
| 54 | 05112 | Fungal Zn(2)-Cys(6) binuclear cluster domain / Fungal specific transcription factor domain | *FZC42* |
| 55 | 01883 | Glucocorticoid receptor-like (DNA-binding domain) / GATA zinc finger | *GAT8* |
| 56 | 04353 | C2H2-type zinc finger | *CLR1* |
| 57 | 05375 | Helix-loop-helix DNA-binding domain | *HLH2* |
| 58 | 03998 | SRF-type transcription factor (DNA-binding and dimerisation | *RLM1* |
| 59 | 00239 | bZIP transcription factor / Basic region leucine zipper | *BAP1* |
| 60 | 06871 | Fungal Zn(2)-Cys(6) binuclear cluster domain / Fungal specific transcription factor domain | *FZC41* |
| 61 | 00156 | C2H2-type zinc finger | *SP1(CRZ1)* |
| 62 | 04268 | GRF zinc finger | *APN2* |
| 63 | 05420 | Zinc-finger double domain / C2H2-type zinc finger | *USV101* |
| 64 | 00018 | Fungal Zn(2)-Cys(6) binuclear cluster domain | *FZC6* |
| 65 | 03346 | bZIP transcription factor | *BZP4* |
| 66 | 00514 | Glucocorticoid receptor-like (DNA-binding domain) / GATA zinc finger | *GAT6* |
| 67 | 05538 | SRR1 domain / C2H2-type zinc finger / DnaJ domain | *JJJ1* |
| 68 | 03409 | "Winged helix" DNA-binding domain / CheY-like / HSF-type DNA-binding | *SKN7* |
| 69 | 06339 | Fungal Zn(2)-Cys(6) binuclear cluster domain / Fungal specific transcription factor domain | *FZC35* |
| 70 | 07011 | Fungal Zn(2)-Cys(6) binuclear cluster domain / Fungal specific transcription factor domain | *FZC22* |
| 71 | 07506 | NF-X1 type zinc finger #3 / R3H domain | *FAP1* |
| 72 | 04807 | Fungal Zn(2)-Cys(6) binuclear cluster domain | *FZC8* |
| 73 | 02435 | PYP-like sensor domain (PAS domain) / Glucocorticoid receptor-like (DNA-binding domain) / GATA zinc finger / AT hook motif | *B W C 2* (*CWC2*) |
| 74 | 02364 | Fungal Zn(2)-Cys(6) binuclear cluster domain | *FZC19* |
| 75 | 03116 | "Winged helix" DNA-binding domain / Fork head domain | *HCM1* |
| 76 | 02877 | Zinc-finger double domain / Fungal Zn(2)-Cys(6) binuclear cluster domain | *FZC51* |
| 77 | 00559 | bZIP transcription factor / Basic region leucine zipper | *BZP3* |
| 78 | 03914 | Fungal Zn(2)-Cys(6) binuclear cluster domain / Fungal specific transcription factor domain | *FZC14* |
| 79 | 00871 | bZIP transcription factor / Basic region leucine zipper | *CLR3* |
| 80 | 06483 | Fungal Zn(2)-Cys(6) binuclear cluster domain | *FZC25* |
| 81 | 07797 | Putative FMN-binding domain | *CRL6* |
| 82 | 05019 | Fungal specific transcription factor domain | *FZC21* |
| 83 | 05380 | Fungal Zn(2)-Cys(6) binuclear cluster domain / Fungal specific transcription factor domain | *FZC44* |
| 84 | 04518 | Fungal specific transcription factor domain / C2H2-type zinc finger | *ZFC5* |
| 85 | 05176 | Homeobox domain | *H0B3* |
| 86 | 05861 | Fork head domain | *FKH101* |
| 87 | 00460 | Helix-loop-helix DNA-binding domain | *LIV1* |
| 88 | 02305 | Fungal Zn(2)-Cys(6) binuclear cluster domain / Fungal specific transcription factor domain | *FZC45* |
| 89 | 03849 | Fungal specific transcription factor domain / Fungal Zn(2)-Cys(6) binuclear cluster domain | *ASG1* |
| 90 | 01014 | C2H2-type zinc finger | *ZFC4* |
| 91 | 01858 | Homeobox domain | *HOB2* |
| 92 | 06719 | Fungal Zn(2)-Cys(6) binuclear cluster domain / Fungal specific transcription factor domain | *FZC49* |
| 93 | 04093 | Fungal Zn(2)-Cys(6) binuclear cluster domain / Fungal specific transcription factor domain | *YRM103* |
| 94 | 04176 | "Winged helix" DNA-binding domain / HSF-type DNA-blinding | *HSF2* |
| 95 | 01431 | Homeobox domain | *H0B1* |
| 96 | 07443 | Helix-loop-helix DNA-binding domain | *HLH4* |
| 97 | 04916 | Fungal Zn(2)-Cys(6) binuclear cluster domain | *FZC16* |
| 98 | 05392 | Zinc-finger double domain / C2H2-type zinc finger | *ZAP104* |
| 99 | 02322 | Fungal Zn(2)-Cys(6) binuclear cluster domain | *FZC17* |
| 100 | 04012 | Fungal Zn(2)-Cys(6) binuclear cluster domain | *FZC18* |
| 101 | 00505 | Fungal Zn(2)-Cys(6) binuclear cluster domain | *FZC28* |
| 102 | 06751 | Helix-loop-helix DNA-binding domain | *HLH3* |
| 103 | 04841 | Fungal Zn(2)-Cys(6) binuclear cluster domain / Fungal specific transcription factor domain | *FZC43* |
| 104 | 03212 | "Winged helix" DNA-binding domain | *HCM101* |
| 105 | 01626 | Zinc finger, ZZ type / Myb-like DNA-binding domain / SWIRM domain | *ADA2* |
| 106 | 3894 | Fungal Zn(2)-Cys(6) binuclear cluster domain | *PDR802* |
| 107 | 2066 | Fungal Zn(2)-Cys(6) binuclear cluster domain | *FZC13* |
| 108 | 6818 | Fungal Zn(2)-Cys(6) binuclear cluster domain / Fungal specific transcription factor domain | *HAP1* |
| 109 | 5940 | C2H2-type zinc finger | *ZFC3* |
| 110 | 1948 | Fungal Zn(2)-Cys(6) binuclear cluster domain / Fungal specific transcription factor domain | *FZC36* |
| 111 | 4804 | Helix-loop-helix DNA-binding domain | *SRE1* |
| 112 | 4352 | Zinc-finger double domain / C2H2-type zinc finger | *ZAP103* |
| 113 | 3768 | Fungal Zn(2)-Cys(6) binuclear cluster domain / Fungal specific transcription factor domain | *FZC32* |
| 114 | 1977 | Fungal Zn(2)-Cys(6) binuclear cluster domain | *FZC39* |
| 115 | 4895 | Fungal Zn(2)-Cys(6) binuclear cluster domain / Fungal specific transcription factor domain | *FZC3* |
| 116 | 4583 | WSTF, HB1, Itc1p, MBD9 motif 2/3 / DDT domain | *DDT1* |
| 117 | 1973 | Zinc-finger double domain / C2H2-type zinc finger / Fungal specific transcription factor domain | *ZFC2* |
| 118 | 2603 | Fungal specific transcription factor domain / C2H2-type zinc finger | *ZFC1* |
| 119 | 4036 | "Winged helix" DNA-binding domain / HSF-type DNA-binding | *HSF3* |
| 120 | 6156 | Fungal Zn(2)-Cys(6) binuclear cluster domain / Fungal specific transcription factor domain | *FZC7* |
| 121 | 03431 | Fungal Zn(2)-Cys(6) binuclear cluster domain / Fungal specific transcription factor domain | *FZC48* |
| 122 | 07922 | Fungal Zn(2)-Cys(6) binuclear cluster domain / Fungal specific transcription factor domain | *FZC4* |
| 123 | 00031 | Fungal Zn(2)-Cys(6) binuclear cluster domain / Fungal specific transcription factor domain | *MLR1* |
| 124 | 03018 | Fungal Zn(2)-Cys(6) binuclear cluster domain / Fungal specific transcription factor domain | *ASG101* |
| 125 | 04263 | Glucocorticoid receptor-like (DNA-binding domain) / Basic region leucine zipper / bZIP transcription factor / GATA zinc finger | *BZP2* |
| 126 | 01708 | GATA zinc finger | *GAT7* |
| 127 | 00332 | Fungal Zn(2)-Cys(6) binuclear cluster domain | *SIP4* |
| 128 | 07724 | Copper fist DNA binding domain | *CUF1* |
| 129 | 03902 | Fungal Zn(2)-Cys(6) binuclear cluster domain/PAS fold | *RDS2* |
| 130 | 03366 | Zinc-finger double domain / C2H2-type zinc finger | *ZNF2* |
| 131 | 05222 | C2H2-type zinc finger / Zinc-finger double domain | *NRGI* |
| 132 | 05049 | Fungal Zn(2)-Cys(6) binuclear cluster domain / Fungal specific transcription factor domain | *P1P201* |
| 133 | 02516 | Helix-loop-helix DNA-binding domain | *HLH5* |
| 134 | 04774 | Fungal Zn(2)-Cys(6) binuclear cluster domain / Fungal specific transcription factor domain | *FZC26* |
| 135 | 03059 | Fungal Zn(2)-Cys(6) binuclear cluster domain | *FZC9* |
| 136 | 00193 | Glucocorticoid receptor-like (DNA-binding domain) / GATA zinc finger | *GAT1* |
| 137 | 03336 | Fungal Zn(2)-Cys(6) binuclear cluster domain / Fungal specific transcription factor domain | *FZC50* |
| 138 | 03086 | Fungal Zn(2)-Cys(6) binuclear cluster domain / Fungal specific transcription factor domain | *FZC20* |
| 139 | 03229 | Homeobox domain / Homeobox KN domain | *YOX101* |
| 140 | 01841 | Glucocorticoid receptor-like [DNA-binding domain) / GATA zinc finger | *GLN3* |
| 141 | 02476 | Fungal Zn(2)-Cys(6) binuclear cluster domain / Fungal specific transcription factor domain | *YRM101* |
| 142 | 05153 | Glucocorticoid receptor-like (DNA-binding domain) / GATA zinc finger / AT hook motif | *GAT5* |
| 143 | 02700 | C2H2-type zinc finger | *ZFC8* |
| 144 | 04586 | Homeobox domain | *HOB7* |
| 145 | 2723 | Fungal Zn(2)-Cys(6) binuclear cluster domain / Fungal specific transcription factor domain | *FZC23* |
| 146 | 3741 | Fungal Zn(2)-Cys(6) binuclear cluster domain / AT hook motif | *FZC31* |
| 147 | 4457 | Fungal Zn(2)-Cys(6) binuclear cluster domain / AT hook motif. | *FZC30* |
| 148 | 6283 | Homeodomain-like | *LIV4* |
| 149 | 7411 | C5HC2 zinc finger / PHD-finger / ARID/BRIGHT DNA binding | *RUM1* |
| 150 | 4836 | Fungal Zn(2)-Cys(6) binuclear cluster domain / Fungal specific transcription factor domain | *FZC10* |
| 151 | 841 | Fungal Zn(2)-Cys(6) binuclear cluster domain / Fungal specific transcription factor domain | *FZC40* |
| 152 | 6223 | MIZ/SP-RING zinc finger | *MIZ1* |
| 153 | 830 | Fungal Zn(2)-Cys(6) binuclear cluster domain / Fungal specific transcription factor domain | *FZC38* |
| 154 | 1551 | Glucocorticoid receptor-like (DNA-binding domain)/ GATA zinc finger | *GAT201* |
| 155 | 4908 | bZIP transcription factor / Basic region leucine zipper | *CLR4* |

To analyze the functions of the transcription factors, the present inventors deleted 155 putative transcription factor genes out of 178 using homologous recombination. To perform a large-scale *in vivo* virulence test, dominant nourseothricin-resistance markers (NATs) containing a series of signature tags were employed.

The genotypes of all transcription factor mutant strains were confirmed by performing Southern blot analysis to verify both the gene deletion and the absence of any ectopic integration of each gene-disruption cassette in transcription factor mutant strains. To accurately validate the phenotype and exclude unlinked mutational effects, the present inventors generated more than two independent transcription factor mutants for all 155 transcription factors, including four known transcription factors (HXL1, ATF1, MBS1 and SKN7) (Non-Patent Document 9, Non-Patent Document 10 and Non-Patent Document 11), and thus obtained a total of 322 strains (see Table 2 below).

**[Table 2]**

| Transcription factor mutants | | | | |
|---|---|---|---|---|
| | H99 ID | Designated name | TF class | Strain information |
| 1 | 02566 | *FKH2* | FKH | YSB1339 |
| 2 | | | | YSB1340 |
| 3 | 00791 | *HLH1* | HLH | YSB1175 |
| 4 | | | | YSB1176 |
| 5 | 07464 | *MBS1* | APS | YSB488 |
| 6 | | | | YSB489 |
| 7 | 03401 | *GAT203* | GAT | YSB569 |
| 8 | | | | YSB570 |
| 9 | 04588 | *ERT1* | FZC | YSB693 |
| 10 | | | | YSB694 |
| 11 | 00828 | *SIP401* | FZC | YSB1358 |
| 12 | | | | YSB1359 |
| 13 | 03561 | *FZC33* | FZC | YSB1074 |
| 14 | | | | YSB1075 |
| 15 | 06762 | *GAT204* | GAT | YSB1311 |
| 16 | | | | YSB1312 |
| 17 | 06276 | *CEP3* | FZC | YSB847 |
| 18 | | | | YSB848 |
| 19 | 05785 | *STB4* | FZC | YSB1013 |
| 20 | | | | YSB1014 |
| 21 | 01438 | *MBS2* | APS | YSB538 |
| 22 | | | | YSB539 |
| 23 | 07593 | *YAP4* | BZP | YSB1587 |
| 24 | | | | YSB1661 |
| 25 | 04837 | *MLN1* | HLH | YSB1172 |
| 26 | | | | YSB1173 |
| 27 | 05093 | *HOB6* | HOM | YSB1255 |
| 28 | | | | YSB1256 |
| 29 | 05642 | *FZC37* | FZC | YSB1329 |
| 30 | | | | YSB1330 |
| 31 | 05431 | *RIM101* | C2Z | YSB1366 |
| 32 | | | | YSB1367 |
| 33 | 04398 | *ARO80* | FZC | YSB714 |
| 34 | | | | YSB715 |
| 35 | 04878 | *FZC1* | FZC | YSB510 |
| 36 | | | | YSB511 |
| 37 | 03183 | *FZC24* | FZC | YSB774 |
| 38 | | | | YSB775 |
| 39 | 03710 | *ECM22* | FZC | YSB476 |
| 40 | | | | YSB478 |
| 41 | 03279 | *CCD4* | HOM | YSB706 |
| 42 | | | | YSB707 |
| 43 | 04637 | *MBF1* | HTH | YSB768 |
| 44 | | | | YSB769 |
| 45 | 06425 | *PPR1* | FZC | YSB1046 |
| 46 | | | | YSB1047 |
| 47 | 04345 | *AR08001* | FZC | YSB661 |
| 48 | | | | YSB662 |
| 49 | 04184 | *FZC47* | FZC | YSB1406 |
| 50 | | | | YSB1407 |
| 51 | 02774 | *MAL13* | FZC | YSB506 |
| 52 | | | | YSB507 |
| 53 | 00670 | *FZC12* | FZC | YSB467 |
| 54 | | | | YSB468 |
| 55 | 05010 | *ZFC7* | C2Z | YSB481 |
| 56 | | | | YSB482 |
| 57 | 04090 | *ATF1* | BZP | YSB676 |
| 58 | | | | YSB678 |
| 59 | 06134 | *8ZP1(HXL1)* | BZP | YSB723 |
| 60 | | | | YSB724 |
| 61 | 04630 | *YAP2* | BZP | YSB1416 |
| 62 | | | | YSB1417 |
| 63 | 07901 | *FZC29* | FZC | YSB718 |
| 64 | | | | YSB719 |
| 65 | 03115 | *FEZ46* | FZC | YSB1209 |
| 66 | | | | YSB1210 |
| 67 | 07924 | *MCM1* | SRF | YSB1302 |
| 68 | | | | YSB1303 |
| 69 | 07435 | *HAP2* | CCA | YSB1104 |
| 70 | | | | YSB1105 |
| 71 | 02555 | *SIP402* | FZC | YSB529 |
| 72 | | | | YSB530 |
| 73 | 04594 | *FZC27* | FZC | YSB582 |
| 74 | | | | YSB583 |
| 75 | 06188 | *FZC15* | FZC | YSB646 |
| 76 | | | | YSB647 |
| 77 | 05170 | *PIP2* | FZC | YSB1249 |
| 78 | | | | YSB1250 |
| 79 | 02241 | *HOB5* | HOM | YSB1585 |
| 80 | | | | YSB1586 |
| 81 | 06921 | *HOB4* | HOM | YSB1435 |
| 82 | | | | YSB1437 |
| 83 | 05186 | *GRF1* | GRF | YSB796 |
| 84 | | | | YSB797 |
| 85 | 00039 | *ZFC6* | C2Z | YSB1953 |
| 86 | | | | YSB1954 |
| 87 | 01454 | *STE12* | C2Z | YSB1542 |
| 88 | | | | YSB1543 |
| 89 | 03527 | *HEL2* | C2Z | YSB1382 |
| 90 | | | | YSB1383 |
| 91 | 05255 | *FZC2* | FZC | YSB1050 |
| 92 | | | | YSB1051 |
| 93 | 01883 | *GAT8* | GAT | YSB471 |
| 94 | | | | YSB472 |
| 95 | 04353 | *CLR1* | C2Z | YSB1396 |
| 96 | | | | YSB1397 |
| 97 | 03998 | *RLM1* | SRF | YSB1300 |
| 98 | | | | YSB1301 |
| 99 | 00239 | *YAP1* | BZP | YSB815 |
| 100 | | | | YSB1290 |
| 101 | 06871 | *FZC41* | FZC | YSB1334 |
| 102 | | | | YSB1335 |
| 103 | 00156 | *SP1(CRZ1)* | C2Z | YSB1263 |
| 104 | | | | YSB1264 |
| 105 | 04268 | *APN2* | GRF | YSB1429 |
| 106 | | | | YSB1430 |
| 107 | 05420 | *USV101* | C2Z | YSB1464 |
| 108 | | | | YSB1465 |
| 109 | 00018 | *FZC6* | FZC | YSB1980 |
| 110 | | | | YSB1981 |
| 111 | 03346 | *BZP4* | BZP | YSB1894 |
| 112 | | | | YSB1895 |
| 113 | 05538 | *JJJ1* | C2Z | YSB1592 |
| 114 | | | | YSB1594 |
| 115 | 03409 | *SKN7* | HSF | YSB349 |
| 116 | | | | YSB350 |
| 117 | 06339 | *FZC35* | FZC | YSB1341 |
| 118 | | | | YSB1342 |
| 119 | 07506 | *FAP1* | NFX | YSB813 |
| 120 | | | | YSB817 |
| 121 | 04807 | *FZC8* | FZC | YSB2112 |
| 122 | | | | YSB2113 |
| 123 | 02435 | *BWC2* | GAT | YSB1839 |
| 124 | | | | YSB1840 |
| 125 | 02364 | *FZC19* | FZC | YSB2115 |
| 126 | | | | YSB2116 |
| 127 | 03116 | *HCM1* | FKH | YSB1850 |
| 128 | | | | YSB1851 |
| 129 | 02877 | *FZC51* | FZC | YSB1842 |
| 130 | | | | YSB1843 |
| 131 | 00559 | *BZP3* | BZP | YSB1099 |
| 132 | | | | YSB1100 |
| 133 | 03914 | *FZC14* | FZC | YSB1846 |
| 134 | | | | YSB1847 |
| 135 | 00871 | *CLR3* | BZP | YSB1834 |
| 136 | | | | YSB1836 |
| 137 | 06483 | *FZC25* | FZC | YSB518 |
| 138 | | | | YSB1822 |
| 139 | 07797 | *CRL6* | FKH | YSB1106 |
| 140 | | | | YSB1107 |
| 141 | 05019 | *FZC21* | FZC | YSB1252 |
| 142 | | | | YSB1253 |
| 143 | 05380 | *FZC44* | FZC | YSB2182 |
| 144 | | | | YSB2183 |
| 145 | 04518 | *ZFC5* | C2Z | YSB2177 |
| 146 | | | | YSB2178 |
| 147 | 05176 | *HOB3* | HOM | YSB2001 |
| 148 | | | | YSB2002 |
| 149 | 05861 | *FKH101* | FKH | YSB1855 |
| 150 | | | | YSB1856 |
| 151 | 00460 | *LIV1* | HLH | YSB2211 |
| 152 | | | | YSB2212 |
| 153 | 02305 | *FZC45* | FZC | YSB2221 |
| 154 | | | | YSB2222 |
| 155 | 03849 | *ASG1* | FZC | YSB3013 |
| 156 | | | | YSB3014 |
| 157 | 01014 | *ZFC4* | C2Z | YSB2231 |
| 158 | | | | YSB2232 |
| 159 | 01858 | *HOB2* | HOM | YSB2282 |
| 160 | | | | YSB2283 |
| 161 | 06719 | *FZC49* | FZC | YSB2171 |
| 162 | | | | YSB2173 |
| 163 | 04093 | *YRM103* | FZC | YSB2298 |
| 164 | | | | YSB2299 |
| 165 | 04176 | *HSF2* | HSF | YSB2295 |
| 166 | | | | YSB2296 |
| 167 | 01431 | *HOB1* | HOM | YSB2308 |
| 168 | | | | YSB2309 |
| 169 | 07443 | *HLH4* | HOM | YSB2244 |
| 170 | | | | YSB2245 |
| 171 | 04916 | *FZC16* | FZC | YSB2326 |
| 172 | | | | YSB2327 |
| 173 | 05392 | *ZAP104* | C2Z | YSB2134 |
| 174 | | | | YSB2135 |
| 175 | 02322 | *FZC17* | FZC | YSB2250 |
| 176 | | | | YSB2251 |
| 177 | 04012 | *FZC18* | FZC | YSB2320 |
| 178 | | | | YSB2321 |
| 179 | 00505 | *FZC28* | FZC | YSB2337 |
| 180 | | | | YSB2338 |
| 181 | 06751 | *HLH3* | HLH | YSB2329 |
| 182 | | | | YSB2330 |
| 183 | 04841 | *FZC43* | FZC | YSB517 |
| 184 | | | | YSB2334 |
| 185 | 03212 | *HCM101* | FKH | YSB2390 |
| 186 | | | | YSB2391 |
| 187 | 01626 | *ADA2* | MYB | YSB2381 |
| 188 | | | | YSB2382 |
| 189 | 03894 | *PDR802* | FZC | YSB2387 |
| 190 | | | | YSB2388 |
| 191 | 02066 | *FZC13* | FZC | YSB2517 |
| 192 | | | | YSB2518 |
| 193 | 06818 | *HAP1* | FZC | YSB2481 |
| 194 | | | | YSB2482 |
| 195 | 05940 | *ZFC3* | C2Z | YSB2108 |
| 196 | | | | YSB2386 |
| 197 | 01948 | *FZC36* | FZC | YSB2335 |
| 198 | | | | YSB2523 |
| 199 | 04804 | *SRE1* | HLH | YSB2493 |
| 200 | | | | YSB2494 |
| 201 | 04352 | *ZAP103* | C2Z | YSB2540 |
| 202 | | | | YSB2541 |
| 203 | 03768 | *FZC32* | FZC | YSB2385 |
| 204 | | | | YSB2526 |
| 205 | 04895 | *FZC3* | FZC | YSB2611 |
| 206 | | | | YSB2664 |
| 207 | 04583 | *DDT1* | DDT | YSB1583 |
| 208 | | | | YSB2633 |
| 209 | 01973 | *ZFC2* | C2Z | YSB2622 |
| 210 | | | | YSB2623 |
| 211 | 06156 | *FZC7* | FZC | YSB2704 |
| 212 | | | | YSB2705 |
| 213 | 03431 | *FZC48* | FZC | YSB2646 |
| 214 | | | | YSB2647 |
| 215 | 07922 | *FZC4* | FZC | YSB2724 |
| 216 | | | | YSB2725 |
| 217 | 00031 | *MLR1* | FZC | YSB2727 |
| 218 | | | | YSB2728 |
| 219 | 03018 | *ASG101* | FZC | YSB2697 |
| 220 | | | | YSB2698 |
| 221 | 04263 | *BZP2* | BZP | YSB2702 |
| 222 | | | | YSB2703 |
| 223 | 01708 | *GAT7* | GAT | YSB2699 |
| 224 | | | | YSB2700 |
| 225 | 00332 | *SIP4* | FZC | YSB2680 |
| 226 | | | | YSB2681 |
| 227 | 07724 | *CUF1* | CDB | YSB2665 |
| 228 | | | | YSB2666 |
| 229 | 03902 | *RDS2* | FZC | YSB1898 |
| 230 | | | | YSB1899 |
| 231 | 03366 | *ZNF2* | C2Z | YSB2740 |
| 232 | | | | YSB2741 |
| 233 | 05222 | *NRG1* | C2Z | YSB3096 |
| 234 | | | | YSB3097 |
| 235 | 05049 | *PIP201* | FZC | YSB3099 |
| 236 | | | | YSB3100 |
| 237 | 02516 | *HLH5* | HLH | YSB2609 |
| 238 | | | | YSB3059 |
| 239 | 04774 | *FZC26* | FZC | YSB3084 |
| 240 | | | | YSB3085 |
| 241 | 03336 | *FZC50* | FZC | YSB3131 |
| 242 | | | | YSB3132 |
| 243 | 03086 | *FZC20* | FZC | YSB3128 |
| 244 | | | | YSB3129 |
| 245 | 03229 | *YOX101* | HOM | YSB3134 |
| 246 | | | | YSB3136 |
| 247 | 01841 | *GLN3* | GAT | YSB3154 |
| 248 | | | | YSB3155 |
| 249 | 02476 | *YRM101* | FZC | YSB2997 |
| 250 | | | | YSB2998 |
| 251 | 05153 | *GAT5* | GAT | YSB3033 |
| 252 | | | | YSB3034 |
| 253 | 02700 | *ZFC8* | C2Z | YSB3031 |
| 254 | | | | YSB3032 |
| 255 | 04586 | *HOB7* | HOM | YSB3026 |
| 256 | | | | YSB3027 |
| 257 | 02723 | *FZC23* | FZC | YSB3105 |
| 258 | | | | YSB3106 |
| 259 | 03741 | *FZC31* | FZC | YSB3093 |
| 260 | | | | YSB3094 |
| 261 | 04457 | *FZC30* | FZC | YSB2447 |
| 262 | | | | YSB2448 |
| 263 | 04836 | *FZC10* | FZC | YSB3083 |
| 264 | | | | YSB3368 |
| 265 | 06223 | *MIZ1* | *MIZ* | YSB2133 |
| 266 | | | | YSB3366 |
| 267 | 01551 | *GAT201* | GAT | YSB3300 |
| 268 | | | | YSB3301 |
| 269 | 04908 | *CLR4* | BZP | YSB3282 |
| 270 | | | | YSB3283 |
| 271 | 07940 | *BZP5* | BZP | YSB1474 |
| 272 | | | | YSB1475 |
| 273 | 05112 | *FZC42* | FZC | YSB687 |
| 274 | | | | YSB690 |
| 275 | 00193 | *GAT1* | GAT | YSB2972 |
| 276 | | | | YSB2973 |
| 277 | 06814 | *SXI1alpha* | HOM | YSB1390 |
| 278 | | | | YSB1391 |
| 279 | | | | YSB1392 |
| 280 | 00896 | *FZC34* | FZC | YSB501 |
| 281 | | | | YSB2979 |
| 282 | 07411 | *RUM1* | PHZ | YSB3164 |
| 283 | | | | YSB3747 |
| 284 | 00830 | *ZC38* | FZC | YSB777 |
| 285 | | | | YSB3791 |
| 286 | 01069 | *FZC11* | FZC | YSB845 |
| 287 | | | | YSB846 |
| 288 | | | | YSB2983 |
| 289 | 03132 | *FZC5* | FZC | YSB1400 |
| 290 | | | | YSB1401 |
| 291 | | | | YSB1404 |
| 292 | 00068 | *MET32* | C2Z | YSB1178 |
| 293 | | | | YSB1179 |
| 294 | | | | YSB1180 |
| 295 | 01173 | *PAN1* | P53 | YSB1181 |
| 296 | | | | YSB1182 |
| 297 | | | | YSB1183 |
| 298 | 00514 | *GAT6* | GAT | YSB1384 |
| 299 | | | | YSB1385 |
| 300 | | | | YSB1386 |
| 301 | 07011 | *FZC22* | FZC | YSB1688 |
| 302 | | | | YSB1689 |
| 303 | | | | YSB2974 |
| 304 | 02603 | *ZFC1* | C2Z | YSB2573 |
| 305 | | | | YSB2574 |
| 306 | | | | YSB2575 |
| 307 | 04036 | *HSF3* | HSF | YSB2527 |
| 308 | | | | YSB2528 |
| 309 | | | | YSB2529 |
| 310 | 03059 | *FZC9* | FZC | YSB2984 |
| 311 | | | | YSB3266 |
| 312 | | | | YSB3267 |
| 313 | 06283 | *LIV4* | MYB | YSB2089 |
| 314 | | | | YSB3755 |
| 315 | | | | YSB3756 |
| 316 | 00841 | *FZC40* | FZC | YSB3088 |
| 317 | | | | YSB3758 |
| 318 | 01977 | *FZC39* | FZC | YSB1820 |
| 319 | | | | YSB2621 |
| 320 | 05375 | *HLH2* | HLH | YSB1147 |
| 321 | | | | YSB1148 |
| 322 | | | | YSB1149 |

For parallel *in vitro* and *in vivo* phenotypic analysis, the present inventors deleted 53 TF genes, which were previously deleted in the CM018 strain (a less virulent H99 strain, Non-Patent Document 12), and derived more than two independent mutants. Certain known transcription factors, including *RIM101, ADA2, CUF1, SXL1, SP-1*/*CRZ1, NRG1, STE12, BWC2, SRE1, ZNF2* and *HAP1*/*HAP2,* were also independently deleted here to accurately compare phenotypes. When two independent transcription factor mutants showed inconsistent phenotypes, additional transcription factor mutants were generated to exclude outlier mutants. The present inventors found that about 8% of gene knockouts (13 transcription factors) exhibited inconsistent phenotypes, potentially attributable to unexpected alterations in the genome. This level (7%) was highly similar to that reported in a similar study on the ascomycete fungal pathogen *Candida albicans* (Non-Patent Document 13). For the remaining 23 transcription factors, transcription factor mutants were not generated. In summary, the present inventors constructed a *Cryptococcus neoformans* transcription factor mutant collection that covers 155 transcription factors and 322 transcription factor mutant strains in total.

Out of the 156 transcription factors whose mutants were constructed, 58 transcription factor genes possess names designated in published studies or reserved by other researchers through registration in FungiDB (www.fungidb.org). For the remaining 98 transcription factors, the present inventors provided gene names by following the systematic genetic nomenclature flowchart in *Cryptococcus neoformans* recently reported (Non-Patent Document 14).

### 1.2: Construction of Transcription Factor Mutants

*Cryptococcus neoformans* transcription factor knockout mutants (hereinafter referred to as TFKO) were constructed in the *C. neoformans* serotype A H99S strain background. Gene-disruption cassettes containing the nourseothricin-resistance marker (NAT) and signature-tagged sequences were generated by overlap polymerase chain reaction (hereinafter referred to as PCR) or double-joint PCR strategies using the primer sets shown in the Sequence List and Table 2 (Non-Patent Document 15 and Non-Patent Document 16). In the overlap PCR process, the 5'- and 3'-flanking regions of the transcription factor genes were amplified by using primers L1 and R1 and primers L2 and R2 (see Table 1), respectively, together with H99 genomic DNA in the first round of PCR. Primers M13Fe (M13 forward extended) and M13Re (M13 reverse extended) were used for amplifying the dominant selectable marker (NAT) containing unique signature-tagged sequences. In the second round of PCR, the TF gene-disruption cassettes were generated by means of overlap PCR performed using primers L1 and R2 and the first-round PCR products as templates. In the double-joint PCR method, the 5'- and 3'-flanking regions of the transcription factor genes were amplified using, respectively, the primer pairs L1/L2 and R1/R2 with H99 genomic DNA in the first round of PCR. The 5'- and 3'-regions of NAT-split markers were amplified using primers M13Fe and NSL and primers M13Re and NSR, respectively, together with pNATSTM (obtained from Joeseph Heitman' Laboratory at Duke University), which harbored unique signature-tagged sequences. The amplified gene-disruption cassettes were combined with 600 µg of gold microcarrier beads (0.6 µm, Bio-Rad) and treated with 10 µL of 2.5M calcium chloride and 2 µL of 1M spermidine. The gold beads combined with the gene-disruption cassettes were introduced into the H99S strains (obtained from Joeseph Heitman' Laboratory at Duke University) using the biolistic transformation apparatus (Non-Patent Document 17). After 4 hours of culture required for recovery, the cells were scraped, transferred onto an yeast extract-peptone dextrose (hereinafter referred to as YPD) medium containing 100µg/ml nourseothricin, and then incubated at 30°C for 3 to 5 days. Stable nourseothricin-resistant transformants were screened by diagnostic PCR using the primer sets listed in Table 3 and the Sequence List.

All transcription factor mutant strains were deposited in the Korean Culture Collection of Microorganisms (KCCM) and the Center of Microbial Pathogenesis at Duke University in USA.

**[Table 3]**

| Construction of primer sets for genes | | |
|---|---|---|
| Gene name | Primer name | Detailed description of primers |
| *FZC5* | L1 | CNAG_03132 5' flanking region primer 1 |
| | L2 | CNAG_03132 5' flanking region primer 2 |
| | R1 | CNAG_03132 3' flanking region primer 1 |
| | R2 | CNAG_03132 3' flanking region primer 2 |
| | SO1 | CNAG_03132 diagnostic screening primer, pairing with B79 |
| | P02 | CNAG_03132 Southern blot probe primer |
| | STM | NAT#5 STM primer |
| | STM common | STM common primer |

### Example 2: Phenotypic Profiling

For the 322 transcription factor mutants constructed, the present inventors performed a series of *in vivo* and *in vitro* phenotypic analyses for the phenotypic classes as follows: growth, differentiation, morphology, stress responses, antifungal drug resistance, virulence-factor production and *in vivo* virulence. This overall phenome data set is illustrated together with a color scale, and data for transcript levels of each transcription factor measured by RNA sequencing analyses under six distinct growth conditions were measured. Red and blue in a thermal map showed decrease and increase, respectively. Phenotype strengths (strong, intermediate and weak) are distinguished in gradients of red or blue. The phenotypic analysis revealed that about 93% of the transcription factor mutants (145/155) exhibited at least one discernable phenotype, suggesting a high functional coverage of this transcription factor mutant collection. 85% of the transcription factors (132/155) have not been functionally characterized before in *Cryptococcus neoformans* strains.

All of these phenome data are publicly available in the *Cryptococcus neoformans* transcription factor database (http://tf.cryptococcus.org).

### 2.1: Genotypic Analysis

The accuracy of the genotypes of the positive transformants was validated by means of Southern blot analysis. Cryptococcus genomic DNA was extracted using the CTAB (cetyl trimethyl ammonium bromide) method (Non-Patent Document 33). Isolated genomic DNA from each TFKO mutant was digested with the indicated restriction enzyme (see Table 4). The digested genomic DNAs were separated by 1% agarose gel electrophoresis. The agarose gel was transferred into the denatured buffer containing 0.5M NaOH and 1.5M NaCl and allowed to stand for 45min. Next, the agarose gel was transferred into the neutralization buffer containing 1.5M NaCl and 0.5M Tris buffer adjusted with pH 8 and allowed to stand for 45min. The digested genomic DNAs were transferred to the nylon membrane using 10x SSC (saline sodium citrate) buffer and fixed by 1,200J/m² ultraviolet exposure. The membrane was hybridized with a gene-specific and radioactively labeled probe using modified church hybridization buffer (1mM EDTA, 0.25M Na₂HPO₄, 1% hydrolysated casein, 7% SDS, 6% H₃PO₄). The membrane was washed for 15minutes with washing buffer 1 (containing 2x SSC and 0.1% SDS) and washing buffer 2 (containing 1x SSC and 0.1% SDS). Next, the membrane was exposed to autography film for 1 day.

**[Table 4]**

| List of transcription factor knockout mutants and restriction enzymes used | | | |
|---|---|---|---|
| | H99 ID | Designated name | Restriction enzyme cut |
| 1 | 02566 | *FKH2* | BamHI |
| 2 | 00791 | *HLH1* | BglII |
| 3 | 01069 | *FZC11* | SphI |
| 4 | 07464 | *MBS1* | SphI |
| 5 | 03401 | *GAT203* | HindIII |
| 6 | 04588 | *ERT1* | EcoRV |
| 7 | 00828 | *SIP401* | EcoRI |
| 8 | 03561 | *FZC33* | EcoRV |
| 9 | 06762 | *GAT204* | KpnI |
| 10 | 06276 | *CEP3* | SphI |
| 11 | 05785 | *STB4* | BamHI |
| 12 | 03132 | *FZC5* | PstI |
| 13 | 01438 | *MBS2* | EcoRI |
| 14 | 07593 | *YAP4* | SmaI |
| 15 | 04837 | *LN1* | SmaI |
| 16 | 05093 | *H0B6* | KpnI |
| 17 | 05642 | *FZC37* | XmaI |
| 18 | 05431 | *RIM101* | ClaI |
| 19 | 04398 | *ARO80* | HincII |
| 20 | 04878 | *FZC1* | EcoRI |
| 21 | 03183 | *FZC24* | EcoRV |
| 22 | 03710 | *ECM22* | HindIII |
| 23 | 03279 | *CCD4* | HindIII |
| 24 | 04637 | *MBF1* | HindIII |
| 25 | 06425 | *PPR1* | EcoRV |
| 26 | 04345 | *AR8001* | BamHI |
| 27 | 04184 | *FZC47* | BglII |
| 28 | 02774 | *MAL13* | EcoRV |
| 29 | 00670 | *FZC12* | StyI |
| 30 | 00068 | *MET32* | AfeI |
| 31 | 05010 | *ZFC7* | ScaI |
| 32 | 04090 | *ATF1* | PstI |
| 33 | 06134 | *BZP1(HXL1)* | BamHI |
| 34 | 04630 | *YAP2* | HindIII |
| 35 | 07901 | *FZC29* | EcoRV |
| 36 | 01173 | *PAN1* | SalI |
| 37 | 03115 | *FZC46* | SacI |
| 38 | 07924 | *MCM1* | XbaI |
| 39 | 07435 | *HAP2* | SalI |
| 40 | 02555 | *SIP402* | MfeI |
| 41 | 04594 | *FZC27* | HindIII |
| 42 | 06188 | *FZC15* | BamHI |
| 43 | 05170 | *PIP2* | BamHI |
| 44 | 02241 | *HOB5* | PstI |
| 45 | 06921 | *HOB4* | KpnI |
| 46 | 05186 | *GRF1* | EcoRI |
| 47 | 00896 | *FZC34* | HindIII |
| 48 | 00039 | *ZFC6* | KpnI |
| 49 | 07940 | *BZP5* | BamHI |
| 50 | 06814 | *SXI1alpha* | SacI |
| 51 | 01454 | *STE12* | BamHI |
| 52 | 03527 | *HEL2* | BamHI |
| 53 | 05255 | *FZC2* | HidIII |
| 54 | 05112 | *FZC42* | BglII, EcoRV |
| 55 | 01883 | *GAT8* | SacI |
| 56 | 04353 | *CLR1* | SalI |
| 57 | 05375 | *HLH2* | HindIII |
| 58 | 03998 | *RLM1* | StyI |
| 59 | 00239 | *YAP1* | BamHI |
| 60 | 06871 | *FZC41* | BamHI, XbaI |
| 61 | 00156 | *SP1(CRZ1)* | EcoRV |
| 62 | 04268 | *APN2* | EcoRV |
| 63 | 05420 | *USV101* | SphI |
| 64 | 00018 | *FZC6* | SphI |
| 65 | 03346 | *BZP4* | PstI |
| 66 | 00514 | *GAT6* | EcoRV |
| 67 | 05538 | *JJJ1* | SphI |
| 68 | 03409 | *SKN7* | BamHI |
| 69 | 06339 | *FZC35* | HindIII |
| 70 | 07011 | *FZC22* | PstI |
| 71 | 07506 | *FAP1* | HindIII |
| 72 | 04807 | *FZC8* | Pst |
| 73 | 02435 | *BWC2* | HindIII |
| 74 | 02364 | *FZC19* | BamHI |
| 75 | 03116 | *HCM1* | SphI |
| 76 | 02877 | *RZC51* | SphI |
| 77 | 00559 | *BZP3* | SphI |
| 78 | 03914 | *FZC14* | PstI |
| 79 | 00871 | *CLR3* | XbaI |
| 80 | 06483 | *FZC25* | PstI |
| 81 | 07797 | *CRL6* | EcoRI |
| 82 | 05019 | *FZC21* | PstI |
| 83 | 05380 | *FZC44* | SacI |
| 84 | 04518 | *ZFC5* | EcoRI |
| 85 | 05176 | *HOB3* | XhoI |
| 86 | 05861 | *FKH101* | EcoRV |
| 87 | 00460 | *LIV1* | SphI |
| 88 | 02305 | *FZC45* | EcoRV |
| 89 | 03849 | *ASG1* | KpnI |
| 90 | 01014 | *ZFC4* | SalI |
| 91 | 01858 | *HOB2* | SphI |
| 92 | 06719 | *FZC49* | PstI |
| 93 | 04093 | *YRM103* | SphI |
| 94 | 04176 | *HSF2* | KpnI |
| 95 | 01431 | *HOB1* | HindIII |
| 96 | 07443 | *HLH4* | SphI |
| 97 | 04916 | *FZC16* | EcoRI |
| 98 | 05392 | *ZAP104* | SalI |
| 99 | 02322 | *FZC17* | XbaI |
| 100 | 04012 | *FZC18* | HindIII |
| 101 | 00505 | *FZC28* | SmaI |
| 102 | 06751 | *HLH3* | SalI |
| 103 | 04841 | *FZC43* | XbaI |
| 104 | 03212 | *HCM101* | SacI, SalI |
| 105 | 01626 | *ADA2* | BamHI |
| 106 | 03894 | *PDR802* | SphI |
| 107 | 02066 | *FZC13* | BamHI |
| 108 | 06818 | *HAP1* | HindIII |
| 109 | 05940 | *ZFC3* | EcoRI |
| 110 | 01948 | *FZC36* | XhoI |
| 111 | 04804 | *SRE1* | BamHI |
| 112 | 04352 | *ZAP103* | SacI, SalI |
| 113 | 03768 | *FZC32* | BamHI |
| 114 | 01977 | *FZC39* | KpnI |
| 115 | 04895 | *FZC3* | KpnI |
| 116 | 04583 | *DDT1* | SphI |
| 117 | 01973 | *ZFC2* | SphI |
| 118 | 02603 | *ZFC1* | HindIII |
| 119 | 04036 | *HSF3* | SphI |
| 120 | 06156 | *FZC7* | EcoRI |
| 121 | 03431 | *FZC48* | EcoRV |
| 122 | 07922 | *FZC4* | EcoRV |
| 123 | 00031 | *MLR1* | PstI, XmaI |
| 124 | 03018 | *ASG101* | XhoI |
| 125 | 04263 | *BZP2* | EcoRV |
| 126 | 01708 | *GAT7* | SacI |
| 127 | 00332 | *SIP4* | PstI |
| 128 | 07724 | *CUF1* | KgnI |
| 129 | 03902 | *RDS2* | EcoRV, EcoRI |
| 130 | 03366 | *ZNF2* | EcoRV |
| 131 | 05222 | *NRG1* | EcoRV |
| 132 | 05049 | *PIP201* | BamHI |
| 133 | 02516 | *HLH5* | BamHI |
| 134 | 04774 | *FZC26* | PstI |
| 135 | 03059 | *FZC9* | EcoRV |
| 136 | 00193 | *GAT1* | SphI |
| 137 | 03336 | *FZC50* | HindIII |
| 138 | 03086 | *FZC20* | KpnI |
| 139 | 03229 | *YOX101* | XbaI |
| 140 | 01841 | *GLN3* | EcoRI |
| 141 | 02476 | *YRM101* | XhoI |
| 142 | 05153 | *GAT5* | SalI |
| 143 | 02700 | *ZFC8* | EcoRV |
| 144 | 4586 | *HOB7* | SphI |
| 145 | 2723 | *FZC23* | EcoRV |
| 146 | 3741 | *FZC31* | ClaI |
| 147 | 4457 | *FZC30* | BamHI |
| 148 | 6283 | *LIV4* | EcoRV |
| 149 | 7411 | *RUM1* | EcoRV |
| 150 | 4836 | *FZC10* | EcoRV |
| 151 | 841 | *FZC40* | EcoRI |
| 152 | 6223 | *MIZ1* | EcoRV |
| 153 | 830 | *FZC38* | SphI |
| 154 | 1551 | *GAT201* | XbaI |
| 155 | 4908 | *CLR4* | *HindIII* |

### 2.2: Analysis of Gene Expression

Gene expression was analyzed by Northern blot analysis. Total RNA was extracted from each sample using Trizol reagent. 10 µg of the RNA was separated in 1% agarose gel made with DEPC (diethyl pyrocarbonate)-treated water and 1x MOPS (3-(N-morpholino)propane sulfonic acid) running buffer by electrophoresis. The gel was washed three times with distilled water, transferred to a nylon membrane (Millipore, INYC00010) using 20 x SSC buffer, and fixed by 1200 J/m² ultraviolet exposure. The membrane was hybridized with a gene-specific and radioactively labeled probe using modified church hybridization buffer (1 mM EDTA; Biosesang Co., Ltd,. E1002), 0.25M Na₂HP0₄ (Sigma, S9763), 1% N-2-Amine (Sigma, C0626), 7% SDS (Bioshop, SDS001) and 0.17% H₃PO₄ (Sigma, #438081)). The membrane was washed with washing buffer 1 (2x SSC and 0.1% SDS) and washing buffer 2 (1x SSC and 0.1% SDS). Next, the membrane was exposed to autography film for 1 to 2 days.

### Example 3: Transcription Factors (TFs) Governing Growth

*C. neoformans* undergoes both saprobic and pathogenic life cycles in natural and animal host environments. Therefore, it must be capable of growing at temperatures ranging from ambient temperature (25°C) to high temperature (37 to 39°C). In order to analyze the growth phenotypes of the transcription factor mutants at various temperatures, the growth of each mutant on yeast extract-peptone dextrose (YPD) medium [yeast extract (Becton, Dickison and company #212750), peptone (Becton, Dickison and company #211677), glucose (Duchefa, #G0802)] at various temperatures (25°C, 30°C, 37°C and 39°C) was observed. Deletion of some transcription factors (BZP2, CUF1, LIV4, GAT5, FZC6 and NRG1) resulted in temperature-independent growth defects. The growth defect of the cuf1 mutant was due to its inability to uptake copper, because external addition of CuSO₄ restored its wild-type (WT) growth.

In the present invention, the growth-defect transcription factor mutants (24 mutants) were classified into two groups: (1) temperature-independent growth-defect transcription factor mutants; and (2) temperature-dependent growth-defect transcription factors. The first transcription factor group (temperature-independent) includes *BZP2, CUF1, HOB1, GAT5, FZC6* and *NRG1.* Deletion of the transcription factors of the second group, including *HXL1, CRX1, ATF1, ADA2, LIV4, AR080, USV101, FZC31, MLN1, FZC30, FZC1, MIZ1, FZC46, APN2, GAT6, MBS2, SRE1,* and *ERT1,* caused growth defects only at high temperature (37 to 39°C). Among these, only *HXL1,* which is a transcription factor downstream of the Ire1 kinase in the UPR signaling pathway, exhibited a severe growth defect at host physiological temperature. By contrast, deletion of *MLN1, MCM1* and *FZC46* promoted the growth of *C. neoformans* at 39°C. Collectively, these results suggest that multiple transcription factors (27 transcription factors) control -both positively and negatively-the growth and thermotolerance of *C. neoformans.*

### Example 4: Transcription Factors Governing Mating

In a natural environment, *C. neoformans* exists mainly in the yeast form but undergoes either bisexual differentiation with cells of the opposite mating type or unisexual differentiation with cells of the same mating type to produce filamentous forms and generate infectious basidiospores. These developmental processes contribute to the generation of the genetic diversity of the pathogen (Non-Patent Document 17).

To analyze mating phenotypes, the present inventors set up unilateral mating crosses by co-culturing each TF mutant (the serotype A MAT strain) with serotype A MATa wild-type KN99a strain (obtained from Joeseph Heitman's laboratory at Duke University). Each strain was cultured in YPD medium at 30°C for 16hours, and equal concentration of cells (10⁷ cells per ml) were mixed, spotted onto V8 mating media (pH 5; per 1 L: 50 ml V8 juice (Campbell), 0.5 g KH₂PO₄ (Bioshop, PPM302), 40 g Agar (Bioshop, AGR001.500)) and incubated in a dark at room temperature for 1 to 2 weeks. Filamentous growth was monitored weekly and photographed using an Olympus BX51 microscope equipped with a SPOT Insight digital camera (Diagnostic Instrument Inc.).

To monitor the expression of pheromone gene, cell fusion assay and Northern blot analysis were performed. For the cell fusion assay, each MAT transcription factor mutant or control strain (YSB119; obtained from Joeseph Heitman's Laboratory at Duke University) containing NAT^{R} marker and MATa control strain (YSB121; obtained from Joeseph Heitman's Laboratory at Duke University) containing neomycin-resistant (Neo^{r}) marker were cultured at 30°C in liquid YPD medium for 16hours, and the concentration of cells was adjusted to 10⁷ cells per ml with distilled water. Each MAT strain and MATa strain were mixed in an equal volume, spotted onto V8 medium and incubated in a dark at room temperature for 24hours. Then, the cells were scraped, resuspended in 1ml distilled water and spread onto YPD medium containing both nourseothricin (100µg/ml) and G418 (50µg/ml, Geneticin, Life technologies). The plates were further incubated at 30°C and the number of colonies on each plate was determined. In monitoring of pheromone gene expression, the MAT and KN99a strains were mixed with equal concentration of cells (10⁸ cells per ml), spread onto the V8 medium and incubated in the dark at room temperature for 18 to 24hours. Then, cells were scraped from the V8 medium, pelleted at 4°C, frozen in liquid nitrogen, and lyophilized overnight. Total RNA was isolated using Trizol reagent according to the protocol described in the prior art document. The RNA was electrophoresed, and then transferred to a membrane. The membrane was hybridized with a mating pheromone gene (MF1)-specific probe amplified with primer B1894 (5'-TTTTACGCTTTTTGCAGATTCCGCCAAA-3'), B195 (5'-GACCACTGTTTCTTTCGTTCT-3') and JEC21 genomic DNA (genomic DNA extracted from the JEC21 strain).

To analyze mating phenotypes, the present inventors set up unilateral mating crosses by coculturing each transcription factor mutant with serotype A MATa KN99a strain. The novel mating-regulating transcription factors in the present invention, deletion of *BZP2, USV101, FZC1* and *ZAP104* severely reduced mating, even in unilateral matings, whereas deletion of *HLH1, HAP2* and *GAT1* highly enhanced mating efficiency. To determine the mating steps in which these transcription factors are involved, the present inventors measured the efficiency of cell fusion and pheromone production, which precede the filamentation step. The *bzp2, usv101, fzc1* and *zap104* mutants lacked the ability to engage in cell fusion with the MATa control strain and also failed to induce pheromone gene (MF1) expression upon mating. Such results suggest that *Bzp2, Usv101, Fzc1* and *Zap104* transcription factors promote pheromone gene expression, which results in a subsequent increase in cell fusion. Conversely, in the *hlh1, hap2* and *gat1* mutants, cell-fusion efficiency was increased two- to three-fold, and pheromone gene expression was highly enhanced. *SKN7,* whose deletion promoted mating, was dispensable for both pheromone gene expression and cell fusion, indicating that it is likely involved in a later stage of mating. Analysis performed according to the present invention suggested that 34 transcription factors are involved in mating.

### Example 5: Transcription Factors Modulating Virulence-Factor Production

To support survival and proliferation within the host, C. *neoformans* is armed with several virulence factors, which include capsule and melanin. Capsule is a glucuronoxylomannan- or galactoxylomannan-based polysaccharide that protects cells from being phagocytosed by host phagocytic cells (Non-Patent Document 18). Melanin, a black-brown pigment made of polyphenol complexes, confers both antiphagocytic and antioxidant activity to cells (Non-Patent Document 19).

### 5.1: Capsule Production

Capsule production was measured in both qualitative and quantitative manners as described in the prior art documents (Non-Patent Document 20 and Non-Patent Document 21). Cells were grown at 30°C in liquid YPD medium for 16hours, spotted onto Dulbecco's Modified Eagle's (DME) solid medium and incubated at 37°C for 2 days. Then, the cells were scraped from DME solid medium and washed with PBS (phosphate buffered saline). For qualitative measurement, capsules were stained by India ink (Bactidrop; Remel), and visualized using an Olympus BX51 microscope equipped with a Spot insight digital camera (Diagnostic Instrument Inc.). For quantitative measurement, the cells collected from DME solid medium were fixed with 10% formalin, and an equal number of cells (2.5 x 10⁷ cells per ml) were loaded into a haematocrit capillary tube, which was subsequently placed vertically to allow the cells to be packed by gravity for 10 days. The packed cell volume ratio was measured by calculating the ratio of the length of the packed cell volume phase to the length of the total volume phase (cells + medium). The relative packed cell volume of each mutant was measured by calculating the ratio of the mutant packed cell volume ratio to the wild-type packed cell volume ratio.

Triplicate technical experiments with two or more independent strains were performed. Statistical difference in relative packed cell volume was determined by one-way analysis of variance with Bonferroni's multiple-comparison test using Prism 6 (GraphPad software).

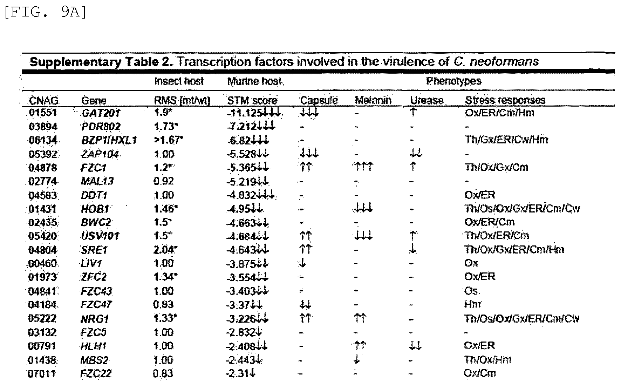

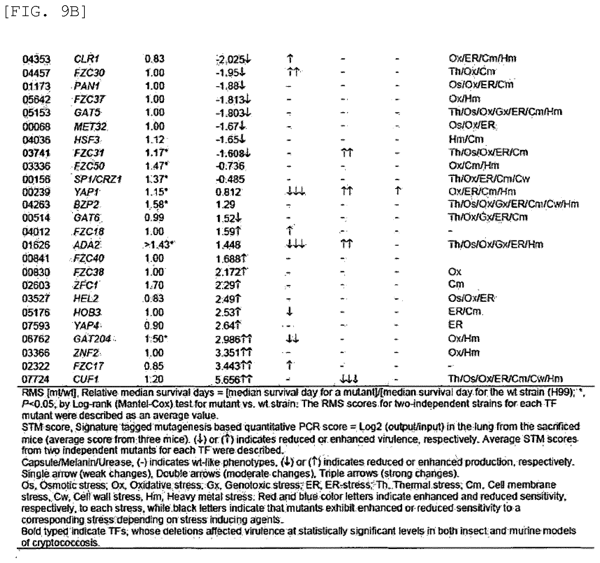

In the present invention, it was found that 49 transcription factors are involved in capsule production (FIG. 8a; 8b-29 negative regulators, 8c-20 positive regulators). Such transcription factors include previously reported capsule-regulating transcription factors, such as Atf1 (Non-Patent Document 10), Mbs1 (Non-Patent Document 11), Gat201 (Non-Patent Document 12) and Ada2 (Non-Patent Document 22). In addition to such capsule-regulating transcription factors, the present inventors identified several novel capsule-regulating transcription factors. The *zap104Δ, bap1Δ* and *rds2Δ* mutants also exhibited severely reduced capsule production compared to the results observed in the previously reported *gat201Δ* and *ada2Δ* mutants. Thus, Ada2, Gat201, Zap104, Bap1 and Rds2 together with 15 other positive regulators were predicted as major positive regulators in capsule production. By contrast, deletion of HOB7, CLR3 and FZC51 greatly enhanced capsule production, suggesting that these transcription factors together 26 other negative regulators are major negative regulators in capsule production.

### 5.2: Measurement of Melanin Production and Analysis of LAC1 Expression

Each transcription factor mutant strain and wild-type strain were cultured in liquid YPD medium at 30°C overnight, spotted on Niger seed agar medium containing 0.1% or 0.3% glucose, and then cultured at 37°C, and the plates were photographed daily to determine melanin production. The present inventors uncovered 27 transcription factors (11 positive regulators and 16 negative regulators) involved in melanin production. A few of transcription factors, including Cuf1, Stel2, Mbs1, Skn7 and Atf1, are previously reported transcription factors (Non-Patent Document 10, Non-Patent Document 11, Non-Patent Document 23, Non-Patent Document 24 and Non-Patent Document 25). In addition to such results, the *fzc8Δ, hob1Δ* and *bzp4Δ* mutants exhibited greatly reduced melanin production; the reduction was similar to that of the *cuf1Δ* mutant.

In addition, in the present invention, the correlation between transcription factors and the expression of LAC1, which is the major laccase involved in melanin synthesis, was examined. First, Northern blot analysis was performed to monitor the induction of LAC1. Each wild-type and each transcription mutant were cultured in YPD medium at 30°C for 16 hours. The cultured cells were adjusted to an OD₆₀₀ of 0.15 (optical density at 600 nm) and inoculated into 150 ml of fresh YPD liquid medium. The cell culture was further cultured at 30°C until it reached at an OD₆₀₀ of about 0.6. To prepare the zero-time sample, 50ml of 150ml cell culture was sampled and 100ml of the remaining culture was pelleted by centrifugation. After removal of the supernatant, the cells were re-suspended in glucose-free YNB liquid medium (Becton, Dickison and company, #291940). During culture, 50 ml of the cell culture was sampled at 1 hr and 2 hr. Total RNA was extracted from each sample, amplified by PCR using H99 genomic DNA, primer B3662 (5'-CTTTCAATCGTCCAAGCG-3') and primer B3663 (5'-CCCCAGTTATCCAAAAAGTC-3'), and subjected to Northern blot analysis using an LAC1-specific probe. As a result, the present inventors found that Hob1 and Fzc8 promote the expression of LAC1, which is the major laccase involved in melanin synthesis (Non-Patent Document 26), under glucose-starvation conditions, whereas Bzp4 and Cuf1 are not directly involved in LAC1 expression. By contrast, deletion of *HLH1, HLH2, BAP1* and *FZC1* greatly enhanced melanin production, although only Hlh1 negatively regulated LAC1 expression. Therefore, the present inventors identified novel positive regulators (Hob1 and Fzc45) and negative regulator (Hlh1) of *LAC1* in *Cryptococcus neoformans.*

### 5.3: Urease Production

In addition to capsule and melanin, crucial factors involved in the virulence of *Cryptococcus neoformans* include urease, a nickel-dependent protein complex (Ure1, Ure4, Ure6 and Ure7) that converts urea into ammonia, which serves as a nitrogen source.

Each wild-type strain and transcription factor mutant strain were cultured at 30°C in liquid YPD medium for 16 hours (overnight) and washed with distilled water, and then the cell density was adjusted to 1 x 10⁷ cells per ml. Next, 5 µl of the cells (5 x 10⁴ cells) were spotted onto Christensen's agar medium. Then, the cells were incubated for 7 to 10 days at 30°C and photographed during incubation.

The present inventors found that 19 transcription factors are involved in either positively (15 transcription factors) or negatively (4 transcription factors) regulating urease production.

### Example 6: Transcription Factors Affecting Infectivity and Virulence of C. neoformans

Identification of transcription factors required for the pathogenicity of *C. neoformans* is critical for future development of novel antifungal drugs and therapeutic methods. The present inventors employed two large-scale assays: (1) a virulence assay conducted in the invertebrate insect larval model system *Galleria mellonella;* and (2) a signature-tagged mutagenesis (STM)-based infectivity assay conducted in a murine inhalation model. These assays using one insect host and one mammalian host model have been widely adopted for large-scale virulence/infectivity assays in other fungi as well as *C. neoformans* (Non-Patent Document 12).

### 6.1: Insect-Based Virulence Assay

Insect-based virulence assay was performed using a modification of the previously known method (Non-Patent Document 21). For the insect-based virulence assay, 15 *Galleria mellonella* caterpillars (body weight: 250±50mg) in the final instar larval stage, reached within 7 days from the day of shipment (Vanderhorst Inc., St Marys, OH, USA), were randomly sorted into each group. Each *C. neoformans* strain was grown overnight at 30°C in YPD medium, washed three times, re-suspended with PBS, and used in hematocyte calculation performed using a hemocytometer. The present inventors inoculated 4,000 *C. neoformans* cells per larva through the second to last prolegs of larvae using a 100-µl Hamilton syringe equipped with a 10-µl-size needle and a repeating dispenser (PB600-1, Hamilton). As a non-infection control, PBS was injected. After injection, larvae were incubated in Petri dishes in humidified plastic containers and monitored daily. Infected larvae were incubated at 37°C and monitored daily. Larvae were considered dead when they displayed no movement when touched. Larvae that transformed into pupae during experiments were censored for statistical analysis. Survival curves were prepared using Prism 6 (GraphPad) and statistically analyzed using the Log-rank (Mantel-Cox) test. The present inventors first monitored the survival curve for a single mutant strain for each transcription factor gene (total 155) and statistically compared it with that of the wild-type strain. In the case of transcription factor mutant that showed statistically significant reduction or enhancement of virulence (P<0.05; Log-rank test) by gene deletion, the present inventors examined a second independent strain.

Each panel indicates virulence assay results for two independent mutants of each transcription factor. The identified mutants include nine transcription factor mutants (*hxl1, ada2, sre1, nrg1, bwc2, crz1, pdr802, gat201* and *gat204*) that were previously reported to show reduced virulence in a murine model of systemic cryptococcosis (Non-Patent Document 9, Non-Patent Document 12, Non-Patent Document 22, Non-Patent Document 27, Non-Patent Document 28, Non-Patent Document 29, Non-Patent Document 30, Non-Patent Document 31 and Non-Patent Document 32). This further indicated a strong correlation between the insect and murine models in terms of the pathogenicity of *C. neoformans.* Besides the deletion of these known TFs, deletion of *HOB1, BZP2, USV101, BAP1, ZFC2, FZC1, FZC50* and *FZC31* significantly reduced the virulence of C. *neoformans.* The present inventors identified 17 transcription factor genes involved in the virulence of *Cryptococcus neoformans* using the insect host model.

### 6.2: Animal Study

Animal care and all experiments were conducted in accordance with the ethical guidelines of the Institutional Animal Care and Use Committee (IACUC) of Yonsei University. The Yonsei University IACUC approved all of the vertebrate studies.

In the signature-tagged mutagenesis (STM)-based mouse infectivity test, transcription factor strains tagged with 44 distinct signature tags (Table 1) were grown at 30°C in YPD medium, washed three times with PBS and then pooled; the same number of cells of each strain were used after counting cells using a haemocytometer. The *ste50* and *ire1* mutants tagged with STM#282 and STM#169 sequences were used as virulent and non-virulent control strains, respectively (Non-Patent Document 9 and Non-Patent Document 33). Thus, the number of transcription factors (TFs) exhibiting the same signature tag is smaller than 4. In the present invention, four STM-based virulence tests were performed. To obtain the input TF genomic DNA library, the pooled TF mutants were 10-fold serially diluted, plated on YPD media, incubated at 30°C for 3 days and collected by scraping for use in isolating genomic DNA. The output TF genomic DNA library was obtained as follows. Seven-week-old female A/Jcr mice (Jackson Laboratory) anaesthetized with intraperitoneal injection of Avertin (2,2,2-tribromoethanol) were infected through intranasal inhalation of 5 x 10⁵ cells (in 50 µl volume) of the pooled TF mutants and sacrificed with an overdose of Avertin at 15 days post infection. For each set of assays, the present inventors used five mice. Two independent mutants for each TF were tested in a separate STM set assay. Mouse lungs were dissected and homogenized in PBS. Each lung-tissue lysate was spread on YPD media containing 100µg/ml of chloramphenicol, incubated at 30°C for 3 days, and then collected by scraping to isolate output genomic DNA. Both input and output genomic DNAs were extracted using the CTAB method (Non-Patent Document 21). Quantitative PCR analysis was performed using various tag-specific primers listed in Table S1 and a MyiQ2 Real-Time PCR detection system (Bio-Rad). The STM score (Log₂[output/input] was calculated according to the method described in the prior art documents (Non-Patent Document 12 and Non-Patent Document 34).

### 6.3: Results of Animal Study

Using the STM-based murine host model, the present inventors identified 40 virulence genes. The STM score for each mutant was calculated based on the quantitative PCR score=Log₂ (output/input) in the lung from the sacrificed mice (average score from three mice). Among all the sets studied, the *ire1Δ* mutant, which is a non-virulent control strain, exhibited a highly reduced STM score (-7.03±1.99), whereas the *ste50Δ* mutant, a virulent control strain, showed an STM score of 0.11±1.13. For supporting the quality of the STM assay, 11 of the 40 transcription factor genes identified in the present invention were previously reported to be involved in virulence (Non-Patent Document 9, Non-Patent Document 12, Non-Patent Document 29 and Non-Patent Document 30). The *gat201Δ* and *pdr802Δ* mutants exhibited drastically reduced STM scores (-11.125 and -7.212, respectively) compared to those described in the prior art document (Non-Patent Document 12). Similarly, the STM scores of the *zap104Δ* and *liv1Δ* mutants were also decreased (-5.528 and -3.875, respectively). However, the zap103Δ mutant showed a very high virulence as described in the prior art document (Non-Patent Document 12) (STM score =2.51). Furthermore, the *hxl1Δ, nrg1Δ* and *bwc2Δ* mutants also showed highly reduced STM scores. 11 of the 40 transcription factors identified by the STM analysis (*GAT201, PDR802, HXL1, BWC2, NRG1, FZC1, HOB1, USV101, ZFC2, SRE1* and *FZC31*) were also discovered using the insect model. The virulence assay data from the insect model were statistically significantly correlated with the STM-based infectivity data from the murine model based on the Pearson correlation coefficient (PCC) analysis. Among the 26 novel virulence-related transcription factors that were screened using only the STM-based murine model, the *fzc31Δ* and *ddt1Δ* mutants exhibited highly reduced STM scores (-4.328 and -4.832, respectively). The phenome database according to the present invention revealed that the *fzc31Δ* mutant exhibited increased susceptibility to osmotic, oxidative and cell membrane stresses, which might collectively affect virulence. By contrast, the only notable phenotype observed in the case of the *ddt1Δ* mutant was a weak dithiothreitol (DTT) sensitivity, which is not likely responsible for the marked decrease in the survival of the mutant in the lung because several other DTT-sensitive TF mutants were as virulent as the WT strain.

### Example 7: Examination of Antifungal Drug Resistance and Susceptibility

For the treatment of cryptococcosis, amphotericin B (AmpB) with or without flucytosine (5-FC) and fluconazole (FCZ) are widely used (Non-Patent Document 2). However, in addition to the toxic side effects of such drugs, the emergence of antifungal drug-resistant Cryptococcus strains has caused serious clinical problems (Non-Patent Document 35). To identify any transcription factors involved in antifungal drug resistance, the present inventors monitored the alteration of antifungal drug susceptibility among the *C. neoformans* TFKO mutant strains.

Cells were grown at 30°C in liquid YPD medium for 16hours, 10-fold serially diluted (1 to 10⁴ dilutions), and spotted on YPD medium containing the indicated concentrations of the following antifungal drugs: fludiooxonil, fluconazole, amphotericin B, and flucytosine. The cells were incubated at 30°C and photographed for 2 to 5 days.

Numerous transcription factors were found to be involved in antifungal drug resistance, implying that Cryptococcus strains can potentially adapt to antifungal drugs in versatile manners. In response to FCZ, 35.5% of transcription factor mutants (55/155) exhibited either increased susceptibility (35 transcription factors) or resistance (20 transcription factors), suggesting that resistance to the azole-based antifungal drug could readily occur through the modulation of diverse transcription factors. However, in response to amphotericin B (AmpB), mutants of 56 genes exhibited differential susceptibility, with 47 transcription factor mutants showing increased susceptibility and only 8 transcription factor mutants exhibiting increased drug resistance. These data support the clinical observation that compared with azole resistance, polyene resistance is rarely observed. Furthermore, it is known that the 5-FC readily elicits the development of drug-resistant strains (Non-Patent Document 36). Supporting such results, the present inventors found that 27 transcription factors differentially regulate flucytosine resistance.

The present inventors noted that the deletion of some transcription factors negatively regulated azole and polyene susceptibility (Table 5 and FIG. 8a), possibly because these transcription factors might directly control *ERG11* expression and sterol biosynthesis and affect polyene-binding capacity. Two of these transcription factors were previously reported to be Erg11 regulators. Sre1, a key sterol regulatory transcription factor, forms a complex with Scp1 as a part of the sterol regulatory element-binding protein pathway in *C. neoformans* (Non-Patent Document 27 and Non-Patent Document 28). Mbs1 negatively regulates basal *ERG11* expression and therefore its deletion increases azole resistance but decreases polyene resistance in *C. neoformans* (Non-Patent Document 11). To further test whether other transcription factors are also involved in ERG11 regulation, the present inventors measured ERG11 expression levels in these TF mutants under both sterol-replete and sterol-depleted conditions (FIG. 8b). To analyze the expression of *ERG2, ERG3, ERG5, ERG11* and *ERG25,* the wild-type, *sre1* and *hob1* mutants were grown in liquid YPD medium overnight. The overnight culture was then inoculated in 100ml of fresh YPD medium at 30°C and grown until the OD₆₀₀ of the culture reached about 1.0. To prepare the zero-time sample, 50ml of cell culture was sampled, and the remaining culture was treated with fluconazole (final concentration: 10µg/ml) for 90min. Total RNA was isolated using TRIzol reagent, and cDNA was synthesized using M-MuLV reverse transcriptase (Thermo scientific). Northern blot analysis was performed with each ERG gene-specific probe that was amplified with ERG gene-specific primers with the total RNA in cells treated or not treated with fluconazole. Primers: B5789 (5'-CAAGAAATGGAGCGTGAG-3') and B5790 (5'-CAGTGTTGTAAAGCGTGATG-3') for ERG2; B1720 (5'-ATCCCTTTTCACCGTCGCTC-3') and B1721 (5'-CGTCGTGGATGAGAATAGTCC-3') for ERG3; B671 (5'-GTTTGTTGCCTGAGAACTGGG-3') and B674 (5'-ATCACTCAACTCGGTCCTCTCGTG-3') for ERG5; B678 (5'-TTCAGGGAACTTGGGAACAGC-3') and B1598 (5'-CAGGAGCAGAAACAAAGC-3') for ERG11; B1718 (5'-TGACCGCCTGTAGATTGTC-3') and B1719 (5'-TAGTCCCACCACCTGAAAC-3') for ERG25. Quantitative real-time PCR was performed with each gene-specific primer using a MyiQ2 Real-Time PCR detection system (Bio-Rad). Primers: B5789 (5'-CAAGAAATGGAGCGTGAG-3') and B6838 (5'-GGGAGGGTCGAGGATGTAGA-3') for ERG2; B1720 (5'-ATCCCTTTTCACCGTCGCTC-3') and B6838 (5'-GGGAGGGTCGAGGATGTAGA-3') for ERG3; B671 (5'-GTTTGTTGCCTGAGAACTGGG-3') and B672 (5'-GTAGATACTGAGAGCCTGCTTGGTG-3') for ERG5; B677 (5'-AATCTCCTTACCAGCCATTCGG-3') and B678 (5'-TTCAGGGAACTTGGGAACAGC-3') for ERG11; B2695 (5'-TCGTCTTTGGCAAGCAGTC-3') and B6840 ((5'-GAAGTCGTGGTGGTCAGCA-3') for ERG25; and B679 (5'-CGCCCTTGCTCCTTCTTCTATG-3') and B680 (5'-TACTCGTCGTATTCGCTCTTCG-3') for ACT1. As expected, basal and induced *ERG11* levels were substantially lower in the sre1 mutant than in the wild-type strain. Notably, deleting *HOB1* markedly increased the basal expression levels of *ERG11.* To determine whether Hob1 is involved in the regulation of other ERG genes, the present inventors monitored the expression of ERG2, ERG3, ERG5 and ERG25 in the wild-type strain, hob1Δ and sre1Δ strains under sterol-replete and -depleted conditions. The expression of all of these ERG genes was induced in response to sterol depletion through fluconazole treatment in the wild-strain strain, but not in the *sre1Δ* strain (FIG. 8c). Deletion of HOB1 markedly induced the basal expression of ERG2 (FIG. 8c). By contrast, under sterol depletion, the induction of *ERG2, ERG3, ERG5, ERG11* and *ERG25* was decreased in the *hob1Δ* mutant (FIG. 8c). The tight regulation of ERG expression appeared to be mostly absent in the *hob1Δ* mutant, indicating that Hob1 is a key regulator of ergosterol gene expression (FIG. 8d). Notably, the transcription factors involved in sterol biosynthesis also appeared to be involved in environmental stress responses and adaptation, which are critical for the survival and proliferation of *C. neoformans* within the host because the pathogen encounters drastic environmental changes during infection (Non-Patent Document 3).

**[Table 5]**

| Transcription factors involved in antifungal agent resistance in *C. neoformans* | | |
|---|---|---|
| Antifungal agents | TF mutants showing increased resistance | TF mutants showing increased susceptibility |
| Azole (Fluconazole) | *HOB1, HAP2*, *SKN7, NRG1, MBS1, PPR1, JJJ1, HCM1, ADA2, FZC9, GAT7, ERT1, FKH2, ECM22, DDT1, GAT5, YRM103, CUF1, FZC51, LIV4,* | *BZP3, HLH3, BZP1*/*HXL1*, *SHE1, RIM101, YAP2, HLH1, YAP4, PIP2, MIZ1, MLN1, HOB6, MBF1, MET32, FZC46, YAP1, FZC14, FZC2, HSF2, ZFC6, FZC45, FZC30, ASG1, STE12, LIV1, FZC22, FZC31, PAN1, BZP2, SP1*/*CRZ1, BZP5, SXI1alpha, FZC34, , FZC17*, *HLH2* |
| Polyene (Amphotericin B) | *SRE1, YAP1, FZC51, SKN7, CLR1*, *BZP4, ATF1, FZC4* | *HOB1, MBS1, JJJ1, ERT1, ECM22, GAT201, ZAP104, SP1*/*CRZ1, FZC6, BZP5, HLH1, PIP2, HCM1, BZP2, USV101, HOB4, STE12, HOB5, GRF1, HEL2, FZC45, ASG1, FZC22, HOB6, PAN1*, *CUF1*, *FZC49, FZC1, BWC2, FAP1, FZC44, FZC8, FZC23, GAT204, NRG1, PIP201, RIM101, HLH3, BZP3, MLN1, MET32, ZFC2, FZC31, RUM1, PDR802, FZC10, HLH2* |
| 5-flucyotosine | *HLH3, RIM101, GAT204, HOB3, FZC50, ZNF2, RDS2*, *FZC31* | *NRG1, ZFC2, YAP1, MBS1, FZC6, YAP2*, *BZP3, JJJ1, HLH1, PIP2, APN2, FZC46, HAP2, FZC51, BZP5, HCM1, FZC19, BZP2, FZC44* |
| Phenylpyrrole Fungicide (Fludioxonil) | *NRG1, JJJ1, SP1*/*CRZ1, SKN7, GAT7, FAP1, ZFC2, GAT204, ZNF2, HEL2*, *FZC50, SRE1* | *USV101, ADA2, YAP1, FZC6, HLH1, PIP2, FZC46, HAP2, BZP1*/*HXL1, FKH2, LIV1, YAP2, BZP2, FZC21, HLH3, YRM101, BZP5, GLN3, ZFC8, DDT1, FZC22, HOB6, RLM1, MLN1*, *PAN1, FZC35, YRM103, ZFC3, ASG1, FZC41, FZC43, FZC51, HAP1, MET32*, *FZC32* |

### Example 8: Examination of Responses to External Stress

To analyze external stress-related phenotypes, cells were grown at 30°C in liquid YPD medium for 16hours, 10-fold serially diluted (1 to 10⁴ dilutions), and spotted on YPD medium containing the indicated concentrations of the following stress inducers: A: osmotic stress (sorbitol); B: oxidative stress [hydrogen peroxide (H₂O₂), tert-butyl hydroperoxide (TH) (an organic peroxide), menadione, diamide] ; C: endoplasmic reticulum (ER) stress [tunicamycin), DTT (dithiothreitol)]; D: genotoxic stress [methyl methanesulfonate (MMS), hydroxyurea (HU)]; E: cell membrane/wall-destabilizing stress [calcofluor white (CFW), Congo red (CR), and sodium dodecyl sulfate (SDS)]; F: heavy-metal stress (CdSO₄). Cells were incubated at 30°C and photographed for 2 to 5 days. The osmotic stress applied was a stress induced by any one selected from the group consisting of 1M to 1.5M sodium chloride (NaCl), 1M to 1.5M potassium chloride (KCl) and 2M sorbitol. It was shown that the antifungal agent-targeting genes against the osmotic stress induced by the sodium chloride were *ada2, aro8001, bap1, bzp2, fzcl3, fzcl9, fzc34, fzc42, fzc43, fzc51, gat5, gat7, hap2, hcm1, hob1, hob6, met32, pan1, rim101* and *skn7* genes, and the antifungal agent-targeting genes against the osmotic stress induced by the potassium chloride were *ada2, bzp2, bzp4, fzc32, fzc35, fzc44, fzc6, hap2, hob1, hob2, nrg1, rim101* and *yrm10.* It was shown that the antifungal agent-targeting genes against the osmotic stress induced by sorbitol were *bzp2, fzc6* and *hob1.*

The oxidative stress was examined by applying each of 2.5 mM to 3.5 mM hydrogen peroxide H₂O₂), 0.7 mM to 0.8 mM tert-butyl hydroperoxide (TH), 0.02 mM to 0.03 mM menadione, and 2 mM to 3 mM diamide (DA).

It was shown that antifungal agent-targeting genes against the oxidative stress induced by the hydrogen peroxide were *ada2, bap1, bzp2, bzp3, cuf1, fzcl3, fzc21, fzc22, fzc27, fzc31, fzc4, fzc46, fzc50, fzc9, gat1, gat201, gat204, gat5, hlh1, hob1, hob4, hob5, hob6, liv1, met32, miz1, nrg1, pan1, rim101, sip402, sp1(crz1), sre1, ste12* and *usv101,* and antifungal agent-targeting genes for the oxidative stress induced by the tert-butyl hydroperoxide were *ada2, asg1, atf1, bap1, bap2, bzp5, clr1, ecm22, fzc1, fzc15, fzc21, fzc31, fzc34, fzc44, fzc49, fzc51, fzc6, gat5, gat8, grf1, hcm1, hlh2, hob1, hob2, hob4, liv1, met32, mwc2, pan1, ppr1, rim101, rlm1, skn7, sre1, usv101, yrm103, zap103, zfc2* and *zfc4.* It was shown that antifungal agent-targeting genes for the oxidative stress induced by the menadione were *bap1, bzp2, ecm22, fzc26, fzc3, fzc34, fzc35, fzc37, fzc4, fzc44, fzc6, gat6, hel2, jjj1, nrg1* and *usv101,* and antifungal agent-targeting genes for the oxidative stress induced by the diamide were *bap1, bap2, bzp2, bzp5, fap1, fkh2, fzcl9, fzc21, fzc27, fzc3, fzc30, fzc31, fzc34, fzc38, fzc4, fzc46, fzc49, fzc8, gat5, gat6, hap2, hlh1, hlh3, hob1, hob6, hsf2, met32, miz1, mln1, pan1, pip2, rum1, sip402, sre1* and *zfc2.*

The endoplasmic reticulum (ER) stress was induced by each of 0.2 µg/ml to 0.3 µg/ml tunicamycin (TM) and 15 mM to 20 mM dithiothreitol (DTT), and as a result, it was shown that antifungal agent-targeting genes for the endoplasmic reticulum (ER) stress induced by the tunicamycin were *bzp1(hxl1), bzp3, fzc2, fzc21, fzc38, fzc40, fzc44, gat7, hlh1, liv4, met32, mln1, pip2, rim101, rlm1, sp1(crz1), sre1* and *ste12.* In addition, it was shown that antifungal agent-targeting genes for the endoplasmic reticulum (ER) stress induced by the dithiothreitol were *ada2, apn2, bzp1(hxl1), bzp2, clr1, cuf1, ddt1, fzc25, fzc31, gat201, gat5, hap2, hlh2, hob1, nrg1, rlm1, sre1* and *usv101.*

The genotoxic stress was induced by each of 0.03% to 0.06% methyl methanesulfonate (MMS) and 50 mM to 100 mM hydroxyurea (HU). As a result, it was shown that an antifungal agent-targeting gene for the genotoxic stress induced by the methyl methanesulfonate was any one gene selected from the group consisting of *apn2, bzp1(hxl1), bzp2, fzc1, fzc38, fzc4, fzc40, fzc6, gat5, gat6, hcm101, hob1, jjj1, miz1* and sre1, and the antifungal agent-targeting genes against the genotoxic stress induced by the hydroxyurea were *ada2, bzp1(hxl1), bzp2, fzc6, gat5, gat6, hcm1, hlh2, hob1, jjj1, mbs1, nrg1, skn7* and *sre1.*

The cell wall or cell membrane stress was induced by each of 3 mg/ml to 5 mg/mg calcofluor white (CFW), 0.8% to 1% Congo red (CR) and 0.03% sodium dodecyl sulfate (SDS). As a result, it was shown that an antifungal agent-targeting gene for the cell wall or cell membrane stress induced by the calcofluor white (CFW) was any one gene selected from the group consisting of *bap2, bzp1(hxl1), bzp2, hap2, hobl, nrg1, pip2, rim101* and *sp1(crz1),* and antifungal agent-targeting genes for the cell wall or cell membrane stress induced by the Congo red were *bap2, bzp1 (hxl1), bzp2, cuf1, hap2, hlh3, hob1, nrg1, rim101* and *sp1(crz1),* and antifungal agent-targeting genes for the cell wall or cell membrane stress induced by the sodium dodecyl sulfate (SDS) were *alpha, asg1, asg101, bap1, bzp2, bzp3, bzp5, clr1, clr4, cuf1, ecm22, erl1, fap1, fzc21, fzc26, fzc30, fzc31, fzc7, gat1, gat201, gat5, gat6, gat7, hap2, hob1, hob3, hob5, jjj1, nrg1, pan1, pip2, rds2, rlm1, rum1, sip4, sp1(crz1), sre1, sxi1, usv101, zfc4* and *zfc6.*

The heavy-metal stress was induced by 20 µM to 30 µM cadmium sulfate (CdSO₄), and as a result, it was shown that antifungal agent-targeting genes for the stress induced by the heavy-metal stress were *aro80, aro8001, bzp2, bzp4, ccd4, cuf1, fap1, fzc10, fzcl9, fzc35, fzc37, fzc46, fzc47, fzc51, fzc6, fzc8, gat5, gln3, hap2, hcm1, hob5, hob6, hob7, mbs2, mln1, pip2, pip201, rum1, skn7, you101, yrm101* and *zfc8.*

Reflecting diverse types of external stresses, 145 transcription factors were identified to be involved in sensing and responding to at least one type of stress. Among these transcription factors, the two sterol regulators, Sre1 and Hob1, appeared to be general stress-responsive transcription factors that govern multiple stress responses and adaptations. Strikingly, the deletion of HOB1 or SRE1 substantially reduced resistance to osmotic/salt, oxidizing/reducing, genotoxic, endoplasmic reticulum (ER) and cell wall/membrane stresses. The hob1 and *sre1* mutants exhibited similar stress resistance and susceptibility patterns under most of the various environmental stresses, and this is in stark contrast to their opposite resistance patterns towards fluconazole (FCZ) and amphotericin B (AmpB). This result strongly suggested that sterol homeostasis is a very important factor in controlling stress response and adaptation in *C*. *neoformans.*

### Example 9: Analysis of Functional Correlation

To understand the correlation among phenotypic traits and virulence, the present inventors measured the degree of linear dependence by calculating all Pearson Correlation Coefficients (PCCs) between two possible *in vitro* and *in vivo* phenotypic combinations tested in the present invention. These correlations are illustrated in a combined network. The correlation network revealed that the virulence of C. *neoformans* is highly correlated to growth at distinct temperatures and osmotic and cell wall/membrane-stress responses and moderately related to oxidative, genotoxic and ER stress responses. Notably, these stress phenotypes were also highly inter-correlated, suggesting that several core stress signaling networks might exist, including the known Hog1, Pkc1/Mpk1 and UPR signaling pathways. By contrast, mating and resistance to antifungal drugs, except to resistance to AmpB, were not significantly related to the virulence of C. *neoformans.* The production of virulence factors did not appear to be correlated to *in vivo* virulence, and this is likely because increased virulence-factor production often did not result in increased virulence. Supporting this, reduced capsule production was found to be highly correlated to reduced virulence by PCC analysis (P<0.05).

### Example 10: Construction of C. neoformans Transcription Factor Database

The genome and transcriptome data collected for 155 transcription factors were processed using the protocol of the standardized genome data in Comparative Fungal Genomics Platform (CFGP 2.0; Non-Patent Document 37). For detailed information of the predicted genes, pre-computed results of eight bioinformatics programs (InterPro scan, Signalp 3.0, PSortII, TargetP, ChloroP, SecretomeP, predictsNLS and TMHMM2) were provided (Non-Patent Document 38, Non-Patent Document 39, Non-Patent Document 40, Non-Patent Document 41, Non-Patent Document 42, Non-Patent Document 43, Non-Patent Document 44 and Non-Patent Document 45).

To browse genomics contexts together with key biological features, Seoul National University Genome Browser (SNUGB; Non-Patent Document 46) was incorporated for use with the *Cryptococcus* Transcription Factor Database. In the pages of Browse Scaffolds, Browse Gene Models and 3 gene-family browsers, direct links to the SNUGB module were provided. MySQL 5.0.81 (source code distribution) and PHP 5.2.6 were used for administrating the database and developing web interfaces, respectively. Web pages were provided through Apache 2.2.9 web server.

### Example 11: Construction of Pearson's Correlation Networks

The present inventors calculated Pearson Correlation Coefficient (PCC) PCC scores by using Prism 5.0 (GraphPad Software Inc.) based on the results of phenotypic tests (strongly resistant phenotype: 3; moderately resistant phenotype: 2; weakly resistant phenotype: 1; wild type-like phenotype: 0; weakly sensitive phenotype: -1; moderately sensitive phenotype: -2; and strongly sensitive phenotype: -3). Networks were visualized using Cytoscape software 3.2.0 based on the PCC scores.

### Accession Number

Name of Depositary Institution: Korean Culture Center of Microorganisms;
Accession Number: KCCM51291;
Date of Deposit: March 23, 2015.

### [Industrial Applicability]

The present invention relates to novel genes that regulate the virulence of *Cryptococcus neoformans* strains, and to the use thereof. According to the present invention, a novel antifungal agent and/or a novel agent for treating meningitis can be screened.

All the documents referred to in the present invention are incorporated herein by reference in its entirety.

### [Sequence List Text]

**General Deposition Number Notice for Microorganism**

| | |
|---|---|
| No. 2015-21 | |
| For the microorganism to which you applied for general deposition under number 2015-21 on March 27, 2015, the general deposit was accepted, and the general deposit number of the microorganism is notified as follows. | |
| Follows | |
| 1. Name of microorganism deposited | *Cryptococcus neoformans var. grubil* H99S and transcription factor mutant library |
| 2. Name of microorganism at listing | |
| 3. Microorganism deposit number | KCCM 51291 |
| 4. Date of deposit | March 23, 2015 |

◇The above microorganism can be distributed without restrictions to researchers both in Korea and abroad for academic and industrial purposes.

### Korean Culture Center of Microorganisms

## Claims

1. A method for screening an antifungal agent, comprising the steps of:
(a) bringing a sample to be analyzed into contact with a *Cryptococcus neoformans* cell containing an antifungal agent-targeting gene;
(b) measuring expression of the antifungal agent-targeting gene in the cell; and
(c) determining that the sample is an antifungal agent, when the expression of the antifungal agent-targeting gene is down-regulated, wherein the antifungal agent-targeting gene is any one gene selected from the group consisting of an antibacterial agent resistance regulatory gene, a growth regulatory gene, a mating regulatory gene, and a gene that regulates responses to external stress,
wherein the antibacterial agent resistance regulatory gene is a gene that regulates resistance to any one antifungal agent selected from the group consisting of azole-, polyene-, 5-flucytocin- and phenylpyrazole-based antifungal agents, wherein the gene that regulates resistance to the azole-based antifungal agent is any one gene selected from the group consisting of *alpha, asg1, bap1, bap2, bzp*/*hxl1, bzp2, bzp3, bzp5, fzc14, fzcl7, fzc2, fzc22, fzc30, fzc31, fzc34, fzc38, fzc40, fzc45, fzc46, hlh1, hlh2, hlh3, hob6, hsf2, liv1, liv4, mbf1, met32, miz1, mln1, pan1, pip2, rim101, sp1*/*crz1, sre1, stel2, sxi1, yap4* and *zfc6;* the gene that regulates resistance to the polyene-based antifungal agent is any one gene selected from the group consisting of *asg1, bwc2, bzp2, bzp3, bzp5, cuf1, ecm22, ert1, fap1, fzc1, fzc22, fzc23, fzc31, fzc38, fzc40, fzc44, fzc45, fzc49, fzc6, fzc8, gat201, gat204, grf1, hcm1, hel2, hlh1, hlh2, hlh3, hob1, hob4, hob5, hob6ᵣ jjj1, liv4, mbs1, met32, mln1, nrg1, pan1, pip2, pip201, rim101, rum1, sp1*/*crz1, stel2, usv101, zap104* and *zfc2;* the gene that regulates resistance to the 5-flucytocin-based antifungal agent is any one gene selected from the group consisting of *apn2, bap1, bap2, bzp3, bzp5, fzcl9, fzc46, fzc51, fzc6, hap2, hcm1, hlh1, jjj1, mbs1, nrg1, pip2* and zfc2; and the gene that regulates resistance to the phenylpyrazole-based antifungal agent is any one gene selected from the group consisting of *ada2, asg1, bap1, bap2, bzp1*/*hxl1, bzp2, bzp5, ddt1, fkh2, fzc21, fzc22, fzc3, fzc32, fzc35, fzc38, fzc41, fzc43, fzc46, fzc51, fzc6, gln3, hap1, hap2, hlh1, hlh3, hob6, liv1, liv4, met32, mln1, pan1, pip2, rlm1, usv101, yrm101, yrm103* and *zfc8,*
the growth regulatory gene is temperature-independent or temperature-dependent, wherein the temperature-independent growth regulatory gene is any one gene selected from the group consisting of *bzp2, cuf1, fzc6, gat5, hob1* and *nrg1,* and the temperature-dependent growth regulatory gene is a gene that regulates growth at a temperature of 37°C to 39°C and is any one gene selected from the group consisting of *ada2, apn2, aro80, atf1, crz1, erl1, fzc1, fzc30, fzc31, gat6, hxl1, liv4, mbs2, miz1, mln1, sre1* and *usv101,*
the mating regulatory gene is any one gene selected from the group consisting of *bzp2, fzc1, skn7, usv101* and *zap104,*
the gene that regulates responses to external stress is a gene that regulates responses to any one stress selected from the group consisting of osmotic stress, oxidative stress, endoplasmic reticulum stress, genotoxic stress, cell wall or cell membrane stress, and heavy-metal stress, wherein the osmotic stress is a stress induced by any one selected from the group consisting of 1M to 1.5M sodium chloride (NaCl), 1M to 1.5M potassium chloride (KC1) and 2M sorbitol, wherein the antifungal agent-targeting gene that regulates responses to the osmotic stress induced by the sodium chloride is any one gene selected from the group consisting of *ada2, aro8001, bap1, bzp2, fzcl3, fzcl9, fzc34, fzc42, fzc43, fzc51, gat5, gat7, hap2, hcm1, hob1, hob6, met32, pan1, rim101* and skn7; the antifungal agent-targeting gene that regulates responses to the osmotic stress induced by the potassium chloride is any one gene selected from the group consisting of ada2, bzp2, bzp4, fzc32, fzc35, fzc44, fzc6, hap2, *hob1,* hob2, *nrg1, rim101* and yrm103; and the antifungal agent-targeting gene that regulates responses to the osmotic stress induced by the sorbitol is any one gene selected from the group consisting of *bzp2, fzc6* and *hob1,*
the oxidative stress is a stress induced by any one selected from the group consisting of 2.5 mM to 3.5 mM hydrogen peroxide (H₂O₂), 0.7 mM to 0.8 mM tert-butyl hydroperoxide (TH), 0.02 mM to 0.03 mM menadione, and 2 mM to 3 mM diamide (DA), the antifungal agent-targeting gene that regulates responses to the oxidative stress induced by the hydrogen peroxide is any one gene selected from the group consisting of *ada2, bap1, bzp2, bzp3, cuf1, fzcl3, fzc21, fzc22, fzc27, fzc31, fzc4, fzc46, fzc50, fzc9, gat1, gat201, gat204, gat5, hlh1, hob1, hob4, hob5, hob6, liv1, met32, miz1, nrg1, pan1, rim101, sip402, sp1(crz1), sre1, ste12* and *usv101*; the antifungal agent-targeting gene that regulates responses to the oxidative stress induced by the tert-butyl hydroperoxide is any one gene selected from the group consisting of ada2, *asg1,* atf1, *bap1,* bap2, bzp5, *clr1,* ecm22, *fzc1,* fzcl5, fzc21, fzc31, fzc34, fzc44, fzc49, fzc51, fzc6, *gat5,* gat8, *grf1, hcm1,* hlh2, hob1, hob2, *hob4, liv1,* met32, mwc2, *pan1,* ppr1, *rim101, rlm1,* skn7, *sre1,* usv101, yrm103, zap103, zfc2 and zfc4; the antifungal agent-targeting gene that regulates responses to the oxidative stress induced by the menadione is any one gene selected from the group consisting of *bap1, bzp2, ecm22, fzc26, fzc3, fzc34, fzc35, fzc37, fzc4, fzc44, fzc6, gat6, hel2, jjj1, nrg1* and *usv101,* and the antifungal agent-targeting gene that regulates responses to the oxidative stress induced by the diamide is any one gene selected from the group consisting of *bap1, bap2, bzp2, bzp5, fap1, fkh2, fzcl9, fzc21, fzc27, fzc3, fzc30, fzc31, fzc34, fzc38, fzc4, fzc46, fzc49, fzc8, gat5, gat6, hap2, hlh1, hlh3, hob1, hob6, hsf2, met32, miz1, mln1, pan1, pip2, rum1, sip402, sre1* and *zfc2,*
the endoplasmic reticulum stress is a stress induced by 0.2 µg/ml to 0.3 µg/ml tunicamycin and 15 mM to 20 mM dithiothreitol (DTT), wherein the antifungal agent-targeting gene that regulates responses to the endoplasmic reticulum stress induced by the tunicamycin is any one gene selected from the group consisting of *bzp1(hxl1), bzp3, fzc2, fzc21, fzc38, fzc40, fzc44, gat7, hlh1, liv4, met32, mln1, pip2, rim101, rlm1, sp1(crz1), sre1* and *stel2;* and the antifungal agent-targeting gene that regulates responses to the endoplasmic reticulum stress induced by the dithiothreitol is any one gene selected from the group consisting of *ada2, apn2, bzp1(hxl1), bzp2, clr1, cuf1, ddt1, fzc25, fzc31, gat201, gat5, hap2, hlh2, hob1, nrg1, rlm1, sre1* and *usv101,*
the genotoxic stress is a stress induced by any one selected from the group consisting of 0.03% to 0.06% methyl methanesulfonate (MMS) and 50 mM to 100 mM hydroxyurea (HU), wherein the antifungal agent-targeting gene that regulates responses to the genotoxic stress induced by the methyl methanesulfonate is any one gene selected from the group consisting of *apn2, bzp1(hxl1), bzp2, fzc1, fzc38, fzc4, fzc40, fzc6, gat5, gat6, hcm101, hob1, jjj1, miz1* and *sre1,* and the antifungal agent-targeting gene that regulates responses to the genotoxic stress induced by the hydroxyurea is any one gene selected from the group consisting of *ada2, bzp1(hxl1), bzp2, fzc6, gat5, gat6, hcm1, hlh2, hob1, jjj1, mbs1, nrg1, skn7* and *sre1,*
the cell wall or cell membrane stress is a stress induced by any one selected from the group consisting of 3 mg/ml to 5 mg/ml calcofluor white (CFW), 0.8% to 1% Congo red (CR) and 0.03% sodium dodecyl sulfate (SDS), wherein the antifungal agent-targeting gene that regulates responses to the cell wall or cell membrane stress induced by the calcofluor white (CFW) is any one gene selected from the group consisting of *bap2, bzp1(hxl1), bzp2, hap2, hob1, nrg1, pip2, rim101* and *sp1(crz1);* the antifungal agent-targeting gene that regulates responses to the cell wall or cell membrane stress induced by the Congo red (CR) is any one gene selected from the group consisting of *bap2, bzp1(hxl1), bzp2, cuf1, hap2, hlh3, hob1, nrg1, rim101* and *spl(crz1);* and the antifungal agent-targeting gene that regulates responses to the cell wall or cell membrane stress induced by the sodium dodecyl sulfate (SDS) is any one gene selected from the group consisting of *alpha, asg1, asg101, bap1, bzp2, bzp3, bzp5, clr1, clr4, cuf1, ecm22, erl1, fap1, fzc21, fzc26, fzc30, fzc31, fzc7, gat1, gat201, gat5, gat6, gat7, hap2, hob1, hob3, hob5, jjj1, nrg1, pan1, pip2, rds2, rlm1, rum1, sip4, sp1(crz1), sre1, sxi1, usv101, zfc4* and *zfc6,* and
the heavy-metal stress is induced by 20 µM to 30 µM cadmium sulfate (CdSO₄), wherein the antifungal agent-targeting gene that regulates responses to the heavy-metal stress induced by 20 µM to 30 µM cadmium sulfate (CdSO₄) is any one gene selected from the group consisting of *aro80, aro8001, bzp2, bzp4, ccd4, cuf1, fap1, fzc10, fzcl9, fzc35, fzc37, fzc46, fzc47, fzc51, fzc6, fzc8, gat5, gln3, hap2, hcm1, hob5, hob6, hob7, mbs2, mln1, pip2, pip201, rum1, skn7, yox101, yrm101* and *zfc8.*

2. The method of claim 1, wherein the resistance is down-regulated.

3. A method for screening an antifungal agent, comprising the steps of:
(a) bringing a sample to be analyzed into contact with a *Cryptococcus neoformans* cell containing an antifungal agent-targeting gene;
(b) measuring expression of the antifungal agent-targeting gene in the cell; and
(c) determining that the sample is an antifungal agent, when the expression of the antifungal agent-targeting gene is up-regulated, wherein the antifungal agent-targeting gene is any one gene selected from the group consisting of an antibacterial agent resistance regulatory gene, a growth regulatory gene, a mating regulatory gene, and a gene that regulates responses to external stress,
wherein the antibacterial agent resistance regulatory gene is a gene that regulates resistance to any one antifungal agent selected from the group consisting of azole-, polyene-, 5-flucytocin- and phenylpyrazole-based antifungal agents, wherein the gene that regulates resistance to the azole-based antifungal agent is any one gene selected from the group consisting of *ada2, cuf1, ddt1, ecm22, erl1, fkh2, fzc51, fzc9, gat1, gat7, hap2, hcm1, hob1, jjj1, mbs1, nrg1, ppr1, skn7* and *yrm103;* the gene that regulates resistance to the polyene-based antifungal agent is any one gene selected from the group consisting of *atf1, bap1, bzp5, clr1, fzc4, fzc51, snk7* and *sre1;* the gene that regulates resistance to the 5-flucytocin-based antifungal agent is any one gene selected from the group consisting of fzc50, gat204, hlh3, hob3, rds2, *rim101* and znf2; and the gene that regulates resistance to the phenylpyrazole-based antifungal agent is any one gene selected from the group consisting of *fap1,* fzc50, gat204, gat7, *hel2,* jjj1, *nrg1,* skn7, sp1/crz1, *sre1,* zfc2 and znf2,
the growth regulatory gene is any one of *fzc46* and *mln1* and regulates growth at 39°C,
the mating regulatory gene is any one gene selected from the group consisting of *gat1, hap2, hlh1* and *skn7,*
the antifungal agent-targeting gene that regulates responses to external stress is a gene that regulates responses to any one stress selected from the group consisting of osmotic stress, oxidative stress, endoplasmic reticulum stress, genotoxic stress, cell wall or cell membrane stress, and heavy-metal stress, wherein the osmotic stress is a stress induced by any one selected from the group consisting of 1M to 1.5M sodium chloride (NaCl), 1M to 1.5M potassium chloride (KC1) and 2M sorbitol, wherein the antifungal agent-targeting gene that regulates responses to the osmotic stress induced by the sodium chloride is any one gene selected from the group consisting of *hlh3, hel2* and *cuf1;* the antifungal agent-targeting gene that regulates responses to the osmotic stress induced by the potassium chloride is any one gene of *fzc36* and *yrm103;* and the antifungal agent-targeting gene that regulates responses to the osmotic stress induced by the sorbitol is *fzc36,*
the oxidative stress is a stress induced by any one selected from the group consisting of 2.5 mM to 3.5 mM hydrogen peroxide (H₂O₂), 0.7 mM to 0.8 mM tert-butyl hydroperoxide (TH), 0.02 mM to 0.03 mM menadione, and 2 mM to 3 mM diamide (DA), wherein the antifungal agent-targeting gene that regulates responses to the oxidative stress induced by the hydrogen peroxide is any one gene selected from the group consisting of *asg101, bwc2, fzc35, fzc45, fzc7* and *znf2;* the antifungal agent-targeting gene that regulates responses to the oxidative stress induced by the tert-butyl hydroperoxide is any one gene selected from the group consisting of *clr3, ddt1, fap1* and *fzc33;* the antifungal agent-targeting gene that regulates responses to the oxidative stress induced by the menadione is any one gene selected from the group consisting of *cuf1, fzc50, hap2, sip4* and *zfc2;* and the antifungal agent-targeting gene that regulates responses to the oxidative stress induced by the diamide is any one gene selected from the group consisting of *asg101, fzc20, fzc26, fzc50, gat1, gat7, hlh5, jjj1, nrg1, pip201, sip4, skn7* and *znf2,*
the endoplasmic reticulum stress is a stress induced by 0.2 µg/ml to 0.3 µg/ml tunicamycin and 15 mM to 20 mM dithiothreitol (DTT), wherein the antifungal agent-targeting gene that regulates responses to the endoplasmic reticulum stress induced by the tunicamycin is any one gene selected from the group consisting of *bap1, bwc2, bzp2, clr1, clr4, cuf1, fzc6, gat5, gat6, hap2, hcm1, hel2, hob1, hob3, jjj1, mbs1, nrg1, ppr1, skn7, zfc2, zfc3 and zfc4,* and the antifungal agent-targeting gene that regulates responses to the endoplasmic reticulum stress induced by the dithiothreitol is any one gene selected from the group consisting of *bzp3, fkh2, fzcl1, fzc20, gat1, gat203, hlh1, mbs1, met32, pan1, pip2, sip401, stb4* and *yap4,*
the genotoxic stress is a stress induced by any one of 0.03% to 0.06% methyl methanesulfonate (MMS) and 50 mM to 100 mM hydroxyurea (HU), wherein the antifungal agent-targeting gene that regulates responses to the genotoxic stress induced by the methyl methanesulfonate is *yox1,* and the antifungal agent-targeting gene that regulates responses to the genotoxic stress induced by the hydroxyurea is *fzc20,*
the cell wall or cell membrane stress is a stress induced by any one of 3 mg/ml to 5 mg/ml calcofluor white (CFW) and 0.03% sodium dodecyl sulfate (SDS), wherein the antifungal agent-targeting gene that regulates responses to the cell wall or cell membrane stress induced by the calcofluor white (CFW) is any one of *fzc9* and *grf1,* and the antifungal agent-targeting gene that regulates responses to the cell wall or cell membrane stress induced by the sodium dodecyl sulfate is any one gene selected from the group consisting of *bwc2, fzc1, fzc22, fzc50, fzc51, fzc6, fzc8, hsf3, skn7* and *zfc1,* and
the heavy-metal stress is induced by 20 µM to 30 µM cadmium sulfate (CdSO₄), wherein the antifungal agent-targeting gene that regulates responses to the heavy-metal stress induced by 20 µM to 30 µM cadmium sulfate (CdSO₄) is any one gene selected from the group consisting of *ada2, asg101, atf1, bzp1(hxl1), clr1, fzc39, fzc7, gat201, gat 204, gat7, hlh2, hsf3, rds2, rlm1, sip4, sre1, zfc3* and *znf2.*

4. The method of claim 3, wherein the resistance is down-regulated.

5. The method of claim 1 or 3, wherein the cell in step (a) is any one cell selected from a cell line library deposited under accession number KCCM51291.

6. A method for screening an antifungal agent, comprising the steps of:
(a) bringing a sample to be analyzed into contact with a *Cryptococcus neoformans* cell containing a virulence regulatory gene;
(b) measuring expression of the virulence regulatory gene in the cell; and
(c) determining that the sample is an antifungal agent, when the expression of the virulence regulatory gene is down-regulated,
wherein the virulence regulatory gene comprises one or more genes selected from the group consisting of a gene that regulates cellular pathogenicity and a gene that regulates virulence-factor production,
wherein the gene that regulates virulence-factor production comprises one or more genes selected from the group consisting of a gene that regulates capsule production, a gene that regulates melanin production, and a gene that regulates urease production, and the gene that regulates cellular pathogenicity comprises one or more genes selected from the group consisting of *aro80, bap1, bzp2, cef3, clr1, ddt1, fzc1, fzcl2, fzc2, fzc22, fzc31, fzc33, fzc37, fzc43, fzc49, fzc5, fzc50, fzc9, gat5, hlh1, hob1, mall3, mbs2, pip2, rum1, usv101, zfc2* and *zfc5.*

7. The method of claim 6, wherein the gene that regulates capsule production comprises one or more genes selected from the group consisting of *bap1, bzp4, fzcl6, fzc33, fzc45, fzc47, gat204, hob3, hob4, hob5, hsf2, liv1, liv4, mcm1, rds2, zap104* and zfc4; the gene that regulates melanin production comprises one or more genes selected from the group consisting of *bzp4, erl1, fzc25, fzc5, fzc8, hob1, liv1, mbs2* and *usv101*; and the gene that regulates urease production comprises one or more genes selected from the group consisting of *zap104, sre1, gat201, fzc46, hlh1* and *fzc21.*

8. A method for screening an antifungal agent, comprising the steps of:
(a) bringing a sample to be analyzed into contact with a *Cryptococcus neoformans* cell containing a virulence regulatory gene;
(b) measuring expression of the virulence regulatory gene in the cell; and
(c) determining that the sample is an antifungal agent, when the expression of the virulence regulatory gene is up-regulated,
wherein the virulence regulatory gene comprises one or more genes selected from the group consisting of a gene that regulates cellular pathogenicity and a gene that regulates virulence-factor production,
wherein the gene that regulates virulence-factor production comprises one or more genes selected from the group consisting of a gene that regulates capsule production, a gene that regulates melanin production, and a gene that regulates urease production, and
the gene that regulates cellular pathogenicity comprises one or more genes selected from the group consisting of *aro8001, ert1, fzcl7, fzc24, fzc38* and *fzc40.*

9. The method of claim 8, wherein the gene that regulates capsule production comprises one or more genes selected from the group consisting of *bzp3, clr1, clr3, crl6, fkh2, fzc1, fzc14, fzc17, fzc18, fzc24, fzc29, fzc30, fzc36, fzc46, fzc49, fzc51, hcm1, hlh3, hlh4, hob7, hpa1, jjj1, mln1, nrg1, sre1, usv101* and *zfc3;* the gene that regulates melanin production comprises one or more genes selected from the group consisting of *ada2, bap1, bzp2, bzp3, fkh2, fzc1, fzc31, gat1, hlh1, hlh2, nrg1, rds2, sip4* and *sip401;* and the gene that regulates urease production comprises one or more genes selected from the group consisting of *atf1, bap1, fkh2, fzc1, fzc14 fzc26, hob4, hob7, mln1, rim1, skn7, sxilalpha, usv101* and *zfc7.*

10. The method of claim 6 or 8, wherein the cell in step (a) is any one cell selected from a cell line library deposited under accession number KCCM51291.

11. A method for screening a meningitis-treating agent, comprising the steps of:
(a) bringing a sample to be analyzed into contact with a *Cryptococcus neoformans* cell containing a virulence regulatory gene;
(b) measuring expression of the virulence regulatory gene in the cell; and
(c) determining that the sample is a meningitis-treating agent, when the expression of the virulence regulatory gene is down-regulated,
wherein the virulence regulatory gene comprises one or more genes selected from the group consisting of a gene that regulates cellular pathogenicity and a gene that regulates virulence-factor production,
wherein the gene that regulates virulence-factor production comprises one or more genes selected from the group consisting of a gene that regulates capsule production, a gene that regulates melanin production, and a gene that regulates urease production, and
the gene that regulates cellular pathogenicity comprises one or more genes selected from the group consisting of *aro80, bap1, bzp2, cef3, clr1, ddt1, fzc1, fzcl2, fzc2, fzc22, fzc31, fzc33, fzc37, fzc43, fzc49, fzc5, fzc50, fzc9, gat5, hlh1, hob1, mall3, mbs2, pip2, rum1, usv101, zfc2* and *zfc5.*

12. The method of claim 11, wherein the gene that regulates capsule production comprises one or more genes selected from the group consisting of *bap1, bzp4, fzcl6, fzc33, fzc45, fzc47, gat204, hob3, hob4, hob5, hsf2, liv1, liv4, mcm1, rds2, zap104* and zfc4; the gene that regulates melanin production comprises one or more genes selected from the group consisting of *bzp4, erl1, fzc25, fzc5, fzc8, hob1, liv1, mbs2* and *usv101*; and the gene that regulates urease production comprises one or more genes selected from the group consisting of *fzc21, fzc46, gat201, hlh1, sre1* and *zap104.*

13. A method for screening a meningitis-treating agent, comprising the steps of:
(a) bringing a sample to be analyzed into contact with a *Cryptococcus neoformans* cell containing a virulence regulatory gene;
(b) measuring expression of the virulence regulatory gene in the cell; and
(c) determining that the sample is a meningitis-treating agent, when the expression of the virulence regulatory gene is up-regulated,
wherein the virulence regulatory gene comprises one or more genes selected from the group consisting of a gene that regulates cellular pathogenicity and a gene that regulates virulence-factor production,
wherein the gene that regulates virulence-factor production comprises one or more genes selected from the group consisting of a gene that regulates capsule production, a gene that regulates melanin production, and a gene that regulates urease production, and
the gene that regulates cellular pathogenicity comprises one or more genes selected from the group consisting of *aro8001, ert1, fzcl7, fzc24, fzc38* and *fzc40.*

14. The method of claim 13, wherein the gene that regulates capsule production comprises one or more gene selected from the group consisting of *bzp3, clr1, clr3, crl6, fkh2, fzc1, fzc14, fzc17, fzc18, fzc24, fzc29, fzc30, fzc36, fzc46, fzc49, fzc51, hcm1, hlh3, hlh4, hob7, hpa1, jjj1, mln1, nrg1, sre1, usv101* and *zfc3;* the gene that regulates melanin production comprises one or more gene selected from the group consisting of *ada2, bap1, bzp2, bzp3, fkh2, fzc1, fzc31, gat1, hlh1, hlh2, nrg1, rds2, sip4* and *sip401;* and the gene that regulates urease production comprises one or more gene selected from the group consisting of *atf1, bap1, fkh2, fzc1, fzc14, fzc26, hob4, hob7, mln1, rim1, skn7, sxilalpha, usv101* and *zfc7.*

15. The method of claim 11 or 13, wherein the cell in step (a) is any one cell selected from a cell line library deposited under accession number KCCM51291.

16. A method for screening an antifungal agent for co-administration, comprising the step:
(a) bringing an antifungal agent into contact with a *Cryptococcus neoformans* cell including a virulence regulatory gene, and measuring expression level of the gene to obtain a first measurement value;
(b) bringing a sample to be analyzed and the antifungal agent into contact with a *Cryptococcus neoformans* cell including a virulence regulatory gene, and measuring expression level of the gene to obtain a second measurement value; and
(c) comparing the first measurement value with the second measurement value, and determining that the sample is an antifungal agent for co-administration, when the second measurement value is down-regulated compared to the second measurement value,
wherein the virulence regulatory gene comprises one or more genes selected from the group consisting of a gene that regulates cellular pathogenicity and a gene that regulates virulence-factor production,
wherein the gene that regulates virulence-factor production comprises one or more genes selected from the group consisting of a gene that regulates capsule production, a gene that regulates melanin production, and a gene that regulates urease production, and
the gene that regulates cellular pathogenicity comprises one or more genes selected from the group consisting of *aro80, bap1, bzp2, cef3, clr1, ddt1, fzc1, fzcl2, fzc2, fzc22, fzc31, fzc33, fzc37, fzc43, fzc49, fzc5, fzc50, fzc9, gat5, hlh1, hob1, mall3, mbs2, pip2, rum1, usv101, zfc2* and *zfc5.*

17. The method of claim 16, wherein the gene that regulates capsule production comprises one or more genes selected from the group consisting of *bap1, bzp4, fzcl6, fzc33, fzc45, fzc47, gat204, hob3, hob4, hob5, hsf2, liv1, liv4, mcm1, rds2, zap104* and zfc4; the gene that regulates melanin production comprises one or more genes selected from the group consisting of *bzp4, erl1, fzc25, fzc5, fzc8, hob1, liv1, mbs2* and *usv101*; and the gene that regulates urease production comprises one or more genes selected from the group consisting of *fzc21, fzc46, gat201, hlh1, sre1* and zap104.

18. A method for screening an antifungal agent for co-administration, comprising the step:
(a) bringing an antifungal agent into contact with a *Cryptococcus neoformans* cell including a virulence regulatory gene, and measuring expression level of the gene to obtain a first measurement value;
(b) bringing a sample to be analyzed and the antifungal agent into contact with a *Cryptococcus neoformans* cell including a virulence regulatory gene, and measuring expression level of the gene to obtain a second measurement value; and
(c) comparing the first measurement value with the second measurement value, and determining that the sample is an antifungal agent for co-administration, when the second measurement value is up-regulated compared to the second measurement value,
wherein the virulence regulatory gene comprises one or more genes selected from the group consisting of a gene that regulates cellular pathogenicity and a gene that regulates virulence-factor production,
wherein the gene that regulates virulence-factor production comprises one or more genes selected from the group consisting of a gene that regulates capsule production, a gene that regulates melanin production, and a gene that regulates urease production, and
the gene that regulates cellular pathogenicity comprises one or more genes selected from the group consisting of *aro8001, ert1, fzcl7, fzc24, fzc38* and *fzc40.*

19. The method of claim 18, wherein the gene that regulates capsule production comprise one or more genes selected from the group consisting of *bzp3, clr1, clr3, crl6, fkh2, fzc1, fzc14, fzc17, fzc18, fzc24, fzc29, fzc30, fzc36, fzc46, fzc49, fzc51, hcm1, hlh3, hlh4, hob7, hpa1, jjj1, mln1, nrg1, sre1, usv101* and *zfc3;* the gene that regulates melanin production comprise one or more genes selected from the group consisting of *ada2, bap1, bzp2, bzp3, fkh2, fzc1, fzc31, gat1, hlh1, hlh2, nrg1, rds2, sip4* and *sip401;* and the gene that regulates urease production comprise one or more genes selected from the group consisting of *atf1, bap1, fkh2, fzc1, fzcl4, fzc26, hob4, hob7, mln1, rim1, skn7, sxilalpha, usv101* and *zfc7.*

20. The method of claim 16 or 18, wherein the cell in steps (a) and (b) is any one cell selected from a cell line library deposited under accession number KCCM51291.
